# EUROPEAN PATENT SPECIFICATION

(11) **EP 4 051 402 B1**
(45) Date of publication and mention of the grant of the patent: **29.04.2026**
(21) Application number: 20803451.2
(22) Date of filing: 29.10.2020
(51) Int. Cl.: B01D 15/38, B01D 15/22, B01D 15/20, B01D 15/16, G01N 30/54, G01N 30/60

(54) **CELL SELECTION AND/OR STIMULATION DEVICES AND METHODS OF USE**
ZELLSELEKTION UND/ODER STIMULATIONSVORRICHTUNGEN UND VERWENDUNGSVERFAHREN
DISPOSITIFS DE SÉLECTION ET/OU STIMULATION DE CELLULES ET PROCÉDÉS D'UTILISATION

(30) Priority: 30.10.2019 US 201962928303 P
(43) Date of publication of application: 07.09.2022
(73) Proprietor: C3S2 GmbH, 81245 München (DE)
(72) Inventor: POLTORAK, Mateusz Pawel, 81245 Munich (DE); RADISCH, Christian, 81245 Munich (DE)
(74) Representative: Schiweck Weinzierl Koch Patentanwälte Partnerschaft mbB
(86) International application number: PCT/EP2020/080476
(87) International publication number: WO 2021/084050

(56) References cited:
- WO-A1-2014/008058
- WO-A2-2006/041487
- US-A1- 2010 005 867
- US-A1- 2017 282 096

## Description

### Field

The present disclosure relates to cell selection and/or stimulation devices and methods of use. In some aspects, the selected and/or stimulated cells are useful for genetic engineering, and ultimately, cell therapy.

### Background

Various cell therapy methods are available for treating diseases and conditions. Among cell therapy methods are methods involving immune cells, such as T cells (e.g., CD4+ and CD8+ T cells), which may be genetically engineered with a recombinant receptor, such as a chimeric antigen receptors. US 2010/0005867 A1 discloses a temperature control unit for a fluidic device comprising a column which may be filled with a package composition. WO 2014/008058 A1 discloses a device for preforming liquid chromatography comprising a chromatography column and an insulating member surrounding the chromatography column. A plurality of heaters may heat the stationary phase of the column, which is placed in an internal cavity between two housing members. US 2017/0282096 A1 discloses also a device comprising a chromatographic column and a surrounding vacuum insulated jacket which could be used for heating. The chromatographic column may comprise two frits between the inlet/outlet and the stationary phase. Improved devices and methods for generating cell populations suitable for use, for example in cell therapy, are needed. Provided are device, articles of manufacture, and methods that meet such needs.

### Summary

In some embodiments, disclosed herein is a housing assembly for column chromatography, comprising: an inlet housing member and an outlet housing member, at least the inlet housing member and the outlet housing member forming an internal cavity configured to house a stationary phase for column chromatography; a temperature control member configured to provide heat to the stationary phase in the internal cavity; and a connector configured to operably connect the internal cavity to a gas source, thereby permitting or effecting intake of gas into the internal cavity, *e.g*., during at least a portion of a chromatography run. In some embodiments, the housing assembly further comprises a side wall member, and the inlet housing member, the outlet housing member, and the side wall member form the internal cavity.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member configured to provide heat to the stationary phase in the internal cavity; and a connector configured to operably connect the internal cavity to a gas source, thereby permitting or effecting intake of gas into the internal cavity.

In some embodiments, provided herein is a housing assembly for column chromatography, comprising: a chromatography column comprising an internal cavity configured to house a stationary phase; a temperature control member configured to provide heat to the stationary phase in the internal cavity; and a connector configured to operably connect the internal cavity to a gas source, thereby permitting or effecting intake of gas into the internal cavity. In some embodiments, the chromatography column comprises an inlet housing member, an outlet housing member, and a side wall member, wherein the inlet housing member, the outlet housing member, and the side wall member form the internal cavity.

In any of the preceding embodiments, the connector can be disposed on the inlet housing member, the outlet housing member, and/or the side wall member.

In any of the preceding embodiments, the connector can be formed between any two or among all three of the inlet housing member, the outlet housing member, and the side wall member.

In any of the preceding embodiments, the housing assembly may comprise a plurality of the connectors.

In any of the preceding embodiments, the connector can be a bonded connector, a screw connector, a luer connector (*e*.*g*., a luer lock connector or a luer slip connector), a barbed connector, or any combination thereof. In any of the preceding embodiments, the connector can be a luer lock connector or a luer slip connector. In any of the preceding embodiments, the connector can comprise a male fitting or a female fitting. In any of the preceding embodiments, the connector can be configured to sealingly engage tubing in fluid communication with the gas source. In any of the preceding embodiments, the connector can comprise one or more valve. In any of the preceding embodiments, the connector can be operably connected to tubing comprising one or more valve.

In any of the preceding embodiments, the connector can comprise one or more filter. In any of the preceding embodiments, the connector can be operably connected to tubing comprising one or more filter. In any of the preceding embodiments, the one or more filter can be a gas filter, *e.g.,* an air filter. In any of the preceding embodiments, the one or more filter can be an air filter. In any of the preceding embodiments, the one or more filter can be a sterile filter and/or a sterilizing filter for sterilization by filtration. In any of the preceding embodiments, the one or more filter can be a sterile filter. In any of the preceding embodiments, the one or more filter can be a sterilizing filter for sterilization by filtration.

In any of the preceding embodiments, the inlet housing member can comprise an upper lid of the housing assembly. In some embodiments, the upper lid is removably attached to the inlet housing member or the side wall member. In some embodiments, the upper lid is integrally formed with the inlet housing member or the side wall member. In any of the preceding embodiments, the connector can be disposed on the upper lid.

In any of the preceding embodiments, the inlet housing member may comprise one or more inlet operably connected to the internal cavity to permit intake of an input composition into the internal cavity. In some embodiments, the one or more inlet is disposed on the upper lid. In some embodiments, the connector and the one or more inlet are disposed on the upper lid at the same or different locations.

In any of the preceding embodiments, fluid path through the one or more inlet can be at an angle of about 90 degrees to the upper lid, while fluid path through the connector can be at an angle of about 45 degrees to the upper lid.

In any of the preceding embodiments, the outlet housing member may comprise a lower lid of the housing assembly. In some embodiments, the lower lid is removably attached to the outlet housing member or the side wall member, or the lower lid is integrally formed with the outlet housing member or the side wall member.

In any of the preceding embodiments, the outlet housing member can comprise one or more outlet operably connected to the internal cavity to permit or effect discharge of an output composition from the internal cavity. In some embodiments, the one or more outlet is disposed on the lower lid. In some embodiments, the connector and the one or more outlet are disposed on the lower lid at the same or different locations. In some embodiments, fluid path through the one or more outlet is at an angle of about 90 degrees to the lower lid.

In any of the preceding embodiments, the gas source can be or comprise a gas reservoir or an outside environment. In any of the preceding embodiments, gas in the gas source may be sterile. In any of the preceding embodiments, the gas can be or comprise air.

In any of the preceding embodiments, the housing assembly can further comprise tubing operably connected to the gas source. In some embodiments, the tubing is configured to sterilely connect the internal cavity to the gas source. In any of the preceding embodiments, the tubing can comprise one or more valve. In any of the preceding embodiments, the tubing may comprise one or more filter.

In any of the preceding embodiments, the housing assembly can further comprise one or more porous member, *e.g*., a cell strainer or a cell sieve. In some embodiments, the one or more porous member can be a cell strainer or a cell sieve. In some embodiments, the housing assembly comprises a first porous member configured to separate the stationary phase and an inlet of the internal cavity, and the first porous member is optionally between the inlet housing member and the side wall member. In some embodiments, the housing assembly further comprises a second porous member configured to separate the stationary phase and an outlet of the internal cavity, and the second porous member is optionally between the outlet housing member and the side wall member.

In any of the preceding embodiments, the housing assembly can comprise a first porous member configured to separate the stationary phase and an inlet of the internal cavity, wherein the first porous member is optionally between the inlet housing member and the side wall member; and/or a second porous member configured to separate the stationary phase and an outlet of the internal cavity, wherein the second porous member is optionally between the outlet housing member and the side wall member. In some embodiments, the first porous member or the second porous member is independently a cell strainer or a cell sieve.

In any of the preceding embodiments, the first porous member can be between the inlet housing member and the side wall member. In any of the preceding embodiments, the second porous member can be between the outlet housing member and the side wall member.

In any of the preceding embodiments, the one or more porous member may have an average pore diameter of about 20 µm, or the one or more porous member may comprise a mesh having a mesh size of about 20 µm.

In any of the preceding embodiments, the temperature control member may be configured to regulate or maintain a temperature of the stationary phase in the internal cavity. In any of the preceding embodiments, the temperature control member can be configured to heat the stationary phase in the internal cavity from a starting temperature (*e.g*., room temperature) to a target temperature between about 35°C and about 39°C (*e.g.,* at or at about 37°C). In any of the preceding embodiments, the target temperature can be at or about 37°C. In some embodiments, the temperature control member is further configured to maintain the stationary phase at the target temperature.

In any of the preceding embodiments, the temperature control member can be configured to heat the stationary phase to a target temperature between about 30°C and about 39°C. In any of the preceding embodiments, the target temperature can be between about 35°C and about 39°C, optionally at or about 37°C. In any of the preceding embodiments, the target temperature can be at or about 37°C. In some embodiments, the temperature control member is further configured to maintain the stationary phase at the target temperature.

In any of the preceding embodiments, the housing assembly can comprise a temperature sensor configured to measure the temperature of the stationary phase in the internal cavity. The temperature sensor may form a part of the temperature control member, or provided separately from the temperature control member. In some embodiments, the temperature sensor is configured to couple to a monitoring/display unit.

In any of the preceding embodiments, the temperature control member may comprise a heating source. In any of the preceding embodiments, the temperature control member may be configured to operably connect to a heating source which is external to the housing assembly.

In any of the preceding embodiments, the temperature control member can comprise a heating element or a plurality of heating elements.

In any of the preceding embodiments, the heating element and/or the plurality of heating elements can be configured to uniformly heat the stationary phase.

In any of the preceding embodiments, the temperature control member can comprise a heating element selected from the group consisting of an electric heating element, an electromagnetic induction heating element, a non-electric heating element, and any combination thereof. In any of the preceding embodiments, the temperature control member can comprise a plurality of heating elements each selected from the group consisting of an electric heating element, an electromagnetic induction heating element, a non-electric heating element, and any combination thereof. In some embodiments, the heating element is an electric heating element. In some embodiments, at least one of the plurality of heating elements is an electric heating element. In some embodiments, the electric heating element comprises a metal plate, a metal rod, a metal wire, or a combination thereof. In some embodiments, the electric heating element can be configured to connect to a power source external to the housing assembly. In some embodiments, the heating element is an electromagnetic induction heating element. In some embodiments, at least one of the plurality of heating elements is an electromagnetic induction heating element. In some embodiments, the electromagnetic induction heating element comprises an induction heating coil surrounding a magnetizable core configured to provide heat to the stationary phase in the internal cavity. In some embodiments, the heating element is a non-electric heating element. In some embodiments, at least one of the plurality of heating elements is a non-electric heating element. In some embodiments, the non-electric heating element comprises a heating channel comprising an inlet and an outlet for a heated fluid, *e.g.,* a heated liquid or gas. In some embodiments, the heated fluid can be a heated liquid or a heated gas. In some embodiments, the heating channel can be a heating coil. In some embodiments, the heated fluid can be heated water. In some embodiments, the heating channel is a heating coil and the heated fluid is heated water. In some embodiments, the inlet for heated water is configured to connect to an external reservoir of heated water.

In any of the preceding embodiments, the heating element may be disposed along and/or around a central axis of the internal cavity. In any of the preceding embodiments, the heating element may be disposed inside the internal cavity, outside the internal cavity, or partially inside and partially outside the internal cavity. In any of the preceding embodiments, the heating element may be disposed inside the side wall member, outside the side wall member, or partially inside and partially outside the side wall member. In any of the preceding embodiments, the heating element can comprise a coil surrounding the inlet housing member, the outlet housing member, and/or the side wall member.

In any of the preceding embodiments, the heating element can comprise a heating channel surrounding the inlet housing member, the outlet housing member, and/or the side wall member. In any of the preceding embodiments, the heating element can comprise a heating coil surrounding the inlet housing member, the outlet housing member, and/or the side wall member. In any of the preceding embodiments, at least one of the plurality of heating elements may be disposed along and/or around a central axis of the internal cavity. In any of the preceding embodiments, at least one of the plurality of heating elements may be disposed inside the internal cavity, outside the internal cavity, or partially inside and partially outside the internal cavity. In any of the preceding embodiments, at least one of the plurality of heating elements may be disposed inside the side wall member, outside the side wall member, or partially inside and partially outside the side wall member.

In any of the preceding embodiments, the heating element and/or at least one of the plurality of heating elements may surround at least a portion of the inlet housing member, at least a portion of the outlet housing member, and/or at least a portion of the side wall member. In any of the preceding embodiments, the heating element and/or at least one of the plurality of heating elements may surround at least a portion of the side wall member.

In any of the preceding embodiments, the plurality of heating elements can be uniformly or about uniformly distributed around the circumference of the side wall member.

In any of the preceding embodiments, at least a portion of the heating element and/or at least a portion of at least one of the plurality of heating elements can be in contact with at least a portion of the inlet housing member, at least a portion of the outlet housing member, and/or at least a portion the side wall member, optionally at least a portion of the side wall member. In any of the preceding embodiments, at least a portion of the heating element and/or at least a portion of at least one of the plurality of heating elements can be in contact with at least a portion of the side wall member.

In any of the preceding embodiments, at least a portion of the heating element and/or at least a portion of at least one of the plurality of heating elements can be not in contact with the inlet housing member, the outlet housing member, or the side wall member.

In any of the preceding embodiments, the housing assembly can further comprise an insulation layer between the heating element and/or the at least one of the plurality of heating elements and at least a portion of the inlet housing member, at least a portion of the outlet housing member, and/or at least a portion of the side wall member. In some embodiments, the insulation layer can comprise a gas, optionally air, or a liquid. In some embodiments, the insulation layer can comprise air.

In any of the preceding embodiments, the heating element can comprise a heating channel and the heating channel can surround at least a portion of the inlet housing member, at least a portion of the outlet housing member, and/or at least a portion of the side wall member

In any of the preceding embodiments, the heating element can comprise a heating coil and the heating coil can surround at least a portion of the inlet housing member, at least a portion of the outlet housing member, and/or at least a portion of the side wall member.

In any of the preceding embodiments, the plurality of heating elements can comprise a plurality of heating channels that surround at least a portion of inlet housing member, at least a portion of the outlet housing member, and/or at least a portion of the side wall member. In any of the preceding embodiments, at least two of the plurality of heating channels can be fluidly coupled to one another.

In any of the preceding embodiments, the plurality of heating elements can be a plurality of electric heating elements that surround at least a portion of the inlet housing member, at least a portion of the outlet housing member, and/or at least a portion of the side wall member. In any of the preceding embodiments, at least two of the plurality of electric heating elements can be electrically coupled to one another. In any of the preceding embodiments, at least one of the plurality of electric heating elements can be configured to electrically connect to a power source external to the housing assembly.

In any of the preceding embodiments, the housing assembly can further comprise a jacket member comprising the heating element or at least one of the plurality of heating elements, wherein the jacket member is configured to surround at least a portion of the inlet housing member, at least a portion of the outlet housing member, and/or at least a portion of the side wall member. In any of the preceding embodiments, the housing assembly can further comprise a jacket member comprising the temperature control member comprising the heating element or at least one of the plurality of heating elements, wherein the jacket member is configured to surround at least a portion of the inlet housing member, at least a portion of the outlet housing member, and/or at least a portion of the side wall member.

In some embodiments, the jacket member surrounds at least a portion of the inlet housing member, at least a portion of the outlet housing member, and/or at least a portion of the side wall member.

In any of the preceding embodiments, the jacket member can be releasably connected together to surround at least a portion of the inlet housing member, at least a portion of the outlet housing member, and/or at least a portion of the side wall member.

In any of the preceding embodiments, the jacket member can be configured to surround at least a portion of the side wall member, optionally can be configured to entirely surround the side wall member. In any of the preceding embodiments, the jacket member can be configured to entirely surround the side wall member. In any of the preceding embodiments, the jacket member may surround at least a portion of the side wall member, optionally may entirely surround the side wall member. In any of the preceding embodiments, the jacket member can be configured to entirely surround the side wall member. In any of the preceding embodiments, the jacket member may entirely surround the side wall member.

In any of the preceding embodiments, the jacket member can comprise two or more jacket components that are configured to together surround the at least a portion of the inlet housing member, the at least a portion of the outlet housing member, and/or the at least a portion of the side wall member, optionally entirely surround the side wall member. In any of the preceding embodiments, the jacket member can comprise two or more jacket components that together are configured to surround at least a portion of the side wall member. In any of the preceding embodiments, the jacket member can comprise two or more jacket components that together are configured to entirely surround the side wall member.

In any of the preceding embodiments, a portion of the one or more inlet of the inlet housing member and/or a portion of the one or more outlet of the outlet housing member can be exposed by the jacket member. In any of the preceding embodiments, a portion of the one or more inlet of the inlet housing member and/or a portion of the one or more outlet of the outlet housing member can be outside the jacket member.

In any of the preceding embodiments, at least a portion of the jacket member can be in contact with at least a portion of the inlet housing member, at least a portion of the outlet housing member, and/or at least a portion of the side wall member, optionally at least a portion of the side wall member. In any of the preceding embodiments, at least a portion of the jacket member can be in contact with at least a portion of the side wall member.

In any of the preceding embodiments, at least a portion of the jacket member can be not in contact with the inlet housing member, the outlet housing member, or the side wall member.

In any of the preceding embodiments, the heating element or the at least one of the plurality of heating elements can be a heating channel comprising a inlet and an outlet for a heated fluid and the jacket member can comprise at least one opening for the inlet for the heating fluid and at least one opening for the outlet for the heated fluid.

In any of the preceding embodiments, the heating element or the at least one of the plurality of heating elements can be an electric heating element and the jacket member can be arranged such that the electric heating element is configured to electrically connect to a power source external to the housing assembly.

In any of the preceding embodiments, the two or more jacket components can be configured to be releasably connected together.

In any of the preceding embodiments, the jacket member can comprise a plurality of heating elements and at least two of the two or more jacket components can each comprise at least one of the plurality of heating elements. In any of the preceding embodiments, the at least two of the two or more jacket components can each further comprise a temperature sensor

In any of the preceding embodiments, the at least two of the two or more jacket components can each comprise a heating channel comprising an inlet and an outlet for a heated fluid, optionally heated water. In some embodiments, the heated fluid can be heated water. In any of the preceding embodiments, the heating channels of the at least two of the two or more jacket components can be fluidly connected to one another. In any of the preceding embodiments, at least one inlet of the heating channels of the at least two of the two or more jacket components can be configured to connect to an external reservoir of heated water.

In any of the preceding embodiments, the at least two of the two or more jacket components can each comprise an electric heating element, optionally an electric heating element that comprises a metal plate. In any of the preceding embodiments, the electric heating elements of the at least two of the two or more jacket components can be electrically coupled to one another. In any of the preceding embodiments, the electric heating elements of the at least two of the two or more jacket components can configured to electrically connect to a power source external to the housing assembly.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member configured to provide heat to the stationary phase in the internal cavity and regulate or maintain a temperature of the stationary phase in the internal cavity; and a connector configured to operably connect the internal cavity to a gas source, thereby permitting or effecting intake of gas into the internal cavity.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member comprising a heating element and configured to provide heat to the stationary phase in the internal cavity and regulate or maintain a temperature of the stationary phase in the internal cavity; and a connector configured to operably connect the internal cavity to a gas source, thereby permitting or effecting intake of gas into the internal cavity.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member comprising a heating element disposed along and/or around a central axis of the internal cavity, the heating element configured to provide heat to the stationary phase in the internal cavity; and a connector configured to operably and sterilely connect the internal cavity to a gas source, thereby permitting or effecting intake of sterile gas into the internal cavity.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member comprising a heating element comprising a metal plate configured to provide heat to the stationary phase in the internal cavity; and a connector configured to operably and sterilely connect the internal cavity to a gas source, thereby permitting or effecting intake of sterile gas into the internal cavity.

In some embodiments, disclosed herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member comprising a heating element comprising a heating coil configured to provide heat to the stationary phase in the internal cavity; and a connector configured to operably and sterilely connect the internal cavity to a gas source, thereby permitting or effecting intake of sterile gas into the internal cavity. In some embodiments, the heating coil comprises an inlet and an outlet for heated water.

In any of the preceding embodiments, the heating coil may surround the inlet housing member, the outlet housing member, and the side wall member.

In some embodiments, provided herein is a housing assembly for column chromatography, comprising: an inlet housing member, an outlet housing member, and a side wall member, where the inlet housing member, the outlet housing member, and the side wall member form an internal cavity configured to house a stationary phase for column chromatography; a temperature control member comprising a heating element configured to provide heat to the stationary phase in the internal cavity; and a connector configured to operably and sterilely connect the internal cavity to a gas filter, thereby permitting or effecting intake of sterile gas into the internal cavity. In some embodiments, the gas filter is an air filter and the sterile gas is sterile air. In any of the preceding embodiments, the housing assembly may further comprise the gas filter.

In some embodiments, the chromatography column can comprise an inlet housing member, an outlet housing member, and a side wall member, wherein the inlet housing member, the outlet housing member, and the side wall member form the internal cavity. In some embodiments, the jacket member can be releasably connected together to surround at least a portion of the inlet housing member, at least a portion of the outlet housing member, and/or at least a portion of the side wall member. In any of the preceding embodiments, the housing assembly can further comprise a connector configured to operably and sterilely connect the internal cavity to a gas filter, thereby permitting or effecting intake of sterile gas into the internal cavity.

In some embodiments, provided herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member configured to regulate or maintain a temperature of the stationary phase, wherein the temperature control member comprises a heating coil configured to provide heat to the stationary phase; a jacket member comprising the heating coil, wherein the jacket member is releasably connected to surround at least a portion of the inlet housing member, the outlet housing member, and the side wall member; and a connector configured to operably and sterilely connect the internal cavity to a gas filter, thereby permitting or effecting intake of sterile gas into the internal cavity.

In some embodiments, the heating coil entirely surrounds the side wall member. In some embodiments, the jacket member can comprise a second heating coil, and the heating coil and the second heating coil can together surround the side wall member.

In some embodiments, provided herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member configured to regulate or maintain a temperature of the stationary phase, wherein the temperature control member comprises an electric heating element that comprises a metal plate and is configured to provide heat to the stationary phase; a jacket member comprising the electric heating element , wherein the jacket member is releasably connected to surround at least a portion of the inlet housing member, the outlet housing member, and the side wall member; and a connector configured to operably and sterilely connect the internal cavity to a gas filter, thereby permitting or effecting intake of sterile gas into the internal cavity.

In some embodiments, the jacket member can comprise a plurality of electric heating elements that comprise metal plates and that are uniformly or about uniformly distributed around the circumference of the side wall member.

In some embodiments, disclosed herein is a housing assembly set, comprising a plurality of the housing assembly of any of the preceding embodiments. In some embodiments, the housing assembly set comprises at least two of the plurality of the housing assembly arranged sequentially. In any of the preceding embodiments, the housing assembly set may comprise at least two of the plurality of the housing assembly arranged in parallel.

In some embodiments, provided herein is a chromatography system, comprising the housing assembly of any of the preceding embodiments and at least one additional chromatography column.

In some embodiments, provided herein is a chromatography kit, comprising the housing assembly or the housing assembly set of any of the preceding embodiments, and a stationary phase for column chromatography. In some embodiments, provided herein is a chromatography kit, comprising the chromatography system of any of the preceding embodiments, and a stationary phase for column chromatography. In some embodiments, provided herein is a chromatography kit, comprising the jacket member of any of the preceding embodiments, a chromatography column, and a stationary phase for column chromatography.

In some embodiments, provided herein is a chromatography column or chromatography column set, comprising the housing assembly or the housing assembly set of any of the preceding embodiments, and a stationary phase for column chromatography in the internal cavity of one or more of the housing assembly. In some embodiments, provided herein is a chromatography column, comprising the jacket member of any of the preceding embodiments and a chromatography column, wherein the internal cavity of the chromatography column comprises a stationary phase for column chromatography. In some embodiments, provided herein is a chromatography column set, comprising at least one jacket member of any of the preceding embodiments and a plurality of chromatography columns, wherein the internal cavity of each of the chromatography column comprises a stationary phase for column chromatography. In some embodiments, the plurality of chromatography columns can be arranged sequentially or in parallel, optionally wherein the plurality of chromatography columns are operably connected. In some embodiments, the plurality of chromatography columns are operably connected. In any of the preceding embodiments, the plurality of chromatograph columns can comprise a first chromatography column and a second chromatography column, wherein the at least one jacket member can be configured to surround the second chromatography column. In some embodiments, the stationary phase comprises a gel filtration matrix. In any of the preceding embodiments, the stationary phase may comprise an affinity chromatography matrix. In any of the preceding embodiments, the stationary phase may be or comprise a non-magnetic material, a non-ferromagnetic material, or non-paramagnetic material. In any of the preceding embodiments, the stationary phase may be or comprises one selected from the group consisting of a cellulose membrane, a plastic membrane, a polysaccharide gel, a polyacrylamide gel, an agarose gel, polysaccharide grafted silica, polyvinylpyrrolidone grafted silica, polyethylene oxide grafted silica, poly(2-hydroxy ethyl aspartamide) silica, poly(N-isopropylacrylamide) grafted silica, a styrenedivinylbenzene gel, a copolymer of an acrylate or an acrylamide and a diol, a co-polymer of a polysaccharide and N,N'-methylenebisacrylamide, and a combination thereof. In any of the preceding embodiments, the stationary phase may be or comprise a monolithic matrix, a particulate matrix, and/or a planar matrix. In some embodiments, the particulate matrix has a mean particle size of about 5 µm to about 200 µm, of about 5 µm to about 600 µm, or of about 5 µm to about 1500 µm. In any of the preceding embodiments, the stationary phase may have a mean pore size of about 1 nm to about 500 nm.

In any of the preceding embodiments, the stationary phase may comprise a selection agent immobilized thereon. In some embodiments, the selection agent is capable of specific binding to a selection marker on the surface of one or more cells. In any of the preceding embodiments, the one or more cells may be or comprise immune cells. In some embodiments, the one or more cells are T cells.

In any of the preceding embodiments, the selection agent can be or comprise an agent selected from the group consisting of antibody fragments, monovalent antibody fragments, proteinaceous binding molecules with immunoglobulin-like functions, molecules containing Ig domains, cytokines, chemokines, aptamers, MHC molecules, MHC-peptide complexes; receptor ligands; and binding fragments thereof. In any of the preceding embodiments, the selection agent can be or comprise an antibody fragment. In any of the preceding embodiments, the selection agent can be or comprise a Fab fragment. In any of the preceding embodiments, the selection agent can be or comprise one selected from the group of divalent antibody fragments consisting of F(ab')₂ fragments and divalent single-chain Fv (scFv) fragments. In any of the preceding embodiments, the selection agent can be or comprise a monovalent antibody fragment selected from the group consisting of Fab fragments, Fv fragments, and scFvs. In any of the preceding embodiments, the selection agent can be or comprise a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers.

In any of the preceding embodiments, the selection agent can further comprise biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag. In any of the preceding embodiments, the selection agent can comprise a streptavidin-binding peptide.

In any of the preceding embodiments, the selection marker can be or comprise a T cell coreceptor. In any of the preceding embodiments, the selection marker can be or comprise a member of a T cell antigen receptor complex. In any of the preceding embodiments, the selection marker can be or comprise a CD3 complex. In any of the preceding embodiments, the selection marker can be or comprise a CD3 chain. In any of the preceding embodiments, the selection marker can be or comprise a CD3γ, CD3δ, CD3ε, or CD3ζ chain. In any of the preceding embodiments, the selection marker can be or comprise CD8. In any of the preceding embodiments, the selection marker can be or comprise CD4. In any of the preceding embodiments, the selection marker can be or comprise CD45RA. In any of the preceding embodiments, the selection marker can be or comprise CD27. In any of the preceding embodiments, the selection marker can be or comprise CD28. In any of the preceding embodiments, the selection marker can be or comprise CCR7.

In any of the preceding embodiments, the specific binding between the selection agent and the selection marker may not result in the induction of a signal, *e.g.,* the induction of a stimulatory or activating or proliferative signal, to the T cells.

In any of the preceding embodiments, the selection agent can be or comprise an anti-CD3 Fab, an anti-CD8 Fab, or an anti-CD4 Fab. In any of the preceding embodiments, the selection agent can be or comprise an anti-CD27 Fab. In any of the preceding embodiments, the selection agent can be directly or indirectly bound to the stationary phase. In any of the preceding embodiments, the selection agent can be bound indirectly to the stationary phase through a selection reagent to which the selection agent reversibly binds.

In any of the preceding embodiments, the selection reagent can be or comprise streptavidin, avidin, a mutein of streptavidin that reversibly binds biotin, a biotin analog or a biologically active fragment thereof; a mutein of avidin or streptavidin that reversibly binds a streptavidin-binding peptide; a reagent that comprises at least two chelating groups K, where the at least two chelating groups are capable of binding to a transition metal ion; an agent capable of binding to an oligohistidine affinity tag; an agent capable of binding to a glutathione-S-transferase; calmodulin or an analog thereof; an agent capable of binding to calmodulin binding peptide (CBP); an agent capable of binding to a FLAG-peptide; an agent capable of binding to an HA-tag; an agent capable of binding to maltose binding protein (MBP); an agent capable of binding to an HSV epitope; an agent capable of binding to a myc epitope; or an agent capable of binding to a biotinylated carrier protein. In any of the preceding embodiments, the selection reagent can be or comprise a mutein of streptavidin that reversibly binds a streptavidin-binding peptide.

In any of the preceeding embodiments, the selection agent immobilized on the stationary phase of at least one of the plurality of chromatography columns can be an anti-CD4 antibody (e.g. an anti-CD4 Fab), and the selection agent immobilized on the stationary phase of at least another one of the plurality of chromatography columns can be an anti-CD8 antibody (e.g. an anti-CD8 Fab). In any of the preceeding embodiments, the selection agent immobilized on the stationary phase of at least one of the plurality of chromatography columns can be an anti-CD4 Fab, and the selection agent immobilized on the stationary phase of at least another one of the plurality of chromatography columns can be an anti-CD8 Fab.

In any of the preceeding embodiments, the selection agent immobilized on the stationary phase of at least one of the plurality of chromatography columns can be an anti-CD3 antibody (e.g. an anti-CD3 Fab) and the selection agent immobilized on the stationary phase of at least another one of the plurality of chromatography coluns can be an antibody targeting CD45RA, CD27, CD28 or CCR7, optionally an anti-CD27 antibody (e.g. an anti-CD27 Fab). In any of the preceeding embodiments, the selection agent immobilized on the stationary phase of at least one of the plurality of chromatography columns can be an anti-CD3 Fab and the selection agent immobilized on the stationary phase of at least another one of the plurality of chromatography coluns can be an anti-CD27 Fab).

In any of the preceeding embodiments, the selection agent immobilized on the stationary phase of at least one of the plurality of chromatography columns can be an anti-CD4 antibody (e.g. anti-CD4 Fab), the selection agent immobilized on at least another of the plurality of chromatography columns can be an anti-CD8 antibody (e.g. anti-CD8 Fab), and the selection agent immobilized on the stationary phase of at least a further one of the plurality of chromatography coluns can be an antibody targeting CD45RA, CD27, CD28 or CCR7, optionally an anti-CD27 antibody (e.g. an anti-CD27 Fab). In any of the preceeding embodiments, the selection agent immobilized on the stationary phase of at least one of the plurality of chromatography columns can be an anti-CD4 Fab, the selection agent immobilized on at least another of the plurality of chromatography columns can be an anti-CD8 Fab, and the selection agent immobilized on the stationary phase of at least a further one of the plurality of chromatography coluns can be an anti-CD27 Fab).

In any of the preceding embodiments, the chromatography kit, chromatography column, or chromatography column set may further comprise one or more stimulatory agent capable of delivering a stimulatory signal in one or more T cells. In some embodiments, the stationary phase comprises at least one of the one or more stimulatory agent. In some embodiments, the one or more stimulatory agent is immobilized on the stationary phase of the chromatography column or chromatography column set. In some embodiments, the one or more stimulatory agent is indirectly immobilized. In some embodiments, the one or more stimulatory agent is indirectly immobilized via a mutein of streptavidin that reversibly binds to a streptavidin-binding peptide. In some embodiments, the chromatography kit can comprise a stimulatory reagent, wherein the stimulatory reagent comprises one or more stimulatory agent capable of delivering a stimulatory signal in one or more T cells. In some embodiments, the at least one or at least one of the one or more stimulatory agent is a first stimulatory agent, and the chromatography kit, chromatography column, or chromatography column set further comprises one or more second stimulatory agent capable of enhancing, dampening, or modifying the stimulatory signal of the first stimulatory agent. In some embodiments, at least one of the second stimulatory agent is capable of specifically binding to a costimulatory molecule on the one or more T cells, *e.g.,* CD28, CD90 (Thy-1), CD95 (Apo-/Fas), CD137 (4-1BB), CD154 (CD40L), ICOS, LAT, CD27, OX40 or HVEM. In any of the preceding embodiments, the stationary phase may comprise at least one of the one or more second stimulatory agent.

In any of the preceding embodiments, the stimulatory signal may be through a TCR/CD3 complex in a T cell, a CD3-containing complex in a T cell, and/or an ITAM-containing molecule in a T cell.

In any of the preceding embodiments, the one or more stimulatory agent can be or comprise an agent selected from the group consisting of antibody fragments, monovalent antibody fragments, proteinaceous binding molecules with immunoglobulin-like functions, molecules containing Ig domains, cytokines, chemokines, aptamers, MHC molecules, MHC-peptide complexes; receptor ligands; and binding fragments thereof. In any of the preceding embodiments, the one or more stimulatory agent can be or comprise an antibody fragment. In any of the preceding embodiments, the one or more stimulatory agent can be or comprise a Fab fragment. In any of the preceding embodiments, the one or more stimulatory agent can be or comprise one selected from the group of divalent antibody fragments consisting of F(ab')₂ fragments and divalent single-chain Fv (scFv) fragments. In any of the preceding embodiments, the one or more stimulatory agent can be or comprise a monovalent antibody fragment selected from the group consisting of Fab fragments, Fv fragments, and scFvs. In any of the preceding embodiments, the one or more stimulatory agent can be or comprise a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers.

In any of the preceding embodiments, the one or more stimulatory agent can further comprise biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag.

In any of the preceding embodiments, the first and second stimulatory agents, independently, can be or comprise an agent selected from the group consisting of antibody fragments, monovalent antibody fragments, proteinaceous binding molecules with immunoglobulin-like functions, molecules containing Ig domains, cytokines, chemokines, aptamers, MHC molecules, MHC-peptide complexes; receptor ligands; and binding fragments thereof. In any of the preceding embodiments, the first and second stimulatory agents, independently, can be or comprise an antibody fragment. In any of the preceding embodiments, the first and second stimulatory agents, independently, can be or comprise a Fab fragment. In any of the preceding embodiments, the first and second stimulatory agents, independently, can be or comprise one selected from the group of divalent antibody fragments consisting of F(ab')₂ fragments and divalent single-chain Fv (scFv) fragments. In any of the preceding embodiments, the first and second stimulatory agents, independently, can be or comprise a monovalent antibody fragment selected from the group consisting of Fab fragments, Fv fragments, and scFvs. In any of the preceding embodiments, the first and second stimulatory agents, independently, can be or comprise a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers. In some embodiments, the first stimulatory reagent is an anti-CD3 Fab and the second stimulatory agent is an anti-CD28 Fab.

In any of the preceding embodiments, the first and second stimulatory agents, independently, can further comprise biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag. In any of the preceding embodiments, the first and second stimulatory agents, independently, can further comprise a streptavidin-binding peptide.

In any of the preceding embodiments, the streptavidin-binding peptide can be selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19).

In any of the preceding embodiments, the first stimulatory agent and the second stimulatory agent can be reversibly bound to an oligomeric stimulatory reagent comprising a plurality of streptavidin or streptavidin mutein molecules, wherein the size of the oligomeric stimulatory reagent comprises i) a radius of greater than 50 nm, ii) a molecular weight of at least 5 × 10⁶ g/mol; and/or (iii) at least 100 streptavidin or streptavidin mutein tetramers per oligomeric stimulatory reagent.

In any of the preceding embodiments, the stimulatory reagent can comprise a plurality of streptavidin or streptavidin mutein molecules, wherein the size of the stimulatory reagent comprises i) a radius of greater than 50 nm, ii) a molecular weight of at least 5 × 10⁶ g/mol; and/or (iii) at least 100 streptavidin or streptavidin mutein tetramers per stimulatory reagent.

In any of the preceding embodiments, the streptavidin mutein can comprise the amino acid sequence Val⁴⁴-Thr⁴⁵-Ala⁴⁶-Arg⁴⁷ at sequence positions corresponding to positions 44 to 47 of SEQ ID NO: 1, or the streptavidin mutein can comprise the amino acid sequence lle⁴⁴-Gly⁴⁵-Ala⁴⁶-Arg⁴⁷ at sequence positions corresponding to positions 44 to 47 of SEQ ID NO: 1. In any of the preceding embodiments, the N-terminal amino acid residue of the streptavidin mutein can be in the region of amino acids 10 to 16 of SEQ ID NO: 1, and the C-terminal amino acid residue of the streptavidin mutein can be in the region of amino acids 133 to 142 of SEQ ID NO: 1. In any of the preceding embodiments, the streptavidin mutein can comprise the amino acid sequence set forth in any of SEQ ID NOs: 3-6, 27, 28, 104, and 105.

In some embodiments, disclosed herein is a device, comprising the housing assembly, the housing assembly set, or the chromatography kit, chromatography column, or chromatography column set of any of the preceding embodiments, and the device further comprises an input composition reservoir operably connected to the internal cavity via an inlet of the inlet housing member. In some embodiments, disclosed herein is a device, comprising the chromatography system of any of the preceding embodiments, and the device further comprises an input composition reservoir operably connected to the internal cavity via an inlet of the inlet housing member. In some embodiments, disclosed herein is a device, comprising the jacket member of any of the preceding embodiments and a chromatography column or chromatography column set, wherein the chromatography column comprises an internal cavity configured to house a stationary phase for column chromatography, and the device further comprises an input composition reservoir operably connected to an inlet of the internal cavity to permit intake of an input composition comprised in the input composition reservoir into the internal cavity. In some embodiments, the input composition comprises or is blood or a blood-derived sample. In some embodiments, the input composition comprises or is a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some embodiments, the apheresis or leukapheresis product is freshly isolated from a subject or thawed from a cryopreserved apheresis or leukapheresis product.

In any of the preceding embodiments, the device can further comprise an output composition reservoir operably connected to the internal cavity via an outlet of the outlet housing member. In any of the preceding embodiments, the device can further comprise an output composition reservoir operably connected to the an outlet of the internal cavity to permit or effect discharge of an output composition comprised in the output composition reservoir from the internal cavity. In some embodiments, the output composition comprises or is enriched T cells. In some embodiments, the enriched T cells have undergone stimulation during chromatography on the chromatography column. In any of the preceding embodiments, the device can be in a closed or sterile system.

In some embodiments, disclosed herein is a method of preparing a chromatography column or chromatography column set, comprising introducing a stationary phase into the housing assembly or the housing assembly set of any of the preceding embodiments.

In some embodiments, disclosed herein is a method of preparing a chromatography column or chromatography column set, comprising introducing the stationary phase of the chromatography kit of any of the preceding embodiments into the housing assembly or housing assembly set of the chromatography kit.

In some embodiments, the competition agent or free binding agent can be an agent that competes for binding with a streptavidin binding peptide of the selection agent to a streptavidin mutein immobilized on the stationary phase. In some embodiments, the competition agent or free binding agent can be a biotin or a biotin analog, optionally wherein the biotin analog is D-biotin. In some embodiments, the competition agent or free binding agent can be D-biotin.

In any of the preceding embodiments, the stimulatory agent may be or comprise an oligomeric stimulatory reagent comprising (i) a plurality of streptavidin or streptavidin mutein molecules and (ii) one or more stimulatory agent capable of delivering a stimulatory signal in one or more T cells, where the size of the oligomeric stimulatory reagent comprises i) a radius of greater than 50 nm, ii) a molecular weight of at least 5 × 10⁶ g/mol; and/or (iii) at least 100 streptavidin or streptavidin mutein tetramers per oligomeric stimulatory reagent. In some embodiments, the streptavidin mutein comprises the amino acid sequence Val⁴⁴-Thr⁴⁵-Ala⁴⁶-Arg⁴⁷ or lle⁴⁴-Gly⁴⁵-Ala⁴⁶-Arg⁴⁷ at sequence positions corresponding to positions 44 to 47 with reference to positions in streptavidin in the sequence of amino acids set forth in SEQ ID NO:1; or the streptavidin mutein comprises the amino acid sequence Val⁴⁴-Thr⁴⁵-Ala⁴⁶-Arg⁴⁷ at sequence positions corresponding to positions 44 to 47 with reference to positions in streptavidin in the sequence of amino acids set forth in SEQ ID NO: 1.

In any of the preceding embodiments, at least one of the one or more stimulatory agent can be capable of delivering a stimulatory signal, wherein the stimulatory signal is through a TCR/CD3 complex in a T cell, a CD3-containing complex in a T cell, and/or an ITAM-containing molecule in a T cell.

In any of the preceding embodiments, the at least one of the one or more stimulatory agent can be a first stimulatory agent capable of delivering the stimulatory signal and the one or more stimulatory agent can further comprise one or more of a second stimulatory agent capable of enhancing, dampening, or modifying the stimulatory signal of the first stimulatory agent. In some embodiments, the second stimulatory agent can be capable of specifically binding to a costimulatory molecule on the one or more T cells. In some embodiments, the costimulatory molecule can be selected from among CD28, CD90 (Thy-1), CD95 (Apo-/Fas), CD137 (4-1BB), CD154 (CD40L), ICOS, LAT, CD27, OX40 or HVEM. In any of the preceding embodiments, the second stimulatory agent can be capable of specifically binding to CD28 and/or the costimulatory molecule is CD28.

In any of the preceding embodiments, the first stimulatory agent can specifically bind CD3 and the second stimulatory agent can specifically bind CD28. In any of the preceding embodiments, the first stimulatory agent can comprise a monovalent antibody fragment that binds to CD3 and the second stimulatory agent can comprise a monovalent antibody fragment that binds to CD28. In some embodiments, the monovalent antibody fragment can be selected from the group consisting of a Fab fragment, an Fv fragment, and a single-chain Fv fragment (scFv). In any of the preceding embodiments, the first stimulatory agent can be an anti-CD3 Fab and the second stimulatory agent can be an anti-CD28 Fab.

In any of the preceding embodiments, during at least a portion of the incubation, the temperature control member can regulate the temperature of the stationary phase to a target temperature between about 30°C and about 39°C

In any of the preceding embodiments, during at least a portion of the incubation, the temperature control member may regulate the temperature of the stationary phase to a target temperature between about 35°C and about 39°C.

In any of the preceding embodiments, during at least a portion of the incubation, the temperature control member can maintain the temperature of the stationary phase at a target temperature between about 30°C and about 39°C.

In any of the preceding embodiments, during at least a portion of the incubation, the temperature control member can maintain the temperature of the stationary phase at a target temperature between about 35°C and about 39°C.

In any of the preceding embodiments, the target temperature is between about 30°C and about 39°C, optionally at or about 37°C. In any of the preceding embodiments, the target temperature can be 37°C or about 37°C.

In any of the preceding embodiments, during at least a portion of the incubation, the connector may allow intake of gas into the internal cavity. In some embodiments, the gas is sterile and is or comprises air. In any of the preceding embodiments, the intake of gas into the internal cavity can be intermittent or continuous during the incubation.

In any of the preceding embodiments, the sample may be or comprise a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some embodiments, the apheresis or leukapheresis product is freshly isolated from a subject. In some embodiments, the apheresis or leukapheresis product is thawed from a cryopreserved apheresis or leukapheresis product.

### Brief Description of the Drawings

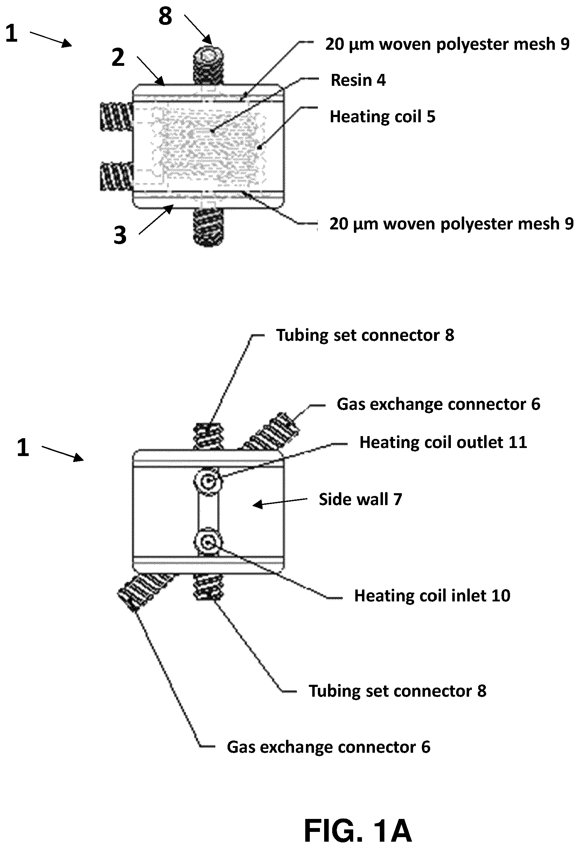

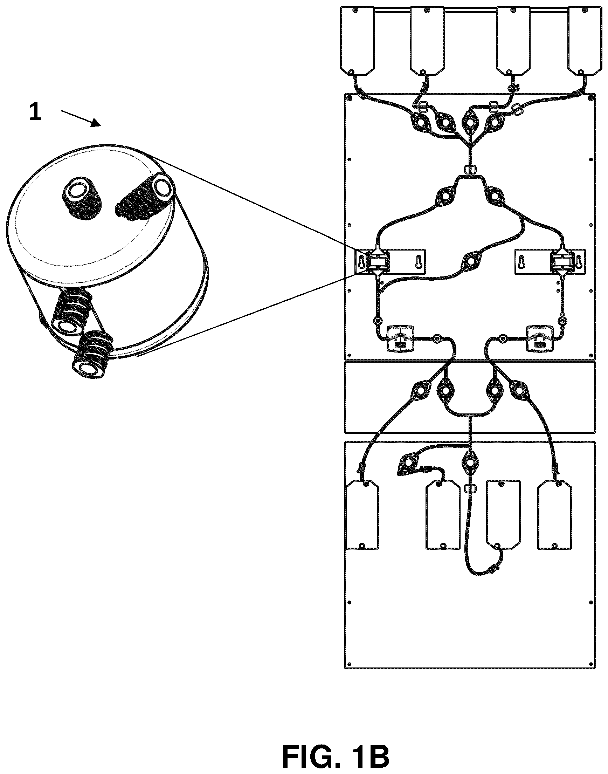

**FIGS. 1A** **and** **1B** provide a schematic representation of an exemplary housing assembly for column chromatography. **FIG. 1A** shows the exemplary housing assembly comprising a temperature control member comprising a heating coil with inlet and outlet for external warm water supply, and a gas supply connector for screw-on air filters. **FIG. 1B** shows the exemplary housing assembly in an exemplary column chromatography system.

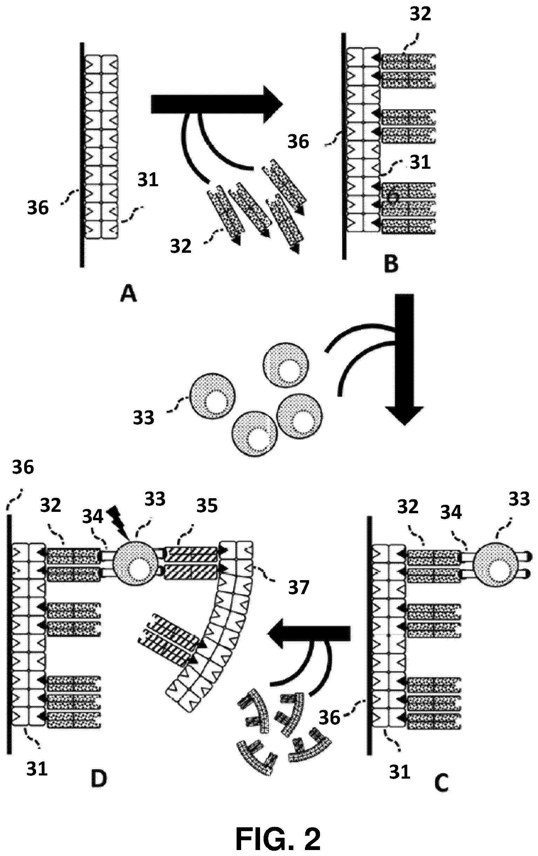

**FIG. 2** provides a schematic representation of an exemplary embodiment for stimulating and selecting for target cells, in which the stimulation is carried out by an incubation of the cells, which occurs, at least in part, in the presence of a support, **36,** drawn here as a stationary phase, having immobilized thereon component(s) of a selection reagent **31** for cell selection (Panel A), which has a binding site for a selection agent **32,** which is capable of binding to a molecule (selection marker) **34** present on some or all of the target cells. The selection agent **32** is added to the support with immobilized selection reagent **31,** under conditions whereby the selection reagent and selection agent reversibly bind, *e.g*., via binding sites, generating an oligomeric complex with the selection agent multimerized thereon (Panel B). The selection agent can include more than one agent. Alternatively, the reversibly bound complex of the selection agent and selection reagent may be added to the stationary phase as a complex for immobilization. As shown, cells **33,** including target cells, are combined with the stationary phase and multimerized selection agent complex, whereby target cells become reversibly immobilized to the support **36,** via the selection agent **32** and reagent (selection marker) **34** (Panel C). Optionally, cells not bound are removed, either prior to addition of stimulatory agents or subsequent thereto. A complex containing multimerized stimulatory agents **35** reversibly bound to an oligomeric stimulatory reagent **37** is added, under conditions whereby the stimulatory agent **35** specifically binds to a molecule on the target cells, thereby inducing or modulating a signal in the immobilized target cells expressing the marker (Panel D).

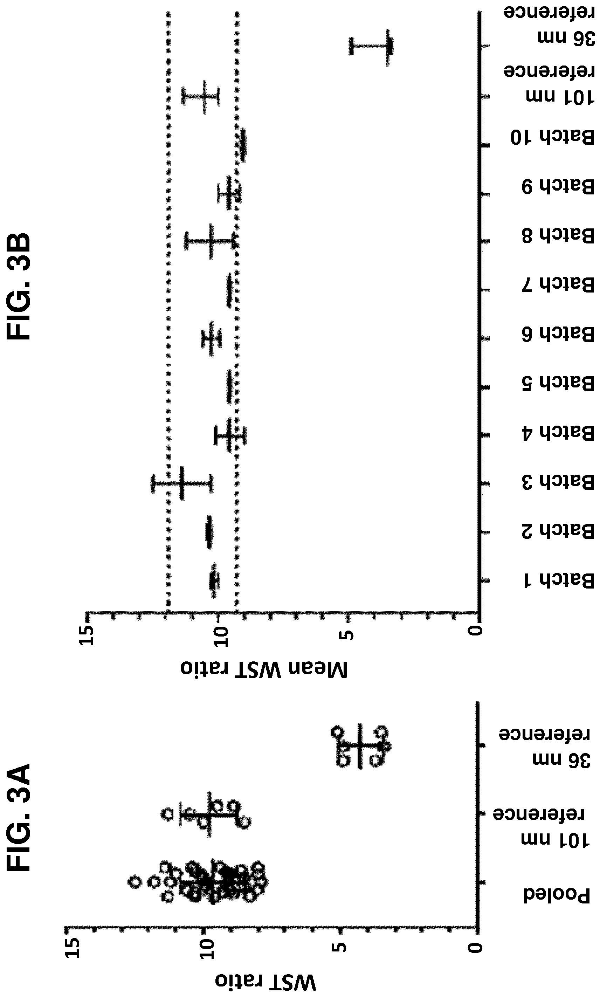

**FIGS. 3A and 3B** show results of a WST metabolic assay of T cells from three different donors incubated with anti-CD3/anti-CD28 multimerized on different batches of oligomeric reagents. **FIG. 3A** summarizes WST metabolic activity, as indicated by WST ratio, for all tested batches (pooled) compared to reference batches containing anti-CD3/anti-CD28 multimerized on an oligomeric backbone with an average hydrodynamic radius of 36 nm or 101 nm. The average WST metabolic activity, as indicated by mean WST ratio, among T cells from the different donors for individual tested batches and reference reagents is shown in **FIG. 3B****.**

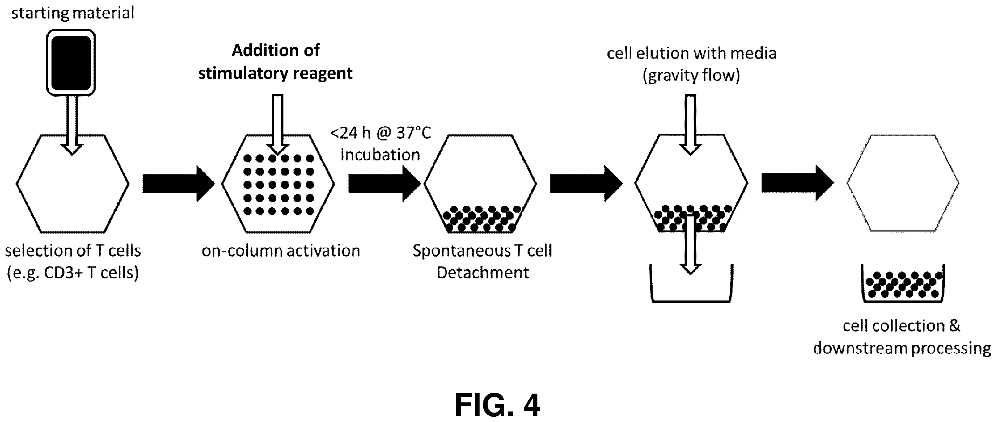

**FIG. 4** provides a schematic representation of an exemplary on-column T cell selection and stimulation process.

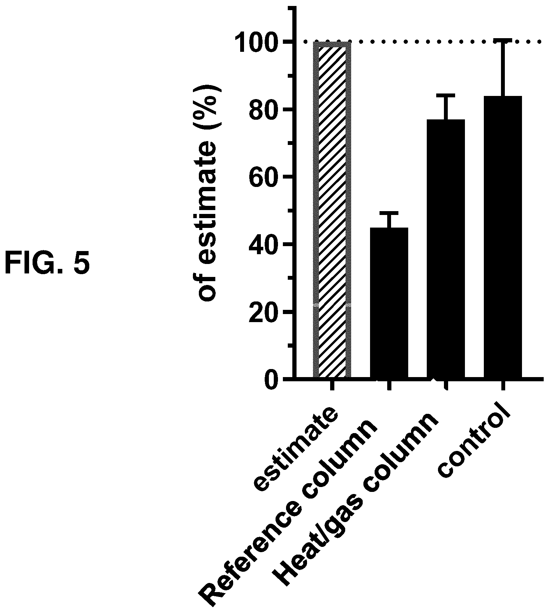

**FIG. 5** shows elution efficiency using an exemplary heat/gas column having a heating element and a gas supply element was approximately two-fold of that using the reference column. The estimate (grey bar) was the theoretical number of captured cells that could be eluted assuming 100% efficiency.

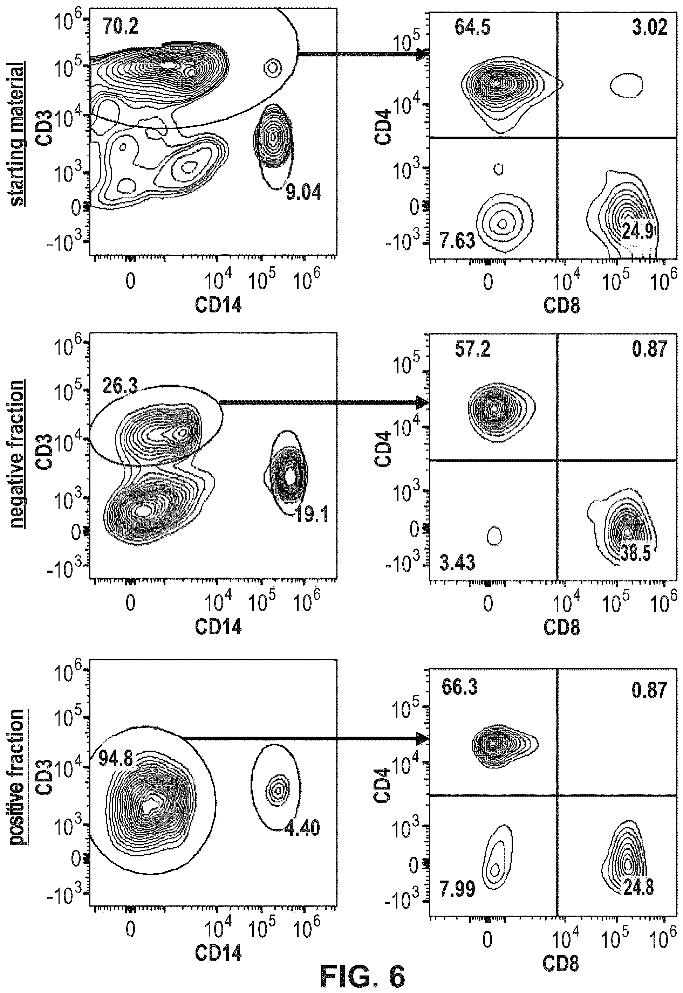

**FIG. 6** shows flow cytometry quantification of cells in the starting material, the negative fraction or the positive fraction, after on-column T cell selection and stimulation using the exemplary column having a heating element and a gas supply element. The cells were stained with antibodies recognizing surface markers including CD3, CD4, CD8, CD45 and CD14.

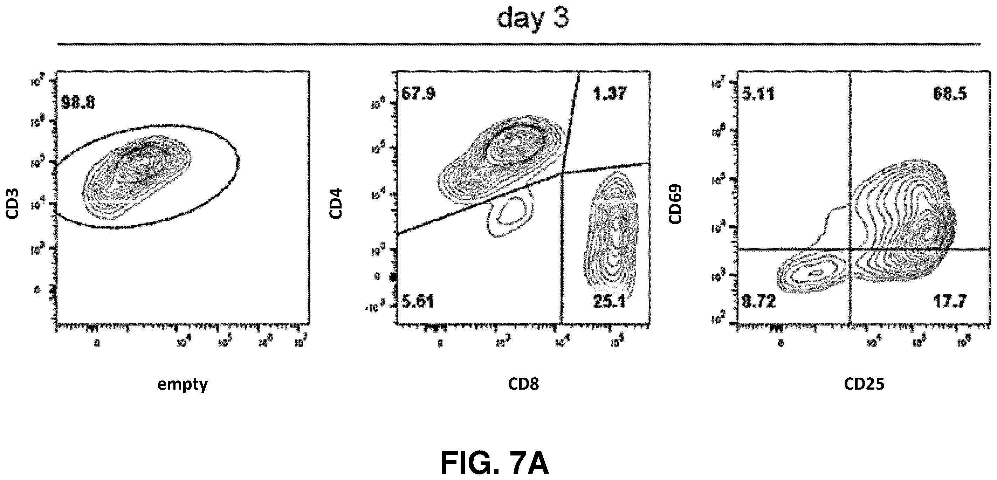

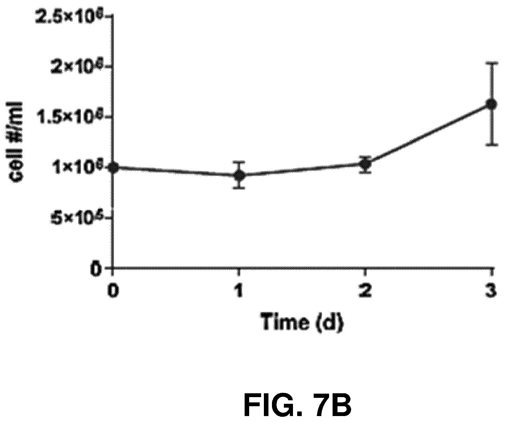

**FIGS. 7A** **and** **7B** show results of T cells after on-column selection and stimulation using the exemplary column having a heating element and a gas supply element. The cells were monitored, at Day 1, Day 2, and Day 3 during the subsequent incubation, for cell number and cell surface expression by flow cytometry after staining the cells with antibodies recognizing CD3, CD4, CD8, and the activation markers CD69 and CD25, and the flow cytometry results are shown in **FIG. 7A****.** Assessment for cell number and fold-expansion following the subsequent incubation showed that the selected and stimulated T cells had started to increase in number at Day 3, as shown in **FIG. 7B****,** consistent with the ability of the cells to proliferate.

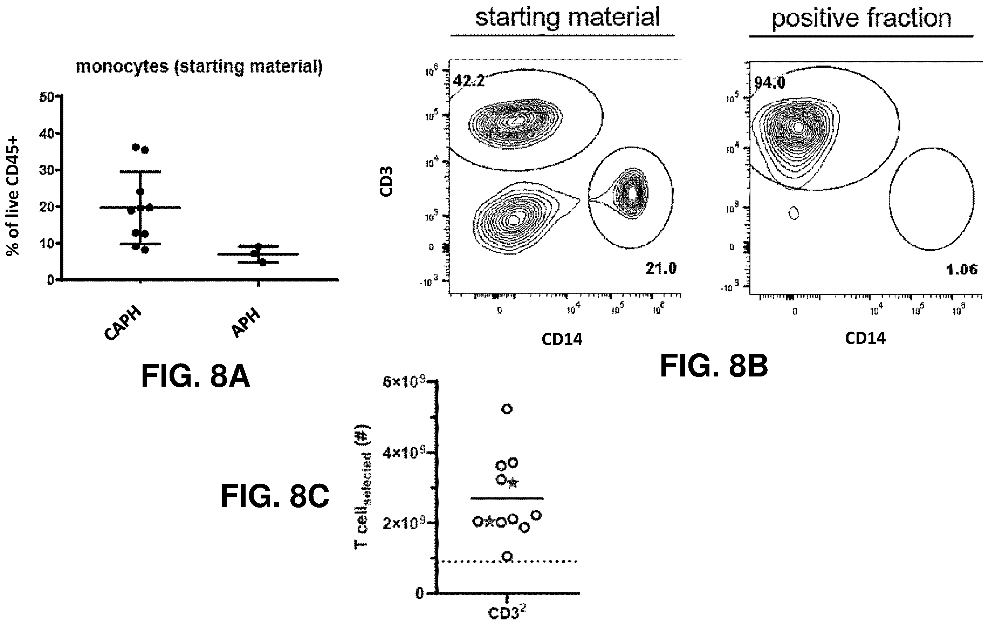

**FIGS. 8A-8C** provide results of on-column T cell selection using a cryopreserved apheresis sample as the starting sample, on the exemplary heat/gas column. **FIG. 8A** shows that cryopreserved apheresis samples (CAPHs) generally have high monocyte content (greater than 20%, as indicated by the % of live CD45+ cells), compared to fresh apheresis samples (APHs). **FIG. 8B** depicts the percentage of cells positive for CD3 or CD14 in the starting material and positive fraction. The numbers of T cells selected using the chromatography column are shown in **FIG. 8C****,** where two sequential selections for CD3 were carried out.

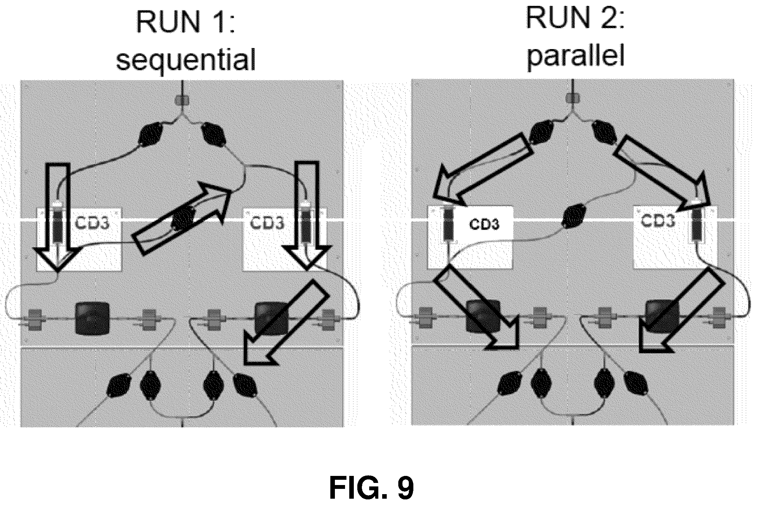

**FIG. 9** provides a schematic representation of a selection and stimulation run using two identical exemplary heat/gas columns that were arranged sequentially (Run 1), and a selection and stimulation using two identical exemplary heat/gas columns that were arranged in parallel (Run 2).

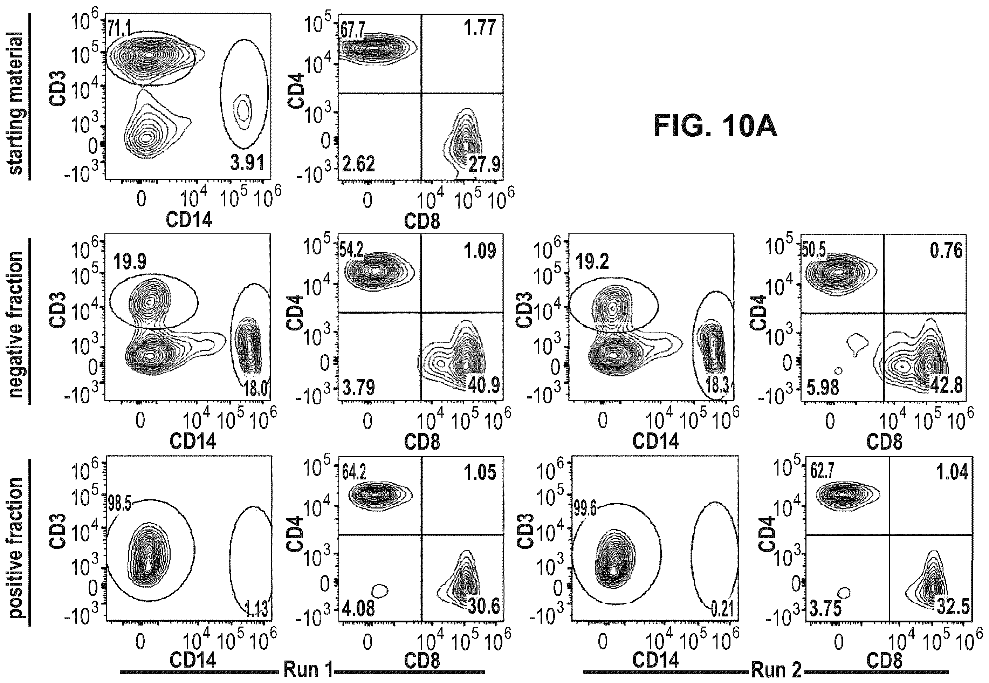

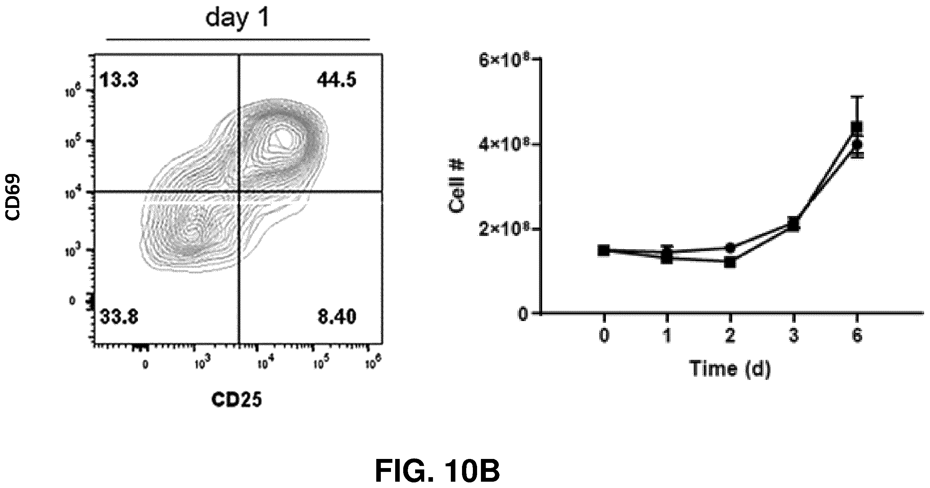

**FIGS. 10A** **and** **10B** provide comparisons of results of T cell selection and stimulation in Run 1 and Run 2. **FIG. 10A** shows flow cytometry analysis of the starting materials, the negative fractions, and the positive fractions, where cells were stained with antibodies recognizing surface markers including CD3, CD4, CD8, and CD14. Cells from the positive fractions were harvested and incubated, and **FIG. 10B****,** left panel, shows expression of activation markers CD25 and CD69 in the cells at Day 1 in incubation Representative results for cell number in Run 1 (▪) and Run 2 (•) during incubation are shown in **FIG. 10B****,** right panel.

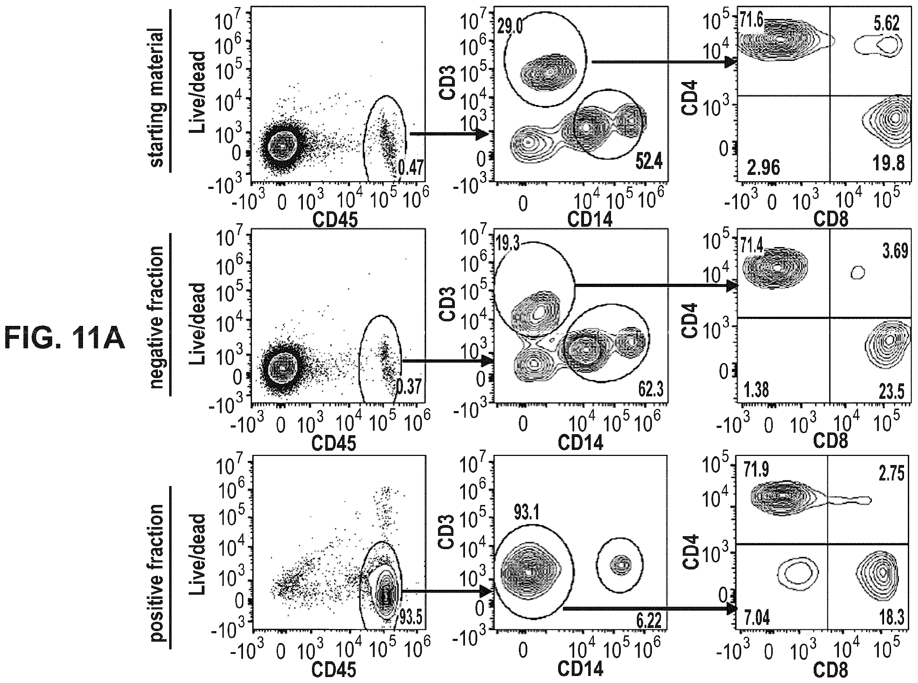

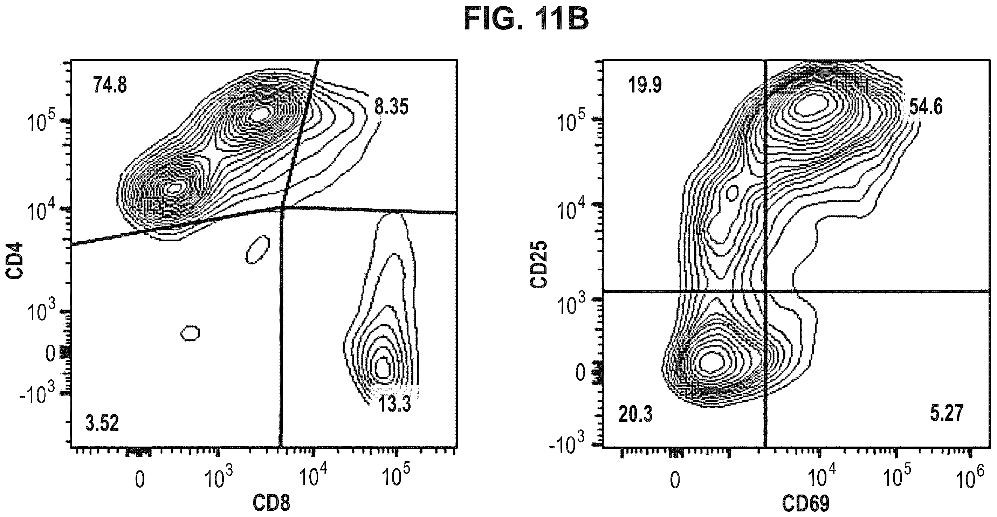

**FIGS. 11A** **and** **11B** provide results of on-column T cell selection using a concentrated blood sample as the starting sample, with CD3 selection and stimulation on two exemplary heat/gas columns arranged in parallel. **FIG. 11A** shows flow cytometry analysis of the starting material, the negative fraction, and the positive fraction, where cells were stained with antibodies recognizing surface markers including CD3, CD4, CD8, and CD14. Cells from the positive fraction were harvested and incubated, and CD4/CD8 and CD25/CD69 expressions of the incubated cells are shown in **FIG. 11B****.**

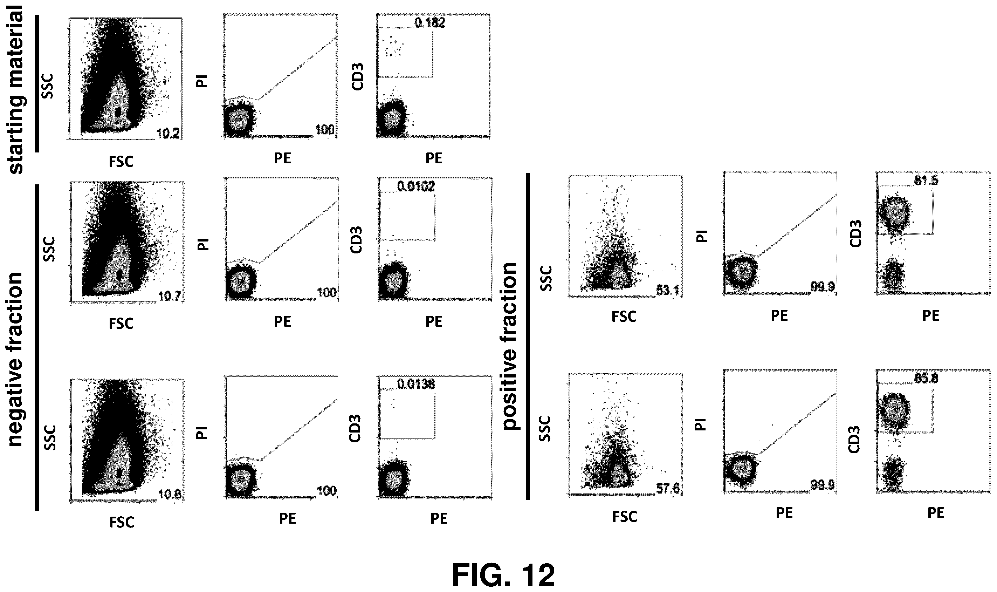

**FIG. 12** provides results of an exemplary process of selecting T cells directly from whole blood, using Sephadex^{®} G-50 as the resin in the exemplary heat/gas chromatography column. The starting material, the negative fractions, and the positive fractions from the CD3+ T cell selection were stained with propidium iodine (PI) and a CD3 antibody and quantified by flow cytometry.

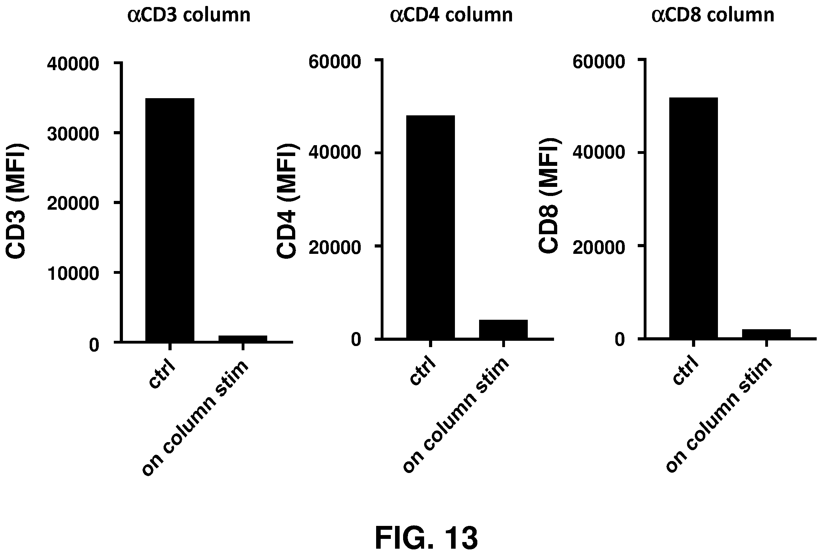

**FIG. 13** shows the effects of 24 hour on-column stimulation with an anti-CD3/anti-CD28 oligomeric stimulatory reagent on CD3, CD4, and CD8 surface expression (assessed as mean fluorescence intensity, MFI) when the respective molecule was used as a selection marker to immobilize the cell on the stationary phase of a chromatography column. Surface expression patterns are compared to control conditions not involving on-column stimulation with an anti-CD3/anti-CD28 oligomeric stimulatory reagent. Cells were isolated from an apheresis sample applied to the stationary phase.

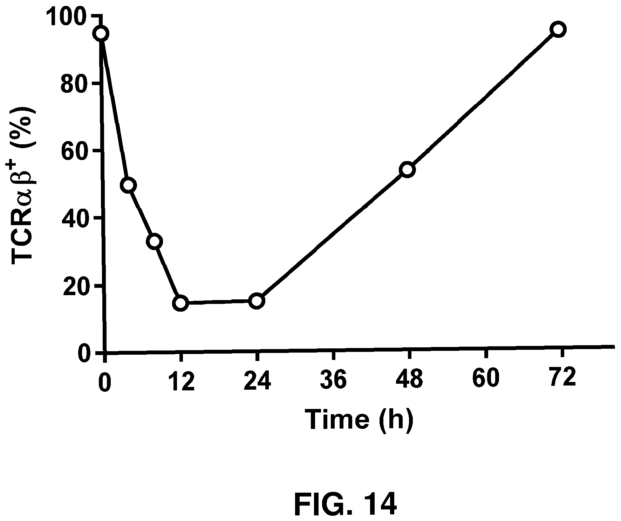

**FIG. 14** shows exemplary kinetics of downregulation and re-expression of the TCR/CD3 complex upon on-column stimulation with an anti-CD3/anti-CD28 oligomeric stimulatory reagent when CD3 was used as a selection marker to immobilize the cell on the column. Cells were isolated from an apheresis sample applied to the stationary phase. An antibody against the alpha-beta TCR chains was used to assess the the CD3/TCR complex.

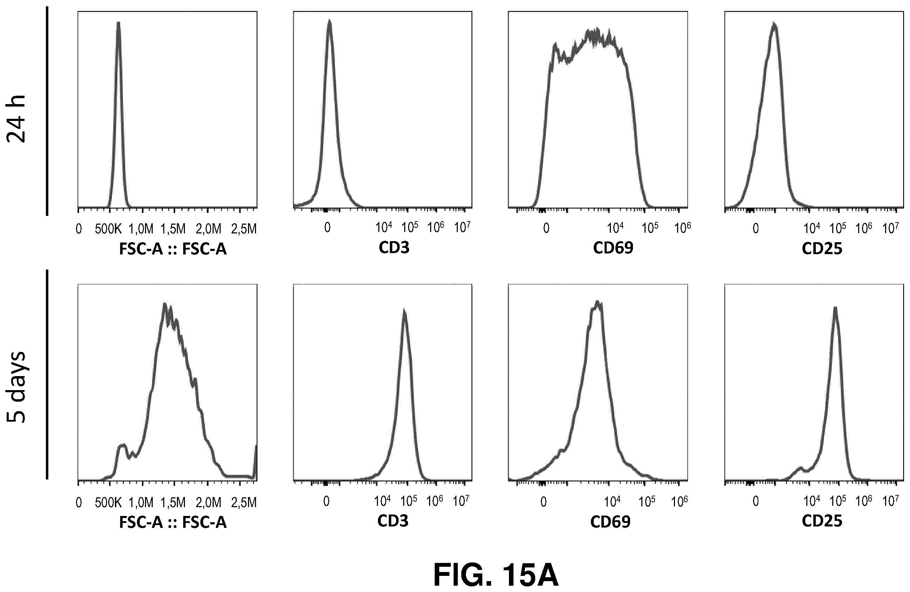

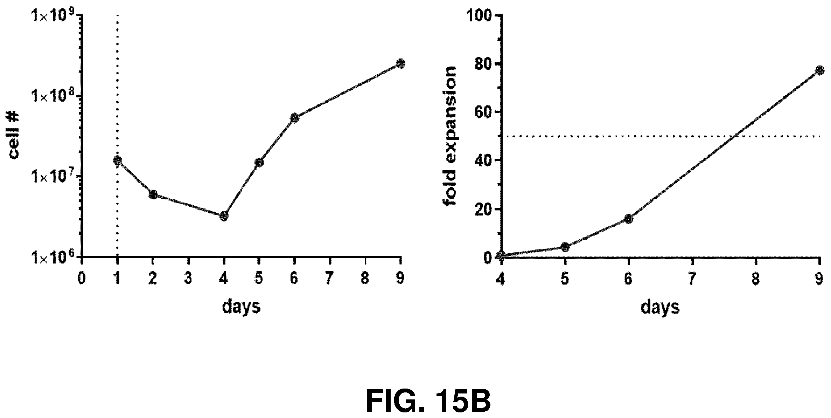

**FIGS. 15A-15B** show phenotypic and functional characteristics of cultured T cells that spontaneously detached during on-column stimulation with an anti-CD3/anti-CD28 oligomeric stimulatory reagent. **FIG. 15A** shows from left to right T cell size and CD3, CD69, and CD25 expression at 24 hours and 5 days following on-column stimulation. **FIG. 15B** shows the proliferative capacity of the spontaneously detached cultured T cells, as indicated by cell number and fold expansion. Cells were isolated from an apheresis sample applied to the stationary phase and collected using a wash step.

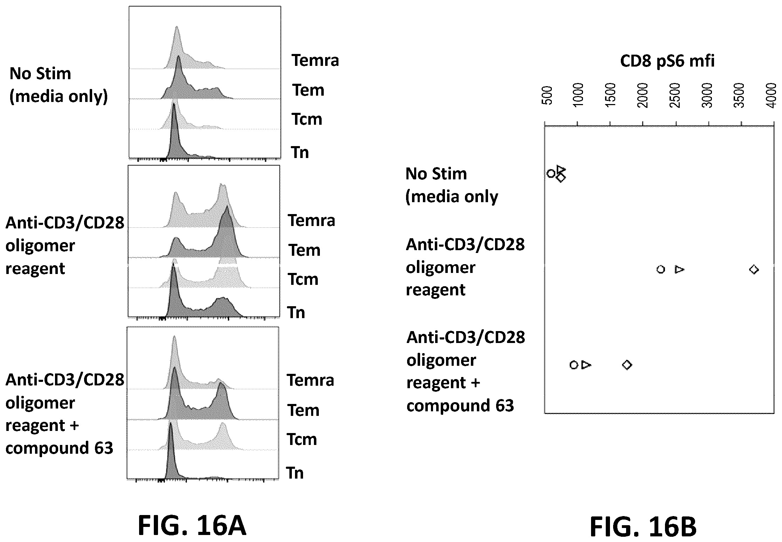

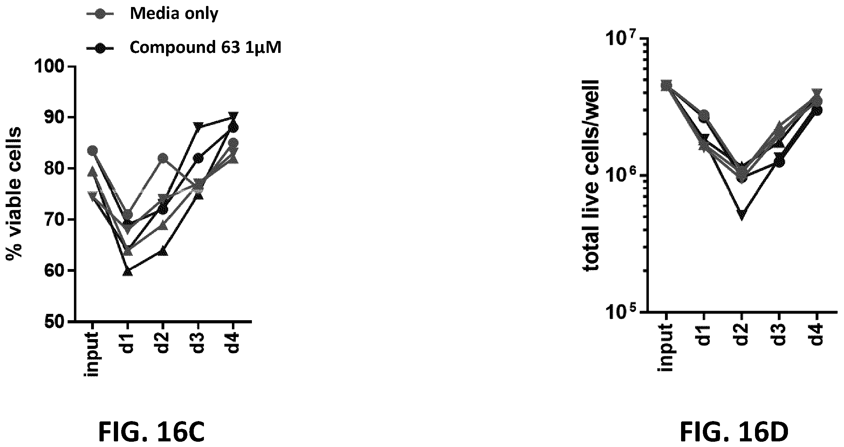

**FIGS. 16A-16D** show exemplary effects of incubating T cells with an anti-CD3/anti-CD28 oligomeric stimulatory reagent in the presence or absence of Compound 63 on mTor signaling and viability and growth kinetics. **FIG. 16A** shows pS6 expression in live CD8+ T cells by memory subset. **FIG. 16B** shows the mean florescence intensity (mfi) of pS6 expression of total CD8 T cells by treatment as indicated. **FIGS. 16C-16D** show viability and total T cell numbers, respectively, over time (as indicated by days; d1, etc) in culture after initiation of stimulation ("input"). In **FIGS. 16C-16D****,** black lines correspond to T cell compositions incubated in the presence of Compound 63, and gray lines correspond to T cell compositions incubated in the absence of Compound 63.

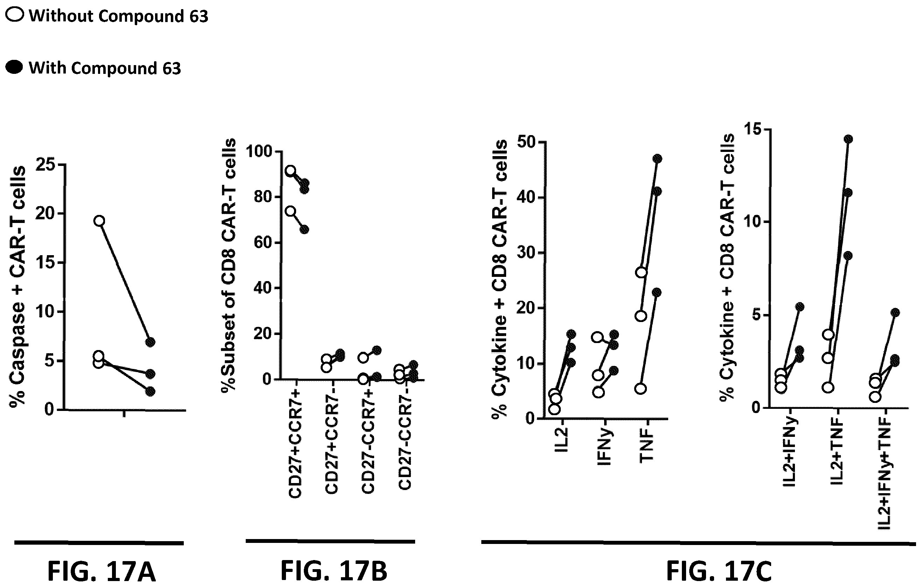

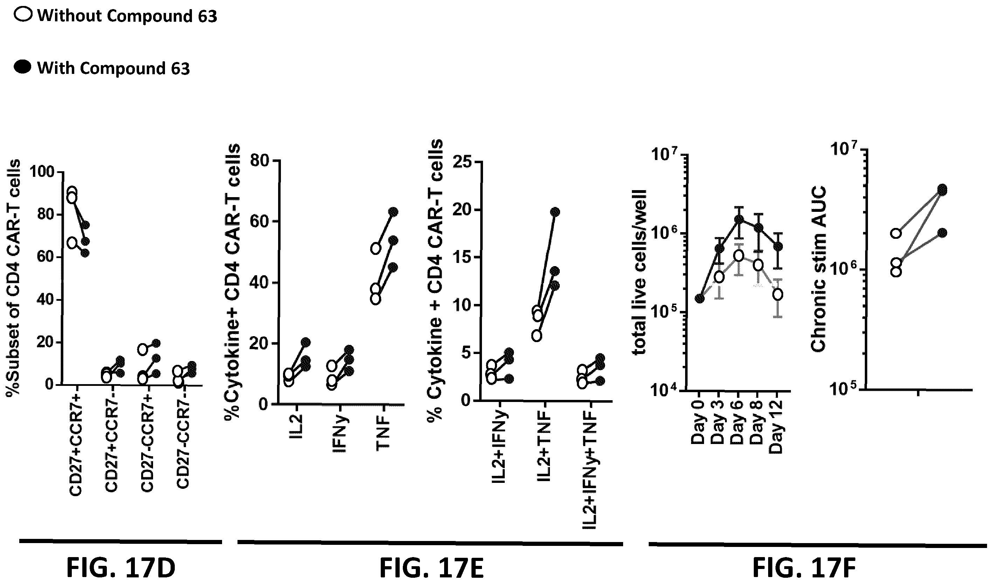

**FIGS. 17A-17F** show exemplary functional and phenotypic properties of cryopreserved CAR-T cells generated using methods employing incubation with an anti-CD3/anti-CD28 oligomeric stimulatory reagent in the presence or absence of Compound 63. **FIG. 17A** shows intracellular expression of Caspase at the time of thaw. **FIGS. 17B** and **17D** show CD8 CAR-T cell and CD4 CAR-T cell phenotypic profiles, respectively, by subset expression of CD27 and/or CCR7. **FIGS. 17C** and **17E** show intracellular IL2, IFNg, or TNF (left panels) or combinations of IL2 and/or IFNg or TNF (right panels) among CD8 CAR-T cells and CD4 CAR-T cells, respectively, stimulated with antigen-bearing targets. **FIG. 17F** shows expansion and survival over 12 days (left panel) and total expansion metric calculated by area under the growth curve (AUC, right panel) for CAR-T cells stimulated with anti-CAR beads.

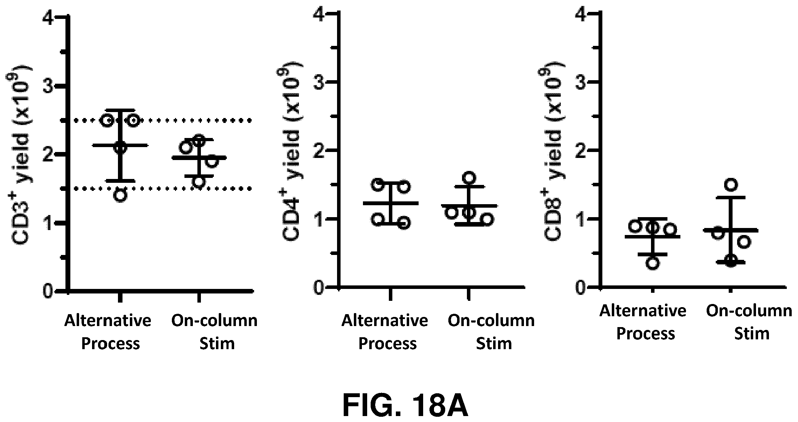

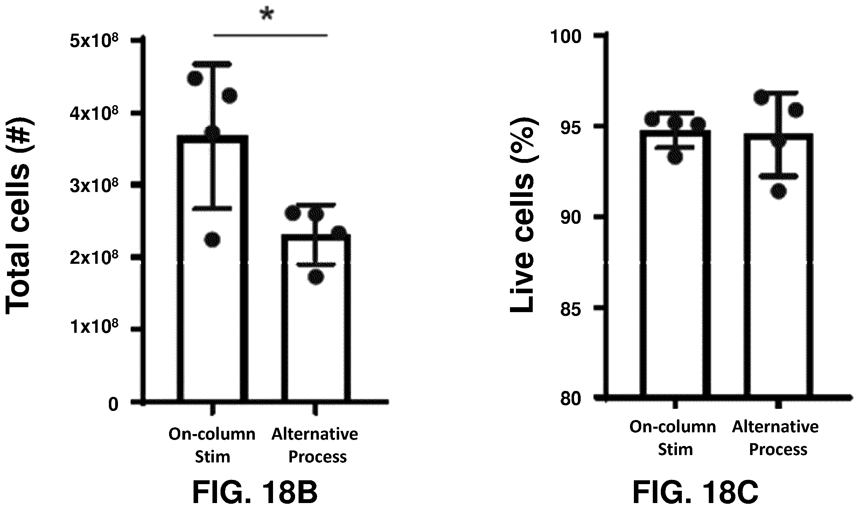

**FIG. 18A** shows CD3+, CD4+ and CD8+ T cell yields following cell selection either using the on-column stimulation process or alternative process described in Example 11. **FIGS. 18B-18C** show the total number of cells **(****FIG. 18B****)** and percentage of live cells **(****FIG. 18C****)** recovered following the use of on-column stimulation or alternative processes described in Example 11.

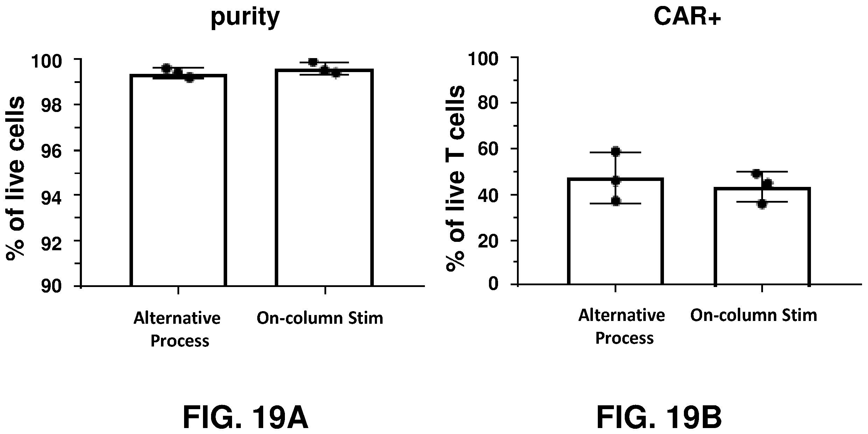

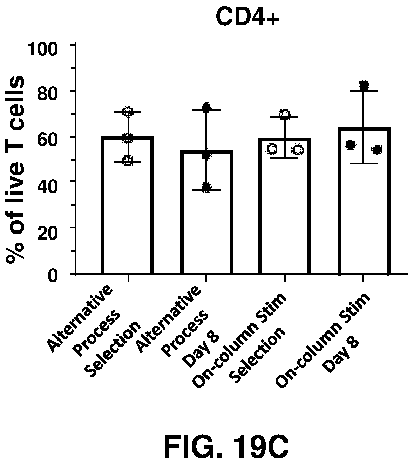

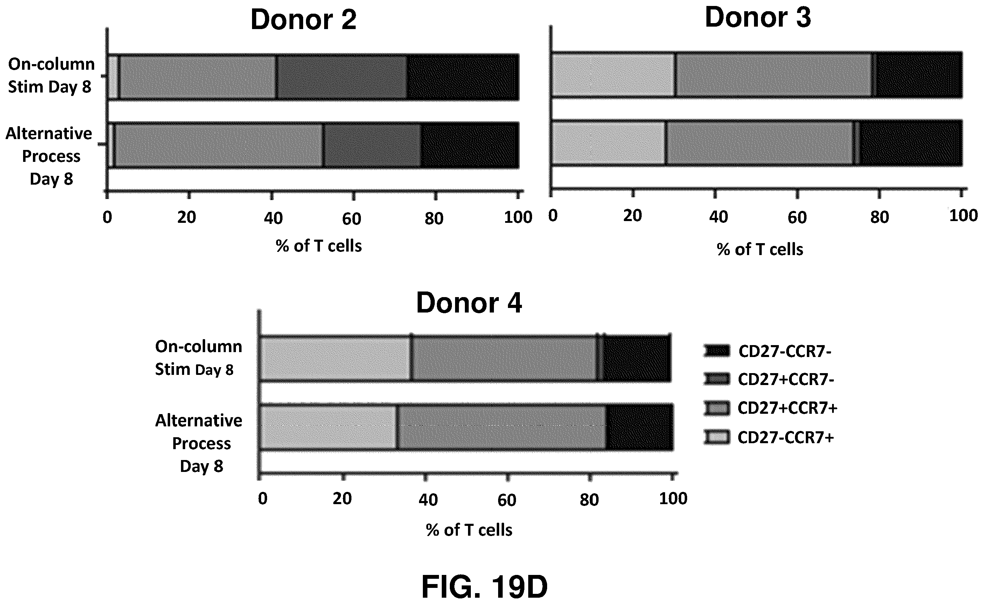

**FIGS. 19A-19D** show the percentage of live cells (e.g., purity; **FIG. 19A****),** the percentage of live cells expressing the exemplary CAR **(****FIG. 19B****),** the percentage of live cells expressing CD4 at selection and on day 8 of the process **(****FIG. 19C****),** and T cell phenotype distributions (percentage) for each donor **(****FIG. 19D****)** on day 5 in culture (day 8 from the beginning of the process) for the on-column stimulation or the alternative processes described in Example 11.

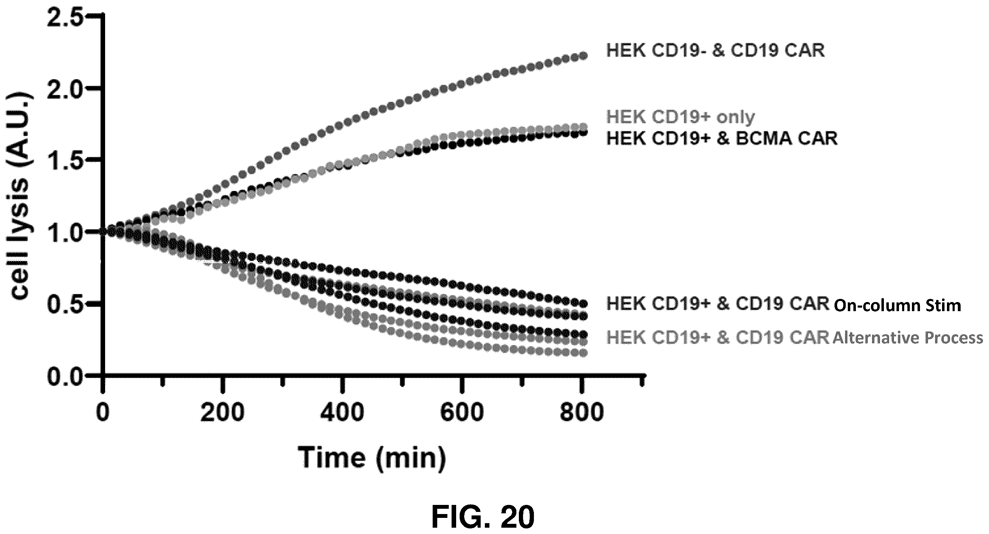

**FIG. 20** shows CD19+ HEK cell lysis over time during culture with anti-CD19 CAR T cells engineered using on-column stimulation or alternative processes, as described in Example 11, and under control conditions.

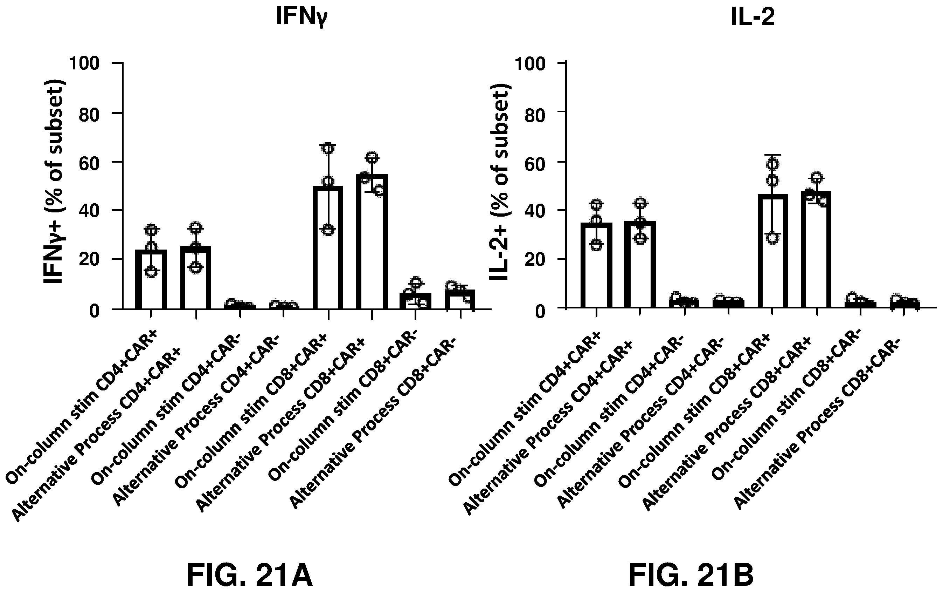

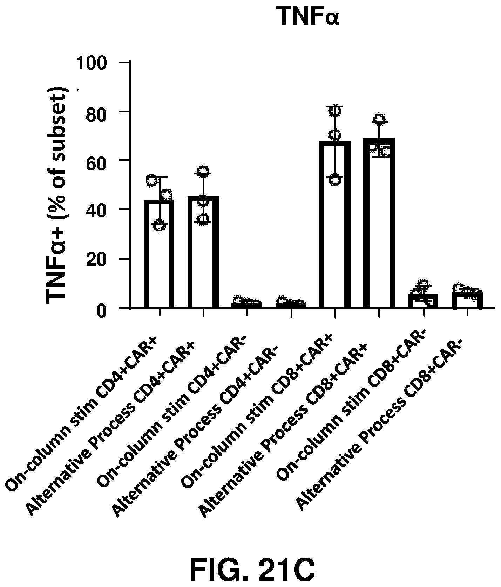

**FIGS. 21A-21C** show antigen-specific CAR T cell IFNg **(****FIG. 21A****),** IL-2 **(****FIG. 21B****),** and TNFα **(****FIG. 21C****)** production for CD4 and CD8 T cells engineered using the on-column stimulation or the alternative processes described in Example 11.

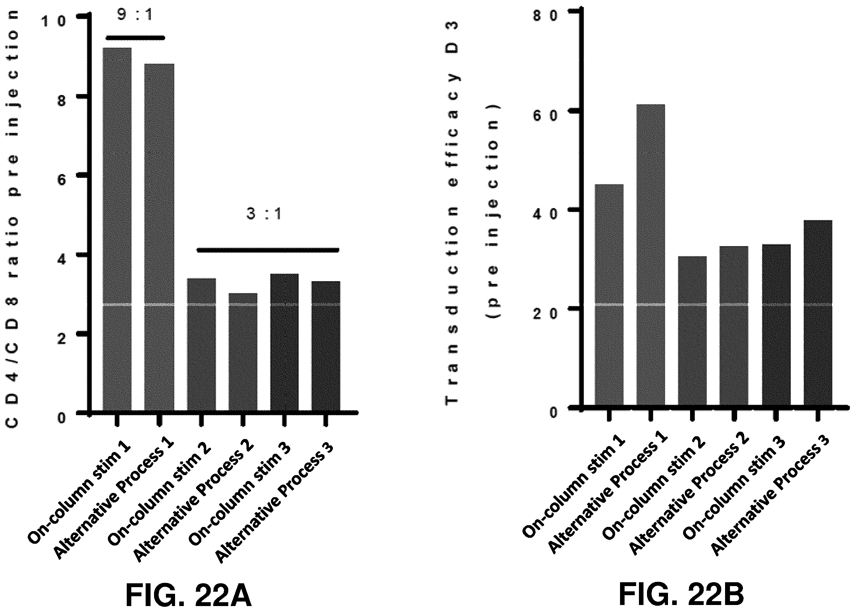

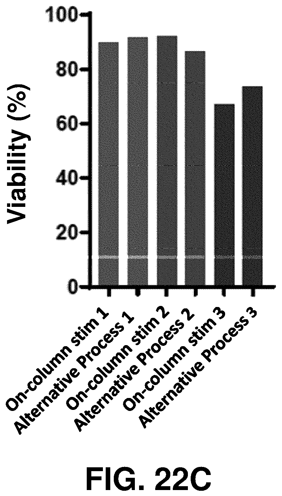

**FIGS. 22A-22C** show the CD4:CD8 ratio **(****FIG. 22A****),** transduction efficiency of engineered T cells (CD4 and CD8 cells combined; **FIG. 22B****),** and the percentage of viable cells **(****FIG. 22C****)** generated using the on-column stimulation or the alternative processes described in Example 11. Three manufacturing runs are shown for each process.

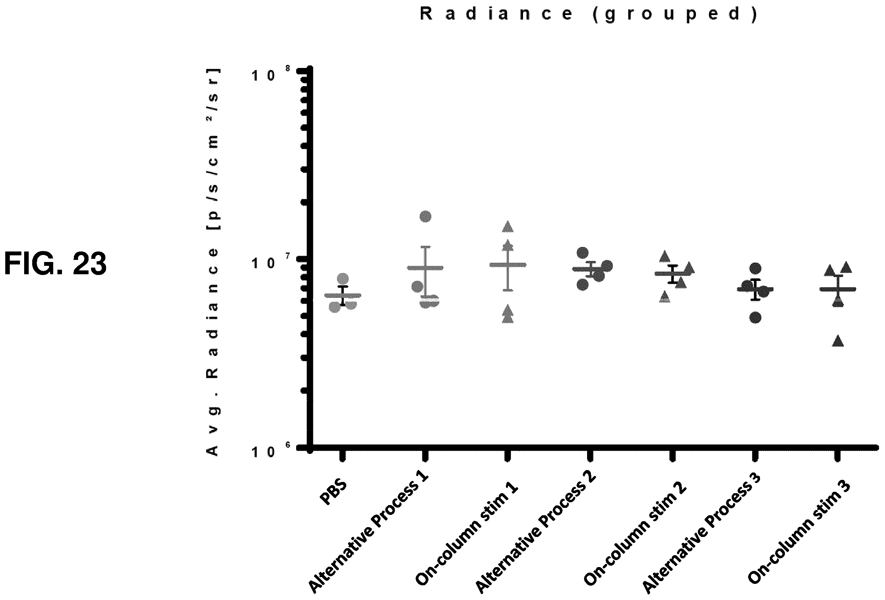

**FIG. 23** shows tumor size by average radiance across treatment groups 6 days after mice were injected (i.v.) with B cell lymphoma cell line (Raji) and prior to the mice being treated with CAR-T cell compositions. Treatment groups refer to CAR-T cell compositions produced by three manufacturing runs each of the on-column stimulation or the alternative processes described in Example 11.

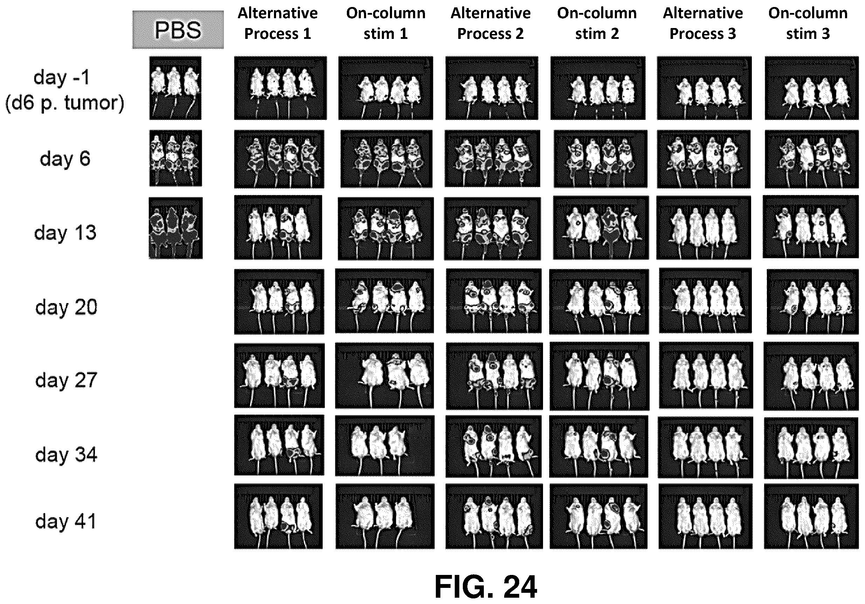

**FIG. 24** shows tumor burden in B cell lymphoma cell line (Raji) injected mice over time for each treatment group. CAR T cell treatment effects are shown for on-column stimulation or the alternative processes described in Example 11, and each of the three manufacturing runs (see **FIGS. 22A-22C****).**

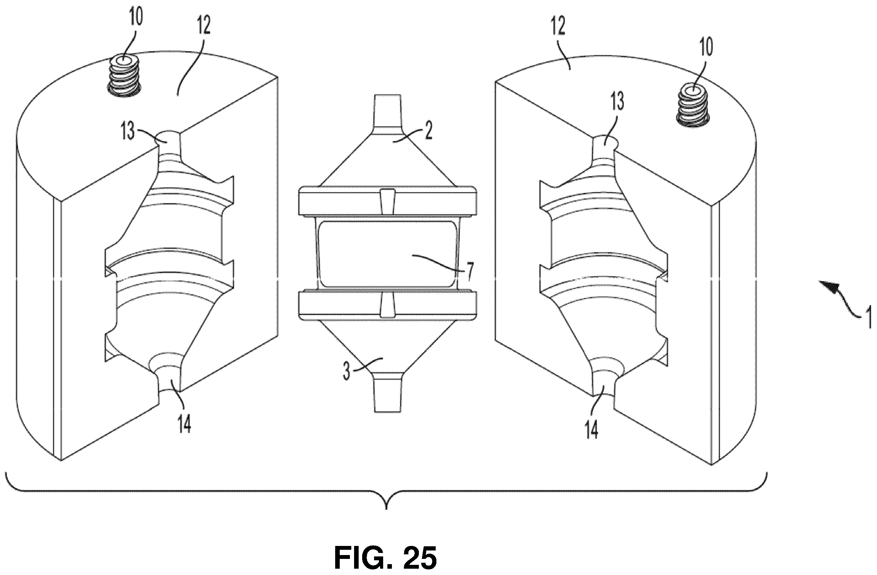

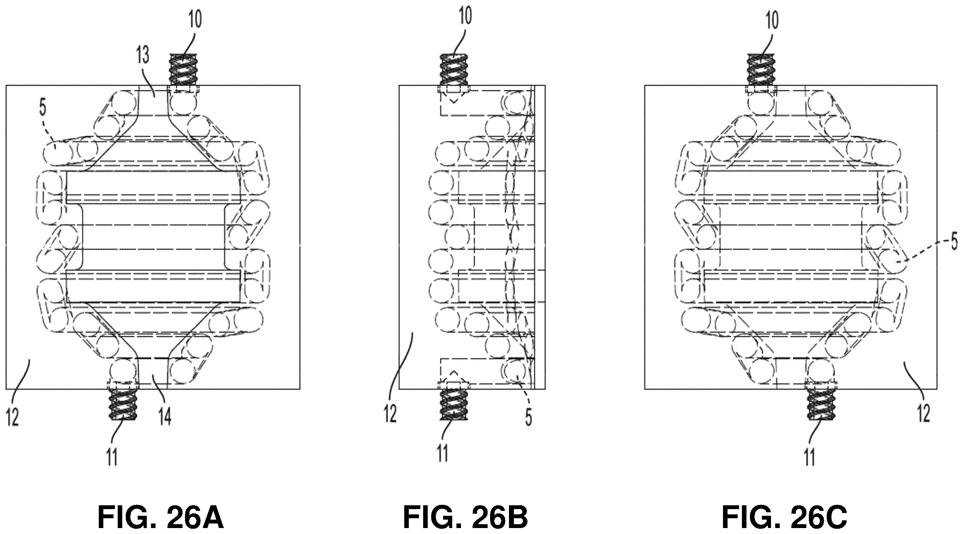

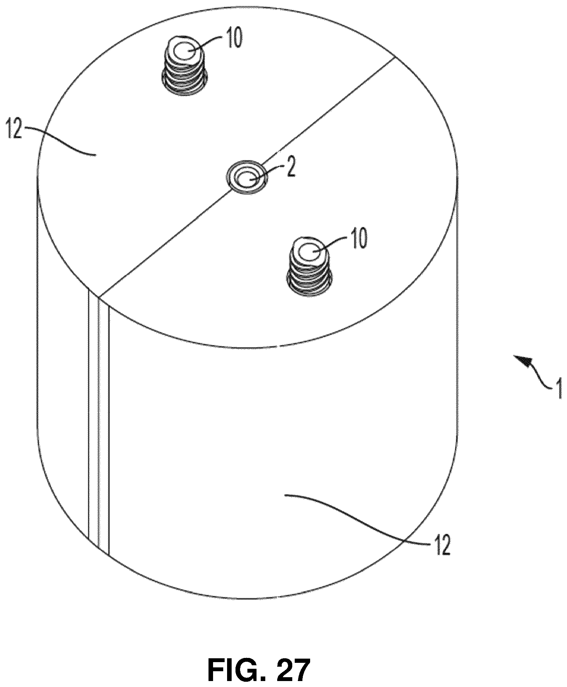

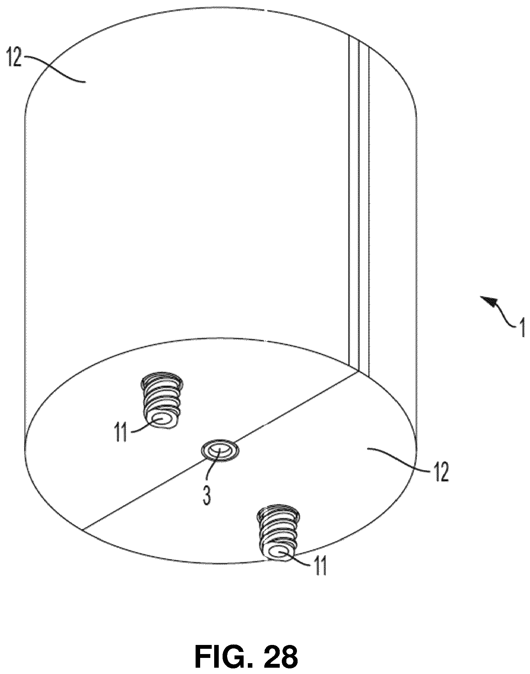

**FIGS. 25-28** provide schematic representations of an exemplary housing assembly for column chromatography. This exemplary housing assembly includes an inlet housing member, an outlet housing member, a side wall member, and a jacket member that surrounds the side wall member as well as portions of the inlet housing member and the outlet housing member. The jacket member of the exemplary housing assembly is made of two jacket components each containing a heating coil with inlet and outlet for external warm water supply. Together, the two jacket components form the jacket member. The exemplary housing assembly also includes a gas supply connector for screw-on air filters (not shown), said gas supply connector connected to an inlet of the inlet housing member. **FIG. 25** shows an exploded view of the exemplary housing assembly. **FIGS. 26A-26C** show views of the interior **(****FIG. 26A****),** side **(****FIG. 26B****),** and exterior **(****FIG. 26C****)** of one jacket component. **FIG. 27** shows a view of the exemplary housing assembly such that the inlets for external warm water supply and a portion of an inlet of the inlet housing member are visible. **FIG. 28** shows a view of the exemplary housing assembly such that the outlets for external warm water supply and a portion of an outlet of the outlet housing member are visible. Optional features (not shown) for this exemplary housing assembly include a first porous member configured to separate the stationary phase and an inlet of the internal cavity (e.g., a woven polyester mesh), a second porous member configured to separate the stationary phase and an outlet of the internal cavity (e.g., a woven polyester mesh), and tubing set connectors.

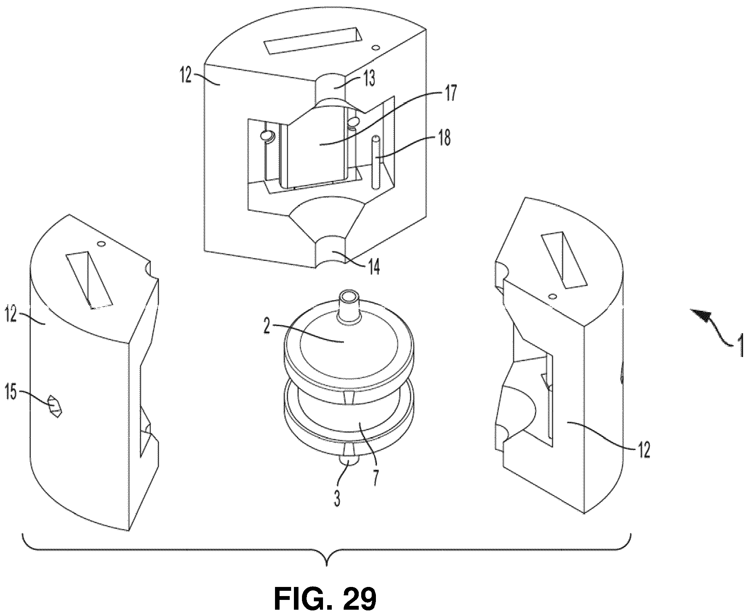

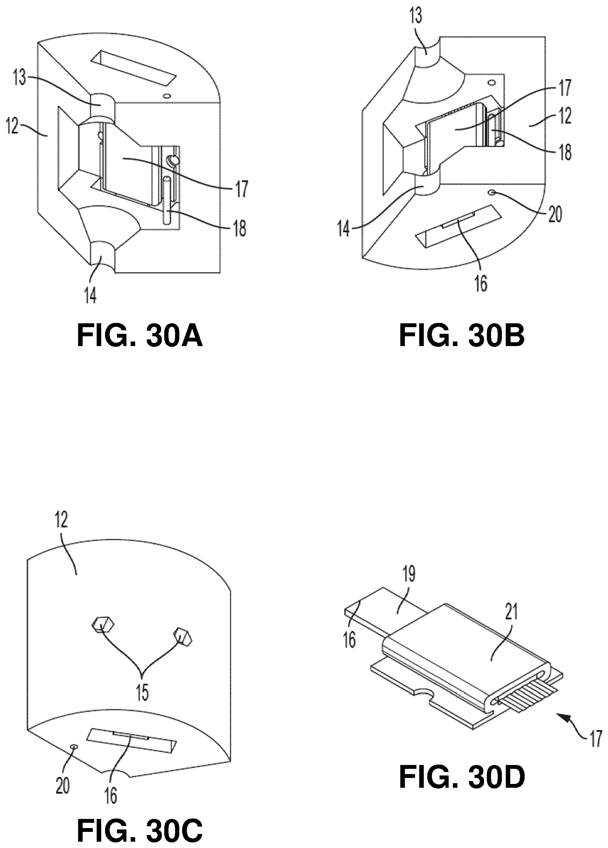

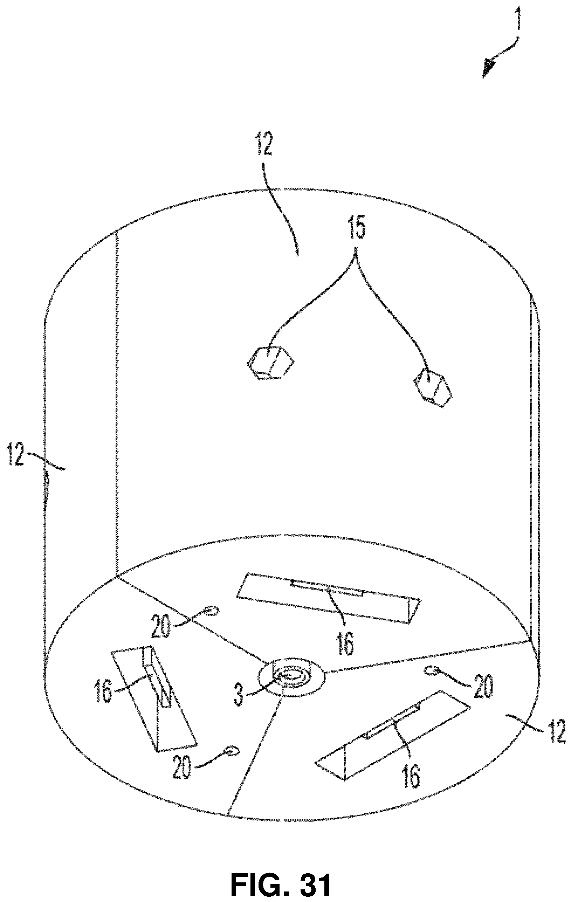

**FIGS. 29-31** provide schematic representations of an exemplary housing assembly for column chromatography. This exemplary housing assembly includes an inlet housing member, an outlet housing member, a sidewall member, and a jacket member that surrounds the side wall member as well as portions of the inlet housing member and the outlet housing member. The jacket member of the exemplary housing assembly is made of three jacket components each containing an electric heating element that includes a metal plate. Together, the three jacket components form the jacket member. The exemplary housing assembly also includes a gas supply connector for screw-on air filters (not shown), said gas supply connector connected to an inlet of the inlet housing member. **FIG. 29** shows an exploded view of the exemplary housing assembly. **FIGS. 30A-30C** show three views of one jacket component. **FIG. 30D** shows the electric heating element. **FIG. 31** shows a view of the exemplary housing assembly such that the electrical connections of the electric heating elements as well as a portion of an outlet of the outlet housing member are visible. Optional features (not shown) for this exemplary housing assembly include a first porous member configured to separate the stationary phase and an inlet of the internal cavity (e.g., a woven polyester mesh), a second porous member configured to separate the stationary phase and an outlet of the internal cavity (e.g., a woven polyester mesh), and tubing set connectors.

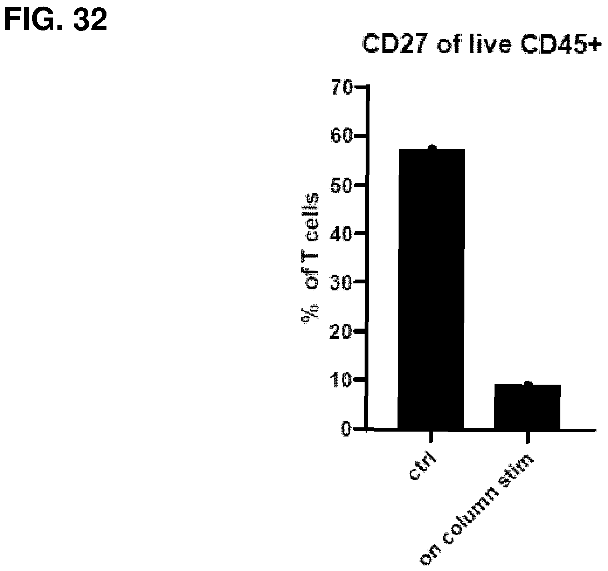

**FIG. 32** shows CD27 surface expression of cells after cells were immobilized on the stationary phase of a heated column using CD27 as a selection marker and stimulated on-column with an anti-CD3/anti-CD28 oligomeric stimulatory reagent. The column was heated using a jacket member containing two heating coils each with inlet and outlet for external warm water supply. The heated column also included a gas supply connector for screw-on air filters. As a control, CD27-selected cells were not subjected to on-column stimulation with an anti-CD3/anti-CD28 oligomeric stimulatory reagent. Cells were isolated from an apheresis sample applied to the stationary phase.

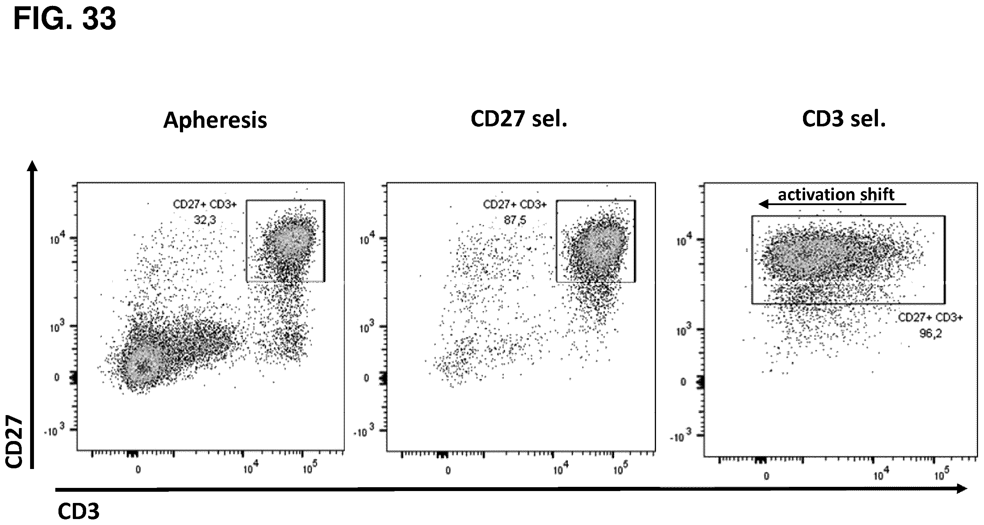

**FIG. 33** shows CD3 and CD27 surface expression of cells sequentially isolated from an apheresis sample using two separate columns. CD27 was used as a selection marker in the first column, and the positive fraction of the first column was passed to a second column with a CD3 selection marker. Immobilized cells in the second column were stimulated with an anti-CD3/anti-CD28 oligomeric stimulatory reagent. The second column was heated using a jacket member containing two heating coils each with inlet and outlet for external warm water supply. The heated column also included a gas supply connector for screw-on air filters.

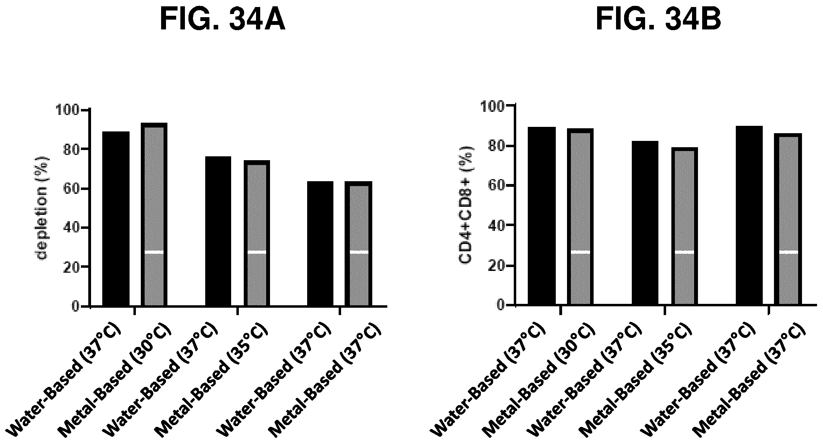

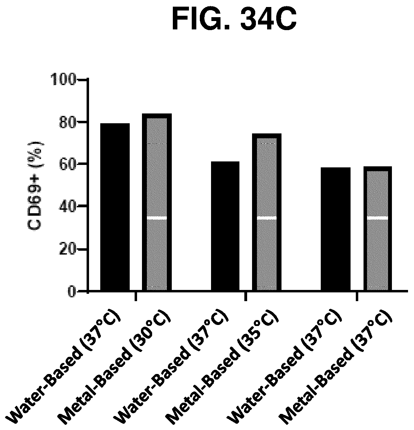

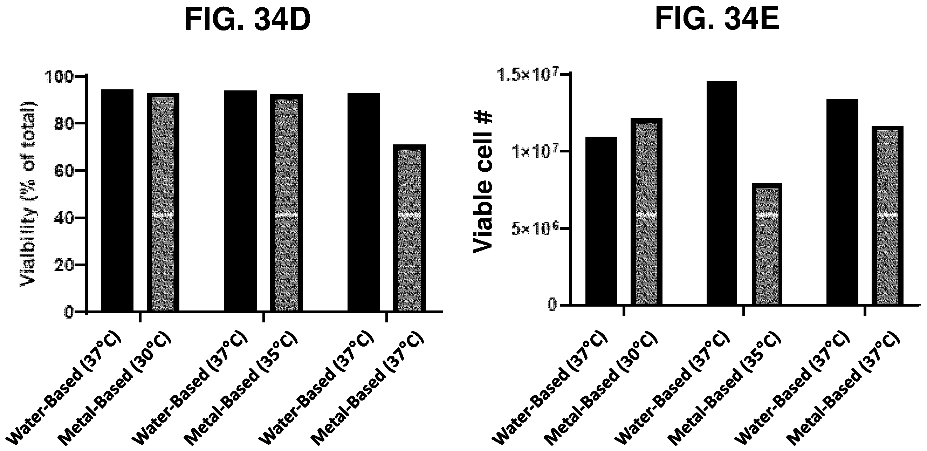

**FIGS. 34A-34E** show CD3+ depletion **(****FIG. 34A****),** CD4 and CD8 expression **(****FIG. 34B****),** CD69 expression **(****FIG. 34C****),** viability **(****FIG. 34D****),** and viable cell number **(****FIG. 34E****)** of cells after on-column stimulation in chromatography columns heated using different heating elements. Columns were heated using jacket members containing two heating coils (water) or three metal plates as electric heating elements (metal). Columns also included a gas supply connector for screw-on air filters.

### Detailed Description

In some aspects, provided herein is a housing assembly for column chromatography. In some aspects, the housing assembly for column chromatography comprises an inlet housing member and an outlet housing member, wherein at least the inlet housing member and the outlet housing member form an internal cavity configured to house a stationary phase for column chromatography; a temperature control member configured to provide heat to the stationary phase in the internal cavity; and a connector configured to operably connect the internal cavity to a gas source, thereby permitting or effecting intake of gas into the internal cavity. In some aspects, provided herein is a housing assembly for column chromatography, comprising: a chromatography column comprising an internal cavity configured to house a stationary phase; a temperature control member configured to provide heat to the stationary phase in the internal cavity; and a connector configured to operably connect the internal cavity to a gas source, thereby permitting or effecting intake of gas into the internal cavity. In some embodiments, the chromatography column comprises an inlet housing member, an outlet housing member, and a side wall member, wherein the inlet housing member, the outlet housing member, and the side wall member form the internal cavity. In some aspects, the temperature control member comprises one or more heating elements. In some aspects, the housing assembly further comprises a jacket member comprising the temperature control member comprising at least one of the one or more heating elements. In some aspects, the jacket member surrounds at least a portion of the inlet housing member and/or at least a portion of the outlet housing member. In some aspects, provided herein is a device comprising a chromatography column housing assembly and a stationary phase housed therein to form a chromatography column. In some aspects, the stationary phase is configured to immobilize target cells thereon. In some aspects, the device is configured to select and/or stimulate a cell or cell population. In some embodiments, the selected and/or stimulated cell or cells are useful in a cell manufacturing process, for examples, for genetic engineering of the cell or cells to manufacture a cell therapy.

Methods for generating suitable cell populations, e.g., selected (enriched) and stimulated cell populations, for use in cell therapies often require separate selection and stimulation steps which can prolong the manufacturing process. Different reagents and systems are available for generating cell populations suitable for use in cell therapy, such as cells engineered to express recombinant proteins (e.g., chimeric antigen receptors)). However, in some aspects, using these reagents or systems may require a long or a relatively long amount of time to generate the cells, at least in part due to the need to perform multiple processing steps. Multiple processing steps may also result in cellular stress, thus affecting the usefulness of the cells in downstream processing. Furthermore, selection techniques may involve steps that contaminate selected cells with selection-related particles, such as, for example, selection agents such as Fab fragments and competition reagents and/or free binding agents used to facilitate detachment of the cells from the stationary phase, thus requiring additional wash steps and/or media exchange to purify the output composition. The additional processing steps may result in cell stress, potentially affecting downstream cell processing or even cell biology, in addition to requiring considerable time to complete. Additional devices and methods for generating cell compositions are needed.

In some aspects, provided herein are devices and methods of using the devices for selecting cells from a sample comprising target cells (e.g., T cells, such as CD3+, CD4+, or CD8+ T cells) and/or stimulating the selected cells. In some aspects, the device comprises an inlet housing member and an outlet housing member, wherein at least the inlet housing member and the outlet housing member form an internal cavity configured to house a stationary phase for column chromatography; a temperature control member configured to provide heat to the stationary phase in the internal cavity; and a connector configured to operably connect the internal cavity to a gas source, thereby permitting or effecting intake of gas into the internal cavity. In some aspects, the stationary phase is configured to immobilize said target cells thereon. In some aspects, the temperature control member comprises one or more heating elements. In some aspects, the device further comprises a jacket member comprising the temperature control member comprising the one or more heating elements. In some aspects, the jacket member is configured to surround at least a portion of the inlet housing member and/or at least a portion of the outlet housing member. The devices provided herein also include jacket members for use in selecting cells from a sample comprising target cells (e.g., T cells, such as CD3+, CD4+, or CD8+ T cells) and/or stimulating the selected cells. In some aspects, the jacket member comprises a temperature control member configured to provide heat to a chromatography column. In some aspects, the jacket member is configured to surround at least a portion of the chromatography column. In some aspects, the jacket member comprises one or more heating elements.

In one aspect, it is found herein that the methods of on-column selection and/or stimulation of the target cells are improved when the temperature of cells immobilized on the stationary phase in the internal cavity of the column chromatography is controlled to maintain the termperature at at or about 37°C or 37°C ±about 5°C. It also is found herein that a column configuration that permits gas exchange (e.g. presence of air in the column) also improves the overall health, fitness or condition of the cells during the on-column selection and/or stimulation. In particular embodiments, the provided devices are able to the control the temperature (e.g., 37°C or 37°C about 5°C) in the column during the selection and stimulation of cells by the provided on-column methods. In particular embodiments, the provided devices able to control the temperature (e.g., 37°C or 37°C about 5°C) and permit gas exchange, e.g. the presence of air, in the column during the selection and stimulation of cells by the provided on-column methods. In some aspects, the provided device can be used in connection with methods for selection and/or stimulation of cells to facilitate or improve cell activation and downstream processing of the cells, such as detachment or elution of cells from the stationary phase for subsequent genentic engineering of the cells.

In one aspect, the temperature control member is configured to provide a temperature appropriate for selection and/or stimulation of the target cells immobilized on the stationary phase in the internal cavity of the column chromatography. For this purpose, a heating and/or cooling means can be provided in the device or in a system comprising the device. In some embodiments, the temperature control member is configured to provide heat to the stationary phase, thereby regulating or maintaining the temperature of the target cells immobilized thereon. In some embodiments, the temperature of the target cells is regulated to and/or maintained at an optimal temperature for cell selection and/or stimulation. In one aspect, the temperature control member is configured to maintain the temperature of target cells immobilized on the stationary phase at an optimal temperature for stimulation by a a stimulatory reagent. In some embodiments, the optimal temperature for stimulation is higher than about 2°C, higher than about 4°C, higher than about 8°C, higher than about 12°C, higher than about 16°C, higher than about 20°C, higher than about 24°C, higher than about 28°C, higher than about 32°C, or higher than about 36°C. In some embodiments, the optimal temperature for stimulation is or is about 37°C. In some embodiments, the temperature of target cells immobilized on the stationary phase is kept at a constant temperature value (e.g., the optimal temperature) during at least portion of the stimulation. In some embodiments, the temperature of target cells immobilized on the stationary phase is kept at a selected temperature value (e.g., the optimal temperature) about 5°C, about 4°C, about 3°C, about 2°C, about 1°C or about 0.5°C during at least portion of the stimulation. In some embodiments, the temperature of target cells immobilized on the stationary phase is kept at 37°C about 5°C, about 4°C, about 3°C, about 2°C, ±about 1°C or ±about 0.5°C during at least portion of the stimulation.

In one aspect, the device comprises a connector configured to operably connect the internal cavity to a gas source, thereby permitting or effecting intake of gas into the internal cavity. Ine one aspect, gas is present in the internal cavity during at least a portion of stimulation of target cells immobilized on the stationary phase of the chromatography column. In some aspects, the gas comprises air.

In some aspects, the devices and methods, which are not in accordance with the claimed invention, provided herein reduce and/or minimize cell handling and processing time in a manufacturing process. In some aspects, the device comprises a stimulating agent configured to stimulate immobilized cells on the stationary phase (also referred to herein as on-column stimulation). In some aspects, the device further comprises one or more member, such as a heating member and/or a gas supply member, that facilitates or promotes cell activation, thereby facilitating or promoting spontaneous detachment of selected and stimulated cells from the stationary phase. In some aspects, the device further comprises one or more member configured to collect the selected and stimulated cells that spontaneously detach from the stationary phase (e.g., due to the stimulation) without the use of a competition agent or a free binding agent to facilitate detachment. In some aspects, the devices and methods (methods are not in accordance with the claimed invention) provided herein are capable of combining cell selection, stimulation, and/or collection steps. In some aspects, the devices and methods provided herein do not require separate steps to facilitate detachment of the selected and stimulated cells from the stationary phase. In some aspects, the devices and methods (methods are not in accordance with the claimed invention) provided herein do not require separate purification steps, e.g., steps to remove agents (e.g., competition agents and/or free binding agents) used to facilitate detachment. As such, the devices and methods (methods are not in accordance with the claimed invention) provided herein reduce the number of processing steps needed to generate a selected and stimulated cell composition suitable for downstream processing (e.g., genetic engineering, expansion, subsequent incubation, stimulation and/or selection (e.g., polishing)), thereby reducing manufacturing time, minimizing potential cell stress, and/or decreasing the potential for contamination. In particular embodiments, the devices and methods (methods are not in accordance with the claimed invention) herein are capable of generating an output composition of selected and stimulated cells suitable for downstream processing within a set amount of time, such as within 24 hours.

The provided devices and methods (methods are not in accordance with the claimed invention) are capable of selecting cells, e.g., CD3+, CD4+, and CD8+ T cells, from other components, such as from other cells in a sample, and immobilizing the cells on a stationary phase of a chromatography column; stimulating the selected cells immobilized on the stationary phase; and collecting selected and stimulated cells in the absence of processing steps to detach the cells from the stationary phase and remove agents used to facilitate said detachment from the output composition of selected and stimulated cells. In particular aspects, the provided devices and methods (methods are not in accordance with the claimed invention) are capable of generating populations of selected and stimulated cells in a shortened amount of time compared to methods that include separate selecting and stimulating steps and require additional steps to detach cells from the stationary phase and remove agents used to facilitate detachment. In certain aspects, the provided devices and methods (methods are not in accordance with the claimed invention) are capable of generating a selected and stimulated cell output population (also referred to as a composition) suitable for downstream processing (e.g., genetic engineering, expansion, and/or subsequent rounds of incubation, stimulation, and/or selection), within 24 hours of initiating stimulation on the column, also referred to herein as on-column stimulation.

If a definition set forth herein is contrary to or otherwise inconsistent with a definition set forth in the patents, applications, published applications and other publications, the definition set forth herein prevails over the definition in the patents, applications, published applications and other publications.

The section headings used herein are for organizational purposes only and are not to be construed as limiting the subject matter described.

### I. DEVICES AND KITS FOR CELL SELECTION, STIMULATION, AND/OR ENGINEERING

In particular aspects, the provided devices and methods herein are capable of selecting and stimulating target cells (e.g., CD3+, CD4+, or CD8+ T cells) on a stationary phase of a chromatography column, where stimulation facilitates downregulation of the molecule used for cell selection (i.e., selection marker), resulting in spontaneous detachment of the cell from the stationary phase. In some embodiments, the stationary phase of the chromatography column is functionalized with an agent (e.g., selection agent) capable of specifically binding to a molecule (e.g., selection marker) on a target cell surface. In this way, when combining a sample comprising target cells containing the selection marker (e.g., CD3, CD4, CD8) with the stationary phase, target cells (e.g., CD3+, CD4+, CD8+ T cells) are indirectly immobilized to the stationary phase. Exemplary selection agents and selection reagents are described in Section II-B-1 and II-B-2. In particular aspects, the target cells (e.g., T cells) are stimulated while immobilized on the stationary phase (e.g., on-column stimulation), for example, by addition of stimulatory agents, stimulatory reagents comprising stimulatory agents, and/or via stimulatory agents coupled directly or indirectly to the stationary phase. Exemplary stimulatory agents and stimulatory reagents comprising stimulatory agents (e.g., oligomeric stimulatory reagents) are described in Sections II-B-1 and II-B-2. Thus, in some aspects, the provided methods and other embodiments are advantageous in that they condense multiple processing steps (e.g., selection and stimulation) and allow the condensed process to occur within the same container and/or closed system, which can provide increased efficiency and sterility.

In certain aspects, the provided devices and methods herein involve the use of oligomeric stimulatory reagents comprising stimulatory agents capable of delivering a stimulatory signal to a target cell (e.g., T cell). Existing reagents for use in stimulating T cells in vitro, such as in the absence of exogenous growth factors or low amounts of exogenous growth factors, are known (see e.g. US Patent 6,352,694 B1 and European Patent EP 0 700 430 B1). In general, such reagents may employ beads, e.g., magnetic beads, of greater than 1 µm in diameter to which various binding agents (e.g. anti-CD3 antibody and/or anti-CD28 antibody) are immobilized. However, in some cases, such magnetic beads are, for example, difficult to integrate into methods for stimulating cells under conditions required for clinical trials or therapeutic purposes since it has to be made sure that these magnetic beads are completely removed before administering the expanded T cells to a subject. In some aspects, such removal, such as by exposing the cells to a magnetic field, may decrease the yield of viable cells available for the cell therapy. In certain cases, such reagents, e.g., stimulatory reagents containing magnetic beads, must be incubated with the cells for a minimal amount of time to allow a sufficient amount of detachment of the T cells from the stimulatory reagent. Furthermore, reagents such as beads are not readily compatible with column chromatography due to physical constraints.

The provided devices and methods herein utilizing oligomeric stimulatory reagents overcome such potential limitations. For example, in some embodiments, the provided methods include addition of a soluble oligomeric reagent not bound to a solid support (e.g., bead) to the stationary phase to initiate stimulation. In some embodiments, the risk of residual reagent output cells generated or produced by the methods is reduced or avoided by use of the oligomeric reagent since addition of a competition reagent or free binding agent can be used to dissociate (e.g., disrupt binding) the oligomeric stimulatory reagents comprising the stimulatory agents from the cells. In some embodiments, this also means that a process that is compliant with GMP standards can be more easily established compared to other methods, such as those where additional measures have to be taken to ensure that the final population for administration is free of beads. Thus, in some aspects, removal or separation of oligomeric stimulatory reagent from cells, such as by the addition of a competition agent or free binding agent, results in little or no cell loss as compared to removal or separation of bead based stimulatory reagents. In some aspects, the timing of the stimulatory reagent or oligomeric stimulatory reagent removal or separation is not limited or is less limited than the removal or separation of bead based stimulatory reagents. Thus, in some aspects, the stimulatory reagent or oligomeric stimulatory reagent may be removed or separated from the cells at any time or step during the provided methods.

In some aspects, the provided devices are improved devices for methods involving the isolation, processing, or manipulation of target cells immobilized on a stationary phase, e.g., methods of on-column selection and/or stimulation of target cells. In some aspects, the provided devices allow for the regulation of the temperature, e.g., heating, of cells immobilized on a stationary phase. In some aspects, the provided devices allow for the maintenance of the temperature of the immobilized cells, e.g., at or about 37°C or 37°C about 5°C. In some aspects, the regulation and maintenance of the temperature of cells by the provided devices improves on-column stimulation of immobilized cells, e.g., by improving the overall health, fitness, or condition of the immobilized cells during on-column stimulation. In some aspects, the provided devices also permit gas exchange, e.g., the presence of air in the stationary phase. In some aspects, gas exchange as permitted by the provided devices improves the overall health, fitness or condition of the immobilized cells during on-column stimulation. Thus, in some aspects, the provided methods of on-column selection and/or stimulation for use in conjunction with the provided devices provide for improved, e.g., healthier, cells for subsequent engineering for use in a therapy, e.g. an autologous cell therapy.

In some embodiments, the devices provided herein can be used to perform any of the methods described in Section II.

In particular aspects, the durations of the provided methods can be measured from when cells, e.g., T cells of an input cell population or sample, are first contacted or exposed to stimulating conditions (e.g., as described herein such as in Section II-D), referred to herein alternatively as the initiation of incubation with a stimulatory agent or under stimulating conditions, e.g., as in when the exposing to the stimulatory reagent is initiated. In some embodiments, the duration of time required to collect an output population (also referred to herein as an output composition) containing stimulated target cells (e.g., CD3+, CD4+, CD8+ T cells) is measured from initiation of incubation (e.g., adding a stimulatory reagent or exposing to a stimulatory reagent). In particular embodiments, the duration of the incubation is, is about, or is less than 24 hours, 23 hours, 22 hours, 21 hours, 20 hours, 19 hours, 18 hours, 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, or 2 hours. In some embodiments, the duration of the provided incubation is, is about, or is less than 75%, 60%, 50%, 40%, 30%, 25%, 15%, or 10% of alternative or existing processes.

It is contemplated herein that the output compositions of selected and stimulated cells may be further processed. For example, the output cells may be genetically engineered to express a recombinant protein, such as a chimeric antigen receptor, and/or the output cells may undergo further incubation, stimulation, expansion, selection (e.g., polishing), and/or formulation.

In certain embodiments, methods of using the provided devices are performed on samples, such as, for example, apheresis, buffy coat, or whole blood. In some embodiments, the samples are biological samples. In some embodiments, the biological samples are collected from human subjects. In some embodiments, the biological samples are collect from patients suffering from a disease or condition. In some embodiments, the methods are performed on populations of cells, e.g., CD4+ and CD8+ T cells, that were previously isolated, enriched, or selected from a sample. In some embodiments, the sample or cells isolated from the sample may have been cryopreserved.

In some embodiments, provided herein are devices, kits, systems, and/or articles of manufacture for cell selection, stimulation, and/or engineering. In some embodiments, provided is an arrangement of a stationary phase for chromatography. In some embodiments, the arrangement further comprises a bioreactor. The bioreactor is suitable for the expansion of cells, and the stationary phase is suitable for cell separation and on-column stimulation. In embodiments, the stationary phase is a gel filtration matrix and/or affinity chromatography matrix, wherein the gel filtration and/or affinity chromatography matrix comprises an selection reagent, wherein the selection reagent comprises a binding site Z1 specifically binding to a binding partner C1 comprised in a selection agent and/or the selection reagent comprises a binding site Z2 specifically binding to a binding partner C2 comprised in a second selection agent. The stationary phase is thereby suitable for immobilizing thereon the first selection agent and/or the second selection agent, the first binding partner C1 and/or the second binding partner C2. In addition the bioreactor and the stationary phase are fluidly connected. This arrangement can be used in a serial expansion and can be integrated into known cell expansion systems such as the Quantum^{®} cell expansion system) or the Xuri Cell Expansion System W25.

In some embodiments, the stationary phase is comprised in a chromatography column. The arrangement may further comprise a second stationary phase which is fluidly connected to the first stationary phase. The secondary stationary phase may be a gel filtration matrix and/or affinity chromatography matrix, wherein the gel filtration and/or affinity chromatography matrix comprises a selection reagent, thereby being suitable of immobilizing the multimerization reagent on the stationary phase. This type of arrangement may facilitate sequential selection of target cells (e.g., T cells, CD4, CD3, CD8 T cells) wherein one of the columns is also suitable for on-column stimulation as described herein.

Provided embodiments in some aspects are directed to a device for purification (e.g. selection) and culture, such as stimulation or expansion, of a composition of cells, in which the device comprises at least one arrangement of a bioreactor and a first stationary phase or a second stationary phase for chromatography as defined above.

The device may further comprise a plurality of arrangements of a bioreactor and a stationary phase being fluidly connected in series.

The device may comprise a sample inlet being fluidly connected stationary phase for chromatography. The device may also comprise a sample outlet for purified and stimulated target cells, the sample outlet being fluidly connected to the stationary phase of the last of the at least one arrangement of a bioreactor and the stationary phase for chromatography.

In some embodiments, the device may be designed as a functionally closed system.

### A. Chromatography Housing Assembly

In some embodiments, provided herein is a chromatography housing assembly (also referred to herein as a housing assembly for column chromatography or a housing assembly) suitable for the chromatography-based cell selection and/or stimulation methods disclosed herein. The chromatography housing assembly may be provided with or without a stationary phase for chromatography.

In one aspect, provided herein is a housing assembly for column chromatography, comprising: an inlet housing member and an outlet housing member, wherein at least the inlet housing member and the outlet housing member form an internal cavity configured to house a stationary phase for column chromatography; a temperature control member configured to provide heat to the stationary phase in the internal cavity; and a connector configured to operably connect the internal cavity to a gas source, thereby permitting or effecting intake of gas into the internal cavity. In one aspect, the housing assembly for column chromatography further comprises a side wall member, wherein the inlet housing member, the outlet housing member, and the side wall member form the internal cavity. The connector can be disposed on the inlet housing member, the outlet housing member, and/or the side wall member. The connector can be a bonded connector, a screw connector, a luer connector (e.g., a luer lock connector or a luer slip connector), a barbed connector, or any combination thereof. In any of the preceding embodiments, the connector can be configured to sealingly engage tubing in fluid communication with the gas source. In any of the preceding embodiments, the connector can comprise one or more filter, and/or the connector can be operably connected to tubing comprising one or more filter. The one or more filter is a gas filter, *e.g.,* an air filter. The one or more filter can be a sterile filter and/or a sterilizing filter for sterilization by filtration. In one aspect, gas is present in the internal cavity during at least a portion of stimulation of target cells immobilized on the stationary phase of the chromatography column. In some aspects, the gas comprises air. In one aspect, stimulation of cells (e.g., lymphocytes such as T cells) in the presence of gas (e.g., air) facilitates cell activation and downstream processing of the cells, such as detachment or elution of cells from the stationary phase and/or genetic engineering of the cells.

In some embodiments, the internal cavity of the housing assembly can accommodate a bed volume between or between about 1 and 40 mL, such as between or between about 1 and 35 mL, 1 and 30 mL, 1 and 25 mL, 1 and 20 mL, 1 and 15 mL, 1 and 10 mL, 1 and 5 mL, 5 and 40 mL, 5 and 35 mL, 5 and 30 mL, 5 and 25 mL, 5 and 20 mL, 5 and 15 mL, 5 and 10 mL, 10 and 40 mL, 10 and 35 mL, 10 and 30 mL, 10 and 25 mL, 10 and 20 mL, 10 and 15 mL, 15 and 40 mL, 15 and 35 mL, 15 and 30 mL, 15 and 25 mL, 15 and 20 mL, 20 and 40 mL, 20 and 35 mL, 20 and 30 mL, 20 and 25 mL, 25 and 40 mL, 25 and 35 mL, 25 and 30 mL, 30 and 40 mL, 30 and 35 mL, or 35 and 40 mL. In some embodiments, the internal cavity of the housing assembly can accommodate a bed volume between or between about 15 and 25 mL. In some embodiments, the internal cavity of the housing assembly can accommodate a bed volume between or between about 15 and 20 mL. In some embodiments, the internal cavity of the housing assembly can accommodate a bed volume between or between about 18 and 20 mL.

In some embodiments, the housing assembly for column chromatography includes one or more connectors, e.g., two or more connectors. In some embodiments, the housing assembly for column chromatography includes two connectors. In some embodiments, the two or more connectors are disposed on at least the inlet housing member. In some embodiments, the two or more connectors are disposed on at least the outlet housing member. In some embodiments, a connector is disposed at least on each of the inlet housing member and the outlet housing member. In some embodiments, both the inlet housing member and the outlet housing member have a connector disposed thereon.

In any of the preceding embodiments, the temperature control member can be configured to regulate or maintain a temperature of the stationary phase in the internal cavity. In any of the preceding embodiments, the temperature control member can be configured to heat the stationary phase in the internal cavity from a starting temperature (*e.g*., room temperature) to a target temperature between about 35°C and about 39°C (*e.g.,* at or at about 37°C). In some embodiments, the temperature control member can be configured to heat the stationary phase to a target temperature between about 30°C and about 39°C. In some embodiments, the starting temperature is about 2°C, about 4°C, about 8°C, about 12°C, about 16°C, about 20°C, about 24°C, about 28°C, about 32°C, about 36°C, or higher than about 36°C. In some embodiments, the temperature for stimulation is or is about 37°C. In some embodiments, the target temperature (e.g., an optimal temperature for cell stimulation) is higher than about 2°C, at or higher than about 4°C, at or higher than about 8°C, at or higher than about 12°C, at or higher than about 16°C, at or higher than about 20°C, at or higher than about 24°C, at or higher than about 28°C, at or higher than about 32°C, at or higher than about 36°C, at or higher than about 37°C, at or higher than about 38°C, at or higher than about 39°C, or at or higher than about 40°C. In some embodiments, the temperature of target cells immobilized on the stationary phase is kept at a constant temperature value (e.g., an optimal temperature) during at least portion of the stimulation. In some embodiments, the temperature of target cells immobilized on the stationary phase is kept at a selected temperature value (e.g., an optimal temperature) about 5°C, about 4°C, about 3°C, about 2°C, ±about 1°C or about 0.5°C during at least portion of the stimulation. In some embodiments, the temperature of target cells immobilized on the stationary phase is kept at 37°C about 5°C, ±about 4°C, ±about 3°C, about 2°C, about 1°C or about 0.5°C during at least portion of the stimulation. In some aspects, stimulation of cells (e.g., lymphocytes such as T cells) at an optimal temperature (e.g., 37°C or 37°C about 5°C) facilitates cell activation and downstream processing of the cells, such as detachment or elution of cells from the stationary phase and/or genetic engineering of the cells. In some aspects, stimulation of (e.g., lymphocytes such as T cells) at an optimal temperature (e.g., 37°C or 37°C ±about 5°C) preserves or maintains the health of the cells during on-column stimulation.

In some aspects, stimulation of cells (e.g., lymphocytes such as T cells) at an optimal temperature (e.g., 37°C or 37°C ±about 5°C) and in the presence of air (e.g., air) in the column facilitates cell activation and downstream processing of the cells, such as detachment or elution of cells from the stationary phase and/or genetic engineering of the cells.

**FIGS. 1A-1B** provide an exemplary housing assembly for column chromatography. In some aspects, housing assembly **1** comprises inlet housing member **2** and outlet housing member **3,** and at least the inlet housing member and the outlet housing member form an internal cavity configured to house a stationary phase, such as resin **4** for column chromatography. In some aspects, the housing assembly further comprises a temperature control member, *e.g*., a temperature control member comprising heating coil **5,** configured to provide heat to the stationary phase in the internal cavity. In some aspects, the housing assembly further comprises a connector, *e.g*., gas exchange connector **6,** configured to operably connect the internal cavity to a gas source, thereby permitting or effecting intake of gas into the internal cavity. In some embodiments, the connector is disposed on the inlet housing member. In some embodiments, the connector is disposed on the outlet housing member. In some embodiments, both the inlet housing member and the outlet housing member have a connector disposed thereon, for example, as shown in **FIG. 1A****,** both inlet housing member **2** and outlet housing member **3** have gas exchange connectors **6** disposed thereon.

In some embodiments, the housing assembly further comprises a side wall member. For example, as shown in **FIG. 1A****,** inlet housing member **2,** outlet housing member **3,** and side wall member **7** together form the internal cavity.

In some embodiments, the connector is disposed on the inlet housing member. In some embodiments, the connector is disposed on the outlet housing member. In some embodiments, the connector is disposed on the side wall member. In some embodiments, both the inlet housing member and the side wall member have a connector disposed thereon. In some embodiments, both the outlet housing member and the side wall member have a connector disposed thereon. In some embodiments, each of the inlet housing member, the outlet housing member, and the side wall member has a connector disposed thereon. In some embodiments, the inlet housing member has at least two connectors disposed thereon. In some embodiments, the outlet housing member has at least two connectors disposed thereon. In some embodiments, the side wall member has at least two connectors disposed thereon.

In some embodiments, the connector is formed between any two or among all three of the inlet housing member, the outlet housing member, and the side wall member. In some aspects, the connector is formed between the inlet housing member and the outlet housing member. In some aspects, the connector is formed between the inlet housing member and the side wall member. In some aspects, the connector is formed between the outlet housing member and the side wall member. In some aspects, at least one connector is formed between the inlet housing member and the side wall member, and at least one connector is formed between the outlet housing member and the side wall member.

In any of the preceding embodiments, the housing assembly can comprise a plurality of the connectors, *e.g*., gas exchange connector **6,** configured to operably connect the internal cavity to a gas source, thereby permitting or effecting intake of gas into the internal cavity. In some embodiments, at least one of the connectors is operably connected to the gas source (directly or indirectly through tubing optionally including one or more filter and/or one or more valve), while at least one other connector is configured to vent.

In any of the preceding embodiments, the connector can be a bonded connector, a screw connector, a luer connector (*e.g*., a luer lock connector or a luer slip connector), a barbed connector, or any combination thereof. In any of the preceding embodiments, the connector can comprises a male fitting or a female fitting. In any of the preceding embodiments, the connector can be configured to sealingly engage tubing in fluid communication with the gas source. In any of the preceding embodiments, the connector can comprise one or more valve. In any of the preceding embodiments, the connector can be operably connected to tubing comprising one or more valve. In any of the preceding embodiments, the connector can comprises one or more filter. In any of the preceding embodiments, the connector can be operably connected to tubing comprising one or more filter. In any of the preceding embodiments, the one or more filter can be a gas filter, *e.g.,* an air filter. In any of the preceding embodiments, the one or more filter can be a sterile filter and/or a sterilizing filter for sterilization by filtration.

In some aspects, the housing assembly comprising an inlet housing member that comprises an upper lid. In some embodiments, the upper lid is removably attached to the inlet housing member or the side wall member. In some embodiments, the upper lid is integrally formed with the inlet housing member or the side wall member. In some embodiments, the connector is disposed on the upper lid.

In any of the preceding embodiments, the inlet housing member can comprise one or more inlet operably connected to the internal cavity to permit intake of an input composition into the internal cavity. For example as shown in **FIG. 1A****,** inlet housing member **2** comprises an inlet, *e.g*., tubing set connector **8,** disposed on the upper lid. In some embodiments, the connector and the one or more inlet are disposed on the upper lid at different locations, *e.g*., as shown in **FIG. 1A** (lower panel) and **FIG. 1B****.** In some embodiments, the connector and the one or more inlet are disposed on the upper lid at the same location. For example, the one or more inlet may be configured to operably connect the internal cavity to a gas source, thereby permitting or effecting intake of gas into the internal cavity, while also configured to operably connect to the internal cavity to permit intake of an input composition into the internal cavity. The one or more inlet may be controllably open or close at certain time points during chromatography for intake of gas, and controllably open or close at other time points during chromatography for intake of the input composition.

In some embodiments, fluid path through the one or more inlet is at an angle of about 90 degrees to the upper lid, while fluid path through the connector is at an angle of about 45 degrees to the upper lid.

In any of the preceding embodiments, the outlet housing member can comprise a lower lid of the housing assembly. In some aspects, the lower lid is removably attached to the outlet housing member or the side wall member. In other aspects, the lower lid is integrally formed with the outlet housing member or the side wall member.

In any of the preceding embodiments, the outlet housing member can comprise one or more outlet operably connected to the internal cavity to permit or effect discharge of an output composition from the internal cavity. In some aspects, the one or more outlet is disposed on the lower lid. In some embodiments, the connector and the one or more outlet are disposed on the lower lid at different locations, *e.g*., as shown in **FIG. 1A** (lower panel). In some embodiments, the connector and the one or more outlet are disposed on the lower lid at the same location. For example, the one or more outlet may be configured to operably connect the internal cavity to a gas source, thereby permitting or effecting intake of gas into the internal cavity, while also configured to operably connect to the internal cavity to permit or effect discharge of an output composition from the internal cavity. The one or more outlet may be controllably open or close at certain time points during chromatography for intake of gas, and controllably open or close at other time points during chromatography for discharge of the output composition from the internal cavity. In some embodiments, fluid path through the one or more outlet is at an angle of about 90 degrees to the lower lid.

In any of the preceding embodiments, the gas source can be or comprise a gas reservoir or an outside environment. In any of the preceding embodiments, gas in the gas source can be sterile. In any of the preceding embodiments, the gas can be or comprise air.

In any of the preceding embodiments, the housing assembly can further comprise tubing operably connected to the gas source. In some embodiments, the tubing is configured to sterilely connect the internal cavity to the gas source. In any of the preceding embodiments, the tubing can comprise one or more valve. In any of the preceding embodiments, the tubing can comprise one or more filter.

In any of the preceding embodiments, the housing assembly can further comprise one or more porous member, *e.g*., a cell strainer or a cell sieve. For example, as shown in **FIG. 1A** (upper panel), housing assembly **1** comprises woven polyester mesh **9.** In some embodiments, the housing assembly comprises a first porous member, *e.g*., woven polyester mesh **9** between inlet housing member 2 and side wall member 7, configured to separate the stationary phase and an inlet of the internal cavity. In some embodiments, the housing assembly further comprises a second porous member, *e.g*., woven polyester mesh **9** between outlet housing member **3** and side wall member **7,** configured to separate the stationary phase and an outlet of the internal cavity.

In any of the preceding embodiments, the one or more porous member can have an average pore diameter of about 20 µm. In any of the preceding embodiments, the one or more porous member can comprises a mesh having a mesh size of about 20 µm.

In any of the preceding embodiments, the temperature control member can be configured to regulate or maintain a temperature of the stationary phase in the internal cavity. In some aspects, the temperature control member is configured to heat the stationary phase in the internal cavity from a starting temperature (e.g., room temperature) to a target temperature between about 35°C and about 39°C *(e.g.,* at or at about 37°C) during a chromatography run. In some aspects, the temperature control member is further configured to maintain the stationary phase at the target temperature.

In any of the preceding embodiments, the housing assembly can further comprise a temperature sensor configured to measure the temperature of the stationary phase in the internal cavity. In one aspect, the temperature sensor is configured to couple to a monitoring/display unit. In some embodiments, the temperature sensor is configured to electrically connect to a power source. In some embodiments, the power source is external to the housing assembly. In some embodiments, the housing assembly further includes the power source.

In any of the preceding embodiments, the temperature control member can comprise a heating source. Alternatively, in any of the preceding embodiments, the temperature control member can be configured to operably connect to a heating source which is external to the housing assembly.

In some embodiments, the temperature control member includes a heating element. In some embodiments, the heating element is configured to uniformly heat the stationary phase.

In any of the preceding embodiments, the temperature control member can comprise a heating element selected from the group consisting of an electric heating element, an electromagnetic induction heating element, a non-electric heating element, and any combination thereof. In one aspect, the heating element is an electric heating element. In some embodiments, the electric heating element comprises a metal plate, a metal rod, a metal wire, or a combination thereof. In one aspect, the heating element is an electromagnetic induction heating element. In some embodiments, the electromagnetic induction heating element comprises an induction heating coil surrounding a magnetizable core configured to provide heat to the stationary phase in the internal cavity. In one aspect, the heating element is a non-electric heating element.

In some embodiments, the non-electric heating element comprises a heating channel comprising an inlet and an outlet for a heated fluid, *e.g.,* a heated liquid or gas. In some embodiments, the heating channel is a heating coil and the heated fluid is heated water. For example, as shown in **FIG. 1A****,** the housing assembly comprises heating coil inlet **10** and heating coil outlet **11.** In some embodiments, the inlet for heated water is configured to connect to an external reservoir of heated water.

In some embodiments, the heating element is an electric heating element. In some embodiments, the electric heating element is configured to electrically connect to a power source. In some embodiments, the power source is external to the housing assembly. In some embodiments, the housing assembly further includes the power source.

In some embodiments, the electric heating element includes a metal plate. In some embodiments, the metal plate is made at least in part of a heat-conductive metal, e.g, aluminum or copper. In some embodiments, the metal plate is made at least in part of aluminum, e.g., made entirely of aluminum. In some embodiments, the electric heating element further includes an electrical isolation layer. In some embodiments, the electrical isolation layer is between at least a portion of the metal plate and at least a portion of other components of the electric heating element. In some embodiments, the electrical isolation layer lines at least a portion of one face of the metal plate, e.g., entirely lines one face of the metal plate.

In some embodiments, at least a portion of the heating element is in contact, e.g., direct contact, with at least a portion of the inlet housing member, at least a portion of the outlet housing member, and/or at least a portion of the side wall member. In some embodiments, at least a portion of the heating element is in contact, e.g., direct contact, with at least a portion of the side wall member. In some embodiments, at least a portion of the heating element is in contact, e.g., direct contact, with at least a portion of the inlet housing member. In some embodiments, at least a portion of the heating element is in contact, e.g., direct contact, with at least a portion of the outlet housing member.

In some embodiments, the heating element is in contact, e.g., direct contact, with at least a portion of the inlet housing member, at least a portion of the outlet housing member, and/or the side wall member. In some embodiments, the heating element is in contact, e.g., direct contact, with at least a portion of the side wall member. In some embodiments, the heating element is in contact, e.g., direct contact, with the side wall member.

In some embodiments, at least a portion of the heating element is not in contact with at least a portion of the inlet housing member, at least a portion of the outlet housing member, and/or at least a portion of the side wall member. In some embodiments, the heating element is not in contact with at least a portion of the inlet housing member, at least a portion of the outlet housing member, and/or at least a portion of the side wall member. In some embodiments, the heating element is not in contact with the inlet housing member, the outlet housing member, or the side wall member.

In any of the preceding embodiments, the heating element can be disposed along and/or around a central axis of the internal cavity. In some aspects, the heating element is disposed inside the internal cavity, outside the internal cavity, or partially inside and partially outside the internal cavity. In some aspects, the heating element is disposed inside the side wall member, outside the side wall member, or partially inside and partially outside the side wall member.

In some embodiments, the heating element is disposed inside the internal cavity. In some embodiments, the heating element includes a non-electric heating element disposed inside the internal cavity. In some embodiments, the heating element includes a heating channel disposed inside the internal cavity. In some embodiments, the heating channel includes an inlet and an outlet for a heated fluid, e.g., heated water. In some embodiments, the inlet for the heated water is configured to connect to an external reservoir of heated water.

In some embodiments, the heating element is disposed outside the internal cavity. In some embodiments, the heating element is disposed outside the side wall member. In some embodiments, the heating element surrounds at least a portion of the inlet housing member, at least a portion of the outlet housing member, and/or at least a portion of the side wall member. In some embodiments, the heating element surrounds, e.g., entirely surrounds, the side wall member. In some embodiments, the heating element surrounds at least a portion of the inlet housing member. In some embodiments, at least a portion of one of the one or more inlet of the inlet housing member, e.g., one or more inlet operably connected to the internal cavity to permit intake of an input composition into the internal cavity, is exposed by the heating element. In some embodiments, at least a portion of one or more inlet of the inlet housing member operably connected to the internal cavity to permit intake of an input composition into the internal cavity is exposed by the heating element. In some embodiments, at least a portion of one of the one or more inlet of the inlet housing member is outside the heating element. In some embodiments, the heating element surrounds at least a portion of the outlet housing member. In some embodiments, at least a portion of one of the one or more outlet of the outlet housing member, e.g. one or more outlet operably connected to the internal cavity to permit or effect discharge of an output composition from the internal cavity, is exposed by the heating element.. In some embodiments, at least a portion of one or more outlet operably connected to the internal cavity to permit or effect discharge of an output composition from the internal cavity is exposed by the heating element. In some embodiments, at least a portion of one of the one or more outlet of the outlet housing member is outside the heating element.

In some embodiments, the heating element includes a heating channel surrounding at least a portion of the inlet housing member, the outlet housing member, and/or the side wall member.

In any of the preceding embodiments, the heating element can comprise a coil surrounding the inlet housing member, the outlet housing member, and/or the side wall member. In any of the preceding embodiments, the heating element can comprise a heating channel surrounding the inlet housing member, the outlet housing member, and/or the side wall member. In any of the preceding embodiments, the heating element can comprise a heating channel surrounding the side wall member.

In some embodiments, the heating element surrounds at least a portion of the side wall member, at least a portion of the outlet housing member, and/or at least a portion of the inlet housing member, and the housing assembly further comprises an insulation layer between the heating element and at least a portion of the inlet housing member, at least a portion of the outlet housing member, and/or at least a portion of the side wall member. In some embodiments, the insulation layer surrounds at least a portion of the inlet housing member, at least a portion of the outlet housing member, and/or at least a portion of the side wall member. In some embodiments, the insulation layer surrounds at least a portion of the side wall member, e.g., entirely surrounds the side wall member. In some embodiments, the insulation layer is a solid layer. In some embodiments, the insulation layer is a liquid layer. In some embodiments, the insulation layer is a gas layer. In some embodiments, the insulation layer is an air layer.

In some embodiments, the temperature control member includes a plurality of heating elements. In some embodiments, the temperature control member includes between or between about 2 and 10 heating elements, between or between about 2 and 8 heating elements, between or between about 2 and 6 heating elements, or between or between about 2 and 4 heating elements, each inclusive. In some embodiments, the temperature control member includes two heating elements. In some embodiments, the temperature control member includes three heating elements. In some embodiments, the temperature control member includes four heating elements.

In some embodiments, the plurality of heating elements are configured to uniformly heat the stationary phase. In some embodiments, the plurality of heating elements are arranged to uniformly heat the stationary phase.

In some embodiments, the plurality of heating elements are each selected from the group consisting of an electric heating element, an electromagnetic induction heating element, a non-electric heating element, and any combination thereof. In some embodiments, the plurality of heating elements are identical. In some embodiments, the plurality of heating elements are a combination of different heating elements.

In some embodiments, the plurality of heating elements include a plurality of non-electric heating elements. In some embodiments, the plurality of heating elements include a plurality of heating channels. In some embodiments, each of the plurality of heating channels has an inlet and outlet for a heated fluid, e.g., heated water. In some embodiments, at least two of the plurality of heating channels are fluidly coupled to one another. In some embodiments, the plurality of heating channels are fluidly coupled to one another. In some embodiments, the inlet of at least one of the plurality of heating channels is configured to connect to an external reservoir of heated fluid, e.g., heated water. In some embodiments, the inlet of each of the plurality of heating channels is configured to connect to an external reservoir of heated fluid.

In some embodiments, the plurality of heating elements include a plurality of electric heating elements, e.g., electric heating elements comprising metal plates. In some embodiments, at least two of the plurality of electric heating elements are electrically coupled to one another. In some embodiments, the plurality of electric heating elements are electrically coupled to one another. In some embodiments, at least one of the plurality of electric heating elements is configured to electrically connect to a power source, e.g., a power source external to or included in the housing assembly. In some embodiments, each of the plurality of electric heating elements is configured to electrically connect to a power source, e.g., a power source external to or included in the housing assembly.

In some embodiments, at least a portion of at least one of the plurality of heating elements is in contact, e.g., direct contact, with at least a portion of the inlet housing member, at least a portion of the outlet housing member, and/or at least a portion of the side wall member. In some embodiments, at least a portion of at least one of the plurality of heating elements is in contact, e.g., direct contact, with at least a portion of the inlet housing member. In some embodiments, at least a portion of at least one of the plurality of heating elements is in contact, e.g., direct contact, with at least a portion of the outlet housing member. In some embodiments, at least a portion of at least one of the plurality of heating elements is in contact, e.g., direct contact, with at least a portion of the side wall member.

In some embodiments, at least a portion of at least one of the plurality of heating elements is in contact, e.g., direct contact, with at least a portion of the inlet housing member, at least a portion of the outlet housing member, and/or at least a portion of the side wall member. In some embodiments, at least one of the plurality of heating elements is in contact, e.g., direct contact, with at least a portion of the inlet housing member, at least a portion of the outlet housing member, and/or at least a portion the side wall member. In some embodiments, at least one of the plurality of heating elements is in contact, e.g., direct contact, with at least a portion of the side wall member. In some embodiments, at least one of the plurality of heating elements is in contact, e.g., direct contact, with the side wall member.

In some embodiments, at least a portion of at least one of the plurality of heating elements is not in contact with at least a portion of the inlet housing member, at least a portion of the outlet housing member, or at least a portion the side wall member. In some embodiments, at least a portion of at least one of the plurality of heating elements is not in contact with the inlet housing member, the outlet housing member, or the side wall member. In some embodiments, at least one of the plurality of heating elements is not in contact with the inlet housing member, the outlet housing member, or the side wall member. In some embodiments, the plurality of heating elements is not in contact with the inlet housing member, the outlet housing member, or the side wall member.

In some embodiments, at least one of the plurality of heating elements is disposed along and/or around a central axis of the internal cavity. In some embodiments, at least one of the plurality of heating elements is disposed inside the internal cavity, outside the internal cavity, or partially inside and partially outside the internal cavity. In some embodiments, at least one of the plurality of heating elements is disposed inside the side wall member, outside the side wall member, or partially inside and partially outside the side wall member. In some embodiments, at least one of the plurality of heating elements is disposed inside the internal cavity, and at least one of the plurality of heating elements is disposed outside the internal cavity. In some embodiments, the plurality of heating elements are disposed inside the internal cavity. In some embodiments, the plurality of heating elements are disposed outside the internal cavity. In some embodiments, the plurality of heating elements are disposed outside the side wall member.

In some embodiments, at least one of the plurality of heating elements is disposed outside the internal cavity. In some embodiments, at least one of the plurality of heating elements surrounds at least a portion of the inlet housing member, at least a portion of the outlet housing member, and/or at least a portion of the side wall member. In some embodiments, at least one of the plurality of heating elements surrounds at least a portion of the side wall member, e.g., entirely surrounds the side wall member. In some embodiments, the plurality of heating elements surrounds at least a portion of the side wall member, e.g., entirely surrounds the side wall member. In some embodiments, at least one of the plurality of heating elements surrounds at least a portion of the inlet housing member. In some embodiments, the plurality of heating elements surrounds at least a portion of the inlet housing member. In some embodiments, at least a portion of one or more inlet of the inlet housing member is exposed by the plurality of heating elements. In some embodiments, at least a portion of one or more inlet of the inlet housing member is outside the plurality of heating elements. In some embodiments, at least one of the plurality of heating elements surrounds at least a portion of the outlet housing member. In some embodiments, the plurality of heating elements surrounds at least a portion of the outlet housing member. In some embodiments, at least a portion of one or more outlet of the outlet housing member is exposed by the plurality of heating elements. In some embodiments, at least a portion of one or more outlet of the outlet housing member is outside the plurality of heating elements.

In some embodiments, the plurality of heating elements are uniformly or about uniformly distributed around the side wall member. In some embodiments, the plurality of heating elements are uniformly or about uniformly distributed around the circumference of the side wall member.

In some embodiments, at least one of the plurality of heating elements surrounds at least a portion of the side wall member, at least a portion of the outlet housing member, and/or at least a portion of the inlet housing member, and the housing assembly further comprises an insulation layer between at least one of the plurality of heating elements and at least a portion of the inlet housing member, at least a portion of the outlet housing member, and/or at least a portion of the side wall member. In some embodiments, the insulation layer surrounds at least a portion of the inlet housing member, at least a portion of the outlet housing member, and/or at least a portion of the side wall member. In some embodiments, the insulation layer surrounds at least a portion of the side wall member, e.g., entirely surrounds the side wall member. In some embodiments, the insulation layer is a liquid layer. In some embodiments, the insulation layer is a gas layer. In some embodiments, the insulation layer is an air layer.

In some embodiments, the heating element is disposed outside the side wall member, and the housing assembly further includes a jacket member (also referred to herein as a jacket) that includes the heating element. In some embodiments, the jacket member includes the temperature control member that includes the heating element disposed outside the side wall member. In some embodiments, the jacket member is any as described in Section I-C.

In some embodiments, at least one of the plurality of heating elements is disposed outside the side wall member, and the housing assembly further includes a jacket member that includes the at least one of the plurality of heating elements. In some embodiments, the jacket member includes the temperature control member that includes the at least one of the plurality of heating elements. In some embodiments, the plurality of heating elements are disposed outside the side wall member, and the housing assembly further includes a jacket member that includes the plurality of heating elements. In some embodiments, the jacket member includes the temperature control member that includes the plurality of heating elements.

In some embodiments, the jacket member is configured to surround at least a portion of the inlet housing member, at least a portion of the outlet housing member, and/or at least a portion of the side wall member. In some embodiments, the jacket member surrounds at least a portion of the inlet housing member, at least a portion of the outlet housing member, and/or at least a portion of the side wall member.

In some embodiments, the jacket member is releasably connected together to surround at least a portion of the inlet housing member, at least a portion of the outlet housing member, and/or at least a portion of the side wall member. In some embodiments, the jacket member is not releasably connected together.

In some embodiments, the jacket member is configured to surround at least a portion of the side wall member. In some embodiments, the jacket member is configured to entirely surround the side wall member. In some embodiments, the jacket member surrounds at least a portion of the side wall member, e.g., entirely surrounds the side wall member. In some embodiments, the jacket member entirely surrounds the side wall member. In some embodiments, the jacket member surrounds at least a portion of the inlet housing member. In some embodiments, one or more inlet of the inlet housing member is exposed by the jacket member. In some embodiments, one or more inlet of the inlet housing member operably connected to the internal cavity to permit intake of an input composition into the internal cavity is exposed by the jacket member. In some embodiments, one or more inlet of the inlet housing member is outside the jacket member. In some embodiments, the jacket member surrounds at least a portion of the outlet housing member. In some embodiments, one or more outlet of the outlet housing member is exposed by the jacket member. In some embodiments, one or more outlet of the outlet housing member operably connected to the internal cavity to permit or effect discharge of an output composition from the internal cavity is exposed by the jacket member. In some embodiments, one or more outlet of the outlet housing member is outside the jacket member.

In some embodiments, the jacket member is in contact, e.g., direct contact, with at least a portion of the inlet housing member, at least a portion of the outlet housing member, and/or at least a portion of the side wall member. In some embodiments, the jacket member is in contact, e.g., direct contact, with at least a portion of the side wall member. In some embodiments, the jacket member is in contact, e.g., direct contact, with the side wall member.

In some embodiments, at least a portion of the jacket member is not in contact with at least a portion of the inlet housing member, at least a portion of the outlet housing member, or at least a portion of the side wall member. In some embodiments, at least a portion of the jacket member is not in contact with the inlet housing member, the outlet housing member, or the side wall member. In some embodiments, the jacket member is not in contact with the inlet housing member, the outlet housing member, or the side wall member.

In some embodiments, the jacket member includes a non-electric heating element, e.g., a heating channel that includes an inlet and an outlet for heated fluid. In some embodiments, the jacket member includes a plurality of non-electric heating elements. In some embodiments, the jacket member includes at least one opening for the inlet for heated fluid. In some embodiments, the jacket member includes at least one opening for the outlet for heated fluid. In some embodiments, the jacket member includes at least two openings for one or more inlets for heated fluid. In some embodiments, the jacket member includes at least two openings for one or more outlets for heated fluid, e.g., heated water. In some embodiments, the jacket member is configured to electrically connect to a power source, e.g., a power source external to or included in the housing assembly.

In some embodiments, the jacket member includes an electric heating element, e.g., an electric heating element that includes a metal plate. In some embodiments, the jacket member includes a plurality of electric heating elements In some embodiments, the jacket member is arranged such that the electric heating element or the plurality of electric heating elements are configured to electrically connect to a power source.

In some embodiments, the jacket member includes at least one temperature sensor configured to measure the temperature of the stationary phase in the internal cavity. In some embodiments, the temperature sensor is configured to electrically connect to a power source, e.g., a power source external to or included in the housing assembly.

In some embodiments, the jacket member includes one or more jacket components. In some embodiments, the one or more jacket components are configured to together form the jacket member. In some embodiments, the one or more jacket components are configured to surround at least a portion of the inlet housing member, at least a portion of the outlet housing member, and/or at least a portion of the side wall member. In some embodiments, the one or more jacket components are configured to be releasably connected together to surround at least a portion of the inlet housing member, at least a portion of the outlet housing member, and/or at least a portion of the side wall member.

In some embodiments, the one or more jacket components are configured to surround at least a portion of the side wall member, e.g., entirely surround the side wall member. In some embodiments, the one or more jacket components are configured to entirely surround the side wall member In some embodiments, the one or more jacket components are configured to surround at least a portion of the inlet housing member. In some embodiments, one or more inlet of the inlet housing member is exposed by the one or more jacket components. In some embodiments, one or more inlet of the inlet housing member is outside the one or more jacket components. In some embodiments, the one or more jacket components are configured to surround at least a portion of the outlet housing member. In some embodiments, one or more outlet of the outlet housing member is exposed by the one or more jacket components. In some embodiments, one or more outlet of the outlet housing member is outside the one or more jacket components.

In some embodiments, the jacket member includes two or more jacket components, for instance between or between about 2 and 10 jacket components, 2 and 8 jacket components, 2 and 6 jacket components, or 2 and 4 jacket components, each inclusive. In some embodiments, the jacket member includes two jacket components. In some embodiments, the jacket member includes three jacket components. In some embodiments, the jacket member includes four jacket components.

In some embodiments, at least two of the two or more jacket components each include a heating element. In some embodiments, the two or more jacket components each include a heating element.

In some embodiments, at least two of the two or more jacket components each include a temperature sensor. In some embodiments, the two or more jacket components each include a temperature sensor.

In some embodiments, at least two of the two or more jacket components each include a non-electric heating element. In some embodiments, at least two of the two or more jacket components each include a heating channel with inlet and outlet for a heated fluid, e.g., heated water. In some embodiments, the two or more jacket components each include a heating channel with inlet and outlet for a heated fluid, e.g., heated water.

In some embodiments, the heating channels of the at least two of the two or more jacket components are fluidly coupled to one another. In some embodiments, the heating channels of the two or more jacket components are fluidly coupled to one another.

In some embodiments, at least one of the two or more jacket components includes an opening for the inlet for a heated fluid, e.g., heated water. In some embodiments, the two or more jacket components each include an opening for the inlet for a fluid, e.g., heated water.

In some embodiments, at least one inlet of the heating channels of the two or more jacket components is configured to connected to an external reservoir of heated fluid. In some embodiments, each inlet of the heating channels of the two or more jacket components is configured to connected to an external reservoir of heated fluid.

In some embodiments, at least one of the two or more jacket components includes an opening for the outlet for a heated fluid, e.g., heated water. In some embodiments, the two or more jacket components each include an opening for the outlet for a fluid, e.g., heated water.

**FIGS. 25-28** provide schematic representations of an exemplary housing assembly for column chromatography. The exemplary housing assembly **1** shown in **FIG. 25** includes inlet housing member **2,** outlet housing member **3,** and side wall member **7** that form an internal cavity configured to house a stationary phase. Housing assembly **1** also includes a gas supply connector for screw-on air filters (not shown) and a temperature control member that includes heating coils **5,** as shown in **FIGS. 26A-26C****.** Heating coils **5** are contained in a jacket member made of two jacket components **12.** The jacket components **12** are configured to together entirely surround side wall member **7** and to surround at least a portion of each of inlet housing member **2** and outlet housing member **3.** Each jacket component **12** includes an inlet groove **13** such that the jacket member exposes an inlet of the inlet housing member **2.** Each jacket component **12** also includes an outlet groove **14** such that the jacket member exposes an outlet of the outlet housing member **3.**

**FIGS. 26A-26C** show interior, side, and exterior views of jacket component **12.** As shown in **FIGS. 26A-26C****,** each jacket component **12** includes a heating coil **5** for a heated fluid, e.g., heated water. The two heating coils **5** of the jacket member are configured to together entirely surround side wall member **7** and to surround at least a portion of each of inlet housing member **2** and outlet housing member **3.** Each jacket component **12** also includes openings for a heating coil inlet **10** and a heating coil outlet **11** of the heating coil. As shown in **FIG. 27****,** the heating coil inlets **10** are parallel to an inlet of inlet housing member **2.** As shown in **FIG. 28****,** the heating coil outlets **1** are parallel to an outlet of outlet housing member **3.**

In some embodiments, at least two of the two or more jacket components each comprise an electric heating element, e.g., an electric heating element that includes a metal plate. In some embodiments, at least two of the two or more jacket components each comprise an electric heating element. In some embodiments, the two or more jacket components each comprise an electric heating element.

In some embodiments, the electric heating elements of the at least two of the two or more jacket components are electrically coupled to one another. In some embodiments, the electric heating elements of the two or more jacket components are electrically coupled to one another.

In some embodiments, at least one of the two or more jacket components is configured to electrically connect to a power source, e.g., a power source external to or included in the housing assembly. In some embodiments, each of the two or more jacket components is configured to electrically connect to a power source, e.g., a power source external to or included in the housing assembly.

In some embodiments, at least one electric heating element of the at least two of the two or more jacket components is configured to electrically connect to a power source, e.g., a power source external to or included in the housing assembly. In some embodiments, each electric heating element of the two or more jacket components is configured to electrically connect to a power source, e.g., a power source external to or included in the housing assembly.

**FIGS. 29-31** provide schematic representations of an exemplary housing assembly for column chromatography. The exemplary housing assembly **1** shown in **FIG. 29** includes inlet housing member **2,** outlet housing member **3,** and side wall member **7** that form an internal cavity configured to house a stationary phase. Housing assembly **1** also includes a gas supply connector for screw-on air filters (not shown) and a temperature control member that includes electric heating elements **17** that include metal plates. Electric heating elements **17** are part of a jacket member made of three jacket components **12.** The jacket components **12** are configured to together entirely surround side wall member **7** and to surround at least a portion of each of inlet housing member **2** and outlet housing member **3.** Each jacket component **12** includes an inlet groove **13** such that the jacket member exposes an inlet of the inlet housing member **2.** Each jacket component **12** also includes an outlet groove **14** such that the jacket member exposes an outlet of the outlet housing member **3.**

**FIGS. 30A-30C** show three views of jacket component **12.** Each jacket component **12** includes an electric heating element **17** and a temperature sensor **18.** Each electric heating element **17** is configured to electrically connect to a power source via heating element electrical connection **16,** and each temperature sensor is configured to electrically connect to a power source via temperature sensor electrical connection **20.** As shown in **FIG. 30D****,** electric heating element **17** also includes an electric isolation layer **19** and an aluminum profile **21.** Each electric heating element **17** is mounted into jacket component **12** at mounting points **15.** Electric heating elements **17** and jacket components **12** are configured such that electric heating elements **17** are uniformly distributed around the circumference of side wall member **7.** As shown in **FIG. 29****,** heating element electrical connections **16** and temperature sensor electrical connections **20** are exposed on the same face of the jacket member as the outlet of outlet housing member **3.**

In one aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member comprising a heating element disposed along and/or around a central axis of the internal cavity, the heating element configured to provide heat to the stationary phase in the internal cavity; and a connector configured to operably and sterilely connect the internal cavity to a gas source, thereby permitting or effecting intake of sterile gas into the internal cavity.

In one aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member configured to regulate or maintain a temperature of the stationary phase and comprising a heating element disposed along and/or around a central axis of the internal cavity, the heating element configured to provide heat to the stationary phase in the internal cavity; and a connector configured to operably and sterilely connect the internal cavity to a gas source, thereby permitting or effecting intake of sterile gas into the internal cavity.

In another aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member comprising a heating element comprising a metal plate configured to provide heat to the stationary phase in the internal cavity; and a connector configured to operably and sterilely connect the internal cavity to a gas source, thereby permitting or effecting intake of sterile gas into the internal cavity.

In another aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member configured to regulate or maintain a temperature of the stationary phase, wherein the temperature control member comprises two heating coils configured to provide heat to the stationary phase; a jacket member comprising the temperature control member comprising the two heating coils, wherein the jacket member is releasably connected together to surround at least a portion of the inlet housing member, the outlet housing member, and the side wall member and the two heating coils entirely surround the side wall member; and a connector configured to operably and sterilely connect the internal cavity to a gas filter, thereby permitting or effecting intake of sterile gas into the internal cavity.

In another aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member configured to regulate or maintain a temperature of the stationary phase, wherein the temperature control member comprises three electric heating elements each comprising a metal plate and that are configured to provide heat to the stationary phase; a jacket member comprising the temperature control member comprising the three electric heating elements, wherein the jacket member is releasably connected together to surround at least a portion of the inlet housing member, the outlet housing member, and the side wall member and the two heating coils entirely surround the side wall member; and a connector configured to operably and sterilely connect the internal cavity to a gas filter, thereby permitting or effecting intake of sterile gas into the internal cavity.

In yet another aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member comprising a heating element comprising a heating coil configured to provide heat to the stationary phase in the internal cavity; and a connector configured to operably and sterilely connect the internal cavity to a gas source, thereby permitting or effecting intake of sterile gas into the internal cavity. In some embodiments, the heating coil comprises an inlet and an outlet for heated water. In some aspects, the heating coil surrounds the inlet housing member, the outlet housing member, and the side wall member.

In one aspect, disclosed herein is a housing assembly for column chromatography, comprising: an inlet housing member, an outlet housing member, and a side wall member, wherein the inlet housing member, the outlet housing member, and the side wall member form an internal cavity configured to house a stationary phase for column chromatography; a temperature control member comprising a heating element configured to provide heat to the stationary phase in the internal cavity; and a connector configured to operably and sterilely connect the internal cavity to a gas filter, thereby permitting or effecting intake of sterile gas into the internal cavity.

In any of the preceding embodiments, the gas filter can be an air filter and the sterile gas can be sterile air. In any of the preceding embodiments, the housing assembly can further comprise the gas filter.

Also disclosed herein is a housing assembly set, comprising a plurality of the housing assembly disclosed herein. The housing assembly set can comprise at least two of the plurality of the housing assembly arranged sequentially. The housing assembly set can comprise at least two of the plurality of the housing assembly arranged in parallel.

Also disclosed herein is a chromatography system, comprising any of the housing assemblies disclosed herein and at least one additional chromatography column. In some embodiments, the at least one additional chromatography column does not include a temperature control member. In some embodiments, the at least one additional chromatography column does not include a connector configured to operably connect an internal cavity of the at least one additional chromatography column to a gas source.

### B. Chromatography Kits, Columns, and Column Sets

In some embodiments, also disclosed herein is a chromatography kit, comprising the housing assembly or the housing assembly set disclosed herein, and a stationary phase for column chromatography. In some embodiments, the housing assembly or the housing assembly set is any as described in Section I-A. In some embodiments, the chromatography kit further comprises one or more stimulatory agent orstimulatory reagent. In some embodiments, the one or more stimulatory agent or stimulatory reagent is any as described in Section II-B-1 or II-B-2.

In some embodiments, also disclosed herein is a chromatography column or chromatography column set, comprising the housing assembly or the housing assembly set disclosed herein, and a stationary phase for column chromatography in the internal cavity of one or more of the housing assembly. In some embodiments, the housing assembly or the housing assembly set is any as described in Section I-A.

In some embodiments, also disclosed herein is a chromatography column, including a jacket member and a chromatography column. In some embodiments, the internal cavity of the chromatography column includes a stationary phase for column chromatography. In some embodiments, the jacket member is any as described in Section I-C.

In some embodiments, also disclosed herein is a chromatography column set, including at least one jacket member and a plurality of chromatography columns. In some embodiments, the jacket member is any as described in Section I-C. In some embodiments, the internal cavity of each of the plurality of chromatography columns comprises a stationary phase for column chromatography. In some embodiments, the plurality of chromatography columns are arranged sequentially. In some embodiments, the plurality chromatography columns are arranged in parallel. In some embodiments, the plurality of chromatography columns are operably connected.

In some embodiments, the plurality of chromatography columns includes a first chromatography column. In some embodiments, the plurality of chromatography columns includes a second chromatography column. In some embodiments, the at least one jacket member is configured to surround the second chromatography column.

In any of the preceding embodiments, the stationary phase can comprise a gel filtration matrix, and/or an affinity chromatography matrix. The stationary phase may comprise a non-magnetic material, a non-ferromagnetic material, or non-paramagnetic material. In other aspects, the stationary phase is selected from the group consisting of a cellulose membrane, a plastic membrane, a polysaccharide gel, a polyacrylamide gel, an agarose gel, polysaccharide grafted silica, polyvinylpyrrolidone grafted silica, polyethylene oxide grafted silica, poly(2-hydroxy ethyl aspartamide) silica, poly(N-isopropylacrylamide) grafted silica, a styrene-divinylbenzene gel, a copolymer of an acrylate or an acrylamide and a diol, a co-polymer of a polysaccharide and N,N'-methylenebisacrylamide, and a combination thereof. The stationary phase can comprises or is a monolithic matrix, a particulate matrix, and/or a planar matrix.

In any of the preceding embodiments, the particulate matrix can have a mean particle size of about 5 µm to about 200 µm, of about 5 µm to about 600 µm, or of about 5 µm to about 1500 µm. In any of the preceding embodiments, the stationary phase can have a mean pore size of about 1 nm to about 500 nm.

In any of the preceding embodiments, the stationary phase can comprise immobilized thereon any of the agents described in Section II-B-1. In some embodiments, the agents are directly immobilized on the stationary phase. In some embodiments, the agents are indirectly immobilized on the stationary phase. In some embodiments, the agents are irreversibly immobilized on the stationary phase. In some embodiments, the agents are reversibly immobilized on the stationary phase. In some embodiments, the agents are reversibly immobilized on the stationary phase via a mutein of streptavidin that reversibly binds to a streptavidin-binding peptide. In some embodiments, the streptavidin mutein and/or the streptavidin-binding peptide are any as described in Section II-B-2.

In any of the preceding embodiments, the stationary phase can comprise a selection agent immobilized thereon. In some aspects, the selection agent is capable of specific binding to a selection marker on the surface of one or more cells. In some embodiments, the one or more cells are immune cells. In some aspects, the one or more cells are T cells.

Also disclosed here in is an apparatus, comprising the housing assembly, the housing assembly set, or the chromatography kit, chromatography column, or chromatography column set, further comprising an input composition reservoir operably connected to the internal cavity via an inlet of the inlet housing member. In some embodiments, the input composition comprises or is blood or a blood-derived sample. In some embodiments, the input composition comprises or is a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some embodiments, the apheresis or leukapheresis product is freshly isolated from a subject or thawed from a cryopreserved apheresis or leukapheresis product. In some embodiments, the apparatus further comprises an output composition reservoir operably connected to the internal cavity via an outlet of the outlet housing member. In some aspects, the output composition comprises or is enriched T cells. In other aspects, the enriched T cells have undergone stimulation during chromatography on the chromatography column. In any of the preceding embodiments, the apparatus can be a closed or sterile system. An exemplary apparatus that includes an exemplary housing assembly **1** is shown in **FIG. 1B****.**

Also disclosed herein is a method of preparing a chromatography column or chromatography column set, comprising introducing a stationary phase into the housing assembly or the housing assembly set disclosed herein. In addition, disclosed herein is a method of preparing a chromatography column or chromatography column set, comprising introducing the stationary phase of the chromatography kit into the housing assembly or housing assembly set of the chromatography kit.

### C. Jacket Members for Chromatography

The devices provided herein also include jacket members for column chromatography. In some aspects, the provided jacket members are devices that allow for improved methods involving the isolation, processing, or manipulation of target cells immobilized on a stationary phase of a chromatography column, e.g., methods of on-column selection and/or stimulation of target cells. In some aspects, the provided jacket members allow for the regulation of the temperature, e.g., heating, of the immobilized cells. In some aspects, the provided jacket members allow for the maintenance of the temperature of the immobilized cells, e.g., at or about 37°C or 37°C ±about 5°C. In some aspects, the regulation and maintenance of the temperature of cells by the provided devices improves on-column manipulation, e.g., stimulation, of immobilized cells, for instance by improving the overall health, fitness, or condition of the immobilized cells during on-column manipulation.

In one aspect, the jacket member includes one or more jacket components configured to surround at least a portion of a chromatography column. In some aspects, the chromatography column is configured to house a stationary phase. In some aspects, the chromatography column includes a stationary phase. In some aspects, the jacket member further includes one or more heating elements. In some aspects, the one or more heating elements are configured to provide heat to the stationary phase. In some embodiments, the one or more heating elements are configured to be part of a temperature control member. In some aspects, the temperature control member is configured to regulate or maintain a temperature of the stationary phase. In some embodiments, the one or more heating elements are any as described in Section I-A. In some embodiments, the temperature control member is any as described in Section I-A.

In some aspects, the one or more jacket components are configured to be releasably connected together to surround the at least a portion of the chromatography column. In other embodiments, the one or more jacket components are configured to be not releasably connected together.

In some embodiments, the jacket member is configured to surround at least a portion of a chromatography column that can accommodate a bed volume between or between about 1 and 40 mL, such as between or between about 1 and 35 mL, 1 and 30 mL, 1 and 25 mL, 1 and 20 mL, 1 and 15 mL, 1 and 10 mL, 1 and 5 mL, 5 and 40 mL, 5 and 35 mL, 5 and 30 mL, 5 and 25 mL, 5 and 20 mL, 5 and 15 mL, 5 and 10 mL, 10 and 40 mL, 10 and 35 mL, 10 and 30 mL, 10 and 25 mL, 10 and 20 mL, 10 and 15 mL, 15 and 40 mL, 15 and 35 mL, 15 and 30 mL, 15 and 25 mL, 15 and 20 mL, 20 and 40 mL, 20 and 35 mL, 20 and 30 mL, 20 and 25 mL, 25 and 40 mL, 25 and 35 mL, 25 and 30 mL, 30 and 40 mL, 30 and 35 mL, or 35 and 40 mL. In some embodiments, the jacket member is configured to surround at least a portion of a chromatography column that can accommodate a bed volume between or between about 15 and 25 mL. In some embodiments, the jacket member is configured to surround at least a portion of a chromatography column that can accommodate a bed volume between or between about 15 and 20 mL. In some embodiments, the jacket member is configured to surround at least a portion of a chromatography column that can accommodate a bed volume between or between about 18 and 20 mL.

In some embodiments, at least a portion of the jacket member is configured to be in contact, e.g., direct contact, with at least a portion of chromatography column. In some embodiments, the jacket member is configured to be in contact, e.g., direct contact, with at least a portion of chromatography column. In some embodiments, the jacket member is configured to be in contact, e.g., direct contact, with the chromatography column.

In some embodiments, at least a portion of the jacket member is configured to not be in contact with at least a portion of the chromatography column. In some embodiments, at least a portion of the jacket member is configured to not be in contact with the chromatography column. In some embodiments, the jacket member is configured to not be in contact with the chromatography column.

In some embodiments, the jacket member is configured so that an insulation layer can be disposed between the one or more jacket components and at the chromatography column. In some embodiments, the jacket member further includes an insulation layer. In some embodiments, the insulation layer is configured to be disposed between the one or more jacket components and at least a portion of the chromatography column. In some embodiments, the insulation layer is configured to be disposed between the one or more jacket components and the chromatography column. In some embodiments, the insulation layer is configured to surround at least a portion of the chromatography column.

In some embodiments, the insulation layer includes a gas layer, e.g., an air layer. In some embodiments, the insulation layer includes a liquid layer. In some embodiments, the insulation layer includes a solid layer.

In some embodiments, the temperature control member can be configured to heat a stationary phase contained in a chromatography column to a target temperature between about 30°C and about 39°C (*e.g.,* at or at about 37°C). In some embodiments, the starting temperature is about 2°C, about 4°C, about 8°C, about 12°C, about 16°C, about 20°C, about 24°C, about 28°C, about 32°C, about 36°C, or higher than about 36°C. In some embodiments, the temperature control member can be configured to heat the stationary phase to or to about 37°C. In some embodiments, the temperature control member can be configured to heat the stationary phase to at or higher than about 2°C, at or higher than about 4°C, at or higher than about 8°C, at or higher than about 12°C, at or higher than about 16°C, at or higher than about 20°C, at or higher than about 24°C, at or higher than about 28°C, at or higher than about 32°C, at or higher than about 36°C, at or higher than about 37°C, at or higher than about 38°C, at or higher than about 39°C, or at or higher than about 40°C. In some embodiments, the temperature control member can be configured to keep the stationary phase at a target temperature. In some embodiments, the temperature control member can be configured to keep the stationary phase at a target temperature about 5°C, about 4°C, ±about 3°C, about 2°C, about 1°C or about 0.5°C. In some embodiments, the temperature control member can be configured to keep the stationary phase at 37°C ±about 5°C, about 4°C, about 3°C, about 2°C, about 1°C or about 0.5°C.

In some embodiments, the temperature control member can be configured to regulate or maintain a temperature of a stationary phase contained in a chromatography column. In some aspects, the temperature control member is configured to heat, e.g., uniformly heat, the stationary phase from a starting temperature (*e.g*., room temperature) to a target temperature between about 30°C and about 39°C (*e.g.,* at or at about 37°C). In some aspects, the temperature control member is further configured to maintain the stationary phases at the target temperature.

In some embodiments, the jacket member can further include a temperature sensor configured to measure the temperature of the stationary phase in the internal cavity. In one aspect, the temperature sensor is configured to couple to a monitoring/display unit. In some embodiments, the temperature sensor is configured to electrically connect to a power source. In some embodiments, the power source is external to the jacket member. In some embodiments, the jacket member further includes the power source.

In any of the preceding embodiments, the temperature control member can comprise a heating source. Alternatively, in any of the preceding embodiments, the temperature control member can be configured to operably connect to a heating source which is external to the housing assembly.

In some embodiments, the temperature control member includes a heating element. In some embodiments, the heating element is configured to uniformly heat the stationary phase.

In any of the preceding embodiments, the temperature control member can comprise a heating element selected from the group consisting of an electric heating element, an electromagnetic induction heating element, a non-electric heating element, and any combination thereof. In one aspect, the heating element is an electric heating element. In some embodiments, the electric heating element comprises a metal plate, a metal rod, a metal wire, or a combination thereof. In one aspect, the heating element is an electromagnetic induction heating element. In some embodiments, the electromagnetic induction heating element comprises an induction heating coil surrounding a magnetizable core configured to provide heat to the stationary phase in the internal cavity. In one aspect, the heating element is a non-electric heating element.

In some embodiments, the non-electric heating element comprises a heating channel comprising an inlet and an outlet for a heated fluid, *e.g.,* a heated liquid or gas. In some embodiments, the heating channel is a heating coil. In some embodiments, the heated fluid is heated water. In some embodiments, the inlet for heated water is configured to connect to an external reservoir of heated water.

In some embodiments, the heating element is an electric heating element. In some embodiments, the electric heating element is configured to electrically connect to a power source. In some embodiments, the power source is external to the jacket member. In some embodiments, the jacket member further includes the power source.

In some embodiments, the electric heating element includes a metal plate. In some embodiments, the metal plate is made at least in part of a heat-conductive metal, e.g, aluminum or copper. In some embodiments, the metal plate is made at least in part of aluminum, e.g., made entirely of aluminum. In some embodiments, the electric heating element further includes an electrical isolation layer, e.g., between at least a portion of the metal plate and at least a portion of other components of the electric heating element. In some embodiments, the electrical isolation layer lines at least a portion of one face of the metal plate, e.g., entirely lines one face of the metal plate.

In some embodiments, at least a portion of the heating element is configured to be in contact, e.g., direct contact, with at least a portion of the chromatography column. In some embodiments, the heating element configured to be in contact, e.g., direct contact, with at least a portion of the chromatography column. In some embodiments, the heating element configured to be in contact, e.g., direct contact, with the chromatography column.

In some embodiments, at least a portion of the heating element is configured to be not in contact with at least a portion of the chromatography column. In some embodiments, the heating element is configured to be not in contact with at least a portion of the chromatography column. In some embodiments, the heating element is configured to be not in contact with the chromatography column.

In some embodiments, the heating element is configured to surround at least a portion of the chromatography column.

In some embodiments, the temperature control member includes a plurality of heating elements. In some embodiments, the temperature control member includes between or between about 2 and 10 heating elements, between or between about 2 and 8 heating elements, between or between about 2 and 6 heating elements, or between or between about 2 and 4 heating elements, each inclusive. In some embodiments, the temperature control member includes two heating elements. In some embodiments, the temperature control member includes three heating elements.

In some embodiments, the plurality of heating elements are configured to uniformly heat the stationary phase.

In some embodiments, the plurality of heating elements are each selected from the group consisting of an electric heating element, an electromagnetic induction heating element, a non-electric heating element, and any combination thereof. In some embodiments, the plurality of heating elements are identical. In some embodiments, the plurality of heating elements are a combination of different heating elements.

In some embodiments, the plurality of heating elements include a plurality of non-electric heating elements. In some embodiments, the plurality of heating elements include a plurality of heating channels. In some embodiments, each of the plurality of heating channels has an inlet and outlet for a heated fluid, e.g., heated water. In some embodiments, at least two of the plurality of heating channels are fluidly coupled to one another. In some embodiments, the plurality of heating channels are fluidly coupled to one another. In some embodiments, the inlet of at least one of the plurality of heating channels is configured to connect to an external reservoir of heated liquid. In some embodiments, the inlet of each of the plurality of heating channels is configured to connect to an external reservoir of heated liquid.

In some embodiments, the plurality of heating elements include a plurality of electric heating elements, e.g., electric heating elements comprising metal plates. In some embodiments, at least two of the plurality of electric heating elements are electrically coupled to one another. In some embodiments, the plurality of electric heating elements are electrically coupled to one another. In some embodiments, at least one of the plurality of electric heating elements is configured to electrically connect to a power source, e.g., a power source external to or included in the housing assembly. In some embodiments, each of the plurality of electric heating elements is configured to electrically connect to a power source, e.g., a power source external to or included in the housing assembly.

In some embodiments, at least a portion of at least one of the plurality of heating elements is configured to be in contact, e.g., direct contact, with at least a portion of the chromatography column. In some embodiments, at least one of the plurality of heating elements is configured to be in contact, e.g., direct contact, with at least a portion of the chromatography column. In some embodiments, at least one of the plurality of heating elements is configured to be in contact, e.g., direct contact, with the chromatography column. In some embodiments, the plurality of heating elements is configured to be in contact, e.g., direct contact, with the chromatography column.

In some embodiments, at least a portion of at least one of the plurality of heating elements is configured to be not in contact with at least a portion of the chromatography column. In some embodiments, at least a portion of at least one of the plurality of heating elements is configured to be not in contact with the chromatography column. In some embodiments, at least one of the plurality of heating elements is configured to be not in contact with the chromatography column. In some embodiments, the plurality of heating elements is configured to be not in contact with the chromatography column.

In some embodiments, the plurality of heating elements are configured to be uniformly or about uniformly distributed around the chromatography column, e.g., around the circumference of the chromatography column.

In some embodiments, the temperature control member includes a non-electric heating element, e.g., a heating channel for heated fluid. In some embodiments, the temperature control member includes a plurality of non-electric heating elements. In some embodiments, the jacket member includes at least opening for one inlet and at least one opening for one outlet for heated fluid, e.g., heated water. In some embodiments, the jacket member includes at least two openings for inlets and/or at least two openings for outlets for heated fluid, e.g., heated water. In some embodiments, the jacket member is configured to electrically connect to a power source, e.g., a power source external to or included in the housing assembly.

In some embodiments, the jacket member includes an electric heating element, e.g., an electric heating element that includes a metal plate. In some embodiments, the jacket member includes a plurality of electric heating elements. In some embodiments, the electric heating element is configured to electrically connect to a power source. In some embodiments, at least one of the plurality of electric heating elements is configured to electrically connect to a power source. In some embodiments, each of the plurality of electric heating elements is configured to electrically connect to a power source.

In some embodiments, at least two of the plurality of electric heating elements are electrically coupled to one another. In some embodiments, the plurality of electric heating elements are electrically coupled to one another.

In some embodiments, the jacket member includes two or more jacket components, for instance between or between about 2 and 10 jacket components, 2 and 8 jacket components, 2 and 6 jacket components, or 2 and 4 jacket components, each inclusive. In some embodiments, the jacket member includes two jacket components. In some embodiments, the jacket member includes three jacket components. In some embodiments, the jacket member includes four jacket components.

In some embodiments, at least two of the two or more jacket components each comprise a heating element. In some embodiments, the two or more jacket components each comprise a heating element.

In some embodiments, at least two of the two or more jacket components each comprise a temperature sensor. In some embodiments, the two or more jacket components each comprise a temperature sensor.

In some embodiments, at least two of the two or more jacket components each comprise a non-electric heating element. In some embodiments, at least two of the two or more jacket components each comprise a heating channel with inlet and outlet for a heated fluid, e.g., heated water. In some embodiments, the two or more jacket components each comprise a heating channel with inlet and outlet for a heated fluid, e.g., heated water.

In some embodiments, the heating channels of the at least two of the two or more jacket components are fluidly coupled to one another. In some embodiments, the heating channels of the two or more jacket components are fluidly coupled to one another.

In some embodiments, at least one of the two or more jacket components includes an opening for the inlet for a heated fluid, e.g., heated water. In some embodiments, the two or more jacket components each include an opening for an inlet for a fluid, e.g., heated water.

In some embodiments, at least one of the two or more jacket components includes an opening for the outlet for a heated fluid, e.g., heated water. In some embodiments, the two or more jacket components each include an opening for an outlet for a fluid, e.g., heated water.

### II. METHODS FOR SELECTING, STIMULATING, AND/OR ENGINEERING CELLS (not part of the claimed invention)

The methods disclosed in the following part II are not in accordance with the claimed invention. Using a device disclosed herein, provided herein are methods for generating an output population of cells (also referred to as an output composition), such as selected and stimulated CD3+ T, CD4+ T, and/or CD8+ T cells, including steps for the selection, stimulation, and collection of the cells. In certain embodiments, the methods provided herein are used in connection with manufacturing, generating, or producing a cell therapy. In some embodiments, the methods of generating or producing the output composition, e.g., selected and stimulated T cells, include one or more of steps for isolating cells from a subject, incubating the cells under stimulatory conditions, and genetically engineering the cells. In some embodiments, the method includes processing steps carried out in an order in which input cells, e.g. primary CD4+ and CD8+ T cells, are isolated, such as selected or separated, from a biological sample and incubated under stimulating conditions and collected in a single step, and subsequently genetically engineered to introduce a recombinant polynucleotide encoding a recombinant receptor into the cells, such as by transduction or transfection; and then collected, harvested, or filled into a container, e.g., a bag or vial, as an output population. In some embodiments, the cells of the output population are re-introduced into the same subject, optionally after cryopreserving and storing the cells. In some embodiments, the output populations of engineered cells are suitable for use in a therapy, e.g., an autologous cell therapy.

Using a device disclosed herein, provided herein are methods for selecting cells from a sample comprising target cells (e.g., T cells, CD3+, CD4+, CD8+ T cells) and immobilizing said target cells on the stationary phase of a chromatography column, stimulating immobilized cells on the stationary phase (also referred to herein as on-column stimulation), and collecting and/or eluting the selected and stimulated cells that spontaneously detach from the stationary phase without the use of competition agents or free binding agents to facilitate detachment. Among the provided methods are methods involving selecting cells from a sample comprising target cells (e.g., T cells, CD3+, CD4+, CD8+ T cells) and immobilizing said target cells on the stationary phase of a chromatography column, stimulating immobilized cells on the stationary phase, and collecting and/or eluting the selected and stimulated cells by gravity flow. In provided embodiments, stimulating target cells (e.g., CD3+, CD4+, or CD8+ T cells) on a stationary phase of a chromatography column, facilitates downregulation of the molecule used for cell selection (i.e., selection marker), resulting in spontaneous detachment or release of the cell from the stationary phase. The release or detachment of the cells can occur without any additional steps or reagents. In some aspects, the cells can be collected by gravity flow, such as by adding a media or other solution to the chromatography column. In particular embodiments, the media or other solution that is added does not contain a competition agents or free binding agents to facilitate detachment of the cells from the stationary phase.

In particular embodiments, the provided methods are carried out to select and stimulate T cells. In some embodiments, the T cells are selected from a biological sample, e.g. apheresis sample, by adding cells of the sample to an affinity chromatography matrix (e.g. stationary phase) immobilized with or bound by a selection agent specific for T cells or a subset thereof, e.g. as described in Section II.B-1. In provided embodiments, the methods include stimulating the cells immobilized on the stationary phase in the presence of one or more stimulatory agents of the T cells. In some embodiments, the one or more stimulatory agents include an agent for delivering a stimulatory signal in the T cells. In some embodiments, the stimulatory signal is through a TCR/CD3 complex in a T cell, a CD3-containing complex in a T cell, and/or an ITAM-containing molecule in a T cell. In some embodiments, the stimulatory agent (e.g. first stimulatory agent) is an agent that binds to CD3, such as an anti-CD3 antibody. In some embodiments, the one or more stimulatory agent further includes a second stimutory agent that is able to further stimulate or enhance a signal in the T cells. In some embodiments, the second stimulatory agent is capable of specifically binding to a costimulatory molecule on the one or more T cells, *e.g.,* CD28, CD90 (Thy-1), CD95 (Apo-/Fas), CD137 (4-1BB), CD154 (CD40L), ICOS, LAT, CD27, OX40 or HVEM. In some embodiments, the second stimulatory agent is an agent that binds to CD28, such as an anti-CD28 antibody. In some embodiments, the one or more stimulatory agents include an anti-CD3 antibody and an anti-CD28 antibody, for example, an anti-CD3 Fab and an anti-CD28 Fab. In some embodiments, the one or more stimulatory agent are immobilized or bound to a reagent (e.g. is a stimulatory reagent) that is added to the chromatography column. In particular embodiments, the stimulatory reagent is soluble polymeric or oligomeric reagent. For instance, the one or more stimulatory agents are functionalized to an oligomeric or polymeric protein as opposed to a solid surface (e.g. bead). Exemplary oligomeric stimulatory reagents for use in the provided methods are described herein, e.g. Section II.B-2. In some embodiments, the oligomeric stimulatory reagents is an oligomeric streptavidin mutein that is functionalized or multimerized with one or more stimulatory agents (e.g. anti-CD3 Fab and anti-CD28 Fab). In provided methods, the selected and stimulated T cells are collected by eluting or washing the selected and stimulated cells by gravity flow.

In some embodiments, said collecting includes washing the stationary phase with media (e.g. serum free media), the media not containing a competition agent or free binding agent to elute the target cells (e.g. T cells) from the stationary phase. In some embodiments, the collecting by gravity flow includes adding media to the stationary phase, the media not comprising a competition agent or free binding agent to elute the T cells from the stationary phase. In some embodiments, said composition containing stimulated T cells does not contain a competition agent or free binding agent. In some embodiments, said competition agent or free binding agent is or contains biotin or a biotin analog, for example a biotin analog that is D-biotin. In some embodiments, the competition agent or free binding agent is D-biotin. In some embodiments, the media for the washing column to elute the cells by gravity flow is a serum-free media that contain recombinant cytokines (e.g. IL-2,

In some embodiments, the method further includes introducing a recombinant nucleic acid molecule into the stimulated T cells of the composition, wherein the nucleic acid molecule encodes a recombinant protein, thereby producing a composition comprising transduced T cells. In some embodiments, the recombinant protein is an antigen receptor. In some embodiments, the recombinant protein is a chimeric antigen receptor.

In some embodiments, the method includes further incubating the composition containing the stimulated cells (e.g. stimulated T cells). In some embodiments, the method includes further incubating the composition containing the cells introduced with the recombinant receptor (e.g. transduced T cells). In some embodiments, the further incubation is carried out at or about 37 °C ± 2 °C. In some embodiments, the further incubation is carried out under conditions that do not expand or substantially expand the cells. In some embodiments, the further incubation is carried out under conditions for expansion (e.g. proliferation) of the cells. In some embodiments, the further incubation is carried out in the presence of a further agent that is capable of delivering a signal to T cells. In some embodiments, the further agent is contained in the media used for washing the stationary phase. In some embodiments, the further agent is capable of enhancing or inducing proliferation of T cells, CD4+ T cells and/or CD8+ T cells. In some embodiments, the further agent is a cytokine selected from among IL-2, IL-15 and IL-7. In some embodiments, the further incubation is carried out for a time that is 72 hours, no more than 48 hours, no more than 24 hours, or no more than 12 hours.

In particular embodiments, using a device disclosed herein, provided herein are methods in connection with generating an output population of cells expressing a recombinant receptor from an initial or input population of cells. In certain embodiments, the input population is produced, generated, and/or made by combining, mixing, and/or pooling cells including from a population of cells containing enriched T cells, enriched CD4+ T cells, and/or enriched CD8+ T cells (herein after also referred to as populations of enriched T cells, populations of enriched CD4+ T cells, and populations of enriched CD8+ T cells, respectively). In some embodiments, the input population of cells is a population of combined, mixed, and/or pooled CD4+ and CD8+ T cells. In certain embodiments, the provided methods are used in connection with genetically engineering the selected and stimulated cells, e.g., to introduce a polynucleotide encoding a recombinant protein by transduction or transfection. In certain embodiments, the methods may be used to isolate select cells from a biological sample (e.g., whole blood, apheresis) to generate an input population of enriched T cells, such as from a biological sample taken, collected, and/or obtained from a subject. In some embodiments, the provided methods may be used in connection with harvesting, collecting, and/or formulating populations of enriched T cells after the cells have been engineered, transduced, and/or cultured.

In certain embodiments, using a device disclosed herein, provided herein are methods in connection with introducing a heterologous or recombinant polynucleotide into the cells, e.g., transducing or transfecting the cells, such as by a method described herein, e.g., in Section II-F. In particular embodiments, the cells are incubated either during or after genetically engineering the cells, for example, for an amount of time sufficient to allow for integration of a heterologous or recombinant polynucleotide encoding a recombinant protein or to allow for the expression of the recombinant protein. In certain embodiments, the cells are incubated for a set or fixed amount of time, such as an amount of time greater than 18 hours or less than 4 days. In some embodiments, the engineering step is started or initiated within a set amount of time from when the stimulating is started or initiated, such as within 24 hours from when the cells are exposed to a stimulatory agent.

In some embodiments, the one or more process steps are carried out, at least in part, in serum free media. In some embodiments, the serum free media is a defined or well-defined cell culture media. In certain embodiments, the serum free media is a controlled culture media that has been processed, e.g., filtered to remove inhibitors and/or growth factors. In some embodiments, the serum free media contains proteins. In certain embodiments, the serum-free media may contain serum albumin, hydrolysates, growth factors, hormones, carrier proteins, and/or attachment factors. In some embodiments, the serum free media includes cytokines. In some embodiments, the serum free media includes cytokines or recombinant cytokines. In some embodiments, the serum free media includes recombinant IL-2, IL-15, and/or IL-7. In some embodiments, the serum free media includes glutamine. In some embodiments, the serum free media includes glutamine and recombinant IL-2, IL-15, and IL-7.

In some embodiments, using a device disclosed herein, provided herein are methods that are carried out such that one, more, or all steps in the preparation of cells for clinical use, e.g., in adoptive cell therapy, are carried out without exposing the cells to non-sterile conditions. In some embodiments, the cells are selected, stimulated, transduced, washed, and formulated, all within a closed, sterile system or device. In some embodiments, the one or more of the steps are carried out apart from the closed system or device. In some such embodiments, the cells are transferred apart from the closed system or device under sterile conditions, such as by sterile transfer to a separate closed system.

In some embodiments, the methods provided herein are performed using any of the devices described in Section I.

In particular embodiments, the sample and/or isolated portions of the sample (e.g., buffy coat, populations of enriched T cells) may be collected, formulated for cryoprotection, frozen (e.g.,cryoprotected), and/or stored below 0°C, below -20°C, or at or below -70C or - 80°C prior to, during, or after any stage or step of the methods as provided herein. In some embodiments, the cells may be stored for an amount of time under 1, 2, 3, 4, 5, 6, 7, 8, 9, or 10 days, or an amount of time under 1, 2, 3, 4, 5, 6, 7, 8 weeks, or for an amount of time at least 1, 2, 3, 4, 5, 6, 7, or 8 weeks, or for more than 8 weeks. After storage, the sample or isolated portion of the sample may be thawed and processing according to the method may be resumed from the same point in the process. In particular embodiments, cultivated and/or formulated populations of enriched T cells are cryoprotected and stored prior to being administered to a subject, e.g., as an autologous cell therapy.

In particular embodiments, at any stage or step in the process, a portion of the cells may be sampled or collected, e.g., cells may be taken from the population of cells (such as a population of T cells) while the population remains in the closed system. In certain embodiments, such cells may be analyzed for makers, features, or characteristics including but not limited to viability, apoptosis, activation, stimulation, growth, and/or exhaustion. In some embodiments, the cells are sampled or collected by an automated process. In some embodiments, the analysis of sampled or collected cells is automated. In particular embodiments, the analysis is performed in a closed system under sterile conditions.

In some embodiments, cells or populations of cells that are produced and/or processed by the provided methods may be compared to cells or populations of cells processed or produced by an exemplary and/or alternative process. In certain embodiments, the alternative and/or exemplary process may differ in one or more specific aspects, but otherwise contains similar or the same features, aspects, steps, stages, reagents, or conditions of the embodiment or aspect of the provided methods that be compared to an exemplary or alternative process. For example, selected and stimulated cells generated by the provided methods, e.g., an output composition of cells, may be compared to cells that were generated with a process that involved separate selection and stimulating steps which required use of a competition agent or free binding agent to detach the selected cells from a stationary phase. In some embodiments, unless otherwise specified, the provided methods and the exemplary or alternative process would have been otherwise similar and/or identical, such as with similar or identical steps for selecting, enriching, stimulating, engineering, transfecting, transducing, cultivating, and/or formulating. In some embodiments, unless otherwise specified, the provided methods and the alternative process select and/or enrich cells from the same or similar types of biological samples, and/or process cells and/or input cells of the same cell type.

In some embodiments, the selected and stimulated cells are a composition containing stimulated T cells in which the T cells have been selected from a biological sample (e.g. apheresis or whole blood sample) containing a plurality of T cells. In some embodiments, the collecting and/or eluting of the selected and stimulated cells that spontaneously detach from the stationary phase is accomplished via gravity flow, for example during a wash step. The methods provided herein combine cell selection, stimulation, and collection and/or elution steps, and do not require separate steps to facilitate detachment of the selected and stimulated cells from the stationary phase and purification steps to remove agents (e.g., competition agents and/or free binding agents) used to facilitate detachment. As such, the methods reduce the number of processing steps needed to generate a selected and stimulated cell composition suitable for downstream processing (e.g., genetic engineering, expansion, subsequent incubation, stimulation and/or selection (e.g., initial selection and/or polishing)), thereby reducing manufacturing time, minimizing potential cell stress, and decreasing the potential for contamination.

In particular embodiments, the methods generate an output composition of selected and stimulated cells suitable for downstream processing within a set amount of time, such as within 24 hours. In particular embodiments, the methods generate an output composition of selected and stimulated cells suitable for downstream processing within a set amount of time, such as within or within about 12, 11, 10, 9, 8, 7, 6, 5, 4, 3, or 2 hours. In particular embodiments, the methods generate an output composition of selected and stimulated cells suitable for downstream processing within a set amount of time, such as within or within about 6, 5, 4, 3, or 2 hours. In some embodiments, the methods generate an output composition of selected and stimulated cells suitable for downstream processing within a set amount of time, such as within or within less than about 6 hours. In some embodiments, the methods generate an output composition of selected and stimulated cells suitable for downstream processing within a set amount of time, such as within or within less than about 5.5 hours. In some embodiments, the methods generate an output composition of selected and stimulated cells suitable for downstream processing within a set amount of time, such as within or within less than about 5 hours. In some embodiments, the methods generate an output composition of selected and stimulated cells suitable for downstream processing within a set amount of time, such as within or within less than about 4.5 hours. In some embodiments, the methods generate an output composition of selected and stimulated cells suitable for downstream processing within a set amount of time, such as within or within less than about 4 hours. In some embodiments, the methods generate an output composition of selected and stimulated cells suitable for downstream processing within a set amount of time, such as within or within less than about 3 hours. In some embodiments, the methods generate an output composition of selected and stimulated cells suitable for downstream processing within a set amount of time, such as within or within less than about 3 to 6 hours. In some embodiments, the methods generate an output composition of selected and stimulated cells suitable for downstream processing within a set amount of time, such as within or within less than about 4 to 6 hours. In some embodiments, the methods generate an output composition of selected and stimulated cells suitable for downstream processing within a set amount of time, such as within or within less than about 5 to 6 hours. In some embodiments, the methods generate an output composition of selected and stimulated cells suitable for downstream processing within a set amount of time, such as within or within less than about 4 to 5 hours. In some embodiments, the methods provided herein generate a composition of engineered T cells (e.g., a therapeutic cell composition) within 5 days. In some embodiments, the methods provided herein generate a composition of engineered T cells (e.g., a therapeutic cell composition) in or in about 4 to 5 days. In some embodiments, the steps provided herein result in a manufacturing process that is or is about 4 or 5 days in length. In some embodiments, the steps provided herein result in a manufacturing process that is about 4 to 5 days in length. In some embodiments, the steps provided herein result in a manufacturing process that is or is about 4 days in length or 96 ± 6 hours in length.

The provided methods include methods for selecting cells, e.g., CD3+, CD4+, and CD8+ T cells, from other components, such as from other cells in a sample, and immobilizing the cells on a stationary phase of a chromatography column; stimulating the selected cells immobilized on the stationary phase; and collecting selected and stimulated cells in the absence of processing steps to detach the cells from the stationary phase and remove agents (e.g., competition agents or free binding agents) used to facilitate said detachment from the output composition of selected and stimulated cells. In particular embodiments, the provided methods include methods for selecting cells, e.g., CD3+, CD4+, and CD8+ T cells, from other components, such as from other cells in a sample, and immobilizing the cells on a stationary phase of a chromatography column; stimulating the selected cells immobilized on the stationary phase; and eluting and/or collecting selected and stimulated cells by gravity flow.

In particular aspects, the provided methods are improved compared to many existing methods for generating engineered cells (e.g. T cells), such as for cell therapy, that include one or more additional steps after cell selection (e.g. immunoaffinity-based selection) prior to stimulating cells. In some embodiments, the one or more additional steps present in existing methods can include an elution step or steps with a competition reagent or free binding agent to recover or collect the selected cells and/or steps to remove reagents used in the selection (e.g. magnetic bead reagents or antibodies). In some embodiments, such additional steps can prolong a process for engineering cells for a cell therapy and/or can result in manipulations of cells during the process that may impact their differentiation state, viability or cell number. In particular aspects, the provided methods generate populations of selected and stimulated cells in a shortened amount of time compared to methods that include separate selecting and stimulating steps and require additional steps to detach cells from the stationary phase and remove agents used to facilitate detachment.

In certain aspects, the methods generate a selected and stimulated cell output population (also referred to as an output composition) suitable for downstream processing (e.g., genetic engineering, expansion, and/or subsequent rounds of incubation, stimulation, and/or selection (e.g., polishing)), within 24 hours of initiating stimulation on the column, also referred to herein as on-column stimulation. In some embodiments, the methods generate a selected and stimulated cell output population (e.g., output composition) suitable for downstream processing (e.g., genetic engineering, expansion, and/or subsequent rounds of incubation, stimulation, and/or selection (e.g., polishing)), within or within about 12, 11, 10, 9, 8, 7, 6, 5, 4, 3, or 2 hours of initiating stimulation on the column. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 6, 5, 4, 3, or 2 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 3 to 6 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 4 to 6 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 5 to 6 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 4 to 5 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 6 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 5.5 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 5 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 4.5 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 4 hours. In some embodiments, the methods generate a selected and stimulated cell output population suitable for downstream processing (e.g., genetic engineering, expansion, and/or subsequent rounds of incubation, stimulation, and/or selection (e.g., polishing)), within or within about 3 hours.

In some embodiments, the methods involve the use of stimulatory agents capable of binding to molecules on the surface of the cells, thereby delivering a stimulatory signal to the cell. In some embodiments, the stimulatory agents are comprised in an oligomeric stimulatory reagent (e.g. a streptavidin mutein oligomer conjugated to anti-CD3 and anti-CD28 Fabs) that can be added to the stationary phase. In some embodiments, the stimulation results in the spontaneous detachment of the selected cells from the stationary phase, thus allowing collection and/or elution of the selected and stimulated cells in the absence of additional processing steps to detach the cells from the stationary phase and remove agents used to facilitate said detachment from the output stimulated cell composition. In some embodiments, the stimulation results in the spontaneous detachment or release of the selected cells from the stationary phase, thus allowing collection and/or elution of the selected and stimulated cells by gravity flow. In some embodiments, gravity flow is relied upon to collect or elute the spontaneously detached cells from the column (e.g., stationary phase). In some embodiments, a wash step, for example in combination with gravity flow, may be used to elute the spontaneously detached cells from the column (e.g., stationary phase). In some embodiments, the wash step can simply include adding cell media (e.g. serum free media) to the column, such as the same media present in the cell input composition prior to adding or immobilizing the cells on the stationary phase. In particular aspects, the methods successfully generate an uncontaminated (e.g., free of agents used for detachment (e.g., competition agents, free binding agents) and/or selection agents) composition of selected and stimulated cells suitable for further processing, e.g., genetic engineering, expansion, incubation, or subsequent rounds of stimulation and/or selection (e.g., polishing), within 24 hours of initiating on-column stimulation. Also provided are articles of manufacture and apparatus thereof

Different methods are available for generating cell populations suitable for use in cell therapy (e.g., selected (enriched) and stimulated cell populations, engineered to express recombinant proteins (e.g., chimeric antigen receptors)). However, in some aspects, these methods may require a long or a relatively long amount of time to generate the cells, at least in part due to the need to perform multiple processing steps. Multiple processing steps may also result in cellular stress, thus affecting the usefulness of the cells in downstream processing. Additional methods for generating cell compositions are needed.

In particular aspects, the provided methods are based on observations that selecting and stimulating target cells (e.g., CD3+, CD4+, or CD8+ T cells) on a stationary phase of a chromatography column, where stimulation facilitates downregulation of the molecule used for cell selection (i.e., selection marker), results in spontaneous detachment of the cell from the stationary phase. In some embodiments, the stationary phase of the chromatography column is functionalized with an agent (e.g., selection agent) capable of specifically binding to a molecule (e.g., selection marker) on a target cell surface. In this way, when combining a sample comprising target cells containing the selection marker (e.g., CD3, CD4, CD8) with the stationary phase (e.g., adding the sample to the stationary phase), target cells (e.g., CD3+, CD4+, CD8+ T cells) are indirectly immobilized to the stationary phase. In particular aspects, the target cells (e.g., T cells) are stimulated while immobilized on the stationary phase (e.g., on-column stimulation), for example, by addition of stimulatory agents, stimulatory reagents comprising stimulatory agents, and/or via stimulatory agents coupled directly or indirectly to the stationary phase. In particular embodiments, the stimulatory agents include agents that activate or stimulate T cells, such as anti-CD3/anti-CD28 antibody (e.g. Fab) agents. Thus, in some aspects, the provided methods and other embodiments are advantageous in that they condense multiple processing steps (e.g., selection and stimulation) and/or eliminate processing steps (e.g., steps for removing selection reagents and/or agents used to facilitate detachment) and allow the condensed process to occur within the same container and/or closed system, which can provide increased efficiency and sterility.

In certain aspects, the methods involve the use of oligomeric stimulatory reagents comprising stimulatory agents capable of delivering a stimulatory signal to a target cell (e.g., T cell). Exemplary oligomeric reagents include streptavidin mutein oligomers that are reversibly bound or conjugated to one or more antibody or fragment thereof capable of delivering a stimulatory signal to a target cell, e.g. a T cell. In some embodiments, the oligomeric stimulatory reagent is a streptavidin mutein oligomer conjugated to anti-CD3 and anti-CD28 Fabs. Existing reagents for use in stimulating T cells in vitro, such as in the absence of exogenous growth factors or low amounts of exogenous growth factors, are known (see e.g. US Patent 6,352,694 B1 and European Patent EP 0 700 430 B1). In general, such reagents may employ beads, e.g., magnetic beads, of greater than 1 µm in diameter to which various binding agents (e.g. anti-CD3 antibody and/or anti-CD28 antibody) are immobilized. However, in some cases, such magnetic beads are, for example, difficult to integrate into methods for stimulating cells under conditions required for clinical trials or therapeutic purposes since it has to be made sure that these magnetic beads are substantially or completely removed before administering the engineered T cells to a subject. In some aspects, such removal, such as by exposing the cells to a magnetic field, may decrease the yield of viable cells available for the cell therapy. In certain cases, such reagents, e.g., stimulatory reagents containing magnetic beads, must be incubated with the cells for a minimal amount of time to allow a sufficient amount of detachment of the T cells from the stimulatory reagent. Furthermore, reagents such as beads are not readily compatible with column chromatography due to physical constraints.

The provided methods utilizing oligomeric stimulatory reagents (e.g. streptavidin mutein oligomer conjugated to anti-CD3 and anti-CD28 antibodies, such as Fabs) overcome such potential limitations. For example, in some embodiments, the provided methods include addition of a soluble oligomeric reagent not bound to a solid support (e.g., bead) to the stationary phase to initiate stimulation. In some embodiments, the provided methods can include steps to reduce or minimize the amount of residual oligomeric stimulatory reagent that may be present at the end of an overall process of engineering cells for a cell therapy. In some embodiments, the risk of residual reagent in output cells, e.g. engineered cells, generated or produced by the methods is reduced or avoided by use of the oligomeric reagent since addition of a competition reagent or free binding agent can be used to dissociate (e.g., disrupt binding) the oligomeric stimulatory reagents from the stimulatory agents in a composition containing the cells. In some embodiments, it also may be sufficient to reduce or remove the oligomeric stimulatory reagent from cells in a composition by one or more washing steps, such as without the need to add a competition reagent or free binding agent, since the oligomeric stimulatory reagent is soluble. In some embodiments, this also means that a process that is compliant with GMP standards can be more easily established compared to other methods, such as those where additional measures have to be taken to ensure that the final population for administration is free of beads. Thus, in some aspects, removal or separation of oligomeric stimulatory reagent from cells, such as by the addition of a competition agent or free binding agent or by one or more washing steps, results in little or no cell loss as compared to removal or separation of bead based stimulatory reagents. In some aspects, the timing of the stimulatory reagent or oligomeric stimulatory reagent reduction, removal or separation is not limited or is less limited than the removal or separation of bead based stimulatory reagents. Thus, in some aspects, the stimulatory reagent or oligomeric stimulatory reagent may be reduced, removed or separated from the cells at any time or step during the provided methods.

Also provided are cells and populations prepared by the methods, including pharmaceutical populations and formulations, and kits, systems, and devices for carrying out the methods. Further provided are methods for use of the cells and populations prepared by the methods, including therapeutic methods, such as methods for adoptive cell therapy, and pharmaceutical populations for administration to subjects.

### A. Samples and Cell Preparation

In particular embodiments, using a device disclosed herein, provided herein are methods, which are not in accordance with the claimed invention, that include selecting and/or enriching cells from a biological sample. In some embodiments, the provided methods include selecting cells or populations thereof from biological samples, such as those obtained from or derived from a subject, such as one having a particular disease or condition or in need of a cell therapy or to which cell therapy will be administered. In some aspects, the subject is a human, such as a subject who is a patient in need of a particular therapeutic intervention, such as the adoptive cell therapy for which cells are being isolated, processed, and/or engineered. Accordingly, the cells in some embodiments are primary cells, e.g., primary human cells. The samples include tissue, fluid, and other samples taken directly from the subject. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the sample is a sample containing T cells. In some embodiments, the sample is a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some embodiments, the sample is an apheresis sample. In some embodiments, the sample is a leukaphresis sample.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca²⁺/Mg²⁺ free PBS. In certain embodiments, components of a blood cell sample are removed and the cells directly resuspended in culture media.

In some embodiments, the sample containing cells (e.g., an apheresis product or a leukapheresis product) is washed in order to remove one or more anti-coagulants, such as heparin, added during apheresis or leukapheresis.

In some embodiments, the sample containing cells (e.g., a whole blood sample, a buffy coat sample, a peripheral blood mononuclear cells (PBMC) sample, an unfractionated T cell sample, a lymphocyte sample, a white blood cell sample, an apheresis product, or a leukapheresis product) is cryopreserved and/or cryoprotected (e.g., frozen) and then thawed prior to any steps for isolating, selecting, activating, stimulating, engineering, transducing, transfecting, incubating, culturing, harvesting, formulating a population of the cells, and/or administering the formulated cell population to a subject.

In particular embodiments, an apheresis product or a leukapheresis product is cryopreserved and/or cryoprotected (e.g., frozen) and then thawed before being subject to a cell selection or isolation step (e.g., a T cell selection or isolation step) as described *infra.* In some embodiments, the thawed cell composition is subjected to dilution (e.g., with a serum-free medium) and/or wash (e.g., with a serum-free medium), which in some cases can remove or reduce unwanted or undesired components. In some cases, the dilution and/or wash removes or reduces the presence of a cryoprotectant, e.g. DMSO, contained in the thawed sample, which otherwise may negatively impact cellular viability, yield, recovery upon extended room temperature exposure. In some embodiments, the dilution and/or wash allows media exchange of a thawed cryopreserved product into a serum-free medium, such as one described herein or in PCT/US2018/064627.

In some embodiments, the serum-free medium comprises a basal medium (e.g.OpTmizer^{™} T-Cell Expansion Basal Medium (ThermoFisher), supplemented with one or more supplement. In some embodiments, the one or more supplement is serum-free. In some embodiments, the serum-free medium comprises a basal medium supplemented with one or more additional components for the maintenance, expansion, and/or activation of a cell (e.g., a T cell), such as provided by an additional supplement (e.g. OpTmizer^{™} T-Cell Expansion Supplement (ThermoFisher)). In some embodiments, the serum-free medium further comprises a serum replacement supplement, for example, an immune cell serum replacement, e.g., ThermoFisher, #A2596101, the CTS^{™} Immune Cell Serum Replacement, or the immune cell serum replacement described in Smith et al. Clin Transl Immunology. 2015 Jan; 4(1): e31. In some embodiments, the serum-free medium further comprises a free form of an amino acid such as L-glutamine. In some embodiments, the serum-free medium further comprises a dipeptide form of L-glutamine (e.g., L-alanyl-L-glutamine), such as the dipeptide in Glutamax^{™} (ThermoFisher). In some embodiments, the serum-free medium further comprises one or more recombinant cytokines, such as recombinant human IL-2, recombinant human IL-7, and/or recombinant human IL-15.

In some embodiments, after a cryopreserved and/or cryoprotected apheresis product or leukapheresis product is subject to a T cell selection or isolation step, no additional cryopreservation and/or cryoprotection step is performed during or between any of the subsequent steps, such as the steps of activating, stimulating, engineering, transducing, transfecting, incubating, culturing, harvesting, formulating a population of the cells, and/or administering the formulated cell population to a subject. For example, T cells selected from a thawed cryopreserved and/or cryoprotected apheresis product or leukapheresis product are not again cryopreserved and/or cryoprotected before being thawed for a downstream process, such as transduction.

In particular embodiments, the cryopreserved and/or cryoprotected apheresis product or leukapheresis product is banked (e.g., without cell selection before freezing the sample), which, in some aspects, can allow more flexibility for subsequent manufacturing steps. In one aspect, banking cells before selection increases cell yields for a downstream process, and banking cells earlier may mean they are healthier and may be easier to meet manufacturing success criteria. In another aspect, once thawed, the cryopreserved and/or cryoprotected apheresis product or leukapheresis product can be subject to one or more different selection methods. Advantages of this approach are, among other things, to enhance the availability, efficacy, and/or other aspects of cells of a cell therapy for treatment of a disease or condition of a subject, such as in the donor of the sample and/or another recipient.

In some embodiments, the sample (e.g. apheresis or leukapheresis sample) is collected and cryopreserved and/or cryoprotected prior to or without prior cell selection (e.g., without prior T cell selection, such as selection by chromatography), at a time after the donor is diagnosed with a disease or condition. In some aspects, the time of cryopreservation also is before the donor has received one or more of the following: any initial treatment for the disease or condition, any targeted treatment or any treatment labeled for treatment for the disease or condition, or any treatment other than radiation and/or chemotherapy. In some embodiments, the sample is collected after a first relapse of a disease following initial treatment for the disease, and before the donor or subject receives subsequent treatment for the disease. The initial and/or subsequent treatments may be a therapy other than a cell therapy. In some embodiments, the collected cells may be used in a cell therapy following initial and/or subsequent treatments. In one aspect, the cryopreserved and/or cryoprotected sample without prior cell selection may help reduce up-front costs, such as those associated with non-treatment patients in a randomized clinic trial who may crossover and require treatment later.

In some embodiments, the sample (e.g. apheresis or leukapheresis sample) is collected and cryopreserved and/or cryoprotected prior to or without prior cell selection (e.g., without prior T cell selection, such as selection by chromatography), at a time after a second relapse of a disease following a second line of treatment for the disease, and before the donor or subject receives subsequent treatment for the disease. In some embodiments, patients are identified as being likely to relapse after a second line of treatment, for example, by assessing certain risk factors. In some embodiments, the risk factors are based on disease type and/or genetics, such as double-hit lymphoma, primary refractory cancer, or activated B-cell lymphoma. In some embodiments, the risk factors are based on clinical presentation, such as early relapse after first-line treatment, or other poor prognostic indicators after treatment (e.g., IPI (International Prognostic Index) > 2).

In some embodiments, the sample (e.g. apheresis or leukapheresis sample) is collected and cryopreserved and/or cryoprotected prior to or without prior cell selection (e.g., without prior T cell selection, such as selection by chromatography), at a time before the donor or subject is diagnosed with a disease. In some aspects, the donor or subject may be determined to be at risk for developing a disease. In some aspects, the donor or subject may be a healthy subject. In certain cases, the donor or subject may elect to bank or store cells without being deemed at risk for developing a disease or being diagnosed with a disease in the event that cell therapy is required at a later stage in life. In some embodiments, a donor or subject may be deemed at risk for developing a disease based on factors such as genetic mutations, genetic abnormalities, genetic disruptions, family history, protein abnormalities (such as deficiencies with protein production and/or processing), and lifestyle choices that may increase the risk of developing a disease. In some embodiments, the cells are collected as a prophylactic.

In some embodiments, the cryopreserved and/or cryoprotected sample of cells (e.g. apheresis or leukapheresis sample), such as a sample of cells that has not been subjected to a prior cell selection (e.g., without prior T cell selection, such as selection by chromatography) is stored, or banked, for a period of time greater than or equal to 12 hours, 24 hours, 36 hours, or 48 hours. In some embodiments, the sample is stored or banked for a period of time greater than or equal to 1 week, 2 weeks, 3 weeks, or 4 weeks. In some embodiments, the sample is placed into long-term storage or long-term banking. In some aspects, the sample is stored for a period of time greater than or equal to 1 month, 2 months, 3 months, 4 months, 5 months, 6 months, 7 months, 8 months, 9 months, 10 months, 1 1 months, 1 year, 2 years, 3 years, 4 years, 5 years, 6 years, 7 years, 8 years, 9 years, 10 years, 1 1 years, 12 years, 13 years, 14 years, 15 years, 16 years, 17 years, 18 years, 19 years, 20 years, 25 years, 30 years, 35 years, 40 years, or more.

In some embodiments, an apheresis or leukapheresis sample taken from a donor is shipped in a cooled environment to a storage or processing facility, and/or cryogenically stored at the storage facility or processed at the processing facility. In some embodiments, before shipping, the sample is processed, for example, by selecting T cells, such as CD4+ and/or CD8+ T cells. In some embodiments, such processing is performed after shipping and before cryogenically storing the sample. In some embodiments, the processing is performed after thawing the sample following cryogenical storage.

By allowing donors to store their cells at a stage when the donors, and thus their cells, have not undergone extensive treatment for a disease and/or prior to contracting of a disease or condition or diagnosis thereof, such cells may have certain advantages for use in cell therapy compared to cells harvested after one or after multiple rounds of treatment. For example, cells harvested before one or more rounds of treatment may be healthier, may exhibit higher levels of certain cellular activities, may grow more rapidly, and/or may be more receptive to genetic manipulation than cells that have undergone several rounds of treatment. Another example of an advantage according to embodiments described herein may include convenience. For example, by collecting, optionally processing, and storing a donor's cells before they are needed for cell therapy, the cells would be readily available if and when a recipient later needs them. This could increase apheresis lab capacity, providing technicians with greater flexibility for scheduling the apheresis collection process.

Exemplary methods and systems for cryogenic storage and processing of cells from a sample, such as an apheresis sample, can include those described in International published application no. WO2018170188. In some embodiments, the method and systems involve collecting apheresis before the patient needs cell therapy, and then subjecting the apheresis sample to cryopreservation for later use in a process for engineering the cells, e.g. T cells, with a recombinant receptor (e.g. CAR). In some cases, such processes can include those described herein. In some embodiments, an apheresis sample is collected from a subject and cryopreserved prior to subsequent T cell selection, activation, stimulation, engineering, transduction, transfection, incubation, culturing, harvest, formulation of a population of the cells, and/or administration of the formulated cell population to a subject. In such examples, the cryopreserved apheresis sample is thawed prior to subjecting the sample to one or more selection steps, such as any as described herein.

In some embodiments, the cryopreserved and/or cryoprotected sample of cells (e.g. apheresis or leukapheresis sample), such as a sample of cells that has not been subject to a prior cell selection (e.g., without prior T cell selection, such as selection by chromatography) is thawed prior to its use for downstream processes for manufacture of a cell population for cell therapy, for example, a T cell population containing CAR+ T cells. In some embodiments, such a cryopreserved and/or cryoprotected sample of cells (e.g. apheresis or leukapheresis sample) is used in connection with the process provided herein for engineering a T cell therapy, such as a CAR+ T cell therapy. In particular examples, no further step of cryopreservation is carried out prior to or during the harvest/formulation steps.

### B. Agent and Reagent Systems

In embodiments, using a device disclosed herein, provided herein are methods, which are not in accordance with the claimed invention, that include selecting and/or enriching cells (e.g. T cells) from a biological sample using an agent that binds to a cell surface markers on cells present in a biological sample (selection agent). In provided embodiments, the biological sample is any as described in Section II.A. In some embodiments, the biological sample is a sample that contains T cells. In provided embodiments, the selection agent is bound or immobilized on a chromatography matrix (e.g. stationary phase) contained in a chromatography column of a device provided herein, and effects specific selection of target cells (e.g. T cells) of interest, as described in Section II.C, thereby immobilizing the target cells (e.g. T cells) to the chromatography matrix (e.g. stationary phase). In some embodiments, the selection agent is capable of being bound indirectly to the chromatography matrix (e.g., stationary phase) through a reagent, e.g., selection reagent. In some embodiments, the selection reagent is bound covalently or non-covalently to the stationary phase of the column. In some embodiments, the selection reagent is a reagent that reversibly immobilizs the selection agent on the chromatography matrix (e.g., stationary phase). Exemplary selection reagents to which a selection agent is bound for use in connection with the provided devices and methods are described in Secion II.B.2.

In some embodiments, the selection reagent to which the selection agent is bound provides a reversible system in which the selection agent is reversibly associated with the reagent. Exemplary reversible systems for selection of cells by chromatography include those described in WO2013/124474. In some embodiments as described further herein, the reversible system employs a reagent composed of streptavidin mutein molecules that reversibly bind to the selection agent via a streptavidin-binding peptide binding partner contained by the selection agent. In some embodiments, adding a free binding partner or competition agent (also called competition substance) disrupts the binding between the selection agent and the reagent, thereby reversing binding of the selection agent from the reagent and releasing the immobilized cells free from the selection reagent. For instance, in the case of a streptavidin mutein/streptavidin binding peptide system an exemplary compeitition agent is biotin or a biotin analog (e.g. D-Biotin).

In some embodiments, reversibility of the binding of the selection agent on the chromatography matrix is not necessary, since on-column stimulation of cells immobilized on the chromatography matrix as provided herein facilitates downregulation of the molecule used for cell selection (i.e., selection marker), resulting in spontaneous detachment or release of the cell from the stationary phase. Thus, the release or detachment of the cells can occur without any additional steps or reagents. In some aspects, the cells can be collected by gravity flow, such as by adding a media or other solution to the chromatography column. In particular embodiments, the media or other solution that is added does not contain a competition agent or free binding agent to facilitate detachment of the cells from the stationary phase. For instance, in the case of a streptavidin mutein/streptavidin binding peptide system, the release or detachment of cells can occur spontaneously such that the cells can be collected by gravity flow after adding a wash or media to the column in which the wash solution or media does not contain a free binding partner or compeitition agent, such as biotin or a biotin analog (e.g. D-Biotin).

In embodiments, using a device disclosed herein, provided herein are methods, which are not in accordance with the claimed invention, that include on-column stimulation of cells (e.g. T cells) immobilized on the chromatography column, such as by the selection agent or selection reagent. In provided embodiments, the stimulation is carried out using one or more agent for stimulating cells to bind to one or more receptor molecule on the cell to deliver a signal to cells (one or more stimulatory agent). In some embodiment, the one or more stimulatory agent is for stimulating T cells and provides a primary signal to the T cells (e.g. via TCR complex signaling) and a costimulatory signal to the T cells (e.g. via signaling from a costimulatory receptor). In some embodiments, the selection agent and at least one of the one or more stimulating agents are different. In some embodiments, the selection agent and each of the one or more stimulating agents are different. In some embodiments, an agent may be used both as a selection agent and as one of the one or more stimulating agent in connection with the provided methods. In some embodiments, the one or more stimulatory agent are bound on a reagent that delivers the stimulatory signal to the cells (e.g. stimulatory reagent). In some embodiments, the reagent contains a plurality of binding sites for binding each of the one or more stimulatory agent such that the stimulatory agents are multimerized on the agent. In particular embodiments, such a stimulatory reagent is an oligomeric or polymeric reagent made up of multiple individual molecules, such as multiple protein units or complexes (e.g. tetramers). Exemplary stimulatory reagents to which the one or more stimulatory agents are bound, including oligomeric stimulatory reagents, for use in connection with the provided devices and methods are described in Secion II.B.2. In particular embodiments, the stimulatory reagent is added to the chromatography column containing the immobilized cells under conditions suitable for delivering a signal in the cells. For instance, the on-column stimulation is carried out at appropriate temperatures as described herein by heating the device as described and provided herein to a physiologic temperature appropriate to permit cellular signaling events in the cells, such as a temperate of at or about between 30 °C and at or about 39 °C, for example at or about 37 °C ± 2 °C, such as at or about 37 °C.

In some embodiments, the stimulatory reagent to which the one or more stimulatory agent are bound provides a reversible system in which the one or more stimulatory agent are reversibly associated with the ewagent. Exemplary reversible systems for stimulation of cells include those described in WO2015/158868, WO2017068421, or WO2018/197949. In some embodiments, the reversible system employs a reagent composed of oligomers or polymers of a streptavidin mutein that reversibly bind to the one or more stimulatory agent via a streptavidin-binding peptide binding partner contained by the one or more stimulatory agent. In some embodiments, adding a free binding partner or competition agent (also called competition substance) disrupts the binding between the one or more stimulatory agent and the reagent, thereby reversing binding of the one or more stimulatory agent from the reagent and terminating or disrupting the stimulatory signal delivered by the one or more stimulatory agents of the stimulatory reagent. For instance, in the case of a streptavidin mutein/streptavidin binding peptide system an exemplary compeitition agent is biotin or a biotin analog (e.g. D-Biotin).

In particular aspects, using a device disclosed herein, provided herein are methods, which are not in accordance with the claimed invention, that employ reversible systems in which at least one agent (e.g., a selection agent or stimulatory agent) capable of binding to a molecule on the surface of a cell (cell surface molecule), is reversibly associated with a reagent (e.g., selection reagent or stimulatory reagent). In some cases, the reagent contains a plurality of binding sites capable of reversibly binding to the agent (e.g., a selection agent or stimulatory agent). In some cases, the reagent (e.g., selection reagent or stimulatory reagent) is a multimerization reagent. In some embodiments, the at least one agent *(e.g.,* a selection agent or stimulatory agent) contains at least one binding site B that can specifically bind an epitope or region of the molecule and also contains a binding partner C that specifically binds to at least one binding site Z of the reagent (e.g., selection reagent or stimulatory reagent). In some cases, the binding interaction between the binding partner C and the at least one binding site Z is a non-covalent interaction. In some embodiments, the binding interaction, such as non-covalent interaction, between the binding partner C and the at least one binding site Z is reversible.

In some embodiments, the reversible association can be mediated in the presence of a substance, such as a competition agent or free binding agent, that is or contains a binding site that also is able to bind to the at least one binding site Z. Generally, the substance (e.g. competition agent or free binding agent) can act as a competitor due to a higher binding affinity for the binding site Z present in the reagent and/or due to being present at higher concentrations than the binding partner C, thereby detaching and/or dissociating the binding partner C from the reagent. In some embodiments, the affinity of the substance (e.g. competition agent or free binding agent) for the at least one binding site Z is greater than the affinity of the binding partner C of the agent *(e.g.,* a selection agent or stimulatory agent) for the at least one binding site Z. Thus, in some cases, the bond between the binding site Z of the reagent and the binding partner C of the agent *(e.g.,* a selection agent or stimulatory agent) can be disrupted by addition of the substance (e.g. competition agent or free binding partner), thereby rendering the association of the agent *(e.g.,* a selection agent or stimulatory agent) and reagent (e.g., selection reagent or stimulatory reagent) reversible.

Reagents that can be used in such reversible systems are described and known in the art, see *e.g.,* U.S. Patent Nos. 5,168,049; 5,506,121; 6,103,493; 7,776,562; 7,981,632; 8,298,782; 8,735,540; 9,023,604; and International published PCT Appl. Nos. WO2013/124474 and WO2014/076277. Non-limiting examples of reagents and binding partners capable of forming a reversible interaction, as well as substances (*e.g*. competition agents or free binding agents) capable of reversing such binding, are described below.

### 1. Agents

In some embodiments, the agent (*e.g.*, selection agent or stimulatory agent) has one or more binding sites, B, for binding to the molecule on the surface of the cell, *e.g.* cell surface molecule. Thus, in some instances, the agent (*e.g*., selection agent or stimulatory agent) contains a binding site B or a plurality of binding sites B, wherein the specific binding between the agent (*e.g*., selection agent or stimulatory agent) and the molecule on the surface of the target cells contains interaction between B and the molecule. In some embodiments, the agent contains only a single binding site, *i.e.* is monovalent. In some embodiments, the agent (*e*.*g*., selection agent or stimulatory agent) has at least two, such as a plurality of binding sites B including three, four or five binding sites B capable of binding to the cell surface molecule. In some such aspects, the at least two or plurality of binding sites B may be identical. In some embodiments, one or more of the at least two or plurality of binding sites B may be different (*e.g*. B1 and B2).

In some embodiments, one or more different agents (*e.g*. one or more different *e.g*., selection agent or stimulatory agent or other agent that binds to a molecule on a cell) are reversibly bound to the reagent (e.g., selection reagent or stimulatory reagent). In some embodiments, at least 2, 3, 4 or more different agents (*e.g*., selection agents or stimulatory agents) are reversibly bound to the same reagent. In some embodiments, at least two different agents (*e.g*., selection agent or stimulatory agents) are reversibly bound to the same reagent, whereby each agent comprises a binding site B or a plurality of binding sites B for specific binding between the agent and the molecule. In some embodiments, the at least two or more agents (*e.g*., selection agent or stimulatory agents) contain the same binding site B, *e.g.* for the binding the same or substantially the same molecule. In some embodiments, the at least two or more agents (*e.g*., selection agents or stimulatory agents) contain different binding sites B, *e.g.* for the binding to different molecules. In some embodiments, a first agent (*e.g.,* a first selection agent or first stimulatory agent) contains a binding site B1, B2, B3, B4, etc. and a second agent (*e.g.,* second selection agent or second stimulatory agent) contains another of a binding site B1, B2, B3, B4, etc. In some embodiments, a first agent (*e.g.* a first selection agent) contains a binding site B1 and a second agent (*e.g.* second selection agent) contains a binding site B3. In some embodiments, a first agent (*e.g.* a first stimulatory agent) contains a binding site B2 and a second agent (*e.g.* a second stimulatory agent) contains a binding site B4. In any of such embodiments, the first agent and second agent can contain a binding partner, C1 or C2. In some embodiments, C1 and C2 can be the same. In some embodiments, C1 and C2 are different. In some embodiments, the first agent and second agent contain the same binding partner, C1.

In some cases, the dissociation constant (K_{D}) of the binding between the agent (e.g., via the binding site B) and the binding site Z of the reagent may have a value in the range from about 10⁻² M to about 10⁻¹³ M or from about 10⁻³ M to about 10⁻¹² M or from about 10⁻⁴ M to about 10⁻¹¹M, or from about 10⁻⁵M to about 10⁻¹⁰M. In some embodiments, the dissociation constant (K_{D}) for the binding between the binding agent and the molecule is of low affinity, for example, in the range of a K_{D} of about 10⁻³ to about 10⁻⁷ M. In some embodiments, the dissociation constant (K_{D}) for the binding between the binding agent and the molecule is of high affinity, for example, in the range of a K_{D} of about 10⁻⁷ to about 1×10⁻¹⁰ M.

In some embodiments, the dissociation of the binding of the agent via the binding site B and the molecule occurs sufficiently fast, for example, to allow the target cell to be only transiently stained or associated with the agent after disruption of the reversible bond between the reagent and the agent. In some cases, when expressed in terms of the k_{off} rate (also called dissociation rate constant for the binding between the agent (via the binding site B) and the molecule, the k_{off} rate is about 0.5×10⁻⁴ sec⁻¹ or greater, about 1×10⁻⁴ sec⁻¹ or greater, about 2×10⁻⁴ sec⁻¹ or greater, about 3 × 10⁻⁴ sec⁻¹ or greater, about 4×10⁻⁴ sec⁻¹ of greater, about 5×10⁻⁴ sec⁻¹ or greater, about 1×10⁻³ sec⁻¹ or greater, about 1.5×10⁻³ sec⁻¹ or greater, about 2×10⁻³ sec⁻¹ or greater, about 3×10⁻³ sec⁻¹ or greater, about 4×10⁻³ sec⁻¹, about 5×10⁻³ sec⁻¹ or greater, about 1×10⁻² sec or greater, or about 5×10⁻¹ sec⁻¹ or greater. It is within the level of a skilled artisan to empirically determine the k_{off} rate range suitable for a particular agent and cell molecule interaction (see *e.g.* U.S. published application No. US2014/0295458). For example, an agent with a rather high k_{off} rate of, for example, greater than 4.0×10⁻⁴ sec⁻¹ may be used so that, after the disruption of the binding complexes, most of the agent can be removed or dissociated within one hour. In other cases, an agent with a lower k_{off} rate of, for example, 1.0×10⁻⁴ sec⁻¹, may be used, so that after the disruption of the binding complexes, most of the agent may be removed or dissociated from the cell within about 3 and a half hours.

In some embodiments, the K_{D} of this bond as well as the K_{D}, k_{off} and kₒₙ rate of the bond formed between the binding site B of the agent (*e.g*., *e.g.,* selection agent or stimulatory agent) and the cell surface molecule can be determined by any suitable means, for example, by fluorescence titration, equilibrium dialysis or surface plasmon resonance.

In some aspects, the cell surface molecule is a molecule against which an agent *(e.g.,* selection agent or stimulatory agent) may be directed. In some embodiments, the cell surface molecule is a peptide or a protein, such as a receptor, *e.g.,* a membrane receptor protein. In some embodiments, the receptor is a lipid, a polysaccharide or a nucleic acid. In some embodiments, a cell surface molecule that is a protein may be a peripheral membrane protein or an integral membrane protein. The cell surface molecule may in some embodiments have one or more domains that span the membrane. As a few illustrative examples, a membrane protein with a transmembrane domain may be a G-protein coupled receptor, such as an odorant receptors, a rhodopsin receptor, a rhodopsin pheromone receptor, a peptide hormone receptor, a taste receptor, a GABA receptor, an opiate receptor, a serotonin receptor, a Ca2+ receptor, melanopsin, a neurotransmitter receptor, such as a ligand gated, a voltage gated or a mechanically gated receptor, including the acetylcholine, the nicotinic, the adrenergic, the norepinephrine, the catecholamines, the L-DOPA-, a dopamine and serotonin (biogenic amine, endorphin/enkephalin) neuropeptide receptor, a receptor kinase such as serine/threonine kinase, a tyrosine kinase, a porin/channel such as a chloride channel, a potassium channel, a sodium channel, an OMP protein, an ABC transporter (ATP-Binding Cassette-Transporter) such as amino acid transporter, the Na-glucose transporter, the Na/iodide transporter, an ion transporter such as Light Harvesting Complex, cytochrome c oxidase, ATPase Na/K, H/K, Ca, a cell adhesion receptor such as metalloprotease, an integrin or a catherin.

In some embodiments, the cell surface molecule may be an antigen defining a desired cell population or subpopulation, for instance a population or subpopulation of blood cells, *e.g.,* lymphocytes *(e.g.,* T cells, T-helper cells, for example, CD4+ T-helper cells, B cells or natural killer cells), monocytes, or stem cells, *e.g.* CD34-positive peripheral stem cells or Nanog or Oct-4 expressing stem cells. Examples of T-cells include cells such as CMV-specific CD8+ T-lymphocytes, cytotoxic T-cells, memory T-cells and regulatory T-cells (Treg). An illustrative example of Treg is CD4 CD25 CD45RA Treg cells and an illustrative example of memory T-cells is CD62L CD8+ specific central memory T-cells. The cell surface molecule may also be a marker for a tumor cell.

As described above, in some embodiments, the agent (*e.g.,* selection agent or stimulatory agent) has, in addition to the binding site B that is able to bind the cell surface molecule, a binding partner C. In some aspects, this binding partner C is able to bind to a binding site Z of the reagent (e.g., selection reagent or stimulatory reagent (e.g., oligomeric stimulatory reagent)) wherein the reagent has one or more binding sites for the binding partner C. In some embodiments, the non-covalent bond that may be formed between the binding partner C that is included in the agent (*e.g.,* selection agent or stimulatory agent) and the binding site(s) Z of the reagent (e.g., selection reagent or stimulatory reagent (e.g., oligomeric stimulatory reagent)) may be of any desired strength and affinity, and may be disruptable or reversible under conditions under which the method is performed. The agent (*e.g.,* receptor-binding agent or selection agent) may include at least one, including two, three or more, additional binding partners C and the reagent (e.g., selection reagent or stimulatory reagent (e.g., oligomeric stimulatory reagent)) may include at least two, such as three, four, five, six, seven, eight or more binding sites Z for the binding partner C that is included in the agent (*e.g.,* selection agent or stimulatory agent). As described in US patent 7,776,562, US patent 8,298,782 or International Patent application WO 2002/054065, any combination of a binding partner C and a reagent with one or more corresponding binding sites Z can be chosen, for example, such that the binding partner C and the binding site Z are able to reversibly bind in a complex, such as to cause an avidity effect.

The binding partner C included in the agent (*e.g.,* selection agent or stimulatory agent) may for instance be hydrocarbon-based (including polymeric) and include nitrogen-, phosphorus-, sulphur-, carben-, halogen- or pseudohalogen groups. In some aspects, it may be an alcohol, an organic acid, an inorganic acid, an amine, a phosphine, a thiol, a disulfide, an alkane, an amino acid, a peptide, an oligopeptide, a polypeptide, a protein, a nucleic acid, a lipid, a saccharide, an oligosaccharide, or a polysaccharide. As further examples, it may also be a cation, an anion, a polycation, a polyanion, a polycation, an electrolyte, a polyelectrolyte, a carbon nanotube or carbon nanofoam. Generally, such a binding partner C has a higher affinity to the binding site of the reagent than to other matter. Examples of a respective binding partner C include, but are not limited to, a crown ether, an immunoglobulin, a fragment thereof and a proteinaceous binding molecule with antibody-like functions.

In some embodiments, the binding partner C that is included in the agent (*e.g.,* selection agent or stimulatory agent) includes biotin and the reagent includes a streptavidin analog or an avidin analog that reversibly binds to biotin. In some embodiments, the binding partner C that is included in the agent (*e.g.,* selection agent or stimulatory agent) includes a biotin analog that reversibly binds to streptavidin or avidin, and the reagent includes streptavidin, avidin, a streptavidin analog or an avidin analog that reversibly binds to the respective biotin analog. In some embodiments, the binding partner C that is included in the agent (*e.g.,* selection agent or stimulatory agent) includes a streptavidin or avidin binding peptide and the reagent includes streptavidin, avidin, a streptavidin analog or an avidin analog that reversibly binds to the respective streptavidin or avidin binding peptide. For purposes herein, the term analog is used interchangeably with the term mutein in reference to a mutant form of a streptavidin (e.g. streptavidin analog or streptavidin mutein) or an avidin (e.g. avidin analog or avidin mutein).

In some embodiments, the reagent (e.g., selection reagent or stimulatory reagent) is or contains a streptavidin, such as a streptavidin mutein including any described above (*e.g.* set forth in SEQ ID NOS: 3-6), and the binding partner C that is included in the agent (*e.g.,* selection agent or stimulatory agent) may include a streptavidin-binding peptide. In some embodiments, the streptavidin-binding peptide may include a sequence with the general formula set forth in SEQ ID NO: 9, such as contains the sequence set forth in SEQ ID NO: 10. In some embodiments, the streptavidin-binding peptide sequence has the general formula set forth in SEQ ID NO: 11, such as set forth in SEQ ID NO: 12. In one example, the streptavidin-binding peptide sequence is Trp-Arg-His-Pro-Gln-Phe-Gly-Gly (also called Strep-tag^{®}, set forth in SEQ ID NO: 7). In one example, the streptavidin-binding peptide sequence is Trp-Ser-His-Pro-Gln-Phe-Glu-Lys (also called Strep-tag^{®} II, set forth in SEQ ID NO: 8). In some embodiments, the streptavidin-binding peptide ligand contains a sequential arrangement of at least two streptavidin-binding modules, wherein the distance between the two modules is at least 0 and not greater than 50 amino acids, wherein one binding module has 3 to 8 amino acids and contains at least the sequence His-Pro-Xaa (SEQ ID NO: 9), where Xaa is glutamine, asparagine, or methionine, and wherein the other binding module has the same or different streptavidin peptide ligand, such as set forth in SEQ ID NO: 11 (see *e.g.* International Published PCT Appl. No. WO02/077018; U.S. Patent No. 7,981,632). In some embodiments, the streptavidin-binding peptide ligand contains a sequence having the formula set forth in any of SEQ ID NO: 13 or 14. In some embodiments, the streptavidin-binding peptide ligand has the sequence of amino acids set forth in any of SEQ ID NOS: 15-19. In most cases, all these streptavidin binding peptides bind to the same binding site, namely the biotin binding site of streptavidin. If one or more of such streptavidin binding peptides is used as binding partners C, *e.g.* C1 and C2, the multimerization reagent is typically a streptavidin mutein.

In some embodiments, the streptavidin-binding peptide may be further modified. In some embodiments, the streptavidin-binding peptide may include the peptide sequence is Trp-Ser-His-Pro-Gln-Phe-Glu-Lys (also called Strep-tag^{®} II, set forth in SEQ ID NO: 8) conjugated with a nickel charged trisNTA (also called His-STREPPER or His/Strep-tag^{®}II Adapter).

In some embodiments, the binding partner C of the agent (*e.g.,* receptor-binding agent or selection agent) includes a moiety known to the skilled artisan as an affinity tag. In such an embodiment, the reagent may include a corresponding binding partner, for example, an antibody or an antibody fragment, known to bind to the affinity tag. As a few illustrative examples of known affinity tags, the binding partner C that is included in the agent (*e.g.,* selection agent or stimulatory agent) may include dinitrophenol or digoxigenin, oligohistidine, polyhistidine, an immunoglobulin domain, maltose-binding protein, glutathione-S-transferase (GST), chitin binding protein (CBP) or thioredoxin, calmodulin binding peptide (CBP), FLAG '-peptide, the HA-tag (sequence: Tyr-Pro-Tyr-Asp-Val-Pro-Asp-Tyr-Ala) (SEQ ID NO: 20), the VSV-G-tag (sequence: Tyr-Thr-Asp-Ile-Glu-Met-Asn-Arg-Leu-Gly-Lys) (SEQ ID NO: 21), the HSV-tag (sequence: Gln-Pro-Glu-Leu-Ala-Pro-Glu-Asp-Pro-Glu-Asp) (SEQ ID NO: 22), the T7 epitope (Ala-Ser-Met-Thr-Gly-Gly-Gln-Gln-Met-Gly) (SEQ ID NO: 23), maltose binding protein (MBP), the HSV epitope of the sequence Gln-Pro-Glu-Leu-Ala-Pro-Glu-Asp-Pro-Glu-Asp (SEQ ID NO: 24) of herpes simplex virus glycoprotein D, the "myc" epitope of the transcription factor c-myc of the sequence Glu-Gln-Lys-Leu-Ile-Ser-Glu-Glu-Asp-Leu (SEQ ID NO: 25), the V5-tag (sequence: Gly-Lys-Pro-Ile-Pro-Asn-Pro-Leu-Leu-Gly-Leu-Asp-Ser-Thr) (SEQ ID NO: 26), or glutathione-S-transferase (GST). In such embodiments, the complex formed between the one or more binding sites Z of the reagent which may be an antibody or antibody fragment, and the antigen can be disrupted competitively by adding the free antigen, *i.e.* the free peptide (epitope tag) or the free protein (such as MBP or CBP). In some embodiments, the affinity tag might also be an oligonucleotide tag. In some cases, such an oligonucleotide tag may, for instance, be used to hybridize to an oligonucleotide with a complementary sequence, linked to or included in the reagent.

Further examples of a suitable binding partner C include, but are not limited to, a lectin, protein A, protein G, a metal, a metal ion, nitrilo triacetic acid derivatives (NT A), RGD-motifs, a dextrane, polyethyleneimine (PEI), a redox polymer, a glycoproteins, an aptamers, a dye, amylose, maltose, cellulose, chitin, glutathione, calmodulin, gelatine, polymyxin, heparin, NAD, NADP, lysine, arginine, benzamidine, poly U, or oligo-dT. Lectins such as Concavalin A are known to bind to polysaccharides and glycosylated proteins. An illustrative example of a dye is a triazine dye such as Cibacron blue F3G-A (CB) or Red HE-3B, which specifically bind NADH-dependent enzymes. Typically, Green A binds to Co A proteins, human serum albumin, and dehydrogenases. In some cases, the dyes 7-aminoactinomycin D and 4',6-diamidino-2-phenylindole bind to DNA. Generally, cations of metals such as Ni, Cd, Zn, Co, or Cu, are typically used to bind affinity tags such as an oligohistidine containing sequence, including the hexahistidine or the His-Asn-His-Arg-His-Lys-His-Gly-Gly-Gly-Cys tag (MAT tag) (SEQ ID NO: 35), and N-methacryloyl-(L)-cysteine methyl ester.

In some embodiments, the binding between the binding partner C that is included in the agent (*e.g.,* selection agent or stimulatory agent) and the one or more binding sites Z of the reagent occurs in the presence of a divalent, a trivalent or a tetravalent cation. In this regard, in some embodiments, the reagent includes a divalent, a trivalent or a tetravalent cation, typically held, *e.g.* complexed, by means of a suitable chelator. In some embodiments, the binding partner C that is included in the agent (*e.g.,* selection agent or stimulatory agent) may include a moiety that includes, *e.g.* complexes, a divalent, a trivalent or a tetravalent cation. Examples of a respective metal chelator, include, but are not limited to, ethylenediamine, ethylene-diaminetetraacetic acid (EDTA), ethylene glycol tetraacetic acid (EGTA), diethylenetri-aminepentaacetic acid (DTPA), N,N-bis(carboxymethyl)glycine (also called nitrilotriacetic acid, NTA), 1,2-bis(o-aminophenoxy)ethane-N,N,N',N'-tetraacetic acid (BAPTA), 2,3-dimer-capto-1-propanol (dimercaprol), porphine and heme. As an example, EDTA forms a complex with most monovalent, divalent, trivalent and tetravalent metal ions, such as *e.g.* silver (Ag⁺), calcium (Ca²⁺), manganese (Mn²⁺), copper (Cu²⁺), iron (Fe²⁺), cobalt (Co ⁺) and zirconium (Zr⁴⁺), while BAPTA is specific for Ca²⁺. As an illustrative example, a standard method used in the art is the formation of a complex between an oligohistidine tag and copper (Cu²⁺), nickel (Ni²⁺), cobalt (Co²⁺), or zinc (Zn²⁺) ions, which are presented by means of the chelator nitrilotriacetic acid (NTA).

In some embodiments, the binding partner C that is included in the agent (*e.g.,* selection agent or stimulatory agent) includes a calmodulin binding peptide and the reagent includes multimeric calmodulin as described in US Patent 5,985,658, for example. In some embodiments, the binding partner C that is included in the agent (*e.g.,* selection agent or stimulatory agent) includes a FLAG peptide and the reagent includes an antibody that binds to the FLAG peptide, *e.g.* the FLAG peptide, which binds to the monoclonal antibody 4E11 as described in US Patent 4,851,341. In one embodiment, the binding partner C that is included in the agent (*e.g.,* selection agent or stimulatory agent) includes an oligohistidine tag and the reagent includes an antibody or a transition metal ion binding the oligohistidine tag. In some cases, the disruption of all these binding complexes may be accomplished by metal ion chelation, *e.g.* calcium chelation, for instance by adding EDTA or EGTA. In some embodiments, calmodulin, antibodies such as 4E11 or chelated metal ions or free chelators may be multimerized by conventional methods, *e.g.* by biotinylation and complexation with streptavidin or avidin or oligomers thereof or by the introduction of carboxyl residues into a polysaccharide, *e.g.* dextran, essentially as described in Noguchi, A, et al. Bioconjugate Chemistry (1992) 3, 132-137 in a first step and linking calmodulin or antibodies or chelated metal ions or free chelators via primary amino groups to the carboxyl groups in the polysaccharide, *e.g.* dextran, backbone using conventional carbodiimide chemistry in a second step. In some such embodiments, the binding between the binding partner C that is included in the agent (*e.g.,* selection agent or stimulatory agent) and the one or more binding sites Z of the reagent can be disrupted by metal ion chelation. The metal chelation may, for example, be accomplished by addition of EGTA or EDTA.

In some embodiments, the agent (*e.g.,* selection agent or stimulatory agent), which specifically bind to the cell surface molecule, may for instance be comprised by an antibody, a fragment thereof, or a proteinaceous binding molecule with antibody-like functions. In some embodiments, the binding site B of the agent is an antibody combining site, such as is or contains one or more complementarity determining regions (CDRs) of an antibody. Examples of (recombinant) antibody fragments include, but are not limited to, Fab fragments, Fv fragments, single-chain Fv fragments (scFv), a divalent antibody fragment such as an (Fab)2'-fragment, diabodies, triabodies (Iliades, P., et al, FEB S Lett (1997) 409, 437-441), decabodies (Stone, E., et al, Journal of Immunological Methods (2007) 318, 88-94) and other domain antibodies (Holt, L.J., et al, Trends Biotechnol. (2003), 21, 11, 484-490). In some embodiments, the agent (e.g., receptor-binding agent or selection agent) may comprise a bivalent proteinaceous artificial binding molecule such as a dimeric lipocalin mutein that is also known as "duocalin".

In some embodiments, the agent (*e.g.,* selection agent or stimulatory agent) may have a single binding site B, *i.e.,* it may be monovalent. Examples of monovalent agents (*e.g.,* selection agent or stimulatory agent) include, but are not limited to, a monovalent antibody fragment, a proteinaceous binding molecule with antibody-like binding properties or an MHC molecule. Examples of monovalent antibody fragments include, but are not limited to a Fab fragment, an Fv fragment, and a single-chain Fv fragment (scFv), including a divalent single-chain Fv fragment.

In some embodiments, the agent (*e.g.,* selection agent or stimulatory agent) is an antibody or an antigen-binding fragment thereof, such as a Fab fragments, Fv fragments, single-chain Fv fragments (scFv), a divalent antibody fragment such as an F(ab')₂-fragment. In some embodiments, the agent (*e.g.,* selection agent or stimulatory agent) is or is derived from a parental antibody that is known to bind to a cell molecule of interest. Various antibody molecules or fragments thereof against cell surface molecules are well known in the art and any of a variety of such can be used as agents in the methods herein. In some embodiments, the agent (*e.g.,* selection agent or stimulatory agent) is an antibody or fragment thereof that contains one or more amino acid replacements in the variable heavy chain of a parental or reference antibody, for example, to generate an antibody with an altered affinity or that exhibits a sufficiently fast off-rate as described above. For example, exemplary of such mutations are known the context of mutants of the anti-CD4 antibody 13B8.2 (see *e.g.,* U.S. Patent Nos. 7,482,000, U.S. Patent Appl. Pub. No. US2014/0295458 or International Patent Application App. No. WO2013/124474), and any of such mutations can be generated in another parental or reference antibody.

In some aspects, the agent (*e.g.,* selection agent or stimulatory agent) that can be monovalent, for example comprise a monovalent antibody fragment or a monovalent artificial binding molecule (proteinaceous or other) such as a mutein based on a polypeptide of the lipocalin family (also known as "Anticalin^{®}), or a bivalent molecule such as an antibody or a fragment in which both binding sites are retained such as an F(ab')₂ fragment.

An example of a proteinaceous binding molecule with antibody-like functions includes a mutein based on a polypeptide of the lipocalin family (see for example, WO 03/029462, Beste et al, Proc. Natl. Acad. Sci. U.S.A. (1999) 96, 1898-1903). Generally, lipocalins, such as the bilin binding protein, the human neutrophil gelatinase-associated lipocalin, human Apo lipoprotein D or human tear lipocalin possess natural ligand-binding sites that can be modified so that they bind a given target. Further examples of a proteinaceous binding molecule with antibody-like binding properties that can be used as agent (*e.g.,* selection agent or stimulatory agent) that specifically binds to the cell surface molecule include, but are not limited to, the so-called glubodies (see *e.g.* international patent application WO 96/23879), proteins based on the ankyrin scaffold (Mosavi, L.K., et al, Protein Science (2004) 13, 6, 1435-1448) or crystalline scaffold (*e.g.* international patent application WO 01/04144) the proteins described in Skerra, J. Mol. Recognit. (2000) 13, 167-187, AdNectins, tetranectins and avimers. Generally, avimers, including multivalent avimer proteins evolved by exon shuffling of a family of human receptor domains, contain so called A-domains that occur as strings of multiple domains in several cell surface receptors (Silverman, J., et al, Nature Biotechnology (2005) 23, 1556-1561). Adnectins, generally derived from a domain of human fibronectin, typically contain three loops that can be engineered for immunoglobulin-like binding to targets (Gill, D.S. & Damle, N.K., Current Opinion in Biotechnology (2006) 17, 653-658). Tetranectins, generally derived from the respective human homotrimeric protein, likewise typically contain loop regions in a C-type lectin domain that can be engineered for desired binding. Peptoids, which can, in some cases, act as protein ligands, typically are oligo(N-alkyl) glycines that differ from peptides in that the side chain is connected to the amide nitrogen rather than the carbon atom. Peptoids are typically resistant to proteases and other modifying enzymes and can have a much higher cell permeability than peptides (see *e.g.* Kwon, Y.-U., and Kodadek, T., J. Am. Chem. Soc. (2007) 129, 1508-1509).

Further examples of suitable proteinaceous binding molecules include, but are not limited to, an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, tendamistat, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an immunoglobulin domain or a an immunoglobulin-like domain (for example, domain antibodies or camel heavy chain antibodies), a C-type lectin domain, a MAM domain, a von Willebrand factor type A domain, a Somatomedin B domain, a WAP -type four disulfide core domain, a F5/8 type C domain, a Hemopexin domain, an SH2 domain, an SH3 domain, a Laminin-type EGF-like domain, a C2 domain, "Kappabodies" (Ill et al. Protein Eng (1997) 10, 949-57, a so called "minibody" (Martin et al, EMBO J (1994) 13, 5303-5309), a diabody (Holliger et al, PNAS USA (1993)90, 6444-6448), a so called "Janusis" (Traunecker et al, EMBO J (1991) 10, 3655-3659, or Traunecker et al, Int J Cancer (1992) Suppl 7, 51-52), a nanobody, a microbody, an affilin, an affibody, a knottin, ubiquitin, a zinc-finger protein, an autofluorescent protein or a leucine-rich repeat protein. In some embodiments, a nucleic acid molecule with antibody-like functions can be an aptamer. Generally, an aptamer folds into a defined three-dimensional motif and shows high affinity for a given target structure.

### a. Selection Agents

In certain aspects, the methods provided herein employ a selection agent. In some embodiments, the agent, as described in Section II-B, is a selection agent. In some embodiments, the selection agent binds to a molecule on the surface of a cell, such as a cell surface molecule. In some instances, the cell surface molecule is a selection marker. In some embodiments, the selection agent is capable of specifically binding to a selection marker expressed by one or more of the cells in a sample. In some embodiments, reference to specific binding to a molecule, such as a cell surace molecule or cell surface receptor, throughout the disclosure does not necessarily mean that the agent binds only to such molecule. For example, an agent that specifically binds to a molecule may bind to other molecules, generally with much lower affinity as determined by, e.g., immunoassays, BIAcore^{®}, KinExA 3000 instrument (Sapidyne Instruments, Boise, ID), or other assays. In some cases, the ability of an agent, under specific binding conditions, to bind to a target molecule such that its affinity or avidity is at least 5 times as great, such as at least 10, 20, 30, 40, 50, 100, 250 or 500 times as great, or even at least 1000 times as great as the average affinity or avidity of the same agent to a collection of random peptides or polypeptides of sufficient statistical size.

In some embodiments, the cells, e.g., target cells (e.g., T cells), have or express a molecule on the cell surface, e.g., a selection marker, such that the cells to be selected are defined by the presence of at least one common specific molecule (e.g., selection marker). In some embodiments, the sample containing the target cell may also contain additional cells that are devoid of the molecule (e.g., selection marker). For example, in some embodiments, T cells may be selected from a sample containing multiple cells types, e.g., red blood cells or B cells. Selection marker and receptor molecule may be used interchangeably herein to refer to a cell surface molecule.

In some embodiments, the selection agent is or contains an agent selected from the group consisting of antibody fragments, monovalent antibody fragments, proteinaceous binding molecules with immunoglobulin-like functions, molecules containing Ig domains, cytokines, chemokines, aptamers, MHC molecules, MHC-peptide complexes; receptor ligands; and binding fragments thereof; and/or the selection agent contains an antibody fragment; the selection agent is or contains a Fab fragment; the selection agent is selected from the group of divalent antibody fragments consisting of F(ab)₂'-fragments and divalent single-chain Fv (scFv) fragments; the selection agent is a monovalent antibody fragment selected from the group consisting of Fab fragments, Fv fragments, and scFvs; and/or the selection agent is a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers.

In some embodiments, the selection agent further contains a binding partner C for binding to the reagent. In some embodiments, the selection agent further contains biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO:15), Trp-Ser-His-Pro-Gln-Phe-Glu-Lys-(GlyGlyGlySer)3-Trp-Ser-His-Pro-Gln-Phe-Glu-Lys (SEQ ID NO: 17), SAWSHPQFEKGGGSGGGSGGSAWSHPQFEK (SEQ ID NO:16), Trp-Ser-His-Pro-Gln-Phe-Glu-Lys-(GlyGlyGlySer)2-Trp-Ser-His-Pro-Gln-Phe-Glu-Lys (SEQ ID NO: 18) and Trp-Ser-His-Pro-Gln-Phe-Glu-Lys-(GlyGlyGlySer)2Gly-Gly-Ser-Ala-Trp-Ser-His-Pro-Gln-Phe-Glu-Lys (SEQ ID NO: 19), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag.

In some embodiments, the reagent is or contains a streptavidin, streptavidin mutein, aviding or avidin mutein, and the selection agent contains a binding partner C that is able to bind the such reagent, such as biotin, a biotin analog or a streptavidin-binding peptide. In some embodiments, the selection agent further comprises biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO:16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19). In particular embodiments, the reagent is or contains a streptavidin mutein (e.g. set forth in SEQ ID NO:6) and the binding partner C is a streptavidin-binding peptide, such as any set forth in any one of SEQ ID NOS: 8 or 15-19. In some embodiments, the the selection agent further comprises a streptavidin-binding peptide having the sequence SAWSHPQFEKGGGSGGGSGGSAWSHPQFEK (SEQ ID NO: 16).

In some aspects, the cell surface molecule, *e.g.,* selection marker, may be an antigen defining a desired cell population or subpopulation, for instance a population or subpopulation of blood cells, e. g. lymphocytes (*e.g.* T cells, T-helper cells, for example, CD4+ T-helper cells, B cells or natural killer cells), monocytes, or stem cells, *e.g.* CD34-positive peripheral stem cells or Nanog or Oct-4 expressing stem cells. In some embodiments, the selection marker can be a marker expressed on the surface of T cells or a subset of T cells, such as CD25, CD28, CD62L, CCR7, CD27, CD127, CD3, CD4, CD8, CD45RA, and/or CD45RO Examples of T-cells include cells such as CMV-specific CD8+ T-lymphocytes, cytotoxic T-cells, memory T-cells and regulatory T-cells (Treg). An illustrative example of Treg includes CD4 CD25 CD45RA Treg cells and an illustrative example of memory T-cells includes CD62L CD8+ specific central memory T-cells.

For example, in some aspects, specific subpopulations of T cells, such as cells positive or expressing high levels of one or more surface markers, e.g., CD28+, CD62L+, CCR7+, CD27+, CD127+, CD3+, CD4+, CD8+, CD45RA+, and/or CD45RO+ T cells, are isolated by positive or negative selection techniques. In some embodiments, such cells are selected by incubation with one or more selection agents that specifically binds to such markers. The selection agent may be any binding molecule, such as an antibody or antibody fragment, that binds to such surface markers to effect the positive or negative selection of T cells or subpopulations thereof.

In some embodiments, T cells are separated from a PBMC sample by negative selection of markers expressed on non-T cells, such as B cells, monocytes, or other white blood cells, such as CD14. In some aspects, a CD4+ or CD8+ selection step is used to separate CD4+ helper and CD8+ cytotoxic T cells. Such CD4+ and CD8+ populations can be further sorted into sub-populations by positive or negative selection for markers expressed or expressed to a relatively higher degree on one or more naive-like, memory, and/or effector T cell subpopulations.

In some embodiments, CD8+ cells are further enriched for or depleted of naive, central memory, effector memory, and/or central memory stem cells, such as by positive or negative selection based on surface antigens associated with the respective subpopulation. In some embodiments, enrichment for central memory T (TCM) cells is carried out to increase efficacy, such as to improve long-term survival, expansion, and/or engraftment following administration, which in some aspects is particularly robust in such sub-populations. See Terakura et al., (2012) Blood.1:72-82; Wang et al. (2012) J Immunother. 35(9):689-701. In some embodiments, combining TCM-enriched CD8+ T cells and CD4+ T cells further enhances efficacy.

In embodiments, memory T cells are present in both CD62L+ and CD62L-subsets of CD8+ peripheral blood lymphocytes. PBMC can be enriched for or depleted of CD62L-CD8+ and/or CD62L+CD8+ fractions, such as using anti-CD8 and anti-CD62L antibodies as selection agents.

In some embodiments, the enrichment for central memory T (T_{CM}) cells is based on positive or high surface expression of CD45RO, CD62L, CCR7, CD28, CD3, and/or CD127; in some aspects, it is based on negative selection for cells expressing or highly expressing CD45RA and/or granzyme B. In some aspects, isolation of a CD8+ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8+ cell population or subpopulation, also is used to generate the CD4+ cell population or sub-population, such that both the positive and negative fractions from the CD4-based separation are retained and used in subsequent steps of the methods, optionally following one or more further positive or negative selection steps. In some embodiments, the selection for the CD4+ cell population and the selection for the CD8+ cell population are carried out simultaneously. In some embodiments, the CD4+ cell population and the selection for the CD8+ cell population are carried out sequentially, in either order. In some embodiments, methods for selecting cells can include those as described in published U.S. App. No. US20170037369.

In particular embodiments, a biological sample, e.g., a sample of PBMCs or other white blood cells, are subjected to selection of CD4+ T cells, where both the negative and positive fractions are retained. In certain embodiments, CD8+ T cells are selected from the negative fraction. In some embodiments, a biological sample is subjected to selection of CD8+ T cells, where both the negative and positive fractions are retained. In certain embodiments, CD4+ T cells are selected from the negative fraction.

In some embodiments, a selection agent that specifically binds CD4 and a selection agent that specifically binds CD8 are used to generate a population enriched in CD4+ T cells and a population enriched in CD8+ T cells, respectively.

In a particular example, a sample of PBMCs or other white blood cell sample is subjected to selection of CD4+ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4+ T helper cells may be sorted into naive, central memory, and effector cells by identifying cell populations that have cell surface antigens. CD4+ lymphocytes can be obtained by standard methods. In some embodiments, naive CD4+ T lymphocytes are CD45RO-, CD45RA+, CD62L+, or CD4+ T cells. In some embodiments, central memory CD4+ cells are CD62L+ and CD45RO+. In some embodiments, effector CD4+ cells are CD62L- and CD45RO-.

In some embodiments, the selection marker is a T cell coreceptor; the selection marker is or contains a member of a T cell antigen receptor complex; the selection marker is or contains a CD3 chain; the selection marker is or contains a CD3 zeta chain; the selection marker is or contains a CD8; the selection marker is or contains a CD4; the selection marker is or contains CD45RA; the selection marker is or contains CD27; the selection marker is or contains CD28; and/or the selection marker is or contains CCR7. In some embodiments, the selection marker is selected from the group consisting of CD3, CD4, and CD8. In some embodiments, the selection marker is CD3.

In some embodiments, the specific binding between the selection agent and the selection marker does not induce a signal, or does not induce a stimulatory or activating or proliferative signal, to the T cells. In some embodiments, the selection agent includes a monovalent antibody fragment that binds to CD3, CD8 or CD4. In some embodiments, the selection agent is an anti-CD3 Fab, an anti-CD8 Fab or an anti-CD4 Fab. In some embodietns, the selection agent is an anti-CD3 Fab. In some embodiments, the anti-CD3 Fab comprises an OKT3 antibody Fab fragment. In some embodiments, the anti-CD3 Fab comprises a variable heavy chain having the sequence set forth by SEQ ID NO:31 and a variable light chain having the sequence set forth by SEQ ID NO:32.

In some embodiments, the selection marker may be CD4 and the selection agent specifically binds CD4. In some aspects, the selection agent that specifically binds CD4 may be selected from the group consisting of an anti-CD4-antibody, a divalent antibody fragment of an anti-CD4 antibody, a monovalent antibody fragment of an anti-CD4-antibody, and a proteinaceous CD4 binding molecule with antibody-like binding properties. In some embodiments, an anti-CD4-antibody, such as a divalent antibody fragment or a monovalent antibody fragment (e.g. CD4 Fab fragment) can be derived from antibody 13B8.2 or a functionally active mutant of 13B8.2 that retains specific binding for CD4. For example, exemplary mutants of antibody 13B8.2 or m13B8.2 are described in U.S. Patent Nos. 7,482,000, U.S. Patent Appl. No. US2014/0295458 or International Patent Application No. WO2013/124474; and Bes, C, et al. J Biol Chem 278, 14265-14273 (2003). The mutant Fab fragment termed "m13B8.2" carries the variable domain of the CD4 binding murine antibody 13B8.2 and a constant domain containing constant human CH1 domain of type gamma for the heavy chain and the constant human light chain domain of type kappa, as described in US Patent 7,482,000. In some embodiments, the anti-CD4 antibody, e.g. a mutant of antibody 13B8.2, contains the amino acid replacement H91A in the variable light chain, the amino acid replacement Y92A in the variable light chain, the amino acid replacement H35A in the variable heavy chain and/or the amino acid replacement R53A in the variable heavy chain, each by Kabat numbering. In some aspects, compared to variable domains of the 13B8.2 Fab fragment in ml3B8.2 the His residue at position 91 of the light chain (position 93 in SEQ ID NO: 30) is mutated to Ala and the Arg residue at position 53 of the heavy chain (position 55 in SEQ ID NO: 29) is mutated to Ala. In some embodiments, the reagent that is reversibly bound to anti-CD4 or a fragment thereof is commercially available or derived from a reagent that is commercially available (*e.g.* catalog No. 6-8000-206 or 6-8000-205 or 6-8002-100; IBA GmbH, Gottingen, Germany). In some embodiments, the selection agent comprises an anti-CD4 Fab fragment. In some embodiments, the anti-CD4 Fab fragment comprises a variable heavy chain having the sequence set forth by SEQ ID NO:29 and a variable light chain having the sequence set forth by SEQ ID NO:30. In some embodiments, the anti-CD4 Fab fragment comprises the CDRs of the variable heavy chain having the sequence set forth by SEQ ID NO:29 and the CDRs of the variable light chain having the sequence set forth by SEQ ID NO:30.

In some embodiments, the selection marker may be CD8 and the selection agent specifically binds CD8. In some aspects, the selection agent that specifically binds CD8 may be selected from the group consisting of an anti-CD8-antibody, a divalent antibody fragment of an anti-CD8 antibody, a monovalent antibody fragment of an anti-CD8-antibody, and a proteinaceous CD8 binding molecule with antibody-like binding properties. In some embodiments, an anti-CD8-antibody, such as a divalent antibody fragment or a monovalent antibody fragment (*e.g.* CD8 Fab fragment) can be derived from antibody OKT8 (*e.g.* ATCC CRL-8014) or a functionally active mutant thereof that retains specific binding for CD8. In some embodiments, the reagent that is reversibly bound to anti-CD8 or a fragment thereof is commercially available or derived from a reagent that is commercially available (e.g. catalog No. 6-8003 or 6-8000-201; IBA GmbH, Gottingen, Germany). In some embodiments, the selection agent comprises an anti-CD8 Fab fragment. In some embodiments, the anti-CD8 Fab fragment comprises a variable heavy chain having the sequence set forth by SEQ ID NO:36 and a variable light chain having the sequence set forth by SEQ ID NO:37. In some embodiments, the anti-CD8 Fab fragment comprises the CDRs of the variable heavy chain having the sequence set forth by SEQ ID NO:36 and the CDRs of the variable light chain having the sequence set forth by SEQ ID NO:37.

In some embodiments, the selection marker may be CD3 and the selection agent specifically binds CD3. In some aspects, the selection agent that specifically binds CD3 may be selected from the group consisting of an anti-CD3-antibody, a divalent antibody fragment of an anti-CD3 antibody, a monovalent antibody fragment of an anti-CD3-antibody, and a proteinaceous CD3 binding molecule with antibody-like binding properties. In some embodiments, an anti-CD3-antibody, such as a divalent antibody fragment or a monovalent antibody fragment (*e.g.* CD3 Fab fragment) can be derived from antibody OKT3 (*e.g.* ATCC CRL-8001; see *e.g.,* Stemberger et al. PLoS One. 2012; 7(4): e35798) or a functionally active mutant thereof that retains specific binding for CD3. In some embodiments, the reagent that is reversibly bound to anti-CD3 or a fragment thereof is commercially available or derived from a reagent that is commercially available (*e.g.* catalog No. 6-8000-201, 6-8001-100; IBA GmbH, Gottingen, Germany). In some embodiments, the selection agent comprises an anti-CD3 Fab fragment. In some embodiments, the anti-CD3 Fab fragment comprises a variable heavy chain having the sequence set forth by SEQ ID NO:31 and a variable light chain having the sequence set forth by SEQ ID NO:32. In some embodiments, the anti-CD3 Fab fragment comprises the CDRs of the variable heavy chain having the sequence set forth by SEQ ID NO:31 and the CDRs of the variable light chain having the sequence set forth by SEQ ID NO:32.

In any of the above examples, the divalent antibody fragment may be an (Fab)2'-fragment, or a divalent single-chain Fv fragment while the monovalent antibody fragment may be selected from the group consisting of a Fab fragment, an Fv fragment, and a single-chain Fv fragment (scFv). In any of the above examples, the proteinaceous binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In some embodiments, the selection agent is directly or indirectly bound to the stationary phase. In some embodiments, the selection agent is bound indirectly to the stationary phase through a selection reagent to which the selection agent reversibly binds. In some embodiments, the the selection reagent is or contains streptavidin, avidin, a mutein of streptavidin that reversibly binds biotin, a biotin analog or a biologically active fragment thereof; a mutein of avidin or streptavidin that reversibly binds a streptavidin-binding peptide; a reagent that contains at least two chelating groups K, wherein the at least two chelating groups are capable of binding to a transition metal ion; an agent capable of binding to an oligohistidine affinity tag; an agent capable of binding to a glutathione-S-transferase; calmodulin or an analog thereof; an agent capable of binding to calmodulin binding peptide (CBP); an agent capable of binding to a FLAG-peptide; an agent capable of binding to an HA-tag; an agent capable of binding to maltose binding protein (MBP); an agent capable of binding to an HSV epitope; an agent capable of binding to a myc epitope; or an agent capable of binding to a biotinylated carrier protein.

In some embodiments, the selection reagent is or contains a streptavidin mutein or an avidin mutein that reversibly binds to biotin or a biologically active fragment. In some embodiments, the selection reagent is or contains a streptavidin mutein or an avidin mutein that reversibly binds to a streptavidin-binding peptide. In some embodiments, the streptavidin or streptavidin mutein molecules reversibly bind to or are capable of reversibly binding to biotin, a biotin analog or a streptavidin-binding peptide.

### b. Stimulatory Agents

In certain aspects, the methods provided herein employ a stimulatory agent. In some embodiments, the agent, as described in Section II-B, is a stimulatory agent. In some embodiments, the stimulatory agent binds to a molecule on the surface of a cell, which binding between the stimulatory agent and the molecule is capable of inducing, delivering, or modulating a stimulatory signal in the cells. In some instances, the cell surface molecule (*e.g.* receptor) is a signaling molecule. In some such cases, the stimulatory agent is capable of specifically binding to a signaling molecule expressed by one or more target cells (e.g., T cells). In some instances, the stimulatory agent is any agent that is capable of inducing or delivering a stimulatory signal in a cell (e.g., a T cell) upon binding to a cell surface molecule, such as a receptor. In some embodiments, the stimulatory signal can be immunostimulatory, in which case the stimulatory agent is capable of inducing, delivering, or modulating a signal that is involved in or that does stimulate an immune response by the cell (*e.g.* T cell), e.g., increase immune cell proliferation or expansion, immune cell activation, immune cell differentiation, cytokine secretion, cytotoxic activity or one or more other functional activities of an immune cell. In some embodiments, the stimulatory signal can be inhibitory, in which case the stimulatory agent is capable of inducing, delivering, or modulating a stimulatory signal in the cell (*e.g.* T cell) that is involved in or that does inhibit an immune response, *e.g.* inhibits or decreases immune cell proliferation or expansion, immune cell activation, immune cell differentiation, cytokine secretion, cytotoxic activity or one or more other functional activities of an immune cell.

In some embodiments, the stimulatory agent is a first stimulatory agent. In some embodiments, the first stimulatory agent binds to a receptor molecule on the surface of the selected cells of the sample. Thus, in some cases, the first stimulatory agent delivers, induces, or modulates a stimulatory signal. In some aspects, the delivering, inducing, or modulating of a stimulatory signal by the first stimulatory agent effects the stimulation of the cells. Thus, in some cases, the first stimulatory agent delivers a stimulatory signal to the cells, thereby stimulating the cells. In some embodiments, the first stimulatory agent further induces downregulation of a selection marker. As used herein, downregulation may encompass a reduction in expression of a selection marker compared to an earlier time point.

In some embodiments, the target cells (e.g., T cells) comprise TCR/CD3 complexes and costimulatory molecules, such as CD28. In this case, the first stimulatory agent binds to a TCR/CD3 complex, thereby delivering a stimulatory signal in the T cells, and the second stimulatory agent binds to costimulatory CD28 molecule. In particular aspects, the first stimulatory agent and/or the second stimulatory agent further induce downregulation of a selection marker (e.g., selection marker used to immobilize the target cells (e.g., T cells)).

In some embodiments, the first stimulatory agent delivers a TCR/CD3 complex-associated stimulatory signal in the cells, *e.g.,* T cells. In some embodiments, the first stimulatory agent specifically binds to a molecule containing an immunoreceptor tyrosine-based activation motif or ITAM. In some aspects, the first stimulatory agent specifically binds CD3. In some cases, a first stimulatory agent that specifically binds CD3 may be selected from the group consisting of an anti-CD3-antibody, a divalent antibody fragment of an anti-CD3 antibody, a monovalent antibody fragment of an anti-CD3-antibody, and a proteinaceous CD3 binding molecule with antibody-like binding properties. The divalent antibody fragment may be a F(ab')₂-fragment, or a divalent single-chain Fv fragment while the monovalent antibody fragment may be selected from the group consisting of a Fab fragment, an Fv fragment, and a single-chain Fv fragment (scFv). In some cases, a proteinaceous CD3 binding molecule with antibody-like binding properties may be an aptamer, a mutein based on a polypeptide of the lipocalin family, a glubody, a protein based on the ankyrin scaffold, a protein based on the crystalline scaffold, an adnectin, or an avimer.

In some embodiments, an anti-CD3 Fab fragment can be derived from the CD3 binding monoclonal antibody produced by the hybridoma cell line OKT3 (ATCC^{®} CRL-8001^{™}; see also U.S. Patent No. 4,361,549). The variable domain of the heavy chain and the variable domain of the light chain of the anti-CD3 antibody OKT3 are described in Arakawa et al J. Biochem. 120, 657-662 (1996) and comprise the amino acid sequences set forth in SEQ ID NOs: 31 and 32, respectively.

In some embodiments, the stimulatory agent is a second stimulatory agent. In some embodiments, the second stimulatory agent binds to a molecule on the surface of the cells, such as a cell surface molecule, *e.g.,* receptor molecule. In some embodiments, the second stimulatory agent is capable of enhancing, dampening, or modifying a stimulatory signal delivered through the molecule bound by the first stimulatory agent. In some embodiments, the second stimulatory agent delivers, induces, or modulates a stimulatory signal, *e.g.,* a second or an additional stimulatory signal. In some aspects, the second stimulatory agent enhances or potentiates a stimulatory signal induced by the first stimulatory agent. In some embodiments, the second stimulatory agent binds to an accessory molecule and/or can stimulate or induce an accessory or secondary stimulatory signal in the cell. In some aspects, the second stimulatory agent binds to a costimulatory molecule and/or provides a costimulatory signal.

In some embodiments, the stimulatory agent, which can be the second stimulatory agent, binds, *e.g.* specifically binds, to a second molecule that can be a costimulatory molecule, an accessory molecule, a cytokine receptor, a chemokine receptor, an immune checkpoint molecule, or a member of the TNF family or the TNF receptor family.

In some embodiments, the molecule on the cell, *e.g.,* T cell, may be CD28 and the stimulatory agent (*e.g.* which can be the second stimulatory agent) specifically binds CD28. In some aspects, the stimulatory agent (*e.g.* which can be the second stimulatory agent) that specifically binds CD28 may be selected from the group consisting of an anti-CD28-antibody, a divalent antibody fragment of an anti-CD28 antibody, a monovalent antibody fragment of an anti-CD28-antibody, and a proteinaceous CD28 binding molecule with antibody-like binding properties. The divalent antibody fragment may be an F(ab')₂-fragment, or a divalent single-chain Fv fragment while the monovalent antibody fragment may be selected from the group consisting of a Fab fragment, an Fv fragment, and a single-chain Fv fragment (scFv). A proteinaceous CD28 binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In some embodiments, an anti-CD28 Fab fragment can be derived from antibody CD28.3 (deposited as a synthetic single chain Fv construct under GenBank Accession No. AF451974.1; see also Vanhove et al, BLOOD, 15 July 2003, Vol. 102, No. 2, pages 564-570) the variable heavy and light chains of which comprise SEQ ID NO: 33 and 34, respectively.

In some embodiments, the one or more stimulatory agent is an anti-CD3 and an anti-CD28 antibody or antigen binding fragment thereof. In some embodiments, the one or more stimulatory agent is an anti-CD3 Fab and an anti-CD28 Fab.

In some embodiments, the molecule on the cell, *e.g.,* T cell, is CD90 and the stimulatory agent (*e.g.* which can be the second stimulatory agent) specifically binds CD90. In some aspects, the stimulatory agent (*e.g.* which can be the second stimulatory agent) that specifically binds CD90 may be selected from the group consisting of an anti-CD90-antibody, a divalent antibody fragment of an anti-CD90 antibody, a monovalent antibody fragment of an anti-CD90-antibody, and a proteinaceous CD90 binding molecule with antibody-like binding properties. The antibody or antigen-binding fragment can be derived from any known in the art. See *e.g.* anti-CD90 antibody G7 (Biolegend, cat. no. 105201).

In some embodiments, the molecule on the cell, *e.g.,* T cell, is CD95 and the stimulatory agent (*e.g.* which can be the second stimulatory agent) specifically binds CD95. In some aspects, the stimulatory agent (*e.g.* which can be the second stimulatory agent) that specifically binds CD95 may be selected from the group consisting of an anti-CD95-antibody, a divalent antibody fragment of an anti-CD95 antibody, a monovalent antibody fragment of an anti-CD95-antibody, and a proteinaceous CD95 binding molecule with antibody-like binding properties. The antibody or antigen-binding fragment can be derived from any known in the art. For example, in some aspects, the anti-CD90 antibody can be monoclonal mouse anti-human CD95 CH11 (Upstate Biotechnology, Lake Placid, NY) or can be anti-CD95 mAb 7C11 or anti-APO-1, such as described in Paulsen et al. Cell Death & Differentiation 18.4 (2011): 619-631.

In some embodiments, the molecule on the cell, *e.g.,* T cell or B cell, may be CD137 and the stimulatory agent (*e.g.* which can be the second stimulatory agent) specifically binds CD137. In some aspects, the stimulatory agent (*e.g.* which can be the second stimulatory agent) that specifically binds CD137 may be selected from the group consisting of an anti-CD137-antibody, a divalent antibody fragment of an anti-CD137 antibody, a monovalent antibody fragment of an anti-CD137-antibody, and a proteinaceous CD137 binding molecule with antibody-like binding properties. The antibody or antigen-binding fragment can be derived from any known in the art. For example, the anti-CD137 antibody can be LOB12, IgG2a or LOB12.3, IgG1 as described in Taraban et al. Eur J Immunol. 2002 Dec;32(12):3617-27. See also *e.g.* US6569997, US6303121, Mittler et al. Immunol Res. 2004;29(1-3):197-208.

In some embodiments, the molecule on the cell, *e.g.* B cell, may be CD40 and the stimulatory agent, *e.g.,* stimulatory agent, (*e.g.* which can be the second stimulatory agent, *e.g.,* second stimulatory agent) specifically binds CD40. In some aspects, the stimulatory agent (which can be the second stimulatory agent, e.g., second stimulatory agent) that specifically binds CD40 may be selected from the group consisting of an anti-CD40-antibody, a divalent antibody fragment of an anti-CD40 antibody, a monovalent antibody fragment of an anti-CD40-antibody, and a proteinaceous CD40 binding molecule with antibody-like binding properties.

In some embodiments, the molecule on the cell, *e.g.,* T cell, may be CD40L (CD154) and the stimulatory agent (*e.g.* which can be the second stimulatory agent) specifically binds CD40L. In some aspects, the stimulatory agent (*e.g.* which can be the second stimulatory agent) that specifically binds CD40L may be selected from the group consisting of an anti-CD40L-antibody, a divalent antibody fragment of an anti-CD40L antibody, a monovalent antibody fragment of an anti-CD40L-antibody, and a proteinaceous CD40L binding molecule with antibody-like binding properties. The antibody or antigen-binding fragment can be derived from any known in the art. For example, the anti-CD40L antibody can in some aspects be Hu5C8, as described in Blair et al. JEM vol. 191 no. 4 651-660. See also *e.g.* WO1999061065, US20010026932, US7547438, WO2001056603.

In some embodiments, the molecule on the cell, *e.g.,* T cell, may be inducible T cell Costimulator (ICOS) and the stimulatory agent, (*e.g.* which can be the second stimulatory agent) specifically binds ICOS. In some aspects, the stimulatory agent (*e.g.* which can be the second stimulatory agent) that specifically binds ICOS may be selected from the group consisting of an anti-ICOS-antibody, a divalent antibody fragment of an anti-ICOS antibody, a monovalent antibody fragment of an anti-ICOS-antibody, and a proteinaceous ICOS binding molecule with antibody-like binding properties. The antibody or antigen-binding fragment can be derived from any known in the art. See *e.g.* US20080279851 and Deng et al. Hybrid Hybridomics. 2004 Jun;23(3):176-82.

In some embodiments, the molecule on the cell, *e.g.,* T cell, may be Linker for Activation of T cells (LAT) and the stimulatory agent (*e.g.* which can be the second stimulatory agent) specifically binds LAT. In some aspects, the stimulatory agent (e.g. which can be the second stimulatory agent) that specifically binds LAT may be selected from the group consisting of an anti-LAT-antibody, a divalent antibody fragment of an anti-LAT antibody, a monovalent antibody fragment of an anti-LAT-antibody, and a proteinaceous LAT binding molecule with antibody-like binding properties. The antibody or antigen-binding fragment can be derived from any known in the art.

In some embodiments, the molecule on the cell, *e.g.,* T cell, may be CD27 and the stimulatory agent (*e.g.* which can be the second stimulatory agent) specifically binds CD27. In some aspects, the stimulatory agent (*e.g.* which can be the second stimulatory agent) that specifically binds CD27 may be selected from the group consisting of an anti-CD27-antibody, a divalent antibody fragment of an anti-CD27 antibody, a monovalent antibody fragment of an anti-CD27-antibody, and a proteinaceous CD27 binding molecule with antibody-like binding properties. The antibody or antigen-binding fragment can be derived from any known in the art. See *e.g.* WO2008051424.

In some embodiments, the molecule on the cell, *e.g.,* T cell, may be OX40 and the stimulatory agent (*e.g.* which can be the second stimulatory agent) specifically binds OX40. In some aspects, the stimulatory agent (*e.g.* which can be the second stimulatory agent) that specifically binds OX40 may be selected from the group consisting of an anti-OX40-antibody, a divalent antibody fragment of an anti-OX40 antibody, a monovalent antibody fragment of an anti-OX40-antibody, and a proteinaceous OX40 binding molecule with antibody-like binding properties. The antibody or antigen-binding fragment can be derived from any known in the art. See *e.g.* WO2013038191, Melero et al. Clin Cancer Res. 2013 Mar 1;19(5):1044-53.

In some embodiments, the molecule on the cell, *e.g.,* T cell, may be HVEM and the stimulatory agent (*e.g.* which can be the second stimulatory agent) specifically binds HVEM. In some aspects, the stimulatory agent (*e.g.* which can be the second stimulatory agent) that specifically binds HVEM may be selected from the group consisting of an anti-HVEM-antibody, a divalent antibody fragment of an anti-HVEM antibody, a monovalent antibody fragment of an anti-HVEM-antibody, and a proteinaceous HVEM binding molecule with antibody-like binding properties. The antibody or antigen-binding fragment can be derived from any known in the art. See *e.g.* WO2006054961, WO2007001459, Park et al. Cancer Immunol Immunother. 2012 Feb;61(2):203-14.

In any of the above examples, the divalent antibody fragment may be a (Fab)2'-fragment, or a divalent single-chain Fv fragment while the monovalent antibody fragment may be selected from the group consisting of a Fab fragment, an Fv fragment, and a single-chain Fv fragment (scFv). In any of the above examples, the proteinaceous binding molecule with antibody-like binding properties may be an aptamer, a mutein based on a polypeptide of the lipocalin family, a glubody, a protein based on the ankyrin scaffold, a protein based on the crystalline scaffold, an adnectin, and an avimer.

In some aspects, the stimulatory agent specifically targets a molecule expressed on the surface of the target cells in which the molecule is a TCR, a chimeric antigen receptor, or a molecule comprising an immunoreceptor tyrosine-based activation motif or ITAM. For example, the molecule expressed on the surface of the target cell is selected from a T cell or B cell antigen receptor complex, a CD3 chain, a CD3 zeta, an antigen-binding portion of a T cell receptor or a B cell receptor, or a chimeric antigen receptor. In some cases, the stimulatory agent targets peptide:MHC class I complexes.

In some embodiments, the stimulatory agent binds to a His-tagged extracellular domain of a molecule expressed on the suface of the target cells. In some cases, the stimulatory agent contains the peptide sequence Trp-Ser-His-Pro-Gln-Phe-Glu-Lys (also called Strep-tag^{®} II, set forth in SEQ ID NO: 8) conjugated with a nickel charged trisNTA (also called His-STREPPER or His/Strep-tag^{®}II Adapter). In some embodiments, the molecule expressed on the surface of the target cells that is His-tagged is CD19.

In some embodiments, the stimulatory agent specifically binds to the antibody portion of the recombinant receptor, *e.g.,* CAR. In some cases, the antibody portion of the recombinant receptor includes at least a portion of an immunoglobulin constant region, such as a hinge region, *e.g.,* an IgG4 hinge region, and/or a CH1/CL and/or Fc region. In some embodiments, the constant region or portion is of a human IgG, such as IgG4 or IgG1. In some cases, the reagent is loaded with αIgG that recognizes the IgG4 spacer.

In some embodiments, the desired target is a T cell receptor and/or a component of a T cell receptor. In certain embodiments, the desired target is CD3. In certain embodiment, the desired target is a T cell costimulatory molecule, e.g., CD28, CD137 (4-1-BB), OX40, or ICOS.

In some embodiments, for example when the stimulatory agent is not bound to a stimulatory agent (e.g., oligomeric stimulatory reagent) or a selection reagent, the stimulatory agent is an antibody, a divalent antibody fragment, a F(ab)₂, or a divalent single-chain Fv fragment.

In some embodiments, the stimulatory agent, or each of the one or more stimulatory agent, further contains a binding partner C for binding to the reagent. In some embodiments, the the stimulatory agent, or each of the one or more stimulatory agent, further contains biotin, a biotin analog that reversibly binds to a streptavidin or avidin, a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO:15), Trp-Ser-His-Pro-Gln-Phe-Glu-Lys-(GlyGlyGlySer)3-Trp-Ser-His-Pro-Gln-Phe-Glu-Lys (SEQ ID NO: 17), SAWSHPQFEKGGGSGGGSGGSAWSHPQFEK (SEQ ID NO:16), Trp-Ser-His-Pro-Gln-Phe-Glu-Lys-(GlyGlyGlySer)2-Trp-Ser-His-Pro-Gln-Phe-Glu-Lys (SEQ ID NO: 18) and Trp-Ser-His-Pro-Gin-Phe-Glu-Lys-(GlyGlyGlyser)2Gly-Gly-Ser-Ala-Trp-Ser-His-Pro-Gln-Phe-Glu-Lys (SEQ ID NO: 19), a calmodulin binding peptide that reversibly binds to calmodulin, a FLAG peptide that reversibly binds to an antibody binding the FLAG peptide, and an oligohistidine tag that reversibly binds to an antibody binding the oligohistidine tag.

In some embodiments, the reagent is or contains a streptavidin, streptavidin mutein, aviding or avidin mutein, and the stimulatory agent, or each of the one or more stimulatory agent, contains a binding partner C that is able to bind the such reagent, such as biotin, a biotin analog or a streptavidin-binding peptide. In some embodiments, th stimulatory agent, or each of the one or more stimulatory agent, further comprises biotin, a biotin analog that reversibly binds to a streptavidin or avidin. a streptavidin-binding peptide selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO:15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO:16), Trp-Ser-His-Pro-Gln-Phe-Glu-Lys-(GlyGlyGlySer)₂-Trp-Ser-His-Pro-Gln-Phe-Glu-Lys (SEQ ID NO: 18) and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19). In particular embodiments, the reagent is or contains a streptavidin mutein (e.g. set forth in SEQ ID NO:6) and the binding partner C is a streptavidin-binding peptide, such as any set forth in any one of SEQ ID NOS: 8 or 15-19. In some embodiments, the stimulatory agent, or each of the one or more stimulatory agent, further comprises a streptavidin-binding peptide having the sequence SAWSHPQFEKGGGSGGGSGGSAWSHPQFEK (SEQ ID NO:16).

### 2. Reagent

In some embodiments, the reagent (e.g., selection agent or stimulatory reagent) contains one or a plurality of binding sites Z that are capable of reversibly binding to a binding partners C comprised by the agent (*e.g.,* a selection agent or stimulatory agent). In some embodiments, the reagent contains a plurality of binding sites Z, which each are able to specifically bind to the binding partner C that is included in the agent (*e.g.,* a selection agent or stimulatory agent), such that the reagent is capable of reversibly binding to a plurality of agents (*e.g.,* a selection agent or stimulatory agent), *e.g.,* is a multimerization reagent (e.g., selection reagent or stimulatory reagent). In some embodiments, the reagent is an oligomer or polymer of individual molecules (*e.g.* monomers) or complexes that make up an individual molecule (*e.g.* tetramer), each containing at least one binding site Z. In some embodiments, the reagent contains at least two binding sites Z, at least three binding sites Z, at least four binding sites Z, such as at least 5, 6, 7, 8, 9, 10, 11, 12, 13, 14, 15, 16, 17, 18, 19, 20, 24, 28, 32, 36, 40, 44, 48, 52, 56, 60, 64, 68, 72 or more binding sites Z. The binding sites can all be the same or the plurality of binding sites can contain one or more different binding sites (*e.g.,* Z1, Z2, Z3, etc.).

In some embodiments, two or more agents (*e.g.,* a selection agents or stimulatory agents) associate with, such as are reversibly bound to, the reagent (e.g., selection reagent or stimulatory reagent), such as via the one or plurality of binding sites Z present on the reagent (e.g., selection reagent or stimulatory reagent). In some cases, this results in the agents (*e.g.,* a selection agents or stimulatory agents) being closely arranged to each other such that an avidity effect can take place if a target cell having (at least two copies of) a cell surface molecule is brought into contact with the agent (*e.g.,* a selection agent or stimulatory agent) that has one or more binding sites B able to bind the particular molecule.

In some embodiments, two or more different agents (*e.g.,* a selection agent or stimulatory agent) that are the same, i.e. containing the same binding site B, can be reversibly bound to the reagent. In some embodiments, it is possible to use at least two different (kinds of) agents (*e.g.,* selection agents or stimulatory agents), and in some cases, three or four different (kinds of) agents, *e.g.* two or more different selection agents and/or stimulatory agents. For example, in some embodiments, the reagent (e.g., selection reagent or stimulatory reagent) can be reversibly bound to a first agent (*e.g.,* a selection agent or stimulatory agent) containing a binding site B1, B2, B3 or B4, etc. and a second agent (*e.g.,* selection agent or stimulatory agent) containing another binding site, *e.g.* another of a binding site B1, B2, B3 or B4. In some cases, the binding site of the first agent and the second agent can be the same. For example, in some aspects, each of the at least two agents (*e.g.,* selection agent or stimulatory agent) can bind to the same molecule. In some cases, the binding site of the first agent and the second agent can be different. In some aspects, each of the at least two agents (*e.g.,* selection agent or stimulatory agent) can bind to a different molecule, such as a first molecule, second molecule and so on. In some cases, the different molecules, such as cell surface molecules, can be present on the same target cell. In other cases, the different molecules, such as cell surface molecules, can be present on different target cells that are present in the same population of cells. In some case, a third, fourth and so on agent (*e.g.,* selection agent or stimulatory agent) can be associated with the same reagent (e.g., selection reagent or stimulatory reagent), each containing a further different binding site.

In some embodiments, the two or more different agents (*e.g.,* selection agent or stimulatory agent) contain the same binding partner C. In some embodiments, the two or more different agents (*e.g.,* selection agent or stimulatory agent) contain different binding partners. In some aspects, a first agent (*e.g.,* selection agent or stimulatory agent) can have a binding partner C1 that can specifically bind to a binding site Z1 present on the reagent (e.g., selection reagent or stimulatory reagent) and a second agent (*e.g.,* selection agent or stimulatory agent) can have a binding partner C2 that can specifically bind to the binding site Z1 or to a binding site Z2 present on the reagent (e.g., selection reagent or stimulatory reagent). Thus, in some instances, the plurality of binding sites Z comprised by the reagent includes binding sites Z1 and Z2, which are capable of reversibly binding to binding partners C1 and C2, respectively, comprised by the agent (*e.g.,* selection agent or stimulatory agent). In some embodiments, C1 and C2 are the same, and/or Z1 and Z2 are the same. In other aspects, one or more of the plurality of binding sites Z can be different. In other instances, one or more of the plurality of binding partners C may be different. It is within a level of a skilled artisan to choose any combination of different binding partners C that are compatible with a reagent containing the binding sites Z, as long as each of the binding partners C are able to interact, such as specifically bind, with one of the binding sites Z.

In some embodiments, the reagent (e.g., selection reagent or stimulatory reagent) is a streptavidin, a streptavidin mutein or analog, avidin, an avidin mutein or analog (such as neutravidin) or a mixture thereof, in which such reagent contains one or more binding sites Z for reversible association with a binding partner C. In some embodiments, the binding partner C can be a biotin, a biotin derivative or analog, or a streptavidin-binding peptide or other molecule that is able to specifically bind to streptavidin, a streptavidin mutein or analog, avidin or an avidin mutein or analog. In some embodiments, the reagent is or contains streptavidin, avidin, an analog or mutein of streptavidin, or an analog or mutein or avidin that reversibly binds biotin, a biotin analog or a biologically active fragment thereof. In some embodiments, the reagent (e.g., selection reagent or stimulatory reagent) is or contains an analog or mutein of streptavidin or an analog or mutein of avidin that reversibly binds a streptavidin-binding peptide. In some embodiments, the substance (*e.g.* competitive agent or free binding agent) can be a biotin, a biotin derivative or analog or a streptavidin-binding peptide capable of competing for binding with the binding partner C for the one or more binding sites Z. In some embodiments, the binding partner C and the substance (*e.g.* competitive agent or free binding agent) are different, and the substance (*e.g.* competitive agent or free binding agent) exhibits a higher binding affinity for the one or more binding sites Z compared to the affinity of the binding partner.

In some embodiments, the streptavidin can be wild-type streptavidin, streptavidin muteins or analogs, such as streptavidin-like polypeptides. Likewise, avidin, in some aspects, includes wild-type avidin or muteins or analogs of avidin such as neutravidin, a deglycosylated avidin with modified arginines that typically exhibits a more neutral pi and is available as an alternative to native avidin. Generally, deglycosylated, neutral forms of avidin include those commercially available forms such as "Extravidin", available through Sigma Aldrich, or "NeutrAvidin" available from Thermo Scientific or Invitrogen, for example.

In some embodiments, the reagent (e.g., selection reagent or stimulatory reagent) is a streptavidin or a streptavidin mutein or analog. In some embodiments, wild-type streptavidin (wt-streptavidin) has the amino acid sequence disclosed by Argarana et al, Nucleic Acids Res. 14 (1986) 1871-1882 (SEQ ID NO: 1). In general, streptavidin naturally occurs as a tetramer of four identical subunits, i.e. it is a homo-tetramer, where each subunit contains a single binding site for biotin, a biotin derivative or analog or a biotin mimic. An exemplary sequence of a streptavidin subunit is the sequence of amino acids set forth in SEQ ID NO: 1, but such a sequence also can include a sequence present in homologs thereof from other *Streptomyces* species. In particular, each subunit of streptavidin may exhibit a strong binding affinity for biotin with an equilibrium dissociation constant (K_{D}) on the order of about 10⁻¹⁴ M. In some cases, streptavidin can exist as a monovalent tetramer in which only one of the four binding sites is functional (Howarth et al. (2006) Nat. Methods, 3:267-73; Zhang et al. (2015) Biochem. Biophys. Res. Commun., 463:1059-63)), a divalent tetramer in which two of the four binding sites are functional (Fairhead et al. (2013) J. Mol. Biol., 426:199-214), or can be present in monomeric or dimeric form (Wu et al. (2005) J. Biol. Chem., 280:23225-31; Lim et al. (2010) Biochemistry, 50:8682-91).

In some embodiments, streptavidin may be in any form, such as wild-type or unmodified streptavidin, such as a streptavidin from a *Streptomyces* species or a functionally active fragment thereof that includes at least one functional subunit containing a binding site for biotin, a biotin derivative or analog or a biotin mimic, such as generally contains at least one functional subunit of a wild-type streptavidin from *Streptomyces avidinii* set forth in SEQ ID NO: 1 or a functionally active fragment thereof. For example, in some embodiments, streptavidin can include a fragment of wild-type streptavidin, which is shortened at the N- and/or C-terminus. Such minimal streptavidins include any that begin N-terminally in the region of amino acid positions 10 to 16 of SEQ ID NO: 1 and terminate C-terminally in the region of amino acid positions 133 to 142 of SEQ ID NO: 1. In some embodiments, a functionally active fragment of streptavidin contains the sequence of amino acids set forth in SEQ ID NO: 2. In some embodiments, streptavidin, such as set forth in SEQ ID NO: 2, can further contain an N-terminal methionine at a position corresponding to Ala13 with numbering set forth in SEQ ID NO: 1. Reference to the position of residues in streptavidin or streptavidin muteins is with reference to numbering of residues in SEQ ID NO: 1.

In some aspects, streptavidin muteins include polypeptides that are distinguished from the sequence of an unmodified or wild-type streptavidin by one or more amino acid substitutions, deletions, or additions, but that include at least one functional subunit containing a binding site for biotin, a biotin derivative or analog or a streptavidin-binding peptide. In some aspects, streptavidin-like polypeptides and streptavidin muteins can be polypeptides which essentially are immunologically equivalent to wild-type streptavidin and are in particular capable of binding biotin, biotin derivatives or biotin analogues with the same or different affinity as wt-streptavidin. In some cases, streptavidin-like polypeptides or streptavidin muteins may contain amino acids which are not part of wild-type streptavidin or they may include only a part of wild-type streptavidin. In some embodiments, streptavidin-like polypeptides are polypeptides which are not identical to wild-type streptavidin, since the host does not have the enzymes which are required in order to transform the host-produced polypeptide into the structure of wild-type streptavidin. In some embodiments, streptavidin also may be present as streptavidin tetramers and streptavidin dimers, in particular streptavidin homotetramers, streptavidin homodimers, streptavidin heterotetramers and streptavidin heterodimers. Generally, each subunit normally has a binding site for biotin or biotin analogues or for streptavidin-binding peptides. Examples of streptavidins or streptavidin muteins are mentioned, for example, in WO 86/02077, DE 19641876 A1, US 6,022,951, WO 98/40396 or WO 96/24606.

In some embodiments, a streptavidin mutein can contain amino acids that are not part of an unmodified or wild-type streptavidin or can include only a part of a wild-type or unmodified streptavidin. In some embodiments, a streptavidin mutein contains at least one subunit that can have one more amino acid substitutions (replacements) compared to a subunit of an unmodified or wild-type streptavidin, such as compared to the wild-type streptavidin subunit set forth in SEQ ID NO: 1 or a functionally active fragment thereof, e.g. set forth in SEQ ID NO: 2. In some embodiments, at least one subunit of a streptavidin mutein can have at least 1, 2, 3, 4, 5, 6, 7, 8, 9, 10, 11, 12, 13, 14, 15, 16, 17, 18, 19 or 20 amino acid differences compared to a wild-type or unmodified streptavidin and/or contains at least one subunit that comprising an amino acid sequence that exhibits at least 85%, 86%, 87%, 88%, 89%, 90%, 91%, 92%, 93%, 94%, 95%, 96%, 97%, 98%, 99% or more sequence identity to the sequence of amino acids set forth in SEQ ID NO: 1 or 2, where such streptavidin mutein exhibits functional activity to bind biotin, a biotin derivative or analog or biotin mimic. In some embodiments, the amino acid replacements (substitutions) are conservative or non-conservative mutations. Examples of streptavidin muteins are known in the art, see *e.g.,* U.S. Pat. No. 5,168,049; 5,506,121; 6,022,951; 6,156,493; 6,165,750; 6,103,493; or 6,368,813; or International published PCT App. No. WO2014/076277.

In some embodiments, streptavidin or a streptavidin mutein includes proteins containing one or more than one functional subunit containing one or more binding sites Z for biotin, a biotin derivative or analog or a streptavidin-binding peptide, such as two or more, three or more, four or more, and, in some cases, 5, 6, 7, 8, 9, 10, 11, 12 or more functional subunits. In some embodiments, streptavidin or streptavidin mutein can include a monomer; a dimer, including a heterodimer or a homodimer; a tetramer, including a homotetramer, a heterotetramer, a monovalent tetramer or a divalent tetramer; or can include higher ordered multimers or oligomers thereof.

In some embodiments, the binding affinity of streptavidin or a streptavidin mutein for a peptide ligand binding partner is less than 1 x 10⁻⁴ M, 5 x 10⁻⁴ M, 1 x 10⁻⁵ M, 5x 10⁻⁵ M, 1 x 10⁻⁶ M, 5 x 10⁻⁶ M or 1 x 10⁻⁷ M, but generally greater than 1 x 10⁻¹³ M, 1 x 10⁻¹² M or 1 x 10⁻¹¹ M. For example, peptide sequences (Strep-tags), such as disclosed in U.S. Pat. No. 5,506,121, can act as biotin mimics and demonstrate a binding affinity for streptavidin, e.g., with a K_{D} of approximately between 10⁻⁴ M and 10⁻⁵ M. In some cases, the binding affinity can be further improved by making a mutation within the streptavidin molecule, see *e.g.* U.S. Pat. No. 6,103,493 or International published PCT App. No. WO2014/076277. In some embodiments, binding affinity can be determined by methods known in the art, such as any described below.

In some embodiments, the reagent (e.g., selection reagent or stimulatory reagent), such as a streptavidin or streptavidin mutein, exhibits binding affinity for a peptide ligand binding partner, which peptide ligand binding partner can be the binding partner C present in the agent (*e.g.,* selection agent or stimulatory agent). In some embodiments, the peptide sequence contains a sequence with the general formula set forth in SEQ ID NO: 9, such as contains the sequence set forth in SEQ ID NO: 10. In some embodiments, the peptide sequence has the general formula set forth in SEQ ID NO: 11, such as set forth in SEQ ID NO: 12. In one example, the peptide sequence is Trp-Arg-His-Pro-Gln-Phe-Gly-Gly (also called Strep-tag^{®}, set forth in SEQ ID NO: 7). In one example, the peptide sequence is Trp-Ser-His-Pro-Gln-Phe-Glu-Lys (also called Strep-tag^{®} II, set forth in SEQ ID NO: 8). In some embodiments, the peptide ligand contains a sequential arrangement of at least two streptavidin-binding modules, wherein the distance between the two modules is at least 0 and not greater than 50 amino acids, wherein one binding module has 3 to 8 amino acids and contains at least the sequence His-Pro-Xaa (SEQ ID NO: 9), where Xaa is glutamine, asparagine, or methionine, and wherein the other binding module has the same or different streptavidin peptide ligand, such as set forth in SEQ ID NO: 11 (see *e.g.* International Published PCT Appl. No. WO02/077018; U.S. Patent No. 7,981,632). In some embodiments, the peptide ligand contains a sequence having the formula set forth in any of SEQ ID NO: 13 or 14. In some embodiments, the peptide ligand has the sequence of amino acids set forth in any of SEQ ID NOS: 15-19.

In some embodiments, the reagent (e.g., selection reagent or stimulatory reagent) is or contains a streptavidin mutein. In some embodiments, the streptavidin muteins contain one or more mutations (*e.g.* amino acid replacements) compared to wild-type streptavidin set forth in SEQ ID NO: 1 or a biologically active portion thereof. For example, biologically active portions of streptavidin can include streptavidin variants that are shortened at the N- and/or the C-terminus, which in some cases is called a minimal streptavidin. In some embodiments, an N-terminally shortened minimal streptavidin, to which any of the mutations can be made, begins N-terminally in the region of the amino acid positions 10 to 16 and terminates C-terminally in the region of the amino acid positions 133 to 142 compared to the sequence set forth in SEQ ID NO: 1. In some embodiments, an N-terminally shortened streptavidin, to which any of the mutations can be made, contains the amino acid sequence set forth in SEQ ID NO: 2. In some embodiments, the minimal streptavidin contains an amino acid sequence from position Ala13 to Ser139 and optionally has an N-terminal methionine residue instead of Ala13. For purposes herein, the numbering of amino acid positions refers throughout to the numbering of wt-streptavidin set forth in SEQ ID NO: 1 *(e.g.* Argarana et al., Nucleic Acids Res. 14 (1986), 1871-1882, cf. also Fig. 3).

In some embodiments, the streptavidin mutein is a mutant as described in U.S. Pat. No. 6,103,493. In some embodiments, the streptavidin mutein contains at least one mutation within the region of amino acid positions 44 to 53, based on the amino acid sequence of wild-type streptavidin, such as set forth in SEQ ID NO: 1. In some embodiments, the streptavidin mutein contains a mutation at one or more residues 44, 45, 46, and/or 47. In some embodiments, the streptavidin mutein contains a replacement of Glu at position 44 of wild-type streptavidin with a hydrophobic aliphatic amino acid, *e.g.* Val, Ala, Ile or Leu, any amino acid at position 45, an aliphatic amino acid, such as a hydrophobic aliphatic amino acid at position 46 and/or a replacement of Val at position 47 with a basic amino acid, *e.g.* Arg or Lys, such as generally Arg. In some embodiments, Ala is at position 46 and/or Arg is at position 47 and/or Val or Ile is at position 44. In some embodiments, the streptavidin mutant contains residues Val⁴⁴-Thr⁴⁵-Ala⁴⁶-Arg⁴⁷, such as set forth in exemplary streptavidin muteins containing the sequence of amino acids set forth in SEQ ID NO: 3 or SEQ ID NO: 4 (also known as streptavidin mutant 1, SAM1). In some embodiments, the streptavidin mutein contains residues Ile⁴⁴-Gly⁴⁵-Ala⁴⁶-Arg⁴⁷, such as set forth in exemplary streptavidin muteins containing the sequence of amino acids set forth in SEQ ID NO: 5 or 6 (also known as SAM2). In some cases, such streptavidin mutein are described, for example, in US patent 6,103,493, and are commercially available under the trademark Strep-Tactin^{®}.

In some embodiment, the streptavidin mutein is a mutant as described in International Published PCT Appl. Nos. WO 2014/076277. In some embodiments, the streptavidin mutein contains at least two cysteine residues in the region of amino acid positions 44 to 53 with reference to amino acid positions set forth in SEQ ID NO: 1. In some embodiments, the cysteine residues are present at positions 45 and 52 to create a disulfide bridge connecting these amino acids. In such an embodiment, amino acid 44 is typically glycine or alanine and amino acid 46 is typically alanine or glycine and amino acid 47 is typically arginine. In some embodiments, the streptavidin mutein contains at least one mutation or amino acid difference in the region of amino acids residues 115 to 121 with reference to amino acid positions set forth in SEQ ID NO: 1. In some embodiments, the streptavidin mutein contains at least one mutation at amino acid position 117, 120 and 121 and/or a deletion of amino acids 118 and 119 and substitution of at least amino acid position 121.

In some embodiments, the streptavidin mutein contains a mutation at a position corresponding to position 117, which mutation can be to a large hydrophobic residue like Trp, Tyr or Phe or a charged residue like Glu, Asp or Arg or a hydrophilic residue like Asn or Gin, or, in some cases, the hydrophobic residues Leu, Met or Ala, or the polar residues Thr, Ser or His. In some embodiments, the mutation at position 117 is combined with a mutation at a position corresponding to position 120, which mutation can be to a small residue like Ser or Ala or Gly, and a mutation at a position corresponding to position 121, which mutation can be to a hydrophobic residue, such as a bulky hydrophobic residue like Trp, Tyr or Phe. In some embodiments, the mutation at position 117 is combined with a mutation at a position corresponding to position 120 of wildtype streptavidin set forth in SEQ ID NO:1 or a biologically active fragment thereof, which mutation can be a hydrophobic residue such as Leu, Ile, Met, or Val or, generally, Tyr or Phe, and a mutation at a position corresponding to position 121 compared to positions of wildtype streptavidin set forth in SEQ ID NO:1 or a biologically active fragment thereof, which mutation can be to a small residue like Gly, Ala, or Ser, or with Gln, or with a hydrophobic residue like Leu, Val, Ile, Trp, Tyr, Phe, or Met. In some embodiments, such muteins also can contain residues Val⁴⁴-Thr⁴⁵-Ala⁴⁶-Arg⁴⁷ or residues Ile⁴⁴-Gly⁴⁵-Ala⁴⁶-Arg⁴⁷. In some embodiments, the streptavidin mutein contains the residues Val⁴⁴ , Thr⁴⁵, Ala⁴⁶, Arg⁴⁷, Glu¹¹⁷, Gly¹²⁰ and Tyr¹²¹. In some embodiments, the mutein streptavidin contains the sequence of amino acids set forth in SEQ ID NO:27 or SEQ ID NO:28, or a sequence of amino acids that exhibits at least 85%, 86%, 87%, 88%, 89%, 90%, 91%, 92%, 93%, 94%, 95%, 96%, 97%, 98%, 99% or more sequence identity to the sequence of amino acids set forth in SEQ ID NO: 27 or SEQ ID NO: 28, contains the residues Val⁴⁴, Thr⁴⁵, Ala⁴⁶, Arg⁴⁷, Glu¹¹⁷, Gly¹²⁰ and Tyr¹²¹ and exhibits functional activity to bind to biotin, a biotin analog or a streptavidin-binding peptide.

In some embodiments, a streptavidin mutein can contain any of the above mutations in any combination, and the resulting streptavidin mutein may exhibit a binding affinity that is less than 2.7 x 10⁻⁴ M for the peptide ligand (Trp-Arg-His-Pro-Gln-Phe-Gly-Gly; also called Strep-tag^{®}, set forth in SEQ ID NO: 7) and/or less than 1.4 x 10⁻⁴ M for the peptide ligand (Trp-Ser-His-Pro-Gln-Phe-Glu-Lys; also called Strep-tag^{®} II, set forth in SEQ ID NO: 8) and/or is less than 1 x 10⁻⁴ M, 5 x 10⁻⁴ M, 1 x 10⁻⁵ M, 5x 10⁻⁵ M, 1 x 10⁻⁶ M, 5 x 10⁻⁶ M or 1 x 10⁻⁷ M, but generally greater than 1 x 10⁻¹³ M, 1 x 10⁻¹² M or 1 x 10⁻¹¹ M for any of the peptide ligands set forth in any of SEQ ID NOS:7-19.

In some embodiments, the streptavidin mutein exhibits the sequence of amino acids set forth in any of SEQ ID NOs: 3-6, 27, or 28, or a sequence of amino acids that exhibits at least 90%, 91%, 92%, 93%, 94%, 95%, 96%, 97%, 98%, 99% or more sequence identity to the sequence of amino acids set forth in any of SEQ ID NO: 3-6, 27, or 28, and exhibits a binding affinity that is less than 2.7 x 10⁻⁴ M for the peptide ligand (Trp Arg His Pro Gln Phe Gly Gly; also called Strep-tag^{®}, set forth in SEQ ID NO: 7) and/or less than 1.4 x 10⁻⁴ M for the peptide ligand (Trp Ser His Pro Gln Phe Glu Lys; also called Strep-tag^{®} II, set forth in SEQ ID NO: 8) and/or is less than 1 x 10⁻⁴ M, 5 x 10⁻⁴ M, 1 x 10⁻⁵ M, 5x 10⁻⁵ M, 1 x 10⁻⁶ M, 5 x 10⁻⁶ M or 1 x 10⁻⁷ M, but generally greater than 1 x 10⁻¹³ M, 1 x 10⁻¹² M or 1 x 10⁻¹¹ M for any of the peptide ligands set forth in any of SEQ ID NOS:7-19.

In some embodiments, the streptavidin mutein comprises the sequence of amino acids set forth in any of SEQ ID NOs: 3-6, 27, or 28, and the streptavidin-binding peptide comprises the sequence of amino acids set forth in any of SEQ ID NOs: 7-19. In some embodiments, the streptavidin mutein comprises the sequence of amino acids set forth in SEQ ID NO: 6, and the streptavidin-binding peptide comprises the sequence of amino acids set forth in any of SEQ ID NOs: 7-19. In some embodiments, the streptavidin mutein comprises the sequence of amino acids set forth in any of SEQ ID NOs: 3-6, 27, or 28, and the streptavidin-binding peptide comprises the sequence of amino acids set forth in SEQ ID NO: 16. In some embodiments, the streptavidin mutein comprises the sequence of amino acids set forth in SEQ ID NO: 6, and the streptavidin-binding peptide comprises the sequence of amino acids set forth in SEQ ID NO: 16.

In some embodiments, the streptavidin mutein also exhibits binding to other streptavidin ligands, such as but not limited to, biotin, iminobiotin, lipoic acid, desthiobiotin, diaminobiotin, HABA (hydroxyazobenzene-benzoic acid) and/or dimethyl-HABA. In some embodiments, the streptavidin mutein exhibits a binding affinity for another streptavidin ligand, such as biotin or desthiobiotin, that is greater than the binding affinity of the streptavidin mutein for a biotin mimic peptide ligand, such as set forth in any of SEQ ID NOS: 7-19. Thus, in some embodiments, biotin or a biotin analog or derivative (*e.g.* desthiobiotin) can be employed as a competition agent in the provided methods. For example, as an example, the interaction of a mutein streptavidin designated Strep-tactin^{®} *(e.g.* containing the sequence set forth in SEQ ID NO: 4) with the peptide ligand designated Strep-tag^{®} II (*e.g.* set forth in SEQ ID NO: 8) is characterized by a binding affinity with a K_{D} of approximately 10⁻⁶ M compared to approximately 10⁻¹³ M for the biotin-streptavidin interaction. In some cases, biotin, which can bind with high affinity to the Strep-tactin^{®} with a K_{D} of between or between about 10⁻¹⁰ and 10⁻¹³ M, can compete with Strep-tag^{®} II for the binding site.

In some cases, the reagent (e.g., selection reagent or stimulatory reagent) contains at least two chelating groups K that may be capable of binding to a transition metal ion. In some embodiments, the reagent (e.g., selection reagent or stimulatory reagent) may be capable of binding to an oligohistidine affinity tag, a glutathione-S-transferase, calmodulin or an analog thereof, calmodulin binding peptide (CBP), a FLAG-peptide, an HA-tag, maltose binding protein (MBP), an HSV epitope, a myc epitope, and/or a biotinylated carrier protein.

In some embodiments, the reagent (e.g., selection reagent or stimulatory reagent) is an oligomer or polymer. In some embodiments, the oligomer or polymer can be generated by linking directly or indirectly individual molecules of the protein as it exists naturally, either by linking directly or indirectly individual molecules of a monomer or a complex of subunits that make up an individual molecule (*e.g.* linking directly or indirectly dimers, trimers, tetramers, etc. of a protein as it exists naturally). For example, a tetrameric homodimer or heterodimer of streptavidin or avidin may be referred to as an individual molecule or smallest building block of a respective oligomer or polymer. In some embodiments, the oligomer or polymer can contain linkage of at least 2 individual molecules of the protein (*e.g.* is a 2-mer), or can be at least a 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 25-mer, 30-mer, 35-mer, 40-mer, 45-mer or 50-mer of individual molecules of the protein (*e.g.,* monomers, tetramers).

Oligomers can be generated using any methods known in the art, such as any described in published U.S. Patent Application No. US2004/0082012. In some embodiments, the oligomer or polymer contains two or more individual molecules that may be crosslinked, such as by a polysaccharide or a bifunctional linker.

In some embodiments, the oligomer or polymer is obtained by crosslinking individual molecules or a complex of subunits that make up an individual molecule in the presence of a polysaccharide. In some embodiments, oligomers or polymers can be prepared by the introduction of carboxyl residues into a polysaccharide, *e.g.* dextran. In some aspects, individual molecules of the reagent (*e.g.,* monomers, tetramers) can be coupled via primary amino groups of internal lysine residues and/or the free N-terminus to the carboxyl groups in the dextran backbone using conventional carbodiimide chemistry. In some embodiments, the coupling reaction is performed at a molar ratio of about 60 moles of individual molecules of the reagent (*e.g.,* monomers, tetramers) per mole of dextran.

In some embodiments, the reagent (e.g., selection reagent or stimulatory reagent) is an oligomer or a polymer of one or more streptavidin or avidin or of any analog or mutein of streptavidin (*e.g.* Strep-Tactin^{®} or Strep-Tactin^{®} XT) or an analog or mutein of avidin (*e.g.* neutravidin). In some embodiments, the binding site Z is a natural biotin binding site of avidin or streptavidin for which there can be up to four binding sites in an individual molecule (*e.g.* a tetramer contains four binding sites Z), whereby a homo-tetramer can contain up to 4 binding sites that are the same, *i.e.* Z1, whereas a hetero-tetramer can contain up to 4 binding sites that may be different, *e.g.* containing Z1 and Z2. In some embodiments, the oligomer is generated or produced from a plurality of individual molecules (*e.g.* a plurality of homo-tetramers) of the same streptavidin, streptavidin mutein, avidin or avidin mutein, in which case each binding site Z, *e.g.* Z1, of the oligomer is the same. For example, in some cases, an oligomer can contain a plurality of binding sites Z1, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more binding sites Z1. In some embodiments, the oligomer is generated or produced from a plurality of individual molecules that can be hetero-tetramers of a streptavidin, streptavidin mutein, avidin or avidin mutein and/or from a plurality of two or more different individual molecules (*e.g.* different homo-tetramers) of streptavidin, streptavidin mutein, avidin or avidin mutein that differ in their binding sites Z, *e.g.* Z1 and Z2, in which case a plurality of different binding sites Z, *e.g.* Z1 and Z2, may be present in the oligomer. For example, in some cases, an oligomer can contain a plurality of binding sites Z1 and a plurality of binding sites Z, which, in combination, can include at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more combined binding sites Z1 and Z2.

In some cases, the respective oligomer or polymer may be crosslinked by a polysaccharide. In one embodiment, oligomers or polymers of streptavidin or of avidin or of analogs of streptavidin or of avidin (*e.g.,* neutravidin) can be prepared by the introduction of carboxyl residues into a polysaccharide, e. g. dextran, essentially as described in Noguchi, A, et al, Bioconjugate Chemistry (1992) 3,132-137 in a first step. In some such aspects, streptavidin or avidin or analogs thereof then may be linked via primary amino groups of internal lysine residue and/or the free N-terminus to the carboxyl groups in the dextran backbone using conventional carbodiimide chemistry in a second step. In some cases, cross-linked oligomers or polymers of streptavidin or avidin or of any analog of streptavidin or avidin may also be obtained by crosslinking via bifunctional molecules, serving as a linker, such as glutardialdehyde or by other methods described in the art.

In some embodiments, the oligomer or polymer is obtained by crosslinking individual molecules or a complex of subunits that make up an individual molecule using a bifunctional linker or other chemical linker, such as glutardialdehyde or by other methods known in the art. In some aspects, cross-linked oligomers or polymers of streptavidin or avidin or of any mutein or analog of streptavidin or avidin may be obtained by crosslinking individual streptavidin or avidin molecules via bifunctional molecules, serving as a linker, such as glutardialdehyde or by other methods described in the art. It is, for example, possible to generate oligomers of streptavidin muteins by introducing thiol groups into the streptavidin mutein (this can, for example, be done by reacting the streptavidin mutein with 2-iminothiolan (Trauts reagent) and by activating, for example in a separate reaction, amino groups available in the streptavidin mutein. In some embodiments, this activation of amino groups can be achieved by reaction of the streptavidin mutein with a commercially available heterobifunctional crosslinker such as sulfosuccinimidyl 4-(N-maleimidomethyl)cyclohexane-1-carboxylate (sulfo SMCC) or Succinimidyl-6-[(β-maleimidopropionamido)hexanoate (SMPH). In some such embodiments, the two reaction products so obtained are mixed together, typically leading to the reaction of the thiol groups contained in the one batch of modified streptavidin mutein with the activated (such as by maleimide functions) amino acids of the other batch of modified streptavidin mutein. In some cases, by this reaction, multimers/oligomers of the streptavidin mutein are formed. These oligomers can have any suitable number of individual molecules, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more, and the oligomerization degree can be varied according to the reaction condition.

In some embodiments, the oligomeric or polymeric reagent (e.g., selection reagent or stimulatory reagent) can be isolated via size exclusion chromatography and any desired fraction can be used as the reagent. For example, in some embodiments, after reacting the modified streptavidin mutein, in the presence of 2-iminothiolan and a heterobifunctional crosslinker such as sulfo SMCC, the oligomeric or polymeric reagent can be isolated via size exclusion chromatography and any desired fraction can be used as the reagent. In some embodiments, the oligomers do not have (and do not need to have) a single molecular weight but they may observe a statistical weight distribution such as Gaussian distribution. In some cases, any oligomer with more than three streptavidin or mutein tetramers, *e.g.,* homotetramers or heterotetramers, can be used as a soluble reagent, such as generally 3 to 50 tetramers, *e.g.,* homotetramers or heterotetramers, 10 to 40 tetramers, *e.g.,* homotetramers or heterotetramers, or 25 to 35 tetramers, *e.g.,* homotetramers or heterotetramers. The oligomers might have, for example, from 3 to 25 streptavidin mutein tetramers, *e.g.,* homotetramers or heterotetramers. In some aspects, with a molecular weight of about 50 kDa for streptavidin muteins, the soluble oligomers can have a molecular weight from about 150 kDa to about 2000 kDa, about 150 kDa to about 1500 kDa, about 150 kDa to about 1250 kDa, about 150 kDa to 1000 kDa, about 150 kDa to about 500 kDa or about 150 kDa to about 300 kDa, about 300 kDa to about 2000 kDa, about 300 kDa to about 1500 kDa, about 300 kDa to about 1250 kDa, about 300 kDa to 1000 kDa, about 300 kDa to about 500 kDa, about 500 kDa to about 2000 kDa, about 500 kDa to about 1500 kDa, about 500 kDa to about 1250 kDa, about 500 kDa to 1000 kDa, about 1000 kDa to about 2000 kDa, about 1000 kDa to about 1500 kDa, about 1000 kDa to about 1250 kDa, about 1250 kDa to about 2000 kDa or about 1500 kDa to about 2000 kDa. Generally, because each streptavidin molecule/mutein has four biotin binding sites, such a reagent can provide 12 to 160 binding sites Z, such as 12 to 100 binding sites Z.

### a. Oligomeric Stimulatory Reagents

In particular embodiments, the stimulatory reagent contains an oligomeric stimulatory reagent, e.g., a streptavidin mutein reagent, that is conjugated, linked, or attached to one or more stimulatory agent. As described above, in some embodiments, the one or more stimulatory agents have an attached binding domain or binding partner (e.g., a binding partner C) that is capable of binding to oligomeric stimulatory reagent at a particular binding sites (e.g., binding site Z). In some embodiments, a plurality of the stimulatory agent is reversibly bound to the oligomeric stimulatory reagent. In various embodiments, the oligomeric stimulatory reagent has a plurality of the particular binding sites, Z, which, in certain embodiments, are reversibly bound to a plurality of stimulatory agents at the binding domain (e.g., binding partner C). In some embodiments, the amount of bound agents are reduced or decreased in the presence of a competition agent, e.g., an agent that is also capable of binding to the particular binding sites (e.g., binding site Z).

In some embodiments, the stimulatory reagent is or includes a reversible system in which at least one stimulatory agent (e.g., a stimulatory agent that is capable of producing a signal in a cell such as a T cell) is associated, e.g., reversibly associated, with the oligomeric stimulatory reagent. In some embodiments, the reagent contains a plurality of binding sites capable of binding, e.g., reversibly binding, to the stimulatory agent. In some cases, the reagent is an oligomeric stimulatory reagent having at least one attached agent capable of producing a signal (e.g., stimulatory signal) in a cell such as a T cell. In some embodiments, the stimulatory agent contains at least one binding site, e.g., a binding site B, that can specifically bind an epitope or region of the molecule and also contains a binding partner, also referred to herein as a binding partner C, that specifically binds to at least one binding site of the oligomeric stimulatory reagent, e.g., binding site Z of the reagent. In some embodiments, the binding interaction between the binding partner C and the at least one binding site Z is a non-covalent interaction. In some cases, the binding interaction between the binding partner C and the at least one binding site Z is a covalent interaction. In some embodiments, the binding interaction, such as non-covalent interaction, between the binding partner C and the at least one binding site Z is reversible.

Substances that may be used as oligomeric stimulatory reagents in such reversible systems are known, see *e.g.,* U.S. Patent Nos. 5,168,049; 5,506,121; 6,103,493; 7,776,562; 7,981,632; 8,298,782; 8,735,540; 9,023,604; and International published PCT Appl. Nos. WO2013/124474 and WO2014/076277. Non-limiting examples of reagents and binding partners capable of forming a reversible interaction, as well as substances (e.g. competition agents) capable of reversing such binding, are described below.

In some embodiments, the oligomeric stimulatory reagent is an oligomer of streptavidin, streptavidin mutein or analog, avidin, an avidin mutein or analog (such as neutravidin) or a mixture thereof, in which such oligomeric stimulatory reagent contains one or more binding sites for reversible association with the binding domain of the stimulatory agent (e.g., a binding partner C). In some embodiments, the binding domain of the stimulatory agent can be a biotin, a biotin derivative or analog, or a streptavidin-binding peptide or other molecule that is able to specifically bind to streptavidin, a streptavidin mutein or analog, avidin or an avidin mutein or analog.

In certain embodiments, one or more stimulatory agents (e.g., agents that are capable of producing a signal in a cell such as a T cell) associate with, such as are reversibly bound to, the oligomeric stimulatory reagent, such as via the plurality of the particular binding sites (e.g., binding sites Z) present on the oligomeric stimulatory reagent. In some cases, this results in the stimulatory agents being closely arranged to each other such that an avidity effect can take place if a target cell having (at least two copies of) a cell surface molecule that is bound by or recognized by the stimulatory agent is brought into contact with the agent.

In some embodiments, the oligomeric stimulatory reagent is a streptavidin oligomer, a streptavidin mutein oligomer, a streptavidin analog oligomer, an avidin oligomer, an oligomer composed of avidin mutein or avidin analog (such as neutravidin) or a mixture thereof. In particular embodiments, the oligomeric stimulatory reagents contain particular binding sites that are capable of binding to a binding domain (e.g., the binding partner C) of a stimulatory agent. In some embodiments, the binding domain can be a biotin, a biotin derivative or analog, or a streptavidin-binding peptide or other molecule that is able to specifically bind to streptavidin, a streptavidin mutein or analog, avidin or an avidin mutein or analog. Examples of streptavidin, a streptavidin mutein, a streptavidin analog, an avidin, an avidin mutein or avidin analog (such as neutravidin) and binding domain molecules, e.g., biotin, a biotin derivative or analog, or a streptavidin-binding peptide or other molecule that is able to specifically bind to streptavidin, a streptavidin mutein or analog, avidin or an avidin mutein or analog, contemplated as comprising the oligomeric stimulatory reagent system are described in Section II-B. The methods provided herein further contemplate that the oligomeric stimulatory reagent may comprise a molecules capable of binding to an oligohistidine affinity tag, a glutathione-S-transferase, calmodulin or an analog thereof, calmodulin binding peptide (CBP), a FLAG-peptide, an HA-tag, maltose binding protein (MBP), an HSV epitope, a myc epitope, and/or a biotinylated carrier protein (*see* Section II-B).

In particular embodiments provided herein, is an oligomeric stimulatory reagent that is composed of and/or contains a plurality of streptavidin or streptavidin mutein tetramers. In certain embodiments, the oligomeric stimulatory reagent provided herein contains a plurality of binding sites that reversibly bind or are capable of reversibly binding to one or more stimulatory agents. In some embodiments, the oligomeric stimulatory reagent has a radius, e.g., an average radius, of between 70 nm and 125 nm, inclusive; a molecular weight of between 1 x 10⁷ g/mol and 1 x 10⁹ g/mol, inclusive; and/or between 1,000 and 5,000 streptavidin or streptavidin mutein tetramers, inclusive. In some embodiments, the oligomeric stimulatory reagent is bound, e.g., reversibly bound, to one or more stimulatory agents such as an agent that binds to a molecule, e.g. receptor, on the surface of a cell. In certain embodiments, the one or more stimulatory agents are agents described herein, e.g., in Section II-B. In some embodiments, the one or more stimulatory agent contains a monovalent binding site (e.g., binding site B). In some embodiments, the monovalent binding site binds to CD3. In some embodiments, the monovalent binding site binds to costimulatory molecule, for example as described herein. In some embodiments, the monovalent binding site binds to CD28. In some embodiments, the one or more stimulatory agents contain a monovalent binding site capable of binding to CD3 and/or CD28. In some embodiments, the stimulatory agent is an anti-CD3 and/or an anti-CD28 antibody or antigen binding fragment thereof, such as an antibody or antigen fragment thereof that contains a binding partner, C, e.g., a streptavidin binding peptide, e.g. Strep-tag^{®} II. In particular embodiments, the one or more agents is an anti-CD3 and/or an anti CD28 Fab containing a binding partner, e.g., a streptavidin binding peptide, e.g. Strep-tag^{®} II. In particular embodiments, the one or more agents comprise a streptavidin-based oligomer, such as a streptavidin mutein oligomer conjugated to Strep-tagged anti-CD3 and Strep-tagged anti-CD28 Fabs. In some embodiments, the oligomeric stimulatory reagent is any as described in WO2015/158868 or WO2018/197949.

In some embodiments, provided herein is an oligomeric stimulatory reagent that is composed of and/or contains a plurality of streptavidin or streptavidin mutein tetramers. In certain embodiments, the oligomeric stimulatory reagent provided herein contains a plurality of binding sites that reversibly bind or are capable of reversibly binding to one or more stimulatory agents. In some embodiments, the oligomeric particle has a radius, e.g., an average radius, of between 80 nm and 120 nm, inclusive; a molecular weight, e.g., an average molecular weight of between 7.5 x 10⁶ g/mol and 2 x 10⁸ g/mol, inclusive; and/or an amount, e.g., an average amount, of between 500 and10,000 streptavidin or streptavidin mutein tetramers, inclusive. In some embodiments, the oligomeric stimulatory reagent is bound, e.g., reversibly bound, to one or more stimulatory agents, such as an agent that binds to a molecule, e.g. receptor, on the surface of a cell. In certain embodiments, the one or more stimulatory agents are agents described herein, e.g., in Section II-B. In some embodiments, the stimulatory agent is an anti-CD3 and/or an anti-CD28 antibody or antigen binding fragment thereof, such as an antibody or antigen fragment thereof that contains a binding partner, C, e.g., a streptavidin binding peptide, e.g. Strep-tag^{®} II. In particular embodiments, the one or more agents is an anti-CD3 and/or an anti CD28 Fab containing a binding partner, e.g., a streptavidin binding peptide, e.g. Twin-Strep-tag (e.g., SEQ ID NO:16).

In some embodiments, the cells are stimulated in the presence of, of about, or of at least 0.01 µg, 0.02 µg, 0.03 µg, 0.04 µg, 0.05 µg, 0.1 µg, 0.2 µg, 0.3 µg, 0.4 µg, 0.5 µg, 0.75 µg, 1 µg, 2 µg, 2.2 µg, 2.4 µg, 2.6 µg, 2.8 µg, 3 µg, 4 µg, 5 µg, 6 µg, 7 µg, 8 µg, 9 µg, or 10 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In some embodiments, the cells are stimulated in the presence of or of about 4 µg per 10⁶ cells. In particular embodiments, the cells are stimulated in the presence of or of about 0.8 µg per 10⁶ cells. In certain aspects, 4 µg of the oligomeric stimulatory reagent is or includes 3 µg of oligomeric particles and 1 µg of attached agents, e.g., 0.5 µg of anti-CD3 Fabs and 0.5 µg of anti-CD28 Fabs.

In some embodiments, the cells are stimulated or subjected to stimulation in the presence of or of about 3 µg of the oligomeric stimulatory reagent (e.g., the streptavidin-based oligomer, such as a such as a streptavidin mutein oligomer, conjugated to Strep-tagged anti-CD3 and Strep-tagged anti-CD28 Fabs) per 10⁶ cells. In some embodiments, the cells are stimulated or subjected to stimulation in the presence of or of about 2.75 µg of the oligomeric stimulatory reagent (e.g., the streptavidin-based oligomer, such as a such as a streptavidin mutein oligomer, conjugated to Strep-tagged anti-CD3 and Strep-tagged anti-CD28 Fabs) per 10⁶ cells. In some embodiments, the cells are stimulated or subjected to stimulation in the presence of or of about 2.5 µg of the oligomeric stimulatory reagent (e.g., the streptavidin-based oligomer, such as a such as a streptavidin mutein oligomer, conjugated to Strep-tagged anti-CD3 and Strep-tagged anti-CD28 Fabs) per 10⁶ cells. In some embodiments, the cells are stimulated or subjected to stimulation in the presence of or of about 2.25 µg of the oligomeric stimulatory reagent (e.g., the streptavidin-based oligomer, such as a such as a streptavidin mutein oligomer, conjugated to Strep-tagged anti-CD3 and Strep-tagged anti-CD28 Fabs) per 10⁶ cells. In some embodiments, the cells are stimulated or subjected to stimulation in the presence of or of about 2 µg of the oligomeric stimulatory reagent (e.g., the streptavidin-based oligomer, such as a such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 1.8 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 1.6 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 1.4 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 1.2 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 1 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In particular embodiments, the cells are stimulated or subjected to stimulation in the presence of or of about 0.8 µg of the oligomeric stimulatory reagent (e.g., the streptavidin-based oligomer, such as a streptavidin mutein oligomer, conjugated to Strep-tagged anti-CD3 and Strep-tagged anti-CD28 Fabs) per 10⁶ cells. In some embodiments, the cells are stimulated or subjected to stimulation in the presence of or of about 10 x 10⁸, 9 x 10⁸, 8 x 10⁸, 7 x 10⁸, 6 x 10⁸, 5 x 10⁸, 4 x 10⁸, 3 x 10⁸, 2 x 10⁸, 1 x 10⁸ oligomeric stimulatory reagents. In some embodiments, the cells are stimulated or subjected to stimulation in the presence of or of about 7 x 10⁸ , 6 x 10⁸, 5 x 10⁸ , 4 x 10⁸, 3 x 10⁸ oligomeric stimulatory reagents. In some embodiments, the cells are stimulated or subjected to stimulation in the presence of or of about 7 x 10⁸ to 3 x 10⁸ oligomeric stimulatory reagents. In some embodiments, the cells are stimulated or subjected to stimulation in the presence of or of about 6 x 10⁸ to 4 x 10⁸ oligomeric stimulatory reagents. In some embodiments, the cells are stimulated or subjected to stimulation in the presence of or of about 6 x 10⁸ to 5 x 10⁸ oligomeric stimulatory reagents. In some embodiments, the cells are stimulated or subjected to stimulation in the presence of or of about 5 x 10⁸ oligomeric stimulatory reagents.

In some embodiments, the cells, e.g., selected cells of a sample, are stimulated or subjected to stimulation in the presence of a ratio of oligomeric stimulatory reagent to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular embodiments, the ratio of oligomeric stimulatory reagent to cells is between 2.5:1 and 0.2:1, between 2:1 and 0.5:1, between 1.5:1 and 0.75:1, between 1.25:1 and 0.8:1, between 1.1:1 and 0.9:1. In particular embodiments, the ratio of oligomeric stimulatory reagent to cells is about 1:1 or is 1:1. In particular embodiments, the ratio of oligomeric stimulatory reagent to cells is about 0.3:1 or is 0.3:1. In particular embodiments, the ratio of oligomeric stimulatory reagent to cells is about 0.2:1 or is 0.2:1.

### C. Cell Selection by Chromatography

Using a device disclosed herein, provided herein are methods, which are not in accordance with the claimed invention, in which cells of a sample, e.g., T cells, are selected by chromatographic isolation, such as by column chromatography including affinity chromatography or gel permeation chromatography. In some embodiments, the method, which is not in accordance with the claimed invention, employs a selection agent that binds to a selection marker that is located on the surface of a target cell, e.g., the cell to be isolated, selected, or enriched. Such methods, which are not in accordance with the claimed invention, may be described as (traceless) cell affinity chromatography technology (CATCH) and may include any of the methods or techniques described in PCT Application Nos. WO2013124474 and WO2015164675. Exemplary selection agents are described in Section II-B-1.

In any of the preceding embodiments, the sample can be or comprise a whole blood sample, a buffy coat sample, a peripheral blood mononuclear cell (PBMC) sample, an unfractionated T cell sample, a lymphocyte sample, a white blood cell sample, an apheresis product, or a leukapheresis product. In some embodiments, the apheresis or leukapheresis product is freshly isolated from a subject. In other embodiments, the apheresis or leukapheresis product is thawed from a cryopreserved apheresis or leukapheresis product. In some embodiments the target cells are T cells.

In some embodiments, the cells, e.g., the target cells, have or express a selection marker as described herein on the cell surface, such that the cells to be isolated, selected, or enriched are defined by the presence of at least one common specific receptor molecule. In some embodiments, the sample containing the target cell may also contain additional cells that are devoid of the selection marker. For example, in some embodiments, T cells are selected, isolated, or enriched from a sample containing multiple cells types, e.g., red blood cells or B cells.

In some embodiments, the selection agent is comprised in a chromatography column, e.g., bound directly or indirectly to the chromatography matrix (e.g., stationary phase). In some embodiments, the selection agent is present on the chromatography matrix (e.g., stationary phase) at the time the sample is added to the column. In some embodiments, the selection agent is capable of being bound indirectly to the chromatography matrix (e.g., stationary phase) through a reagent, e.g., selection reagent. In some embodiments, the selection reagent is bound covalently or non-covalently to the stationary phase of the column. In some embodiments, the selection reagent is reversibly immobilized on the chromatography matrix (e.g., stationary phase). In some cases, the selection reagent is immobilized on the chromatography matrix (e.g., stationary phase) via covalent bonds. In some aspects, the selection reagent is reversibly immobilized on the chromatography matrix (e.g., stationary phase) non-covalently.

In some embodiments, the selection agent may be present, for example bound directly to (e.g., covalently or non-covalently) or indirectly via a selection reagent, on the chromatography matrix (e.g., stationary phase) at the time the sample is added to the chromatography column (e.g., stationary phase). Thus, upon addition of the sample, target cells can be bound by the selection agent and immobilized on the chromatography matrix (e.g., stationary phase) of the column. Alternatively, in some embodiments, the selection agent can be added to the sample. In this way, the selection agent binds to the target cells (e.g., T cells) in the sample, and the sample can then be added to a chromatography matrix (e.g., stationary phase) comprising the selection reagent, where the selection agent, already bound to the target cells, binds to the selection reagent, thereby immobilizing the target cells on the chromatography matrix (e.g., stationary phase). In some embodiments, the selection agent binds to the selection reagent as described herein via binding partner C, as described herein, comprised in the selection agent.

In some embodiments, two or more selection agents associate with, such as are reversibly or irreversibly bound to, the selection reagent, such as via the one or plurality of binding sites Z present on the selection reagent. In some cases, this results in the selection agents being closely arranged to each other such that an avidity effect can take place if a target cell having (at least two copies of) a cell surface molecule (e.g., selection marker) is brought into contact with the selection agent that is able to bind the particular molecule (e.g., selection marker).

In some embodiments, two or more different selection agents that are the same, i.e. have the same selection marker binding specificity, can be reversibly bound to the selection reagent. In some embodiments, it is possible to use at least two different selection agents, and in some cases, three or four different selection agents that bind to different selection markers. In some aspects, each of the at least two selection agents can bind to a different molecule (e.g., selection marker), such as a first molecule, second molecule and so on. In some cases, the different molecules (e.g., selection agents), such as cell surface molecules, can be present on the same target cell. In other cases, the different molecules (e.g., selection markers), such as cell surface molecules, can be present on different target cells that are present in the same population of cells. In some case, a third, fourth and so on selection agent can be associated with the same reagent, each containing a further different binding site.

In some embodiments, the two or more different selection agents contain the same binding partner C. In some embodiments, the two or more different selection agents contain different binding partners. In some aspects, a first selection agent can have a binding partner C1 that can specifically bind to a binding site Z1 present on the selection reagent and a second selection agent can have a binding partner C2 that can specifically bind to the binding site Z1 or to a binding site Z2 present on the selection reagent. Thus, in some instances, the plurality of binding sites Z comprised by the selection reagent includes binding sites Z1 and Z2, which are capable of reversibly binding to binding partners C1 and C2, respectively, comprised by the selection agent. In some embodiments, C1 and C2 are the same, and/or Z1 and Z2 are the same. In other aspects, one or more of the plurality of binding sites Z can be different. In other instances, one or more of the plurality of binding partners C may be different. It is within a level of a skilled artisan to choose any combination of different binding partners C that are compatible with a selection reagent containing the binding sites Z, as long as each of the binding partners C are able to interact, such as specifically bind, with one of the binding sites Z.

In some embodiments, a reversible bond formed between binding partner C and binding site Z can be disrupted by a competitive agent and/or free binding agent. In some embodiments, a competitive agent and/or free binding agent can be a biotin, a biotin derivative or analog or a streptavidin-binding peptide capable of competing for binding with the binding partner C for the one or more binding sites Z. In some embodiments, the binding partner C and the competitive agent and/or free binding agent are different, and the competitive agent and/or free binding agent exhibit a higher binding affinity for the one or more binding sites Z compared to the affinity of the binding partner. In particular aspects of any of the methods provided herein, addition of a competitive agent and/or free binding agent to the stationary phase of the chromatography column to disrupt the binding of the selection agent to the selection reagent is not required to detach the target cells (e.g., T cells) from the chromatography matrix (e.g., stationary phase).

In some embodiments, the cells, e.g., the target cells of the sample, may be depleted from the sample, such as by rinsing, releasing, or washing the remaining sample from the chromatography matrix (e.g., stationary phase). In some embodiments, one or more (e.g., 2, 3, 4, 5, 6) wash steps are used to remove unbound cells and debris from the chromatography matrix (e.g., stationary phase). In some embodiments, the sample is allowed to penetrate the matrix for at least or about 5, 10, 16, 20, 25, 30, 35, 40, 45, 50, 55, 60, 70, 80, 90, 100, or 120 minutes before one or more wash steps are performed.

Any material may be employed as a chromatography matrix (e.g., stationary phase). In general, a suitable chromatography material is essentially innocuous, i.e. not detrimental to cell viability, such as when used in a packed chromatography column under desired conditions. In some embodiments, the stationary phase remains in a predefined location, such as a predefined position, whereas the location of the sample is being altered. Thus, in some embodiments the stationary phase is the part of a chromatographic system through which the mobile phase flows (either by flow through or in a batch mode) and where distribution of the components contained in the liquid phase (either dissolved or dispersed) between the phases occurs.

In some embodiments, the chromatography matrix has the form of a solid or semisolid phase, whereas the sample that contains the target cell to be isolated/separated is a fluid phase. The chromatography matrix can be a particulate material (of any suitable size and shape) or a monolithic chromatography material, including a paper substrate or membrane. Thus, in some aspects, the chromatography can be both column chromatography as well as planar chromatography. In some embodiments, in addition to standard chromatography columns, columns allowing a bidirectional flow such as PhyTip^{®} columns available from PhyNexus, Inc. San Jose, CA, U.S.A. or pipette tips can be used for column based/flow through mode based methods. Thus, in some cases, pipette tips or columns allowing a bidirectional flow are also comprised by chromatography columns useful in the present methods. In some cases, such as where a particulate matrix material is used, the particulate matrix material may, for example, have a mean particle size of about 5 µm to about 200 µm, or from about 5 µm to about 400 µm, or from about 5 µm to about 600 µm. In some aspects, the chromatography matrix may, for example, be or include a polymeric resin or a metal oxide or a metalloid oxide. In some aspects, such as where planar chromatography is used, the matrix material may be any material suitable for planar chromatography, such as conventional cellulose-based or organic polymer based membranes (for example, a paper membrane, a nitrocellulose membrane or a polyvinylidene difluoride (PVDF) membrane) or silica coated glass plates. In one embodiment, the chromatography matrix/stationary phase is a non-magnetic material or non-magnetizable material.

In some embodiments, non-magnetic or non-magnetizable chromatography stationary phases that are suitable in the present methods include derivatized silica or a crosslinked gel. In some aspects, a crosslinked gel may be based on a natural polymer, such as on a polymer class that occurs in nature. For example, a natural polymer on which a chromatography stationary phase may be based is a polysaccharide. In some cases, a respective polysaccharide is generally crosslinked. An example of a polysaccharide matrix includes, but is not limited to, an agarose gel (for example, Superflow^{™} agarose or a Sepharose^{®} material such as Superflow^{™} Sepharose^{®} that are commercially available in different bead and pore sizes) or a gel of crosslinked dextran(s). A further illustrative example is a particulate cross-linked agarose matrix, to which dextran is covalently bonded, that is commercially available (in various bead sizes and with various pore sizes) as Sephadex^{®} or Superdex^{®}, both available from GE Healthcare. Another illustrative example of such a chromatography material is Sephacryl^{®} which is also available in different bead and pore sizes from GE Healthcare.

In some embodiments, a crosslinked gel may also be based on a synthetic polymer, such as on a polymer class that does not occur in nature. In some aspects, such a synthetic polymer on which a chromatography stationary phase is based is a polymer that has polar monomer units, and which is therefore in itself polar. Thus, in some cases, such a polar polymer is hydrophilic. Hydrophilic molecules, also termed lipophobic, in some aspects contain moieties that can form dipole-dipole interactions with water molecules. In general, hydrophobic molecules, also termed lipophilic, have a tendency to separate from water.

Generally, a chromatographic method is a fluid chromatography, typically a liquid chromatography. In some aspects, the chromatography can be carried out in a flow through mode in which a fluid sample containing the cells, e.g., the target cells, is applied, for example, by gravity flow or by a pump on one end of a column containing the chromatography matrix and in which the fluid sample exists the column at the other end of the column. In addition the chromatography can be carried out in an "up and down" mode in which a fluid sample containing the cells to be isolated is applied, for example, by a pipette on one end of a column containing the chromatography matrix packed within a pipette tip and in which the fluid sample enters and exists the chromatography matrix /pipette tip at the other end of the column. Alternatively, the chromatography can also be carried out in a batch mode in which the chromatography material (stationary phase) is incubated with the sample that contains the cells, for example, under shaking, rotating or repeated contacting and removal of the fluid sample, for example, by means of a pipette.

In some aspects, any material may be employed as chromatography matrix in the context of the provided embodiments, as long as the material is suitable for the chromatographic isolation, e.g., selection of cells. In particular aspects, a suitable chromatography material is at least innocuous or essentially innocuous, e.g., not detrimental to cell viability, when used in a packed chromatography column under desired conditions for cell isolation and/or cell separation. In some aspects, the chromatography matrix remains in a predefined location, typically in a predefined position, whereas the location of the sample to be separated and of components included therein, is being altered. Thus, in some aspects, the chromatography matrix is a "stationary phase."

Typically, the respective chromatography matrix has the form of a solid or semisolid phase, whereas the sample that contains the target cell to be isolated/separated is a fluid phase. The mobile phase used to achieve chromatographic separation is likewise a fluid phase. The chromatography matrix can be a particulate material (of any suitable size and shape) or a monolithic chromatography material, including a paper substrate or membrane. Thus, the chromatography can be both column chromatography as well as planar chromatography. In addition to standard chromatography columns, columns allowing a bidirectional flow or pipette tips can be used for column based/flow through mode based chromatographic separation of cells as described here. In some aspects, a particulate matrix material is used, and the particulate matrix material may, for example, have a mean particle size of about 5 µm to about 200 µm, or from about 5 µm to about 400 µm, or from about 5 µm to about 600 µm. In some aspects, planar chromatography is used, and the matrix material may be any material suitable for planar chromatography, such as conventional cellulose-based or organic polymer based membranes (for example, a paper membrane, a nitrocellulose membrane or a polyvinylidene difluoride (PVDF) membrane) or silica coated glass plates.

In some aspects, the chromatography matrix/stationary phase is a non-magnetic material or non-magnetisable material. Such material may include derivatized silica or a crosslinked gel. A crosslinked gel (which is typically manufactured in a bead form) may be based on a natural polymer, such as a crosslinked polysaccharide. Suitable examples include but are not limited to agarose gels or a gel of crosslinked dextran(s). A crosslinked gel may also be based on a synthetic polymer, i.e. on a polymer class that does not occur in nature. Usually such a synthetic polymer on which a chromatography stationary phase for cell separation is based is a polymer that has polar monomer units, and which is therefore in itself polar.

Illustrative examples of suitable synthetic polymers are polyacrylamide(s), a styrene-divinylbenzene gel and a copolymer of an acrylate and a diol or of an acrylamide and a diol. An illustrative example is a polymethacrylate gel, commercially available as a Fractogel^{®}. A further example is a copolymer of ethylene glycol and methacrylate, commercially available as a Toyopearl^{®}. In some embodiments a chromatography stationary phase may also include natural and synthetic polymer components, such as a composite matrix or a composite or a co-polymer of a polysaccharide and agarose, e.g. a polyacrylamide/agarose composite, or of a polysaccharide and N,N'-methylenebisacrylamide. An illustrative example of a copolymer of a dextran and N,N'-methylenebisacryl-amide is the above-mentioned Sephacryl^{®} series of material. A derivatized silica may include silica particles that are coupled to a synthetic or to a natural polymer. Examples of such embodiments include, but are not limited to, polysaccharide grafted silica, polyvinyl-pyrrolidone grafted silica, polyethylene oxide grafted silica, poly(2-hydroxyethylaspartamide) silica and poly(N-isopropylacrylamide) grafted silica.

Other components present in a sample such as stimulatory agents and/or stimulatory reagents (e.g., oligomeric stimulatory reagents) may have a size that is below the exclusion limit of the pores and this can enter the pores of the size exclusion chromatography matrix. Of such components that are able to partially or fully enter the pore volume, larger molecules, with less access to the pore volume will usually elute first, whereas the smallest molecules elute last. In some embodiments the exclusion limit of the size exclusion chromatography matrix is selected to be below the maximal width of the target cell. Hence, components that have access to the pore volume will usually remain longer in/on the size exclusion chromatography matrix than target cell. Thus, target cells can be collected in the eluate of a chromatography column separately from other matter/components of a sample. Therefore components such as a stimulatory reagent elute at a later point of time from a gel filtration matrix than the target cell.

A chromatography matrix employed in the provided embodiments may also include magnetically attractable matter such as one or more magnetically attractable particles or a ferrofluid. A respective magnetically attractable particle may comprise a selection reagent with a binding site (e.g., selection agent) that is capable of binding to and immobilizing the target cell on the chromatography matrix. Magnetically attractable particles may contain diamagnetic, ferromagnetic, paramagnetic or superparamagnetic material. Superparamagnetic material responds to a magnetic field with an induced magnetic field without a resulting permanent magnetization. Magnetic particles based on iron oxide are for example commercially available as Dynabeads^{®} from Dynal Biotech, as magnetic MicroBeads from Miltenyi Biotec, as magnetic porous glass beads from CPG Inc., as well as from various other sources, such as Roche Applied Science, BIOCLON, BioSource International Inc., micromod, AMBION, Merck, Bangs Laboratories, Polysciences, or Novagen Inc., to name only a few. Magnetic nanoparticles based on superparamagnetic Co and FeCo, as well as ferromagnetic Co nanocrystals have been described, for example by Hütten, A. et al. (J. Biotech. (2004), 112, 47-63). However, in some embodiments a chromatography matrix employed in the provided embodiments is void of any magnetically attractable matter.

In line with the co-pending International Patent Application PCT/EP2012/063969, published as WO 2013/011011, the strength of the binding between the selection agent and a selection marker on a target cell may not be essential for the reversibility of the binding of the target cell to the selection reagent via the selection agent. Rather, irrespective of the strength of the binding, meaning whether the dissociation constant (K_{D}) for the binding between the selection agent via the binding site B and the selection marker is of low affinity, for example, in the range of a K_{D} of about 10⁻³ to about 10⁻⁷ M, or of high affinity, for example, in the range of a K_{D} of about 10⁻⁷ to about 1 x 10⁻¹⁰ M, a target cell can be reversibly stained as long as the dissociation of the binding of the selection agent via the binding site B and the receptor molecule occurs sufficiently fast. In this regard the dissociation rate constant (k_{off}) for the binding between the selection agent via the binding site B and the selection agent may have a value of about 3 × 10⁻⁵ sec⁻¹ or greater (this dissociation rate constant is the constant characterizing the dissociation reaction of the complex formed between the binding site B of the receptor binding reagent and the receptor molecule on the surface of the target cell). The association rate constant (kₒₙ) for the association reaction between the binding site B of the selection agent and the selection marker on the surface of the target cell may have any value. In order to ensure a sufficiently reversible binding between the selection marker and selection agent it is advantageous to select the k_{off} value of the binding equilibrium to have a value of about 3 × 10⁻⁵ sec⁻¹ or greater, of about 5 × 10⁻⁵ sec⁻¹ or greater, such as or as about 1 × 10⁻⁴ sec⁻¹ or greater, 5 × 10⁻⁴ sec⁻¹ or greater, 1 × 10⁻³ sec⁻¹ or greater, 5 × 10⁻³ sec⁻¹ or greater, a 1 × 10⁻² sec⁻¹ or greater, 1 × 10⁻¹ sec⁻¹ or greater or 5 × 10⁻¹ sec⁻¹ or greater. It is noted here that the values of the kinetic and thermodynamic constants as used herein, refer to conditions of atmospheric pressure, i.e. 1.013 bar, and room temperature, i.e. 25 °C.

In some embodiments, multiple rounds of cell selection steps are carried out, where the positively or negatively selected fraction from one step is subjected to another selection step, such as a subsequent positive or negative selection. In certain embodiments, methods, techniques, and reagents for selection, isolation, and enrichment are described, for example, in PCT Application No. WO2015164675.

In some embodiments, a single selection step can be used to isolate target cells (e.g., CD3+ T cells) from a sample. In some embodiments, the single selection step can be performed on a single chromatography column. In some examples, a single selection step can deplete cells expressing multiple markers simultaneously. Likewise, multiple cell types can simultaneously be positively selected. In certain embodiments, selection steps are repeated and or performed more than once, where the positively or negatively selected fraction from one step is subjected to the same selection step, such as a repeated positive or negative selection. In some examples, a single selection step is repeated and/or performed more than once, for example to increase the purity of the selected cells and/or to further remove and/or deplete the negatively selected cells from the negatively selected fraction. In certain embodiments, one or more selection steps are performed two times, three times, four times, five times, six times, seven times, eight times, nine times, ten times, or more than ten times. In certain embodiments, the one or more selection steps are performed and/or repeated between one and ten times, between one and five times, or between three and five times. In some embodiments, two selection steps are performed.

Cell selection may be performed using one or more chromatography columns. In some embodiments, the one or more chromatography columns are included in a closed system. In some embodiments, the closed system is an automated closed system, for example requiring minimal or no user (e.g., human) input. In some embodiments, cell selection is performed sequentially (e.g., a sequential selection technique). In some embodiments, the one or more chromatography columns are arranged sequentially. For example, a first column may be oriented such that the output of the column (e.g., eluent) can be fed, e.g., via connected tubing, to a second chromatography column. In some embodiments, a plurality of chromatography columns may be arranged sequentially. In some embodiments, cell selection may be achieved by carrying out sequential positive and negative selection steps, the subsequent step subjecting the negative and/or positive fraction from the previous step to further selection, where the entire process is carried out in the same tube or tubing set. In some embodiments, a sample containing target cells is subjected to a sequential selection in which a first selection is effected to enrich for one of the CD4+ or CD8+ populations, and the non-selected cells from the first selection are used as the source of cells for a second selection to enrich for the other of the CD4+ or CD8+ populations. In some embodiments, a further selection or selections can be effected to enrich for sub-populations of one or both of the CD4+ or CD8+ population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a sequential selection in which a first selection is effected to enrich for a CD3+ population, and the selected cells are used as the source of cells for a second selection to enrich for CD3+ populations. In some embodiments, a sample containing target cells is subjected to a sequential selection in which a first selection is effected to enrich for a CD3- population on a first stationary phase (e.g., in a first chromatography column), and the flow through containing unbound cells is used as the source of cells for a second selection to enrich for a CD3+ population on a second stationary phase (e.g., in a second chromatography column), wherein the first and second stationary phases are arranged sequentially. In some embodiments, a further selection or selections can be effected to enrich for sub-populations of the CD3 + population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a sequential selection in which a first selection is effected to enrich for a CD3+ population, and the selected cells are used as the source of cells for a second selection to enrich for CD4+ populations. In some embodiments, a further selection or selections can be effected to enrich for sub-populations of the CD3+CD4+ population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a sequential selection in which a first selection is effected to enrich for a CD3+ population, and the selected cells are used as the source of cells for a second selection to enrich for CD8+ populations. In some embodiments, a further selection or selections can be effected to enrich for sub-populations of the CD3+CD8+ population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some aspects, specific subpopulations of T cells (e.g., CD3+ cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative sequential selection techniques. The methods of sequential selections can be carried out in either order.

In some embodiments, a sample containing target cells is subjected to a sequential selection in which a first selection is effected to enrich for a CD3+ population on a first stationary phase (e.g., in a first chromatography column), and the selected cells are used as the source of cells for a second selection to enrich for subpopulations of CD3+ population on a second stationary phase (e.g., in a second chromatography column), wherein the first and second stationary phases are arranged sequentially. In some embodiments, a further selection or selections can be effected to enrich for sub-populations of the CD3 + population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+.

In some embodiments, a sample containing target cells is subjected to a sequential selection in which a first selection is effected to enrich for a a marker of central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+ on a first stationary phase (e.g., in a first chromatography column), and the selected cells are used as the source of cells for a second selection to enrich for subpopulations of CD3+ population on a second stationary phase (e.g., in a second chromatography column), wherein the first and second stationary phases are arranged sequentially.

In some embodiments, cell selection is performed in parallel (e.g., parallel selection technique). In some embodiments, the one or more chromatography columns are arranged in parallel. For example, two or more columns may be arranged such that a sample is loaded onto two or more columns at the same time via tubing that allows for the sample to be added to each column, for example, without the need for the sample to traverse through a first column. For example, using a parallel selection technique, cell selection may be achieved by carrying out positive and/or negative selection steps simultaneously, for example in a closed system where the entire process is carried out in the same tube or tubing set. In some embodiments, a sample containing target cells is subjected to a parallel selection in which the sample is loaded onto two or more chromatography columns, where each column effects selection of a cell population. In some embodiments, the two or more chromatography columns effect selection of CD3+, CD4+, or CD8+ populations individually. In some embodiments, the two or more chromatography columns, including affinity chromatography or gel permeation chromatography, independently effect selection of the same cell population. For example, the two or more chromatography columns may effect selection of CD3+ cells. In some embodiments, the two or more chromatography columns, including affinity chromatography or gel permeation chromatography, independently effect selection of different cell populations. For example, the two or more chromatography columns independently may effect selection of CD3+ cells, CD4+ cells, and CD8+ cells. In some embodiments, a further selection or selections, for example using sequential selection techniques, can be effected to enrich for sub-populations of one or all cell populations selected via parallel selection. For example, selected cells may be further selected for central memory T (T_{CM}) cells, naïve T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a parallel selection in which parallel selection is effected to enrich for a CD3+ population on the two or more columns. In some embodiments, a further selection or selections can be effected to enrich for sub-populations of the CD3+ population, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a parallel selection in which a selection is effected to enrich for a CD3+ population and a CD4+ population on the two or more columns, independently. In some embodiments, a further selection or selections can be effected to enrich for sub-populations of the CD3+ and CD4+ populations, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a parallel selection in which parallel selection is effected to enrich for a CD3+ population and a CD8+ population. In some embodiments, a further selection or selections can be effected to enrich for sub-populations of the CD3+ and CD8+ populations, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. In some embodiments, a sample containing target cells is subjected to a parallel selection in which parallel selection is effected to enrich for a CD4+ population and a CD8+ population. In some embodiments, a further selection or selections can be effected to enrich for sub-populations of the CD4+ and CD8+ populations, for example, central memory T (T_{CM}) cells, naive T cells, and/or cells positive for or expressing high levels of one or more surface markers, e.g., CD28+, CD62L+, CCR7+, CD27+, CD127+, CD4+, CD8+, CD45RA+, and/or CD45RO+. It is contemplated that in some aspects, specific subpopulations of T cells (e.g., CD3+, CD4+, CD8+ T cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative parallel selection techniques. In some embodiments, sequential and parallel selection techniques can be used in combination.

In some embodiments, two columns are used for parallel selection. In some embodiments, the two columns select for the same cell type (e.g., same selection marker). In some embodiments, the two columns each select for CD3+ T cells.

In some embodiments, cell selection is carried out by positive or negative selection to deplete CD57+ cells and to enrich for T cells. Exemplary methods for depleting for CD57+ cells are described in WO2020/097132. In some embodiments, specific subpopulations of T cells, such as cells positive or expressing high levels of one or more surface markers, e.g., CD3+, CD4+, CD8+, or CD57+ T cells, are isolated by positive or negative selection techniques. In some embodiments, such cells are selected by incubation with one or more selection agent, such as an antibody or antibody fragment, that specifically binds to such markers. In certain embodiments, CD57+ cells are depleted from a sample, e.g. PBMC sample, by negative selection of cells positive for CD57 expression, and the non-selected cells (CD57- cells) are used as the source of cells for a second selection to enrich for T cells on a second stationary phase (e.g., in a second chromatography column), wherein the first and second stationary phases are arranged sequentially. For instance, in some embodiments CD57+ cells are depleted from a sample, e.g. PBMC sample, by negative selection of cells positive for CD57 expression, and the non-selected cells (CD57- cells) are used as the source of cells for a second selection to enrich for CD3+ population on a second stationary phase (e.g., in a second chromatography column), wherein the first and second stationary phases are arranged sequentially.

In embodiments using multiple columns, e.g. in sequential selections, such as a first chromatography column and a second chromatography column, the provided methods are carried such that the one or more stimulatory agent or stimulatory reagent is added to the last chromatograpy column (e.g. second chromatography column) used in the final step of selecting or enriching subpopulations of cells. In particular embodiments, the chromatography column to which the one or more stimulatory agent or stimulatory reagent is to be added is subjected to heating using the provided devices herein. For instance, the temperature control member is configured to regulate the temperatue to a target temperature above room temperature of the last or final chromatography column (e.g. second chromatography column) used for the selecting or enriching of subpopulations of cells, and to which the one or more stimulatory agent or reagent is to be added. In some embodiments, the target temperature is a physiologic temperature that maximizes the health and activity of the cells to provide for efficient or effective delivery of the stimulatory signal in the one or more T cells.

In general, binding capacity of a stationary phase (e.g., selection resin) affects how much stationary phase is needed in order to select a certain number of target moieties, e.g., target cells such as T cells. The binding capacity, e.g., the number of target cells that can be immobilized per mL of the stationary phase (e.g., selection resin), can be used to determine or control the number of captured target cells on one or more columns. One or more chromatography column can be used for the on-column cell selection and stimulation disclosed herein. When multiple columns are used, they can be arranged sequentially, in parallel, or in a suitable combination thereof. Thus, the binding capacity of a stationary phase (e.g., selection resin) can be used to standardize the reagent amount in a single-column approach or the reagent amount for each column in a multiple-column approach. In some embodiments, 1mL of the stationary phase is capable of accommodating up to 0.1 billion ± 0.025 billion cells. In some embodiments, the stationary phase is or is about 5 mL, 10 mL, 15 mL, 20 mL, 25 mL, 30 mL, 35 mL, or 40 mL. In some embodiments, the stationary phase is or is about 10 mL and is capable of accommodating up to 1 billion ± 0.25 billion cells. In some embodiments, the stationary phase is or is about 20 mL and is capable of accommodating up to 2 billion ± 0.5 billion cells. In some embodiments, the stationary phase is or is about 40 mL and is capable of accommodating between about 3 billion and about 5 billion cells.

In some embodiments, the binding capacity of the stationary phase used herein is the maximum number of target cells (e.g., CD3+ T cells, CD4+ T cells, or CD8+ T cells) bound to the stationary phase at given solvent and cell concentration conditions, when an excess of target cells are loaded onto the stationary phase. In some embodiments, the binding capacity is or is about 100 million ± 25 million target cells (e.g., T cells) per mL of stationary phase. In some embodiments, the static binding capacity of the stationary phase (e.g., selection resin) disclosed herein ranges between about 75 million and about 125 million target cells per mL of stationary phase. In one aspect, the binding capacity of the stationary phase used herein for on-column cell selection and stimulation is a static binding capacity. In some embodiments, the static binding capacity is the maximum amount of cells capable of being immobilized on the stationary phase, e.g., at certain solvent and cell concentration conditions. In some embodiments, the static binding capacity of the stationary phase (e.g., selection resin) disclosed herein ranges between about 50 million and about 100 million target cells per mL of stationary phase. In some embodiments, the static binding capacity is or is about 100 million ± 25 million target cells (e.g., T cells) per mL of stationary phase. In some embodiments, the static binding capacity of the stationary phase (e.g., selection resin) disclosed herein ranges between about 75 million and about 125 million target cells per mL of stationary phase. In some embodiments, the static binding capacity of the stationary phase (e.g., selection resin) is between about 10 million and about 20 million, between about 20 million and about 30 million, between about 30 million and about 40 million, between about 40 million and about 50 million, between about 50 million and about 60 million, between about 60 million and about 70 million, between about 70 million and about 80 million, between about 80 million and about 90 million, between about 90 million and about 100 million, between about 110 million and about 120 million, between about 120 million and about 130 million, between about 130 million and about 140 million, between about 140 million and about 150 million, between about 150 million and about 160 million, between about 160 million and about 170 million, between about 170 million and about 180 million, between about 180 million and about 190 million, or between about 190 million and about 200 million target cells per mL of stationary phase.

In some embodiments, the binding capacity of the stationary phase used herein is the number of target cells (e.g., CD3+ T cells, CD4+ T cells, or CD8+ T cells) that bind to the stationary phase under given flow conditions before a significant breakthrough of unbound target cells occurs. In one aspect, the binding capacity of the stationary phase used herein for on-column cell selection and stimulation is a dynamic binding capacity, i.e., the binding capacity under operating conditions in a packed chromatography column during sample application. In some embodiments, the dynamic binding capacity is determined by loading a sample containing a known concentration of the target cells and monitoring the flow-through, and the target cells will bind the stationary phase to a certain break point before unbound target cells will flow through the column. In some embodiments, the dynamic binding capacity is or is about 100 million ± 25 million target cells (e.g., T cells) per mL of stationary phase. In some embodiments, the dynamic binding capacity of the stationary phase (e.g., selection resin) disclosed herein is between or is between about 75 million and about 125 million target cells per mL of stationary phase. In some embodiments, the dynamic binding capacity of the stationary phase (e.g., selection resin) disclosed herein ranges between about 50 million and about 100 million target cells per mL of stationary phase. In some embodiments, the dynamic binding capacity of the stationary phase (e.g., selection resin) is between about 10 million and about 20 million, between about 20 million and about 30 million, between about 30 million and about 40 million, between about 40 million and about 50 million, between about 50 million and about 60 million, between about 60 million and about 70 million, between about 70 million and about 80 million, between about 80 million and about 90 million, between about 90 million and about 100 million, between about 110 million and about 120 million, between about 120 million and about 130 million, between about 130 million and about 140 million, between about 140 million and about 150 million, between about 150 million and about 160 million, between about 160 million and about 170 million, between about 170 million and about 180 million, between about 180 million and about 190 million, or between about 190 million and about 200 million target cells per mL of stationary phase.

In some embodiments, the stationary phase is 20 mL. In some embodiments, the stationary phase has a binding capacity of 2 billion ± 0.5 billion cells.

In some embodiments, one or more (e.g., 2, 3, 4, 5, 6) wash steps are used to remove unbound cells and debris from the chromatography matrix (e.g., stationary phase), resulting in an enriched population of selected cells immobilized on the chromatography matrix of the chromatography column. In certain embodiments, the isolation and/or selection results in one or more populations of enriched T cells immobilized on the chromatography matrix of the column that includes at least 60%, at least 65%, at least 70%, at least 75%, at least 80%, at least 85%, at least 90%, at least 95%, at least 98%, at least 99%, at least 99.5%, at least 99.9%, or at or at about 100% T cells or a subset or subpopulation thereof. In certain embodiments, the isolation and/or selection results in one or more populations of enriched T cells immobilized on the chromatography matrix of the column that includes at least 60%, at least 65%, at least 70%, at least 75%, at least 80%, at least 85%, at least 90%, at least 95%, at least 98%, at least 99%, at least 99.5%, at least 99.9%, or at or at about 100% CD3+ T cells or a subset or subpopulation thereof.

### D. On-Column Cell Selection with Stimulation (Methods are not part of the claimed invention)

Using a device disclosed herein, provided herein are methods, which are not in accordance with the claimed invention, that include combining the cell selection by column chromatography step as described in Section II.C with stimulation. In certain embodiments, the cells of a sample are selected using any of the exemplary selection agents described in Section II-B-1, which is not in accordance with the claimed invention,. Thus, in certain aspects, stimulation, which are not in accordance with the claimed invention, is performed during the selection step when cells are immobilized on the column (e.g., by the selection agent). In some embodiments, the stimulating conditions include conditions that stimulate, and/or are capable of delivering a stimulatory signal in a cell, e.g., a CD3+, CD4+, or CD8+ T cell. For instance, the selection is to enrich or select for T cells or certain subsets thereof, and the stimulating conditons include conditions tht stimulate a signal generated from a component of the TCR complex (e.g. CD3) and/or a costimulatory molecule (e.g. CD28). In some embodiments, the stimulating conditions are or include incubating target cells (e.g., T cells) immobilized on the chromatography matrix (e.g., stationary phase) with a stimulatory agent, e.g., an agent that delivers a stimulatory signal, or is capable of delivering a stimulatory signal, thereby stimulating the selected cell. In some embodiments, the selected cell is a T cell or a subset thereof and the stimulatory agent binds to and stimulates and/or activates a component of the TCR complex (e.g. CD3) and/or a costimulatory molecule (e.g. CD28). In certain embodiments, stimulating a population of cells under stimulating conditions generates or produces a population of selected and stimulated cells (also referred to herein as a stimulated population of cells).

In certain embodiments, the cells of a sample are selected and stimulated prior to introducing a heterologous or recombinant polynucleotide into the cells, such as by a method, step, or technique described herein, e.g., in Section II-F, which is not in accordance with the claimed invention,.

The following method is not part of the claimed invention. In particular aspects, the initiation of the stimulation (also referred to herein as initiation of incubation) occurs when the target cells (e.g., T cells) of the sample immobilized on the chromatography matrix (e.g., stationary phase) are first contacted or exposed to a stimulatory agent. In provided embodiments, prior to initiation of the stimulation, a sample containing target cells (e.g. T cells) are added to a column containing a chromatography matrix to which a selection agent is bound or immobilized for specific selection of target cells of interest, as described in Section II.C, and the cells are allowed to incubate under conditions for immobilizing the target cells onto the chromatography matrix (e.g. stationary phase). In some embodiments, the cells are allowed to penetrate the column for about 5, 10, 15, 20, 25, 30, 35, 40, 45, 50, 55, 60, 70, 80, 90, 100 or 120 minutes prior to addition of the stimulatory reagent (e.g., oligomeric stimulatory reagent) or stimulatory agents. In some embodiments, the column is washed at least one (1, 2, 3, 4, 5) time prior to addition of the stimulatory reagent (e.g., oligomeric stimulatory reagent) or stimulatory agents. In some embodiments, the stimulatory agents or stimulatory reagent including stimulatory agents (e.g., oligomeric stimulatory reagent) is added at, at about, or at least, 30, 35, 40, 45, 50, 55, or 60 minutes after the sample is added to the chromatography column (e.g., stationary phase). In some embodiments, the stimulatory agents or stimulatory reagent including stimulatory agents (e.g., oligomeric stimulatory reagent) is added from between about 15 to about 120 minutes, inclusive, after the sample is added to the column. In some embodiments, the stimulatory agents or stimulatory reagent including stimulatory agents (e.g., oligomeric stimulatory reagent) is added from between about 15 to about 100 minutes, inclusive, after the sample is added to the column. In some embodiments, the stimulatory agents or stimulatory reagent including stimulatory agents (e.g., oligomeric stimulatory reagent) is added from between about 15 to about 90 minutes, inclusive, after the sample is added to the column. In some embodiments, the stimulatory agents or stimulatory reagent including stimulatory agents (e.g., oligomeric stimulatory reagent) is added from between about 15 to about 80 minutes, inclusive, after the sample is added to the column. In some embodiments, the stimulatory agents or stimulatory reagent including stimulatory agents (e.g., oligomeric stimulatory reagent) is added from between about 15 to about 70 minutes, inclusive, after the sample is added to the column. In some embodiments, the stimulatory agents or stimulatory reagent including stimulatory agents (e.g., oligomeric stimulatory reagent) is added from between about 15 to about 60 minutes, inclusive, after the sample is added to the column. In some embodiments, the stimulatory agents or stimulatory reagent including stimulatory agents (e.g., oligomeric stimulatory reagent) is added from between about 15 to about 50 minutes, inclusive, after the sample is added to the column. In some embodiments, the stimulatory agents or stimulatory reagent including stimulatory agents (e.g., oligomeric stimulatory reagent) is added from between about 15 to about 40 minutes, inclusive, after the sample is added to the column. In some embodiments, the stimulatory agents or stimulatory reagent including stimulatory agents (e.g., oligomeric stimulatory reagent) is added from between about 15 to about 30 minutes, inclusive, after the sample is added to the column. In some embodiments, the stimulatory agents or stimulatory reagent including stimulatory agents (e.g., oligomeric stimulatory reagent) is added from between about 30 to about 120 minutes, inclusive, after the sample is added to the column. In some embodiments, the stimulatory agents or stimulatory reagent including stimulatory agents (e.g., oligomeric stimulatory reagent) is added from between about 30 to about 100 minutes, inclusive, after the sample is added to the column. In some embodiments, the stimulatory agents or stimulatory reagent including stimulatory agents (e.g., oligomeric stimulatory reagent) is added from between about 30 to about 90 minutes, inclusive, after the sample is added to the column. In some embodiments, the stimulatory agents or stimulatory reagent including stimulatory agents (e.g., oligomeric stimulatory reagent) is added from between about 30 to about 80 minutes, inclusive, after the sample is added to the column. In some embodiments, the stimulatory agents or stimulatory reagent including stimulatory agents (e.g., oligomeric stimulatory reagent) is added from between about 30 to about 70 minutes, inclusive, after the sample is added to the column. In some embodiments, the stimulatory agents or stimulatory reagent including stimulatory agents (e.g., oligomeric stimulatory reagent) is added from between about 30 to about 60 minutes, inclusive, after the sample is added to the column. In some embodiments, the stimulatory agents or stimulatory reagent including stimulatory agents (e.g., oligomeric stimulatory reagent) is added from between about 30 to about 50 minutes, inclusive, after the sample is added to the column. In some embodiments, the stimulatory agents or stimulatory reagent including stimulatory agents (e.g., oligomeric stimulatory reagent) is added from between about 30 to about 40 minutes, inclusive, after the sample is added to the column. In some embodiments, at least one wash step is performed prior to adding the stimulatory agents or reagent including stimulatory agents (e.g., oligomeric stimulatory reagent) to the column.

In some embodiments, the stimulation, e.g. incubating the immobilized cells under stimulating conditions, is performed for, for about, or for less than one day. In some embodiments, the stimulation, e.g. incubating the immobilized cells under stimulating conditions, is performed for, for about, or for less than, 24, 23, 22, 21, 20, 19, 18, 17, 16, 15, 14, 13, 12, 11, 10, 9, 8, 7, 6, 5, 4, 3, or 2 hours. In some embodiments, the stimulation, e.g. incubating the selected cells under stimulating conditions, is performed for between or between about 2 to 24, 3 to 24, 4 to 24, 5, to 24, 6 to 24, 7 to 24, 8 to 24, 9 to 24, 10 to 24, 11 to 24, 12 to 24, 13 to 24, 14 to 24, 15 to 24, 16 to 24, 17 to 24, 18 to 24, 19 to 24, 20 to 24, 21 to 24, 22 to 24, 23 to 24, 2 to 23, 2 to 22, 2 to 21, 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3 hours. In some embodiments, the stimulation, e.g. incubating the immobilized cells under stimulating conditions, is performed for, for about, or for less than, 24 hours. In some embodiments, the stimulation, e.g. incubating the immobilized cells under stimulating conditions, is performed for, for about, or for less than, 12 hours. In some embodiments, the stimulation, e.g. incubating the immobilized cells under stimulating conditions, is performed for, for about, or for less than, 5 hours. In some embodiments, the stimulation, e.g. incubating the immobilized cells under stimulating conditions, is performed for, for about, or for less than, 4 hours. In some embodiments, the stimulation, e.g. incubating the immobilized cells under stimulating conditions, is performed for, for about, or for less than, 2 hours.

In particular embodiments, an amount of, of about, or of at least 50 x 10⁶, 100 x 10⁶, 150 x 10⁶, 200 x 10⁶, 250 x 10⁶, 300 x 10⁶, 350 x 10⁶, 400 x 10⁶, 450 x 10⁶, 500 x 10⁶, 550 x 10⁶, 600 x 10⁶, 700 x 10⁶, 800 x 10⁶, 900 x 10⁶, 1,000 x 10⁶, 1250 x 10⁶, 1500 x 10⁶, 1750 x 10⁶, 2000 x 10⁶, 2250 x 10⁶, 2500 x 10⁶, 2750 x 10⁶, 3000 x 10⁶, 3250 x 10⁶, 3500 x 10⁶, 3750 x 10⁶, 4000 x 10⁶ , 4250 x 10⁶, 4500 x 10⁶, 4750 x 10⁶, or 5000 x 10⁶ cells selected from the sample are stimulated, e.g., incubated under stimulating conditions. In some embodiments, the selected cells are immobilized on a single column (e.g., containing a chromatography matrix). For example, the total amount of selected cells from the sample are immobilized on a single column and the immobilized cells on the single column are incubated under stimulating conditions. In some embodiments, the selected cells are immobilized on two columns (e.g., each containing a chromatography matrix). For example, the total amount of selected cells from the sample are immobilized on two columns (e.g., each column (e.g., chromatography matrix) contains half or about half of the total amount of cells immobilized thereon) and the immobilized cells on the two columns are incubated under stimulating conditions. In certain embodiments, the cells, e.g., selected cells (e.g., T cells) immobilized on the chromatography matrix (e.g., stationary phase), are stimulated e.g., incubated under stimulating conditions such as in the presence of a stimulatory agent, at a density of, of about, or at least 0.01 x 10⁶ cells/mL, 0.1 x 10⁶ cells/mL, 0.5 x 10⁶ cells/mL, 1.0 x 10⁶ cells/mL, 1.5 x 10⁶ cells/mL, 2.0 x 10⁶ cells/mL, 2.5 x 10⁶ cells/mL, 3.0 x 10⁶ cells/mL, 4.0 x 10⁶ cells/mL, 5.0 x 10⁶ cells/mL, 10 x 10⁶ cells/mL, 50 x 10⁶ cells/mL, 75 x 10⁶ cells/mL, 100 x 10⁶ cells/mL, 125 x10⁶ cells/mL, 150 x 10⁶ cells/mL, or 200 x 10⁶ cells/mL. In certain embodiments, the cells, e.g., selected cells (e.g., T cells) immobilized on the stationary phase, are stimulated e.g., incubated under stimulating conditions such as in the presence of a stimulatory agent, at a density of, of about, or at least 3.0 x 10⁶ cells/mL. In certain embodiments, the cells, e.g., selected cells (e.g., T cells) immobilized on the stationary phase, are stimulated or subjected to stimulation, e.g., incubated under stimulating conditions such as in the presence of a stimulatory agent, at a density of or of about 100 ± 25 million cells/mL. In certain embodiments, the selected cells are viable cells.

In some embodiments, the stimulatory agent or stimulatory reagent including stimulatory agents is added to the column at a concentration of, of about, or at least 0.25, 0.5, 0.75, 1, 1.25, 1.5, 1.75, 2, 2.25, 2.5, 2.75, 3 µg per 1 x 10⁶ cells. In some embodiments, the stimulatory agent or stimulatory reagent including stimulatory agents is added to the column containing immobilized cells at a concentration of, of about, or at least 0.75, 1, 1.25, 1.5, 1.75, 2, 2.25 µg per 1 x 10⁶ cells. In some embodiments, the stimulatory agent or stimulatory reagent including stimulatory agents is added to the column at a concentration of or of about 1 to 2 µg per 1 x 10⁶ cells. In some embodiments, the stimulatory reagent is an oligomeric stimulatory reagent, such as described in Section II.B-2. In some embodiments the oligomeric stimulatory reagent is added to the column containing immobilized cells at a concentration of between or between about 1 to 2 µg per 1 x 10⁶ cells. In some embodiments, 5 x 10⁸ oligomeric stimulatory reagents are added to the column containing immobilized cells. In cases where two or more columns contain immobilized cells for stimulation, the concentration or amount of stimulatory agent or stimulatory reagent including stimulatory agents (e.g., oligomeric stimulatory reagent) decribed herein is added or applied to each column.

In some embodiments, the conditions for stimulation can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells. In some embodiments, temperature is or is about 37 °C. In some embodiments, the oxygen and carbon dioxide content is controlled using gas exchange.

In particular embodiments, the stimulating conditions include incubating the cells, e.g., selected cells of a sample, with and/or in the presence of one or more cytokines. In particular embodiments, the one or more cytokines are recombinant cytokines. In some embodiments, the one or more cytokines are human recombinant cytokines. In certain embodiments, the one or more cytokines bind to and/or are capable of binding to receptors that are expressed by and/or are endogenous to the selected cells (e.g., T cells). In particular embodiments, the one or more cytokines are or include a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more cytokines is or includes IL-15. In particular embodiments, the one or more cytokines is or includes IL-7. In particular embodiments, the one or more cytokines is or includes IL-2.

In certain embodiments, the amount or concentration of the one or more cytokines are measured and/or quantified with International Units (IU). International units may be used to quantify vitamins, hormones, cytokines, vaccines, blood products, and similar biologically active substances. In some embodiments, IU are or include units of measure of the potency of biological preparations by comparison to an international reference standard of a specific weight and strength e.g., WHO 1st International Standard for Human IL-2, 86/504. International Units are the only recognized and standardized method to report biological activity units that are published and are derived from an international collaborative research effort. In particular embodiments, the IU for population, sample, or source of a cytokine may be obtained through product comparison testing with an analogous WHO standard product. For example, in some embodiments, the IU/mg of a population, sample, or source of human recombinant IL-2, IL-7, or IL-15 is compared to the WHO standard IL-2 product (NIBSC code: 86/500), the WHO standard IL-17 product (NIBSC code: 90/530) and the WHO standard IL-15 product (NIBSC code: 95/554), respectively.

In some embodiments, the biological activity in IU/mg is equivalent to (ED50 in ng/ml)-1 x106. In particular embodiments, the ED50 of recombinant human IL-2 or IL-15 is equivalent to the concentration required for the half-maximal stimulation of cell proliferation (XTT cleavage) with CTLL-2 cells. In certain embodiments, the ED50 of recombinant human IL-7 is equivalent to the concentration required for the half-maximal stimulation for proliferation of PHA-activated human peripheral blood lymphocytes. Details relating to assays and calculations of IU for IL-2 are discussed in Wadhwa et al., Journal of Immunological Methods (2013), 379 (1-2): 1-7; and Gearing and Thorpe, Journal of Immunological Methods (1988), 114 (1-2): 3-9; details relating to assays and calculations of IU for IL-15 are discussed in Soman et al. Journal of Immunological Methods (2009) 348 (1-2): 83-94.

In some embodiments, the cells, e.g., selected cells of a sample, are stimulated in the presence of a cytokine, e.g., a recombinant human cytokine, at a concentration of between 1 IU/mL and 1,000 IU/mL, between 10 IU/mL and 50 IU/mL, between 50 IU/mL and 100 IU/mL, between 100 IU/mL and 200 IU/mL, between 100 IU/mL and 500 IU/mL, between 250 IU/mL and 500 IU/mL, or between 500 IU/mL and 1,000 IU/mL.

In some embodiments, the cells, e.g., selected cells of a sample, are stimulated in the presence of IL-2, e.g., human recombinant IL-2, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular embodiments, cells, e.g., selected cells of a sample, are stimulated in the presence of recombinant IL-2 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 100 IU/mL. In some embodiments, the cells, e.g., selected cells of a sample, are stimulated in the presence of or of about 100 IU/mL of recombinant IL-2, e.g., human recombinant IL-2.

In some embodiments, the cells, e.g., selected cells of a sample, are stimulated in the presence of recombinant IL-7, e.g., human recombinant IL-7, at a concentration between 100 IU/mL and 2,000 IU/mL, between 500 IU/mL and 1,000 IU/mL, between 100 IU/mL and 500 IU/mL, between 500 IU/mL and 750 IU/mL, between 750 IU/mL and 1,000 IU/mL, or between 550 IU/mL and 650 IU/mL. In particular embodiments, the cells, e.g., the input cells, are stimulated in the presence of IL-7 at a concentration at or at about 50 IU/mL,100 IU/mL, 150 IU/mL, 200 IU/mL, 250 IU/mL, 300 IU/mL, 350 IU/mL, 400 IU/mL, 450 IU/mL, 500 IU/mL, 550 IU/mL, 600 IU/mL, 650 IU/mL, 700 IU/mL, 750 IU/mL, 800 IU/mL, 750 IU/mL, 750 IU/mL, 750 IU/mL, or 1,000 IU/mL. In particular embodiments, the cells, e.g., selected cells of a sample, are stimulated in the presence of or of about 600 IU/mL of IL-7.

In some embodiments, the cells, e.g., selected cells of a sample, are stimulated in the presence of recombinant IL-15, e.g., human recombinant IL-15, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular embodiments, cells, e.g., a cell of the input population, are stimulated in the presence of recombinant IL-15 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 200 IU/mL. In some embodiments, the cells, e.g., selected cells of a sample, are stimulated in the presence of or of about 100 IU/mL of recombinant IL-15, e.g., human recombinant IL-2.

In particular embodiments, the cells, e.g., selected cells of a sample, are stimulated under stimulating conditions in the presence of IL-2, IL-7, and/or IL-15. In some embodiments, the IL-2, IL-7, and/or IL-15 are recombinant. In certain embodiments, the IL-2, IL-7, and/or IL-15 are human. In particular embodiments, the one or more cytokines are or include human recombinant IL-2, IL-7, and/or IL-15. In certain embodiments, the cells, e.g., selected cells of a sample, are stimulated under stimulating conditions in the presence of recombinant IL-2, IL-7, and IL-15. In some embodiments, the stimulating conditions further comprise glutamine.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some aspects, stimulation is carried out in accordance with techniques such as those described in US Patent No. 6,040,1 77 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some embodiments, the stimulation is performed in serum free media. In some embodiments, the serum free media is a defined and/or well-defined cell culture media. In certain embodiments, the serum free media is a controlled culture media that has been processed, e.g., filtered to remove inhibitors and/or growth factors. In some embodiments, the serum free media contains proteins. In certain embodiments, the serum-free media may contain serum albumin, hydrolysates, growth factors, hormones, carrier proteins, and/or attachment factors.

In some embodiments, stimulation, e.g., incubation under stimulatory conditions, is carried out at room temperature (e.g., at or about 23 °C). In some embodiments, stimulation, e.g., incubation under stimulatory conditions, is carried out between about 30°C and about 39°C, such as at or about 37 °C. In particular embodiments, the methods of on-column stimulation is carried out using the device provided herein so that the cells immobilized or bound to the chromatography matrix (e.g. stationary phase) of the chromatography column are exposed to a temperature of between about 30°C and about 39°C, such as at or about 37 °C, during the stimulation. In some embodiments, the device provided herein regulates the temperature to a target temperature of between about 30°C and about 39°C, such as at or about 37 °C, such as increases the temperature from an initial starting temperature (e.g. room temperature) to the target temperature. In some embodiments, the device provided herein maintains the temperature to a target temperature of between about 30°C and about 39°C, such as at or about 37 °C, for example, provides for a constant or near constant target temperature during the time of stimulation of the cells on the column.

In some embodiments, the methods provided herein are carried out at or at about 37 °C.

### 1. Incubation with Stimulatory Agents and Reagents for On-Column Stimulation (not part of the claimed invention)

Using a device disclosed herein, provided herein are methods, which are not in accordance with the claimed invention, that comprise incubating the target cells (e.g., T cells) immobilized on a chromatography matrix (e.g., stationary phase) with one or more stimulatory agents. Exemplary stimulatory agents are described in Section II-B-1. In some embodiments, the stimulatory agents are comprised in a stimulatory reagent. Exemplary stimulatory reagents are described in Section II-B-2. In some embodiments, the stimulatory agents are bound directly or indirectly to the chromatography matrix (e.g., stationary phase) of the chromatography column. In some embodiments, the stimulatory agents are bound indirectly to the chromatography matrix (e.g., stationary phase) of the chromatography column, for example through a selection reagent as described above or a stimulatory reagent as described herein. In some embodiments, the stimulatory agents are comprised in a stimulatory reagent. In some embodiments, the stimulatory reagent is bound to the chromatography matrix (e.g., stationary phase) of the chromatography column. In some embodiments, the stimulatory reagent is covalently bound to the chromatography matrix (e.g., stationary phase). In some embodiments, the stimulatory agent is non-covalently bound to the chromatography matrix (e.g., stationary phase).

In some embodiments, the stimulatory reagent is not bound to or associated with, a solid support, stationary phase, a bead, a microparticle, a magnetic particle, and/or a matrix. In some embodiments, the stimulatory reagent is flexible, does not contain a metal or magnetic core, is comprised entirely or primarily of organic multimer, and/or is not rigid. In some embodiments, the stimulatory reagent is soluble. In some embodiments, the stimulatory reagent is an oligomeric stimulatory reagent. In some embodiments, the oligomeric stimulatory reagent is soluble. Thus, in some embodiments, the stimulatory reagent, such as oligomeric stimulatory reagent, is not associated with the column. In some embodiments, the stimulatory reagent, such as oligomeric stimulatory reagent, is added to the column.

In certain embodiments, the initiation of the stimulation occurs when the cells are incubated or contacted with the stimulatory agent. Thus, in some embodiments, where the stimulatory agent is bound directly or indirectly, e.g., through a selection reagent or stimulatory reagent, to the chromatography matrix (e.g., stationary phase) of the column, initiation of the stimulation occurs when the sample comprising the target cells is added to the chromatography matrix (e.g., stationary phase) of the column. In some embodiments, when the stimulatory agents are comprised in a stimulatory reagent not associated (e.g., bound) with a chromatography matrix (e.g., stationary phase), the initiation of the stimulation occurs when the stimulatory reagent (e.g., oligomeric stimulatory reagent) is added to the stationary phase upon which the target cells of the sample are immobilized. In some embodiments, when the stimulatory agent is not bound directly or indirectly to the chromatography matrix (e.g., stationary phase) and is not comprised in a stimulatory reagent (e.g., oligomeric stimulatory reagent), initiation of the stimulation occurs when the stimulatory agent is added to the chromatography matrix (e.g., stationary phase).

In some embodiments, the stimulating conditions or stimulatory reagents (e.g., oligomeric stimulatory reagents) include one or more stimulatory agent, which is capable of activating an intracellular signaling domain of a TCR complex. In some embodiments, a stimulatory reagent agent as contemplated herein can include, but is not limited to, RNA, DNA, proteins (e.g., enzymes), antigens, polyclonal antibodies, monoclonal antibodies, antibody fragments, carbohydrates, lipids lectins, or any other biomolecule with an affinity for a desired target. In some embodiments, the desired target is a T cell receptor and/or a component of a T cell receptor. In certain embodiments, the desired target is CD3. In certain embodiment, the desired target is a T cell costimulatory molecule, e.g., CD28, CD137 (4-1-BB), OX40, or ICOS.

In some embodiments, the stimulatory reagent (e.g., oligomeric stimulatory reagent) contains one or more stimulatory agents that bind to one or more of the following macromolecules on a cell (e.g., a T cell): CD2, CD3, CD4, CD5, CD8, CD25, CD27, CD28, CD29, CD31, CD44, CD45RA, CD45RO, CD54 (ICAM-1), CD127, MHCI, MHCII, CTLA-4, ICOS, PD-1, OX40, CD27L (CD70), 4-1BB (CD137), 4-1BBL, CD30L, LIGHT, IL-2R, IL-12R, IL-1R, IL-15R; IFN-gammaR, TNF-alphaR, IL-4R, IL- 10R, CD18/CDl la (LFA-1), CD62L (L-selectin), CD29/CD49d (VLA-4), Notch ligand (e.g. Delta-like 1/4, Jagged 1/2, etc.), CCR1, CCR2, CCR3, CCR4, CCR5, CCR7, and CXCR3 or fragment thereof including the corresponding ligands to these macromolecules or fragments thereof. In some embodiments, a stimulatory agent specifically binds to one or more of the following macromolecules on a cell (e.g. a T cell): CD28, CD62L, CCR7, CD27, CD127, CD3, CD4, CD8, CD45RA, and/or CD45RO.

In some embodiments, the stimulatory agent is an antibody that binds to and/or recognizes one or more components of a T cell receptor. In particular embodiments, the stimulatory agent is an anti-CD3 antibody. In certain embodiments, the stimulatory agent is an antibody that binds to and/or recognizes a costimulatory molecule. In certain embodiments, the stimulatory agent is an anti-CD28 antibody. In some embodiments, the stimulatory reagent comprises an anti-CD28 antibody and an anti-CD3 antibody (e.g., stimulatory agents). In some embodiments, the first stimulatory agent is an anti-CD3 Fab, for example as described herein, and the second stimulatory agent is an anti-CD28 Fab, for example as described herein.

In any of the preceding embodiments, the stimulatory reagent can comprise or be an oligomeric stimulatory reagent comprising (i) a plurality of streptavidin or streptavidin mutein molecules and (ii) one or more stimulatory agent capable of delivering a stimulatory signal in one or more T cells, wherein the size of the oligomeric stimulatory reagent comprises i) a radius of greater than 50 nm, ii) a molecular weight of at least 5 × 10⁶ g/mol; and/or (iii) at least 100 streptavidin or streptavidin mutein tetramers per oligomeric stimulatory reagent. For example, the streptavidin mutein can comprise the amino acid sequence Va1⁴⁴-Thr⁴⁵-Ala⁴⁶-Arg⁴⁷ or Ile⁴⁴-Gly⁴⁵-Ala⁴⁶-Arg⁴⁷ at sequence positions corresponding to positions 44 to 47 with reference to positions in streptavidin in the sequence of amino acids set forth in SEQ ID NO:1. In other examples, the streptavidin mutein comprises the amino acid sequence Va1⁴⁴-Thr⁴⁵-Ala⁴⁶-Arg⁴⁷ at sequence positions corresponding to positions 44 to 47 with reference to positions in streptavidin in the sequence of amino acids set forth in SEQ ID NO: 1. In some embodiments, the stimulatory reagent comprises an anti-CD28 antibody and an anti-CD3 antibody (e.g., stimulatory agents). In some embodiments, the first stimulatory agent is an anti-CD3 Fab, for example as described herein, and the second stimulatory agent is an anti-CD28 Fab, for example as described herein.

In some embodiments, for example when the stimulatory agent is not bound to a stimulatory reagent (e.g., oligomeric stimulatory reagent) or a selection reagent, the stimulatory agent is an antibody, a divalent antibody fragment, a F(ab)₂, or a divalent single-chain Fv fragment.

In some embodiments, the cells, e.g., selected cells of a sample, are stimulated in the presence of a ratio of stimulatory reagent to cells at or at about 3:1, 2.5:1, 2:1, 1.5:1, 1.25:1, 1.2:1, 1.1:1, 1:1, 0.9:1, 0.8:1, 0.75:1, 0.67:1, 0.5:1, 0.3:1, or 0.2:1. In particular embodiments, the ratio of stimulatory reagent to cells is between 2.5:1 and 0.2:1, between 2:1 and 0.5:1, between 1.5:1 and 0.75:1, between 1.25:1 and 0.8:1, between 1.1:1 and 0.9:1. In particular embodiments, the ratio of stimulatory reagent to cells is about 1:1 or is 1:1. In particular embodiments, the ratio of stimulatory reagent to cells is about 0.3:1 or is 0.3:1. In particular embodiments, the ratio of stimulatory reagent to cells is about 0.2:1 or is 0.2:1.

In some embodiments, the cells are stimulated in the presence of, of about, or of at least 0.01 µg, 0.02 µg, 0.03 µg, 0.04 µg, 0.05 µg, 0.1 µg, 0.2 µg, 0.3 µg, 0.4 µg, 0.5 µg, 0.75 µg, 1 µg, 2 µg, 3 µg, 4 µg, 5 µg, 6 µg, 7 µg, 8 µg, 9 µg, or 10 µg of the stimulatory reagent per 10⁶ cells. In some embodiments, the cells are stimulated in the presence of or of about 4 µg of the stimulatory reagent per 10⁶ cells. In particular embodiments, the cells are stimulated in the presence of or of about 0.8 µg of the stimulatory reagent per 10⁶ cells. In particular embodiments, the cells are stimulated or subjected to stimulation in the presence of or of about 3 µg of the stimulatory reagent per 10⁶ cells. In particular embodiments, the cells are stimulated or subjected to stimulation in the presence of or of about 2.5 µg of the stimulatory reagent per 10⁶ cells. In particular embodiments, the cells are stimulated or subjected to stimulation in the presence of or of about 2 µg of the stimulatory reagent per 10⁶ cells. In particular embodiments, the cells are stimulated or subjected to stimulation in the presence of or of about 1.8 µg of the stimulatory reagent per 10⁶ cells. In particular embodiments, the cells are stimulated or subjected to stimulation in the presence of or of about 1.6 µg of the stimulatory reagent per 10⁶ cells. In particular embodiments, the cells are stimulated or subjected to stimulation in the presence of or of about 1.4 µg of the stimulatory reagent per 10⁶ cells. In particular embodiments, the cells are stimulated or subjected to stimulation in the presence of or of about 1.2 µg of the stimulatory reagent per 10⁶ cells. In particular embodiments, the cells are stimulated or subjected to stimulation in the presence of or of about 1 µg of the stimulatory reagent per 10⁶ cells. In particular embodiments, the cells are stimulated or subjected to stimulation in the presence of or of about 0.8 µg of the stimulatory reagent per 10⁶ cells.

In some embodiments, using a device disclosed herein, the methods, which are not in accordance with the claimed invention, provided herein of on-column selection and stimulation of target cells (e.g. T cells) is carried out using a heat/gas column (e.g., housing assembly for column chromatography) as disclosed herein. In some embodiments, any of the methods of on-column selection and stimulation of T cells described herein are performed using a device disclosed herein.

In some embodiments, on-column selection and stimulation is carried out using a chromatography column or column set containing a housing assembly for chromatography in which the stationary phase of the chromatography column is functionalized with a selection agent for selecting or enriching the target cells (e.g. T cells). The housing assembly for the provided chromatography based on-column selection and stimulation methods also contains a temperature control member, e.g. containing one or more heating elements, for regulating and/or maintaining the temperature of the stationary phase in the internal cavity of the column, and a connector configured to operably connect the internal cavity to a gas source, thereby permitting or effecting intake of gas into the internal cavity. In some embodiments, the chromatography column contains an inlet housing member and an outlet housing member, wherein at least the inlet housing member and the outlet housing member form an internal cavity configured to house a stationary phase for column chromatography. Exemplary housing assembles for column chromatography for use in any of the preceding embodiments are described in Section I-A. Exemplary chromatography columns and chromatography column sets for use in any of the preceding embodiments are described in Section I-B.

In one aspect, the method comprises incubating, in the chromatography column or chromatography column set disclosed herein, a sample comprising a plurality of T cells with one or more stimulatory agent to deliver a stimulatory signal in one or more T cells of the plurality of T cells, wherein the plurality of T cells are immobilized on the stationary phase, thereby generating a composition comprising stimulated T cells as the output composition of the chromatography column or chromatography column set. In one aspect, the stationary phase comprises a selection agent that specifically binds to a selection marker on the surface of the one or more T cells. In one aspect, specific binding of the selection agent to the selection marker expressed by the one or more T cells effects the immobilization of the one or more T cells on the stationary phase.

Also disclosed herein in some embodiments is a method of on-column stimulation of T cells, the method comprising: (a) adding a sample comprising a plurality of T cells to the stationary phase in the chromatography column or chromatography column set disclosed herein, the stationary phase comprising a selection agent that binds to a selection marker on the surface of one or more of the plurality of T cells, thereby immobilizing the one or more of the plurality of T cells on the stationary phase; and (b) adding, to the stationary phase in the chromatography column or chromatography column set, a stimulatory reagent comprising one or more stimulatory agent capable of delivering_a stimulatory signal in one or more of the plurality of T cells, thereby initiating incubation of the stimulatory reagent with the one or more T cells, thereby generating a composition comprising stimulated T cells as the output composition of the chromatography column or chromatography column set.

In another aspect, disclosed herein is a method of on-column stimulation of T cells, which are not in accordance with the claimed invention, comprising: (a) combining (i) a sample comprising a plurality of T cells and (ii) the stationary phase in the chromatography kit dislosed herein, the stationary phase comprising a selection agent capable of specifically binding to a selection marker expressed on the surface of one or more of the plurality of T cells, wherein specific binding of the selection agent to a selection marker effects the immobilization of the plurality of T cells on the stationary phase; and (b) adding, to the stationary phase, a stimulatory reagent comprising one or more stimulatory agent capable of delivering a stimulatory signal in T cells, thereby initiating incubation of the stimulatory reagent with the one or more T cells, wherein the combining step and/or the adding step is performed inside or outside the internal cavity of the chromatography column or chromatography column set of the chromatography kit, thereby generating a composition comprising stimulated T cells as the output composition of the chromatography column or chromatography column set. The method in some embodiments further comprises: after the initiation of the incubation, collecting the one or more T cells from the stationary phase. In some embodiments, the one or more T cells are collected from the stationary phase within 24 hours of the initiation of the incubation. In some aspects, the one or more T cells are collected from the stationary phase by gravity flow, and the collecting step is performed without the addition of a competition agent or free binding agent to elute the plurality of T cells from the stationary phase.

In any of the preceding embodiments, during at least a portion of the incubation, the temperature control member can regulate the temperature of the stationary phase to a target temperature of greater than room temperature. In any of the preceding embodiments, during at least a portion of the incubation, the temperature control member can regulate the temperature of the stationary phase to a target temperature that provides a physiologic temperature to the cells during the incubation with the one or more stimulatory agents or stimulatory reagent. In any of the preceding embodiments, during at least a portion of the incubation, the temperature control member can regulate the temperature of the stationary phase to a target temperature of between about 30°C and about 39°C. In any of the preceding embodiments, during at least a portion of the incubation, the temperature control member can regulate the temperature of the stationary phase to a target temperature between about 35°C and about 39°C. For example, the target temperature is 37°C or about 37°C.

In any of the preceding embodiments, during at least a portion of the incubation, the temperature control member can maintain the temperature of the stationary phase to a target temperature of greater than room temperature. In any of the preceding embodiments, during at least a portion of the incubation, the temperature control member can maintain the temperature of the stationary phase to a target temperature that provides a physiologic temperature to the cells during the incubation with the one or more stimulatory agents or stimulatory reagent. In any of the preceding embodiments, during at least a portion of the incubation, the temperature control member can maintain the temperature of the stationary phase to a target temperature of between about 30°C and about 39°C. In some aspects, during at least a portion of the incubation, the temperature control member maintains the temperature of the stationary phase at a target temperature between about 35°C and about 39°C. For example, the target temperature is 37°C or about 37°C.

In any of the preceding embodiments, during at least a portion of the incubation, the connector can allow intake of gas into the internal cavity. For example, the gas is sterile and is or comprises air, and the intake of gas into the internal cavity can be intermittent or continuous during the incubation.

### E. Elution (not part of the claimed invention)

In any of the preceding embodiments, the method, which are not in accordance with the claimed invention, can further comprise: after the initiation of the incubation, collecting the one or more T cells from the stationary phase. In one aspect, the one or more T cells are collected from the stationary phase within 24 hours of the initiation of the incubation. In some embodiments, the one or more T cells are collected from the stationary phase by gravity flow, which is not in accordance with the claimed invention.

In any of the preceding embodiments, the collecting step can be performed without the addition of a competition agent or free binding agent to elute the plurality of T cells from the stationary phase.

Using a device disclosed herein, provided herein are methods, which are not in accordance with the claimed invention, that comprise elution of cells, e.g., target cells (e.g., T cells) following incubation with a stimulatory agent from the chromatography column that is accomplished without the use of a competition agent or free binding agent as described herein. In some embodiments, the elution comprises, consists essentially of, or consists of a washing step, e.g., using a wash media.

In some embodiments, during incubation with the stimulatory agent, cells immobilized via the selection agent on the chromatography matrix (e.g., stationary phase) spontaneously detach from the selection agent. In some embodiments, spontaneous detachment occurs within 24, 23, 22, 21, 20, 19, 18, 17, 16, 15, 14, 13, 12, 11, 10, 9, 8, 7, 6, 5, 4, 3, or 2 hours from the start of the incubation with a stimulatory agent. In some embodiments, spontaneous detachment occurs within about 2 to 24, 3 to 24, 4 to 24, 5, to 24, 6 to 24, 7 to 24, 8 to 24, 9 to 24, 10 to 24, 11 to 24, 12 to 24, 13 to 24, 14 to 24, 15 to 24, 16 to 24, 17 to 24, 18 to 24, 19 to 24, 20 to 24, 21 to 24, 22 to 24, 23 to 24, 2 to 23, 2 to 22, 2 to 21, 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3 hours following the start of incubation with the stimulatory agent. In some embodiments, detachment from the column occurs within or within about 4 to 5 hours, e.g., 4.5 hours following the start of incubation with the stimulatory agent. In some embodiments, the majority of the plurality of target cells (e.g., T cells) immobilized via the selection agent on the chromatography matrix (e.g., stationary phase) detach in less than 24 hours from the start of the incubation with a stimulatory agent. In some embodiments, the majority of the plurality of target cells (e.g., T cells) immobilized via the selection agent on the chromatography matrix (e.g., stationary phase) detach in less than 12 hours from the start of the incubation with a stimulatory agent. In some embodiments, the majority of the plurality of target cells (e.g., T cells) immobilized via the selection agent on the chromatography matrix (e.g., stationary phase) detach in less than 5 hours from the start of the incubation with a stimulatory agent. In some embodiments, the majority of the plurality of target cells (e.g., T cells) immobilized via the selection agent on the chromatography matrix (e.g., stationary phase) detach in less than 4 hours from the start of the incubation with a stimulatory agent. In some embodiments, the majority of the plurality of target cells (e.g., T cells) immobilized via the selection agent on the chromatography matrix (e.g., stationary phase) detach in less than 2 hours from the start of the incubation with a stimulatory agent.

In some embodiments, the spontaneously detached cells are eluted and/or collected via gravity flow from the chromatography column. In some embodiments, the spontaneously detached cells are eluted from the chromatography column using a wash step. In some embodiments, at least one wash step is performed at, at about, or at least 2, 3, 4, 5, 6, 7, 8, 9, 10, 11, 12, 13, 14, 15, 16, 17, 18, 19, 20, 21, 22, 23, or 24 hours after initiation of the incubation with the stimulatory agent or stimulatory reagent containing stimulatory agents. In some embodiments, one or more wash steps are performed at, at about, or at least 2, 3, 4, 5, 6, 7, 8, 9, 10, 11, 12, 13, 14, 15, 16, 17, 18, 19, 20, 21, 22, 23, or 24 hours after initiation of the incubation with the stimulatory agent or stimulatory reagent containing stimulatory agents. In some embodiments, one or more wash steps are performed within about 2 to 24, 3 to 24, 4 to 24, 5, to 24, 6 to 24, 7 to 24, 8 to 24, 9 to 24, 10 to 24, 11 to 24, 12 to 24, 13 to 24, 14 to 24, 15 to 24, 16 to 24, 17 to 24, 18 to 24, 19 to 24, 20 to 24, 21 to 24, 22 to 24, 23 to 24, 2 to 23, 2 to 22, 2 to 21, 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3 hours following the start of incubation with the stimulatory agents or stimulatory reagent including stimulatory agents.

In some embodiments, the eluting and/or collecting step following the selection and on-column stimulation steps is performed within or within about 2 days after the sample is added to the chromatography column (e.g., stationary phase). In some embodiments, the eluting and/or collecting step following the selection and on-column stimulation steps is performed within or within about 1 to 2 days after the sample is added to the chromatography column (e.g., stationary phase). In some embodiments, the eluting and/or collecting step following the selection and on-column stimulation steps is performed within or within about 1 day after the sample is added to the chromatography column (e.g., stationary phase). In some embodiments, the eluting and/or collecting step following the selection and on-column stimulation steps is performed less than 1 day after the sample is added to the chromatography column (e.g., stationary phase). In some embodiments, the eluting and/or collecting step following the selection and on-column stimulation steps is performed within or within about 48, 36, 24, 12, 6, 4, or 2 hours, inclusive, after the sample is added to the chromatography column (e.g., stationary phase). In some embodiments, the collecting or eluting step following the selection and on-column stimulation steps is performed within or within about 2 to 48, 2 to 36, 2 to 24, 2 to 12, 2 to 6, 2 to 4, 4 to 48, 4 to 36, 4 to 24, 4 to 12, 4 to 6, 6 to 48, 6 to 36, 6 to 24, 6 to 12, 12 to 48, 12 to 36, 12 to 24, 24 to 48, 24 to 36, or 36 to 48 hours after the sample is added to the chromatography column (e.g., stationary phase). In some embodiments, the process duration, including steps from selection and on-column stimulation to elution or collecting, is less than 48, 36, 24, 12, 6, 4, or 2 hours. In some embodiments, the process duration, including steps from selection and on-column stimulation to elution or collecting, is less than 36 hours. In some embodiments, the process duration, including steps from selection and on-column stimulation to elution or collecting, is less than 24 hours. In some embodiments, the process duration, including steps from selection and on-column stimulation to elution or collecting, is less than 12 hours. In some embodiments, the process duration, including steps from selection and on-column stimulation to elution or collecting, is, is about, or is less than 7 hours. In some embodiments, the process duration, including steps from selection and on-column stimulation to elution or collecting, is, is about, or is less than 6.5 hours. In some embodiments, the process duration, including steps from selection and on-column stimulation to elution or collecting, is, is about, or is less than 6 hours. In some embodiments, the process duration, including steps from selection and on-column stimulation to elution or collecting, is, is about, or is less than 5.5 hours. In some embodiments, the process duration, including steps from selection and on-column stimulation to elution or collecting, is, is about, or is less than 5 hours. In some embodiments, the process duration, including steps from selection and on-column stimulation to elution or collecting, is, is about, or is less than 4.5 hours. In some embodiments, the process duration, including steps from selection and on-column stimulation to elution or collecting, is, is about, or is less than 4 hours.

In some embodiments, the spontaneously detached cells are collected via gravity flow from the chromatography column. In some embodiments, the spontaneously detached cells are eluted from the chromatography column using a wash step. In some embodiments, the wash media is a culture media. Thus, in some embodiments, the eluted cells can proceed directly to downstream processing (e.g., subsequent selections steps, stimulating steps, incubating steps, genetic engineering). In some embodiments, the wash media comprises serum free basal media containing glutamine and recombinant IL-2, IL-15, and IL-7.

In some embodiments, the eluate comprises stimulatory reagent (e.g., oligomeric stimulatory reagent). In some embodiments, the collected cells are still bound to the stimulatory agents (e.g., stimulatory agents bound to the oligomeric stimulatory reagent). As such, the collected cells may still be considered under stimulating conditions. In some embodiments, the stimulatory agents contained in the eluate are bound to the eluted cell and the stimulatory reagent (e.g., oligomeric stimulatory reagent). As such, the collected and/or eluted cells may still be considered under stimulating conditions. In some embodiments, the detached and eluted cells are under stimulating conditions (e.g., still being stimulated). In some embodiments, the eluted cells may continue under stimulating conditions, for example as described in Section II-D.

In some embodiments, the column and collection containers are connected in a closed system. In some embodiments, the closed system is sterile. In some embodiments, the selection, stimulation, and elution steps are performed by an automated system with minimal or no manual, such as human, operation or interference.

### F. Genetic Engineering (not part of the claimed invention)

In some embodiments, provided herein are methods, which are not in accordance with the claimed invention, that comprise genetically engineering the cells (e.g., output composition), e.g., introducing a heterologous or recombinant polynucleotide encoding a recombinant protein into cells that have been selected and stimulated using a device disclosed herein. Such recombinant proteins may include recombinant receptors, such as any described in Section III. Introduction of the polynucleotides, e.g., heterologous or recombinant polynucleotides, encoding the recombinant protein into the cell may be carried out using any of a number of known vectors. Such vectors include viral, including lentiviral and gammaretroviral, systems. Exemplary methods, which are not in accordance with the claimed invention, include those for transfer of heterologous polynucleotides encoding the receptors, including via viral, e.g., retroviral or lentiviral, transduction. In some embodiments, a population of stimulated cells (e.g., output composition) is genetically engineered, such as to introduce a heterologous or recombinant polynucleotide encoding a recombinant receptor, thereby generating a population of transformed cells (also referred to herein as a transformed population of cells).

In particular embodiments, the cells (e.g., T cells, CD3+, CD4+ T cells) are genetically engineered, transformed, or transduced after the cells have undergone on-column stimulation, such as by any of the methods, which are not in accordance with the claimed invention, provided herein, e.g., in Section II-D. In particular embodiments, the cells (e.g., T cells, CD3+, CD4+ T cells) are genetically engineered, transformed, or transduced after the cells have undergone on-column stimulation, such as by any of the methods, which are not in accordance with the claimed invention, provided herein, e.g., in Section II-D, and collection by gravity flow, e.g. in Section II.E. In particular embodiments, the one or more stimulated populations have been previously cryoprotected and stored, and are thawed prior to genetically engineering, transforming, transfecting, or transducing the cells.

In particular embodiments, the cells (e.g., T cells, CD3+, CD4+ T cells) are genetically engineered, transformed, or transduced after the cells are stimulated or cultured under stimulatory conditions (e.g., on-column stimulation). In particular embodiments, the cells are genetically engineered, transformed, or transduced at, at about, or within 72 hours, 60 hours, 48 hours, 36 hours, 24 hours, 12 hours, 5 hours, 4 hours, or 2 hours, inclusive, from the initiation of the stimulation. In some embodiments, the cells are genetically engineered at or at about 2, 3, 4, 5, or 6 hours from the initiation of on-column stimulation. In some embodiments, the cells are genetically engineered at or at about 4 to 5 hours from the initiation of on-column stimulation. In some embodiments, the cells are still under stimulating conditions during genetic engineering. In certain embodiments, the cells are genetically engineered, transformed, or transduced between or between about 2 hours and 6 hours or 6 hours and 12 hours,after the initiation of the stimulation. In certain embodiments, the cells are genetically engineered, transformed, or transduced between or between about 12 hours and 48 hours, 16 hours and 36 hours, or 18 hours and 30 hours after the initiation of the stimulation. In particular embodiments, the cells are genetically engineered, transformed, or transduced between or between about 18 hours and 30 hours after the initiation of the stimulation. In particular embodiments, the cells are genetically engineered, transformed, or transduced at or at about 22 hours or 24 hours after the initiation of the stimulation. In particular embodiments, the cells are genetically engineered, transformed, or transduced at or at about 6 hours or 12 hours after the initiation of the stimulation. In particular embodiments, the cells are genetically engineered, transformed, or transduced at or at about 4 hours or 5 hours after the initiation of the stimulation. In particular embodiments, the cells are genetically engineered, transformed, or transduced at or at about 2 hours or 3 hours after the initiation of the stimulation.

In certain embodiments, methods for genetic engineering, which are not in accordance with the claimed invention, are carried out by contacting or introducing one or more cells of a population (e.g., output composition) with a nucleic acid molecule or polynucleotide encoding the recombinant protein, e.g. a recombinant receptor. In certain embodiments, the nucleic acid molecule or polynucleotide is heterologous to the cells. In particular embodiments, heterologous nucleic acid molecule or heterologous polynucleotide is not native to the cells. In certain embodiments, the heterologous nucleic acid molecule or heterologous polynucleotide encodes a protein, e.g., a recombinant protein, that is not natively expressed by the cell. In particular embodiments, the heterologous nucleic acid molecule or polynucleotide is or contains a nucleic acid sequence that is not found in the cell prior to the contact or introduction.

In some embodiments, the cells, e.g., output composition, are engineered, e.g., transduced or in the presence of a transduction adjuvant. Exemplary transduction adjuvants include, but are not limited to, polycations, fibronectin or fibronectin-derived fragments or variants, and RetroNectin. In certain embodiments, the cells are engineered in the presence of polycations, fibronectin or fibronectin-derived fragments or variants, and/or RetroNectin. In particular embodiments, the cells are engineered in the presence of a polycation that is polybrene, DEAE-dextran, protamine sulfate, poly-L-lysine, or a cationic liposome. In particular embodiments, the cells are engineered in the presence of protamine sulfate.

In some embodiments, the genetic engineering, e.g., transduction, is carried out in serum free media. In some embodiments, the serum free media is a defined or well-defined cell culture media. In certain embodiments, the serum free media is a controlled culture media that has been processed, e.g., filtered to remove inhibitors and/or growth factors. In some embodiments, the serum free media contains proteins. In certain embodiments, the serum-free media may contain serum albumin, hydrolysates, growth factors, hormones, carrier proteins, and/or attachment factors. In some embodiments, the media comprises glutamine.

In particular embodiments, the cells are engineered in the presence of one or more cytokines. In certain embodiments, the one or more cytokines are recombinant cytokines. In particular embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines is or includes a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more cytokines is or includes IL-15. In particular embodiments, the one or more cytokines is or includes IL-7. In particular embodiments, the one or more cytokines is or includes recombinant IL-2.

In particular embodiments, cells, e.g., stimulated cells are engineered under stimulating conditions in the presence of IL-2, IL-7, and/or IL-15. In certain embodiments, the IL-2, IL-7, and/or IL-15 are recombinant. In certain embodiments, the IL-2, IL-7, and/or IL-15 are human. In particular embodiments, the one or more cytokines are or include human recombinant IL-2, IL-7, and/or IL-15. In certain embodiments, the cells are engineered, e.g., transduced or under stimulating conditions in the presence of recombinant IL-2, IL-7, and IL-15.

In some embodiments, the cells are genetically engineered, transformed, or transduced in the presence of the same or similar media as was present during the stimulation. In some embodiments, the cells are genetically engineered, transformed, or transduced in media having the same cytokines as the media present during stimulation. In certain embodiments, the cells are genetically engineered, transformed, or transduced, in media having the same cytokines at the same concentrations as the media present during stimulation.

### 1. Transduction (not part of the claimed invention)

In some embodiments, provided herein are methods, which are not in accordance with the claimed invention, that comprise genetically engineering the cells (e.g., output composition) by introducing the polynucleotide, e.g., the heterologous or recombinant polynucleotide, by transduction into the cells that have been selected and stimulated using a device disclosed herein. In some embodiments, the cells are transduced with a viral vector. In particular embodiments, the cells are transduced with a viral vector. In some embodiments, the virus is a retroviral vector, such as a gammaretroviral vector or a lentiviral vector. Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, the transduction, which is not in accordance with the claimed invention, is carried out by contacting one or more cells of a population (e.g., output composition) with a nucleic acid molecule encoding the recombinant protein, e.g. recombinant receptor. In some embodiments, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some embodiments, the provided methods are used in connection with transducing a viral vector containing a polynucleotide encoding a recombinant receptor into, into about, or into less than 300 x 10⁶ cells, e.g., viable T cells of a stimulated cell population. In certain embodiments, at or about 100 x 10⁶ cells, e.g., viable T cells of a stimulated cell population are transduced. In some embodiments, 1 x 10⁶ cells per mL e.g., viable T cells of a stimulated cell population are transduced or subjected to transduction. In some embodiments, the viral vector dose is or is about 10, 9, 8, 7, 6, 5, 4, 3, 2, or 1 µL per 1 x 10⁶ cells. In some embodiments, the viral vector dose is between or is between about 6 to 4 µL per 1 x 10⁶ cells. In some embodiments, the viral vector dose is or is about 5 µL per 1 x 10⁶ cells.

In some embodiments, the transduction is performed in serum free media. In some embodiments, the transduction is performed in the presence of IL-2, IL-7, and IL-15. In particular embodiments, the cells, e.g., the cells of the stimulated cell population (e.g., output composition) contain at least 80%, at least 85%, at least 90%, or at least 95% cells that are CD4+ T cells or CD8+ T cells. In some embodiments, the transduction is performed for between 24 and 48 hours, between 36 and 12 hours, between 18 and 30 hours, or for or for about 24 hours. In certain embodiments, the transduction step is initiated within two days, within 36 hours, within 30 hours, within 24 hours, within 12 hours, within 6 hours, within 5 hours, within 4 hours, or within 2 hours of the start or initiation of the incubation, e.g., the incubation under stimulating conditions. In some embodiments, the transduction, including post-transduction incubation, is performed for or for about 72 hours ± 6 hours. In some embodiments, the transduction is performed for or for about 0.5, 1, 1.5, 2, 2.5, 3, 3.5, 4, 4.5, or 5 hours. In some embodiments, the transduction is performed for or for about 0.5, 1, 1.5, or 2 hours. In some embodiments, the transduction is performed for or for about 0.5 to 1.5 hours. In some embodiments, the transduction is performed for or for about 1 hour.

In certain embodiments, the transduction step is initiated within two days, within 36 hours, within 30 hours, within 24 hours, within 12 hours, within 6 hours, within 5 hours, within 4 hours, or within 2 hours of the start or initiation of the incubation, e.g., the incubation under stimulating conditions. In certain embodiments, the transduction step is initiated within 4 to 5 hours of the start or initiation of the incubation, e.g., the incubation under stimulating conditions. In certain embodiments, the transduction step is initiated at about 20 hours of the start or initiation of the incubation, e.g., the incubation under stimulating conditions. In certain embodiments, the transduction step is initiated at or at about 4 to 5 hours of the start or initiation of the incubation, e.g., the incubation under stimulating conditions.

In some embodiments, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in International Publication Number WO2016/073602. This instrumentation in some embodiments is contained within a cabinet. In some embodiments, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some embodiments, the system comprises a series of containers, e.g., bags, tubing, stopcocks, clamps, connectors, and a centrifuge chamber. In some embodiments, the containers, such as bags, include one or more containers, such as bags, containing the cells to be transduced and the viral vector particles, in the same container or separate containers, such as the same bag or separate bags. In some embodiments, the system further includes one or more containers, such as bags, containing medium, such as diluent and/or wash solution, which is pulled into the chamber and/or other components to dilute, resuspend, and/or wash components and/or populations during the methods. The containers can be connected at one or more positions in the system, such as at a position corresponding to an input line, diluent line, wash line, waste line and/or output line.

In some embodiments, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some embodiments, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some embodiments, the population containing cells and population containing viral vector particles, and optionally air, can be combined or mixed prior to providing the populations to the cavity. In some embodiments, the population containing cells and population containing viral vector particles, and optionally air, are provided separately and combined and mixed in the cavity. In some embodiments, a population containing cells, a population containing viral vector particles, and optionally air, can be provided to the internal cavity in any order. In any of such some embodiments, a population containing cells and viral vector particles is the input population once combined or mixed together, whether such is combined or mixed inside or outside the centrifugal chamber and/or whether cells and viral vector particles are provided to the centrifugal chamber together or separately, such as simultaneously or sequentially.

In some embodiments, intake of the volume of gas, such as air, occurs prior to the incubating the cells and viral vector particles, such as rotation, in the transduction method. In some embodiments, intake of the volume of gas, such as air, occurs during the incubation of the cells and viral vector particles, such as rotation, in the transduction method.

In some embodiments, the liquid volume of the cells or viral vector particles that make up the transduction population, and optionally the volume of air, can be a predetermined volume. The volume can be a volume that is programmed into and/or controlled by circuitry associated with the system.

In some embodiments, intake of the transduction population, and optionally gas, such as air, is controlled manually, semi-automatically and/or automatically until a desired or predetermined volume has been taken into the internal cavity of the chamber. In some embodiments, a sensor associated with the system can detect liquid and/or gas flowing to and from the centrifuge chamber, such as via its color, flow rate and/or density, and can communicate with associated circuitry to stop or continue the intake as necessary until intake of such desired or predetermined volume has been achieved. In some aspects, a sensor that is programmed or able only to detect liquid in the system, but not gas (e.g. air), can be made able to permit passage of gas, such as air, into the system without stopping intake. In some such embodiments, a non-clear piece of tubing can be placed in the line near the sensor while intake of gas, such as air, is desired. In some embodiments, intake of gas, such as air, can be controlled manually.

In aspects of the provided methods, which are not in accordance with the claimed invention, the internal cavity of the centrifuge chamber is subjected to high speed rotation. In some embodiments, rotation is effected prior to, simultaneously, subsequently or intermittently with intake of the liquid input population, and optionally air. In some embodiments, rotation is effected subsequent to intake of the liquid input population, and optionally air. In some embodiments, rotation is by centrifugation of the centrifugal chamber at a relative centrifugal force at the inner surface of side wall of the internal cavity and/or at a surface layer of the cells of at or about or at least at or about 200 g, 300 g, 400 g, 500 g, 600 g, 700 g, 800 g, 1000 g, 1100 g, 1500, 1600 g, 1800 g, 2000 g, 2200 g, 2500 g, 3000 g, 3200 g, 3500 g or 4000 g. In some embodiments, rotation is by centrifugation at a force that is greater than or about 1100 g, such as by greater than or about 1200 g, greater than or about 1400 g, greater than or about 1600 g, greater than or about 1800 g, greater than or about 2000 g, greater than or about 2400 g, greater than or about 2800 g, greater than or about 3000 g or greater than or about 3200 g. In particular embodiments, the rotation by centrifugation is at a force between 600 g and 800 g. In particular embodiments, the rotation by centrifugation is at a force of or of about 693 g. In some embodiments, rotation is by centrifugation at a force that is or is about 1600g.

In some embodiments, the gas, such as air, in the cavity of the chamber is expelled from the chamber. In some embodiments, the gas, such as air, is expelled to a container that is operably linked as part of the closed system with the centrifugal chamber. In some embodiments, the container is a free or empty container. In some embodiments, the air, such as gas, in the cavity of the chamber is expelled through a filter that is operably connected to the internal cavity of the chamber via a sterile tubing line. In some embodiments, the air is expelled using manual, semi-automatic or automatic processes. In some embodiments, air is expelled from the chamber prior to, simultaneously, intermittently or subsequently with expressing the output population containing incubated cells and viral vector particles, such as cells in which transduction has been initiated or cells have been transduced with a viral vector, from the cavity of the chamber.

In some embodiments, the transduction and/or other incubation is performed as or as part of a continuous or semi-continuous process, which are not in accordance with the claimed invention. In some embodiments, a continuous process involves the continuous intake of the cells and viral vector particles, e.g., the transduction composition (either as a single pre-existing composition or by continuously pulling into the same vessel, e.g., cavity, and thereby mixing, its parts), and/or the continuous expression or expulsion of liquid, and optionally expelling of gas (e.g. air), from the vessel, during at least a portion of the incubation, e.g., while centrifuging. In some embodiments, the continuous intake and continuous expression are carried out at least in part simultaneously. In some embodiments, the continuous intake occurs during part of the incubation, e.g., during part of the centrifugation, and the continuous expression occurs during a separate part of the incubation. The two may alternate. Thus, the continuous intake and expression, while carrying out the incubation, can allow for a greater overall volume of sample to be processed, e.g., transduced.

In some embodiments, the incubation is part of a continuous process, the method including, during at least a portion of the incubation, effecting continuous intake of said transduction composition into the cavity during rotation of the chamber and during a portion of the incubation, effecting continuous expression of liquid and, optionally expelling of gas (e.g. air), from the cavity through the at least one opening during rotation of the chamber.

In some embodiments, the semi-continuous incubation, which are not in accordance with the claimed invention, is carried out by alternating between effecting intake of the composition into the cavity, incubation, expression of liquid from the cavity and, optionally expelling of gas (e.g. air) from the cavity, such as to an output container, and then intake of a subsequent (e.g., second, third, etc.) composition containing more cells and other reagents for processing, e.g., viral vector particles, and repeating the process. For example, in some embodiments, the incubation is part of a semi-continuous process, the method including, prior to the incubation, effecting intake of the transduction composition into the cavity through said at least one opening, and subsequent to the incubation, effecting expression of fluid from the cavity; effecting intake of another transduction composition comprising cells and the viral vector particles into said internal cavity; and incubating the another transduction composition in said internal cavity under conditions whereby said cells in said another transduction composition are transduced with said vector. The process may be continued in an iterative fashion for a number of additional rounds. In this respect, the semi-continuous or continuous methods may permit production of even greater volume and/or number of cells.

In some embodiments, a portion of the transduction incubation is performed in the centrifugal chamber, which is performed under conditions that include rotation or centrifugation.

In particular embodiments, transduction of the cells with the viral vector, which is not in accordance with the claimed invention, is or includes spinoculation, e.g., centrifugation of a mixture containing the cells and the viral particles. In some embodiments, the composition containing cells and viral particles can be rotated, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm). In some embodiments, the rotation is carried at a force, e.g., a relative centrifugal force, of from or from about 100 g to 4000 g (e.g. at or about or at least at or about 100 g, 200 g, 300 g, 400 g, 500 g, 600 g, 700 g, 800 g, 900 g, 1000 g, 1500 g, 2000 g, 2500 g, 3000 g or 3500 g), as measured for example at an internal or external wall of the chamber or cavity.

In some embodiments, the cells are spinoculated with the viral vector at a force, e.g., a relative centrifugal force, of between or between about 100 g and 4000 g, 200 g and 1,000 g, 500 g and 1200 g, 1000 g and 2000 g, 600 g and 800 g, 1200 g and 1800 g, or 1500 g and 1800 g. In certain embodiments, the cells are spinoculated with the viral vector particle for, for at least, or for about 100 g, 200 g, 300 g, 400 g, 500 g, 600 g, 700 g, 800 g, 900 g, 1000 g, 1200g, 1500 g, 1600g, 2000 g, 2500 g, 3000 g, 3200 g, or 3500 g. In some embodiments, the cells are transduced with the viral vector at a force of or of about 692 g. In particular embodiments, the cells are transduced with the viral vector at a force of or of about 1600 g. In some embodiments, the force is the force at the internal surface of the side wall of the internal cavity and/or at a surface layer of the cells.

In certain embodiments, the cells are spinoculated, e.g., the cell composition containing cells and viral vector is rotated, for greater than or about 5 minutes, such as greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes; or between or between about 5 minutes and 120 minutes, 30 minutes and 90 minutes, 15 minutes and 60 minutes, 15 minutes and 45 minutes, 30 minutes and 60 minutes or 45 minutes and 60 minutes, each inclusive. In some embodiments, the cells are spinoculated with the viral vector for or for about 30 minutes. In certain embodiments, the cells are spinoculated with the viral vector for or for about 60 minutes.

In some embodiments, the method of transduction, which is not in accordance with the claimed invention, includes a spinoculation, e.g., a rotation or centrifugation of the transduction composition, and optionally air, in the centrifugal chamber for greater than or about 5 minutes, such as greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes. In some embodiments, the transduction composition, and optionally air, is rotated or centrifuged in the centrifugal chamber for greater than 5 minutes, but for no more than 60 minutes, no more than 45 minutes, no more than 30 minutes or no more than 15 minutes. In particular embodiments, the transduction includes rotation or centrifugation for or for about 60 minutes.

In some embodiments, the method of transduction, which is not in accordance with the claimed invention, includes rotation or centrifugation of the transduction composition, and optionally air, in the centrifugal chamber for between or between about 10 minutes and 60 minutes, 15 minutes and 60 minutes, 15 minutes and 45 minutes, 30 minutes and 60 minutes or 45 minutes and 60 minutes, each inclusive, and at a force at the internal surface of the side wall of the internal cavity and/or at a surface layer of the cells of, of about, or at 1000 g, 1100 g, 1200 g, 1400 g, 1500 g, 1600 g, 1800 g, 2000 g, 2200 g, 2400 g, 2800 g, 3200 g or 3600 g. In particular embodiments, the method of transduction includes rotation or centrifugation of the transduction composition, e.g., the cells and the viral vector particles, at or at about 1600 g for or for about 60 minutes.

In some embodiments, the method of transduction does not include rotation or centrifugation.

### 2. Viral Vector Particles (not part of the claimed invention)

In some embodiments, for transduction in methods, which are not in accordance with the claimed invention, provided herein, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

The viral vector genome is typically constructed in a plasmid form that can be transfected into a packaging or producer cell line. In any of such examples, the nucleic acid encoding a recombinant protein, such as a recombinant receptor, is inserted or located in a region of the viral vector, such as generally in a non-essential region of the viral genome. In some embodiments, the nucleic acid is inserted into the viral genome in the place of certain viral sequences to produce a virus that is replication defective.

Any of a variety of known methods can be used to produce retroviral particles whose genome contains an RNA copy of the viral vector genome. In some embodiments, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some embodiments, the packaging plasmid can contain all retroviral, such as HIV-1, proteins other than envelope proteins (Naldini et al., 1998). In other embodiments, viral vectors can lack additional viral genes, such as those that are associated with virulence, e.g. vpr, vif, vpu and nef, and/or Tat, a primary transactivator of HIV. In some embodiments, lentiviral vectors, such as HIV-based lentiviral vectors, comprise only three genes of the parental virus: gag, pol and rev, which reduces or eliminates the possibility of reconstitution of a wild-type virus through recombination.

In some embodiments, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some aspects, in order to prevent replication of the genome in the target cell, however, endogenous viral genes required for replication are removed and provided separately in the packaging cell line.

In some embodiments, a packaging cell line is transfected with one or more plasmid vectors containing the components necessary to generate the particles. In some embodiments, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some embodiments, multiple vectors are utilized to separate the various genetic components that generate the retroviral vector particles. In some such embodiments, providing separate vectors to the packaging cell reduces the chance of recombination events that might otherwise generate replication competent viruses. In some embodiments, a single plasmid vector having all of the retroviral components can be used.

In some embodiments, the retroviral vector particle, such as lentiviral vector particle, is pseudotyped to increase the transduction efficiency of host cells. For example, a retroviral vector particle, such as a lentiviral vector particle, in some embodiments is pseudotyped with a VSV-G glycoprotein, which provides a broad cell host range extending the cell types that can be transduced. In some embodiments, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some embodiments, the packaging cell line provides the components, including viral regulatory and structural proteins, that are required in trans for the packaging of the viral genomic RNA into lentiviral vector particles. In some embodiments, the packaging cell line may be any cell line that is capable of expressing lentiviral proteins and producing functional lentiviral vector particles. In some aspects, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some embodiments, the packaging cell line stably expresses the viral protein(s). For example, in some aspects, a packaging cell line containing the gag, pol, rev and/or other structural genes but without the LTR and packaging components can be constructed. In some embodiments, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some embodiments, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some embodiments is then collected, optionally concentrated, and used for gene transfer. For example, in some aspects, after cotransfection of the packaging plasmids and the transfer vector to the packaging cell line, the viral vector particles are recovered from the culture media and titered by standard methods used by those of skill in the art.

**In** some embodiments, a retroviral vector, such as a lentiviral vector, can be produced in a packaging cell line, such as an exemplary HEK 293T cell line, by introduction of plasmids to allow generation of lentiviral particles. In some embodiments, a packaging cell is transfected and/or contains a polynucleotide encoding gag and pol, and a polynucleotide encoding a recombinant receptor, such as an antigen receptor, for example, a CAR. In some embodiments, the packaging cell line is optionally and/or additionally transfected with and/or contains a polynucleotide encoding a rev protein. In some embodiments, the packaging cell line is optionally and/or additionally transfected with and/or contains a polynucleotide encoding a non-native envelope glycoprotein, such as VSV-G. In some such embodiments, approximately two days after transfection of cells, e.g. HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g. antigen receptor, such as CAR, can be detected.

### 3. Incubating the Cells (not part of the claimed invention)

In embodiments of methods, which are not in accordance with the claimed invention, herein that comprise genetic engineering, such as by transforming (e.g. transducing) the cells (e.g. output composition) with a viral vector, the methods can further include one or more steps of incubating the cells after the introducing or contacting of the cells with the viral vector. In some embodiments, cells, e.g., cells of the transformed cell population, are incubated subsequent to processes for genetically engineering, transforming, transducing, or transfecting the cells to introduce the viral vector into the cells. In particular embodiments, the incubation results in a population of incubated cells (also referred to herein as an incubated cell population).

In some embodiments, the cells are incubated after the introducing of the heterologous or recombinant polynucleotide, e.g., viral vector particles is carried out without further processing of the cells. In particular embodiments, prior to the incubating, the cells are washed, such as to remove or substantially remove exogenous or remaining polynucleotides encoding the heterologous or recombinant polynucleotide, e.g. viral vector particles, such as those remaining in the media after the genetic engineering process following the spinoculation.

In some such embodiments, the further incubation, which is not in accordance with the claimed invention, is effected under conditions to result in integration of the viral vector into a host genome of one or more of the cells. It is within the level of a skilled artisan to assess or determine if the incubation has resulted in integration of viral vector particles into a host genome, and hence to empirically determine the conditions for a further incubation. In some embodiments, integration of a viral vector into a host genome can be assessed by measuring the level of expression of a recombinant protein, such as a heterologous protein, encoded by a nucleic acid contained in the genome of the viral vector particle following incubation. A number of well-known methods for assessing expression level of recombinant molecules may be used, such as detection by affinity-based methods, e.g., immunoaffinity-based methods, e.g., in the context of cell surface proteins, such as by flow cytometry. In some examples, the expression is measured by detection of a transduction marker and/or reporter construct. In some embodiments, nucleic acid encoding a truncated surface protein is included within the vector and used as a marker of expression and/or enhancement thereof.

In certain embodiments, the incubation is performed under static conditions, such as conditions that do not involve centrifugation, shaking, rotating, rocking, or perfusion, e.g., continuous or semi-continuous perfusion of the media. In some embodiments, either prior to or shortly after, e.g., within 5, 15, or 30 minutes, the initiation of the incubation, the cells are transferred (e.g., transferred under sterile conditions) to a container such as a bag or vial, and placed in an incubator.

In some embodiments, at least a portion of the incubation is carried out in the internal cavity of a centrifugal chamber, such as described in International Publication Number WO2016/073602.

In some embodiments, the cells that have been introduced with a polynucleotide encoding the heterologous or recombinant polypeptide, e.g., the viral vectors, are transferred into a container for the incubation. In some embodiments, the container is a vial. In particular embodiments, the container is a bag. In some embodiments, the cells, and optionally the heterologous or recombinant polypeptide, are transferred into the container under closed or sterile conditions. In some embodiments, the container, e.g., the vial or bag, is then placed into an incubator for all or a portion of the incubation. In particular embodiments, incubator is set at, at about, or at least 16°C, 24°C, or 35°C. In some embodiments, the incubator is set at 37°C, at about at 37°C, or at 37°C ±2°C, ±1°C, ±0.5°C, or ±0.1°C.

In some aspects, the conditions for the incubation can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the incubation is performed in serum free media. In some embodiments, the serum free media is a defined and/or well-defined cell culture media. In certain embodiments, the serum free media is a controlled culture media that has been processed, e.g., filtered to remove inhibitors and/or growth factors. In some embodiments, the serum free media contains proteins. In certain embodiments, the serum-free media may contain serum albumin, hydrolysates, growth factors, hormones, carrier proteins, and/or attachment factors.

In particular embodiments, the cells are incubated in the presence of one or more cytokines. In certain embodiments, the one or more cytokines are recombinant cytokines. In particular embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines is or includes a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more cytokines is or includes IL-15. In particular embodiments, the one or more cytokines is or includes IL-7. In particular embodiments, the one or more cytokines is or includes recombinant IL-2.

In particular embodiments, the cells are incubated in the presence of IL-2, IL-7, and/or IL-15. In certain embodiments, the IL-2, IL-7, and/or IL-15 are recombinant. In certain embodiments, the IL-2, IL-7, and/or IL-15 are human. In particular embodiments, the one or more cytokines are or include human recombinant IL-2, IL-7, and/or IL-15. In certain embodiments, the cells are incubated in the presence of recombinant IL-2, IL-7, and IL-15.

In some embodiments, the cells, e.g., the transformed cells, are incubated with a cytokine, e.g., a recombinant human cytokine, at a concentration of between 1 IU/mL and 1,000 IU/mL, between 10 IU/mL and 50 IU/mL, between 50 IU/mL and 100 IU/mL, between 100 IU/mL and 200 IU/mL, between 100 IU/mL and 500 IU/mL, between 250 IU/mL and 500 IU/mL, or between 500 IU/mL and 1,000 IU/mL.

In some embodiments, the cells, e.g., the transformed cells, are incubated with IL-2, e.g., human recombinant IL-2, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular embodiments, cells, e.g., transformed cells, are incubated with recombinant IL-2 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 100 IU/mL. In some embodiments, the cells, e.g., the transformed cells, are incubated in the presence of or of about 100 IU/mL of recombinant IL-2, e.g., human recombinant IL-2.

In some embodiments, the cells, e.g., the transformed cells, are incubated with recombinant IL-7, e.g., human recombinant IL-7, at a concentration between 100 IU/mL and 2,000 IU/mL, between 500 IU/mL and 1,000 IU/mL, between 100 IU/mL and 500 IU/mL, between 500 IU/mL and 750 IU/mL, between 750 IU/mL and 1,000 IU/mL, or between 550 IU/mL and 650 IU/mL. In particular embodiments, the cells, e.g., the transformed cells, are incubated with IL-7 at a concentration at or at about 50 IU/mL, 100 IU/mL, 150 IU/mL, 200 IU/mL, 250 IU/mL, 300 IU/mL, 350 IU/mL, 400 IU/mL, 450 IU/mL, 500 IU/mL, 550 IU/mL, 600 IU/mL, 650 IU/mL, 700 IU/mL, 750 IU/mL, 800 IU/mL, 750 IU/mL, 750 IU/mL, 750 IU/mL, or 1,000 IU/mL. In particular embodiments, the cells, e.g., the transformed cells, are incubated in the presence of or of about 600 IU/mL of IL-7.

In some embodiments, the cells, e.g., the transformed cells, are incubated with recombinant IL-15, e.g., human recombinant IL-15, at a concentration between 1 IU/mL and 500 IU/mL, between 10 IU/mL and 250 IU/mL, between 50 IU/mL and 200 IU/mL, between 50 IU/mL and 150 IU/mL, between 75 IU/mL and 125 IU/mL, between 100 IU/mL and 200 IU/mL, or between 10 IU/mL and 100 IU/mL. In particular embodiments, cells, e.g., transformed cells, are incubated with recombinant IL-15 at a concentration at or at about 50 IU/mL, 60 IU/mL, 70 IU/mL, 80 IU/mL, 90 IU/mL, 100 IU/mL, 110 IU/mL, 120 IU/mL, 130 IU/mL, 140 IU/mL, 150 IU/mL, 160 IU/mL, 170 IU/mL, 180 IU/mL, 190 IU/mL, or 200 IU/mL. In some embodiments, the cells, e.g., the transformed cells, are incubated in the presence of or of about 100 IU/mL of recombinant IL-15, e.g., human recombinant IL-2.

In particular embodiments, the cells, e.g., transformed cells, are incubated in the presence of IL-2, IL-7, and/or IL-15. In some embodiments, the IL-2, IL-7, and/or IL-15 are recombinant. In certain embodiments, the IL-2, IL-7, and/or IL-15 are human. In particular embodiments, the one or more cytokines are or include human recombinant IL-2, IL-7, and/or IL-15. In certain embodiments, the cells are incubated in the presence of recombinant IL-2, IL-7, and IL-15.

In some embodiments, the cells are incubated in the presence of the same or similar media as was present during the stimulation of the cells, such as carried out in connection with methods or processes of stimulation (e.g., on-column stimulation) described above. In some embodiments, the cells are incubated in media having the same cytokines as the media present during stimulation of the cells, such as carried out in connection with methods or processes of stimulation described above. In certain embodiments, the cells are incubated in media having the same cytokines at the same concentrations as the media present during stimulation of the cells, such as carried out in connection with methods or processes of stimulation described above.

In some embodiments, the cells are incubated in the absence of recombinant cytokines.

In some embodiments, all or a portion of the incubation is performed in basal media, which is not in accordance with the claimed invention,. In some embodiments, the basal medium contains a mixture of inorganic salts, sugars, amino acids, and, optionally, vitamins, organic acids and/or buffers or other well known cell culture nutrients. In addition to nutrients, the medium also helps maintain pH and osmolality. In some aspects, the reagents of the basal media support cell growth, proliferation and/or expansion. A wide variety of commercially available basal media are well known to those skilled in the art, and include Dulbeccos' Modified Eagles Medium (DMEM), Roswell Park Memorial Institute Medium (RPMI), Iscove modified Dulbeccos' medium and Hams medium. In some embodiments, the basal medium is Iscove's Modified Dulbecco's Medium, RPMI- 1640, or α-MEM.

In some embodiments, the basal media is a balanced salt solution (e.g., PBS, DPBS, HBSS, EBSS). In some embodiments, the basal media is selected from Dulbecco's Modified Eagle's Medium (DMEM), Minimal Essential Medium (MEM), Basal Medium Eagle (BME), F-10, F-12, RPMI 1640, Glasgow's Minimal Essential Medium (GMEM), alpha Minimal Essential Medium (alpha MEM), Iscove's Modified Dulbecco's Medium, and M199. In some embodiments, the base media is a complex medium (e.g., RPMI-1640, IMDM). In some embodiments, the base medium is OpTmizer^{™} CTS^{™} T-Cell Expansion Basal Medium (ThermoFisher).

In certain embodiments, the basal media is supplemented with additional additives. In some embodiments, the basal media is not supplemented with any additional additives. Additives to cell culture media may include, but is not limited to nutrients, sugars, e.g., glucose, amino acids, vitamins, or additives such as ATP and NADH.

In some embodiments, the basal medium is free of a protein. In some embodiments, the basal medium is free of a human protein (e.g., a human serum protein). In some embodiments, the basal medium is serum-free. In some embodiments, the basal medium is free of serum derived from human. In some embodiments, the basal medium is free of a recombinant protein. In some embodiments, the basal medium is free of a human protein and a recombinant protein. In some embodiments, the basal medium is free of a human protein and a recombinant protein. In some embodiments, the basal medium is free of one or more or all cytokines as described herein.

In some embodiments, all or a portion of the incubation, e.g., for the non-expanded process, is performed in a basal medium without any additional additives or recombinant cytokines. In some embodiments, the basal media is a CTS OpTmizer basal media (Thermofisher) without any additional additives or recombinant cytokines. In some embodiments, all or a portion of the incubation, e.g., for the non-expanded process, is performed in a media comprising a basal medium and glutamine, e.g., a CTS OpTmizer basal media (Thermofisher) with glutamine.

In some embodiments, all or a portion of the incubation, e.g., of the non-expanded process, is performed in a media comprising a basal medium (e.g., a CTS OpTmizer basal media (Thermofisher)) without one or more recombinant cytokines, such as recombinant human IL-2, recombinant human IL-7, and/or recombinant human IL-15. In some embodiments, the medium is supplemented with one or more additional non-serum component. In some embodiments, the one or more supplement is serum-free. In some embodiments, the serum-free medium further comprises a free form of an amino acid such as L-glutamine. In some embodiments, the serum-free medium does not comprise a serum replacement supplement. In some embodiments, the serum-free medium does not comprise a dipeptide form of L-glutamine (e.g., L-alanyl-L-glutamine). In some embodiments, the serum-free medium does not comprise any recombinant cytokine. In some embodiments, the serum-free medium comprises a basal medium supplemented with a T cell supplement and a free form of L-glutamine, and does not contain any immune cell serum replacement, any dipeptide form of L-glutamine, or any recombinant cytokine. In some embodiments, the serum-free medium comprises a basal medium (e.g. OpTmizer^{™} T-Cell Expansion Basal Medium supplemented), L-glutamine and one or more additional compoents such as provided by a supplement (e.g. OpTmizer^{™} T-Cell Expansion Supplement).

In particular embodiments, the cells are incubated in the serum free medium at a concentration of or of about 0.25×10⁶ cells/mL, 0.5×10⁶ cells/mL, 0.75×10⁶ cells/mL, 1.0×10⁶ cells/mL, 1.25×10⁶ cells/mL, 1.5×10⁶ cells/mL, 1.75×10⁶ cells/mL, or 2.0×10⁶ cells/mL. In particular embodiments, the cells are incubated in the serum free medium at a concentration between or between about 0.25×10⁶ cells/mL to 1.0×10⁶ cell/mL. In particular embodiments, the cells are incubated in the serum free medium at a concentration between or between about 0.25×10⁶ cells/mL to 0.75×10⁶ cell/mL. In particular embodiments, the cells are incubated in the serum free medium at a concentration between or between about 0.5×10⁶ cells/mL to 0.75×10⁶ cell/mL. In particular embodiments, the cells are incubated in the serum free medium at a concentration between or between about 0.25×10⁶ cells/mL to 0.5×10⁶ cell/mL. In particular embodiments, the cells are incubated in the serum free medium at a concentration of or of about 0.75×10⁶ cells/mL. In particular embodiments, the cells are incubated in the serum free medium at a concentration of or of about 0.5×10⁶ cells/mL. In some embodiments, the incubating is for or for about between 18 hours and 30 hours. In particular embodiments, the incubating is for or for about 24 hours or for for for about one day.

In particular embodiments, the cells are incubated in the absence of cytokines. In particular embodiments, the cells are incubated in the absence of any recombinant cytokine. In particular embodiments, the cells are incubated in the absence of one or more recombinant cytokine, such as recombinant IL-2, IL-7, and/or IL-15.

In some embodiments, all or a portion of the incubation, e.g., for the non-expanded process, is performed in a media comprising a basal media, glutamine, and one or more recombinant cytokines, e.g., a CTS OpTmizer basal media (Thermofisher) with glutamine and recombinant IL-2, IL-7, and/or IL-15. In some embodiments, all or a portion of the incubation, e.g., for the non-expanded process, is performed in a media comprising a basal media, glutamine, one or more recombinant cytokines, and a T cell supplement, e.g., a CTS OpTmizer basal media (Thermofisher) with glutamine, recombinant IL-2, IL-7, and/or IL-15, and an OpTmizer^{®} supplement (Thermofisher). In some embodiments, all or a portion of the incubation, e.g., for the non-expanded process, is performed in a media comprising a basal media, glutamine, one or more recombinant cytokines, a T cell supplement, and one or more serum-substituting proteins, e.g., a CTS OpTmizer basal media (Thermo fisher) with glutamine, recombinant IL-2, IL-7, and/or IL-15, an OpTmizer^{®} supplement (Thermofisher), and serum-substituting proteins such as one or more of albumin, insulin or transferrin.

In some embodiments, the basal medium further comprises glutamine, such as L-glutamine. In some aspects, the glutamine is a free form of glutamine, such as L-glutamine. In some embodiments, the concentration of the glutamine, such as L-glutamine, in the basal medium is about or less than about about 0.5mM-1mM, 0.5mM-1.5mM, 0.5mM-2mM, 0.5mM-2.5mM, 0.5mM-3mM, 0.5mM-3.5mM, 0.5mM-4mM, 0.5mM-4.5mM, 0.5mM-5mM, 1mM-1.5mM, 1mM-2mM, 1mM-2.5mM, 1mM-3mM, 1mM-3.5mM, 1mM-4mM, 1mM-4.5mM, 1mM-5mM, 1.5mM-2mM, 1.5mM-2.5mM, 1.5mM-3mM, 1.5mM-3.5mM, 1.5mM-4mM, 1.5mM-4.5mM, 1.5mM-5mM, 2mM-2.5mM, 2mM-3mM, 2mM-3.5mM, 2mM-4mM, 2mM-4.5mM, 2mM-5mM, 2.5mM-3mM, 2.5mM-3.5mM, 2.5mM-4mM, 2.5mM-4.5mM, 2.5mM-5mM, 3mM-3.5mM, 3mM-4mM, 3mM-4.5mM, 3mM-5mM, 3.5mM-4mM, 3.5mM-4.5mM, 3.5mM-5mM, 4mM-4.5mM, 4mM-5mM, or 4.5mM-5mM, each inclusive. In some embodiments, the concentration of glutamin, such as L-glutamine, in the basal medium is at least about 0.5mM, 1mM, 1.5mM, 2mM, 2.5mM, 3mM, 3.5mM, 4mM, 4.5mM, or 5mM. In some embodiments, the concentration of glutamine, such as L-glutamine, in the basal medium is at most about 2mM, 2.5mM, 3mM, 3.5mM, 4mM, 4.5mM, 5mM. In some embodiments, the concentration of glutamine, such as L-glutamine, in the basal medium is about 2 mM.

In some embodiments, the basal medium further may comprises a protein or a peptide. In some embodiments, the at least one protein is not of non-mammalian origin. In some embodiments, the at least one protein is human or derived from human. In some embodiments, the at least one protein is recombinant. In some embodiments, the at least one protein includes albumin, transferrin, insulin, fibronectin, aprotinin or fetuin. In some embodiments, the protein comprises one or more of albumin, insulin or transferrin, optionally one or more of a human or recombinant albumin, insulin or transferrin.

In some embodiments, the protein is an albumin or albumin substitute. In some embodiments, the albumin is a human derived albumin. In some embodiments, the albumin is a recombinant albumin. In some embodiments, the albumin is a natural human serum albumin. In some embodiments, the albumin is a recombinant human serum albumin. In some embodiments, the albumin is a recombinant albumin from a non-human source. Albumin substitutes may be any protein or polypeptide source. Examples of such protein or polypeptide samples include but are not limited to bovine pituitary extract, plant hydrolysate (e.g., rice hydrolysate), fetal calf albumin (fetuin), egg albumin, human serum albumin (HSA), or another animal-derived albumins, chick extract, bovine embryo extract, AlbuMAX^{®} I, and AlbuMAX^{®} II. In some embodiments, the protein or peptide comprises a transferrin. In some embodiments, the protein or peptide comprises a fibronectin. In some embodiments, the protein or peptide comprises aprotinin. In some embodiments, the protein comprises fetuin.

In some embodiments, the one or more additional protein is part of a serum replacement supplement that is added to the basal medium. Examples of serum replacement supplements include, for example, Immune Cell Serum Replacement (ThermoFisher, #A2598101) or those described in Smith et al. Clin Transl Immunology. 2015 Jan; 4(1): e31.

In certain embodiments, the cells are incubated after the introducing of the polynucleotide encoding the heterologous or recombinant protein, e.g., viral vector, for, for about, or for at least 18 hours, 24 hours, 30 hours, 36 hours, 40 hours, 48 hours, 54 hours, 60 hours, 72 hours, 84 hours, 96 hours, or more than 96 hours. In some embodiments, the incubating is performed for an amount of time between 30 minutes and 2 hours, between 1 hour and 8 hours, between 6 hours and 12 hours, between 12 hours and 18 hours, between 16 hours and 24 hours, between 18 hours and 30 hours, between 24 hours and 48 hours, between 24 hours and 72 hours, between 42 hours and 54 hours, between 60 hours and 120 hours between 96 hours and 120 hours, between 90 hours and between 1 days and 7 days, between 3 days and 8 days, between 1 day and 3 days, between 4 days and 6 days, or between 4 days and 5 days prior to the genetic engineering. In some embodiments, the incubating is for or for about between 18 hours and 30 hours. In particular embodiments, the incubating is for or for about 24 hours.

In certain embodiments, the total duration of the incubation is, is about, or is at least 12 hours, 18 hours, 24 hours, 30 hours, 36 hours, 42 hours, 48 hours, 54 hours, 60 hours, 72 hours, 84 hours, 96 hours, 108 hours, or 120 hours. In particular embodiments, the incubation is completed at, at about, or within 120 hours, 108 hours, 96 hours, 84 hours, 72 hours, 60 hours, 54 hours, 48 hours, 42 hours, 36 hours, 30 hours, 24 hours, 18 hours, or 12 hours. In some embodiments, the total duration of the incubation is between or between about 12 hour and 120 hours, 18 hour and 96 hours, 24 hours and 72 hours, or 24 hours and 48 hours, inclusive. In some embodiments, the total duration of the incubation is between or about between 1 hour and 48 hours, 4 hours and 36 hours, 8 hours and 30 hours or 12 hours and 24 hours, inclusive. In particular embodiments, the incubation is performed for or for about 24 hours, 48 hours, or 72 hours. In particular embodiments, the incubation is performed for 24 hours ± 6 hours, 48 hours ± 6 hours, or 72 hours ± 6 hours.

In particular embodiments, the incubation is initiated at, at about, or is at least 12 hours, 18 hours, 24 hours, 30 hours, 36 hours, 42 hours, 48 hours after the initiation of the stimulation. In particular embodiments, the incubation is initiated at, at about, or within 120 hours, 108 hours, 96 hours, 84 hours, 72 hours, 60 hours, 54 hours, 48 hours, 42 hours, 36 hours, 30 hours, 24 hours, 18 hours, or 12 hours of the initiation of the stimulation.

In some embodiments, the incubation is completed between or between about 24 hour and 120 hours, 36 hour and 108 hours, 48 hours and 96 hours, or 48 hours and 72 hours, inclusive, after the initiation of the stimulation. In some embodiments, the incubation is completed at, about, or within 120 hours, 108 hours, 96 hours, 72 hours, 48 hours, or 36 hours from the initiation of the stimulation. In particular embodiments, the incubation is completed after hours 24 hours ± 6 hours, 48 hours ± 6 hours, or 72 hours ± 6 hours after the initiation of the stimulation. In particular embodiments, the incubation is performed for or for about 72 hours or for or for about 3 days. In some embodiments, the incubation is performed for a duration sufficient to allow integration of the polynucleotide encoding the heterologous or recombinant protein into the genome of the cells. In particular embodiments, the incubation is initiated at, at about, or is at least 12 hours, 18 hours, 24 hours, 30 hours, 36 hours, 42 hours, 48 hours after the initiation of the stimulation. In particular embodiments, the incubation is initiated at, at about, or is at least 0.5 days, one day, 1.5 days, or 2 days after the initiation of the stimulation. In particular embodiments, the incubation is initiated at, at about, or within 120 hours, 108 hours, 96 hours, 84 hours, 72 hours, 60 hours, 54 hours, 48 hours, 42 hours, 36 hours, 30 hours, 24 hours, 18 hours, or 12 hours of the initiation of the stimulation. In particular embodiments, the incubation is initiated at, at about, or within 11 hours, 10 hours, 9 hours, 8 hours, 7 hours, 6 hours, 5 hours, or 4 hours of the initiation of the stimulation. In particular embodiments, the incubation is initiated at, at about, or within 5 days, 4 days, 3 days, 2 days, one day, or 0.5 days of the initiation of the stimulation.

In some embodiments, the incubation is completed between or between about 24 hour and 120 hours, 36 hour and 108 hours, 48 hours and 96 hours, or 48 hours and 72 hours, inclusive, after the initiation of the stimulation. In some embodiments, the incubation is completed at, about, or within 120 hours, 108 hours, 96 hours, 72 hours, 48 hours, or 36 hours from the initiation of the stimulation. In some embodiments, the incubation is completed at, about, or within 5 days, 4.5 days. 4 days,3 days, 2 dayrs, or 1.5 days from the initiation of the stimulation. In particular embodiments, the incubation is completed after hours 24 hours ± 6 hours, 48 hours ± 6 hours, or 72 hours ± 6 hours after the initiation of the stimulation. In some embodiments, the incubation is completed after or after about 72 hours or after or after about 3 days.

In some of any of the embodiments above, the engineered cells are not incubated under cultivating conditions to expand the cell population (e.g., viable T cell count). In some any of the above embodiments, the cells are not incubated under cultivating conditions that increase the amount of viable cells during the incubation or cultivation. For example, in some aspects, the cells are not incubation under conditions (e.g., cultivating conditions) that increase the amount of total viable cells at the end of the incubation as compared to the number of total viable cells at the beginning of the incubation. In some embodiments, the cells are incubated under conditions that may result in expansion, but the incubating conditions are not carried out for purposes of expanding the cell population. In some embodiments, cells that have been incubated under conditions that do not promote or facilitate expansion and proliferation may be referred to as non-expanded or minimally expanded.

In some embodiments, the transduced or engineered cells are incubated under cultivating conditions that promote proliferation and/or expansion subsequent to a step of genetically engineering, e.g., introducing a recombinant polypeptide to the cells by transduction or transfection. In particular embodiments, the cells are cultivated after the cells have been transduced or transfected with a recombinant polynucleotide, e.g., a polynucleotide encoding a recombinant receptor. In some embodiments, the cultivation produces one or more cultivated compositions of engineered T cells. In some embodiments, such cultivating conditions may be designed to induce proliferation, expansion, activation, and/or survival of cells in the population. In particular embodiments, the cultivating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to promote growth, division, and/or expansion of the cells. In some embodiments, cells that have been incubated under conditions that promote proliferation and/or expansion may be referred to as expanded cells.

In particular embodiments, the cells are incubated under cultivating conditions (e.g., cultivated) at a concentration of or of about 0.25×10⁶ cells/mL, 0.5×10⁶ cells/mL, 0.75×10⁶ cells/mL, 1.0×10⁶ cells/mL, 1.25×10⁶ cells/mL, 1.5×10⁶ cells/mL, 1.75×10⁶ cells/mL, or 2.0×10⁶ cells/mL. In particular embodiments, the cells are incubated under cultivating conditions at a concentration between or between about 0.25×10⁶ cells/mL to 1.0×10⁶ cell/mL. In particular embodiments, the cells are incubated under cultivating conditions at a concentration between or between about 0.25×10⁶ cells/mL to 0.75×10⁶ cell/mL. In particular embodiments, the cells are incubated under cultivating conditions at a concentration between or between about 0.5×10⁶ cells/mL to 0.75×10⁶ cell/mL. In particular embodiments, the cells are incubated under cultivating conditions at a concentration between or between about 0.25×10⁶ cells/mL to 0.5×10⁶ cell/mL. In particular embodiments, the cells are incubated under cultivating conditions at a concentration of or of about 0.75×10⁶ cells/mL. In particular embodiments, the cells are incubated under cultivating conditions at a concentration of or of about 0.5×10⁶ cells/mL.

In some embodiments, the engineered cells are cultivated (e.g., cultured) in a container that can be filled, e.g. via the feed port, with cell media and/or cells for culturing added cells. The cells can be from any cell source for which culture of the cells is desired, for example, for expansion and/or proliferation of the cells.

In some aspects, the culture media is an adapted culture medium that supports that growth, expansion or proliferation of the cells, such as T cells. In some aspects, the medium can be a liquid containing a mixture of salts, amino acids, vitamins, sugars or any combination thereof. In some embodiments, the culture media further contains one or more stimulating conditions or agents, such as to stimulate the expansion or proliferation of cells during the incubation. In some embodiments, the stimulating condition is or includes one or more cytokines, such as selected from IL-2, IL-7 or IL-15. In some embodiments, the cytokine is a recombinant cytokine. In particular embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines is or includes a member of the 4-alpha-helix bundle family of cytokines. In some embodiments, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more cytokines is or includes IL-15. In particular embodiments, the one or more cytokines is or includes IL-7. In particular embodiments, the one or more cytokines is or includes recombinant IL-2.

In some embodiments, the concentration of the one or more cytokine in the culture media during the cultivating, independently, is from or from about 1 IU/mL to 1500 IU/mL, such as from or from about 1 IU/mL to 100 IU/mL, 2 IU/mL to 50 IU/mL, 5 IU/mL to 10 IU/mL, 10 IU/mL to 500 IU/mL, 50 IU/mL to 250 IU/mL or 100 IU/mL to 200 IU/mL, 50 IU/mL to 1500 IU/mL, 100 IU/mL to 1000 IU/mL or 200 IU/mL to 600 IU/mL. In some embodiments, the concentration of the one or more cytokine, independently, is at least or at least about 1 IU/mL, 5 IU/mL, 10 IU/mL, 50 IU/mL, 100 IU/mL, 200 IU/mL, 500 IU/mL, 1000 IU/mL or 1500 IU/mL.

In some embodiments, the composition of engineered cells is cultivated at a temperature of 25 to 38°C, such as 30 to 37°C, for example at or about 37 °C ± 2 °C. In some embodiments, the cu;tivating condition is carried out for a time period until the culture, e.g. cultivation or expansion, results in a desired or threshold density, concentration, number or dose of cells. In some embodiments, the incubation is carried out for a time period until the culture, e.g. cultivation or expansion, results in a desired or threshold density, concentration, number or dose of viable cells. In some embodiments, the incubation is greater than or greater than about or is for about or 24 hours, 48 hours, 72 hours, 96 hours, 5 days, 6 days, 7 days, 8 days, 9 days or more.

In some embodiments, the cells are incubated or cultivated under conditions to maintain a target amount of carbon dioxide in the cell culture. In some aspects, this ensures optimal cultivation, expansion and proliferation of the cells during the growth. In some aspects, the amount of carbon dioxide (CO₂) is between 10% and 0% (v/v) of said gas, such as between 8% and 2% (v/v) of said gas, for example an amount of or about 5% (v/v) CO₂.

In particular embodiments, the cultivation is performed in a closed system. In certain embodiments, the cultivation is performed in a closed system under sterile conditions. In some embodiments the composition of engineered cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some embodiments, the bioreactor is used to perfuse and/or mix the cells during at least a portion of the cultivation step.

In some embodiments, cells cultivated while enclosed, connected, and/or under control of a bioreactor undergo expansion during the cultivation more rapidly than cells that are cultivated without a bioreactor, e.g., cells that are cultivated under static conditions such as without mixing, rocking, motion, and/or perfusion. In some embodiments, cells cultivated while enclosed, connected, and/or under control of a bioreactor reach or achieve a threshold expansion, cell count, and/or density within 14 days, 10 days, 9 days, 8 days, 7 days, 6 days, 5 days, 4 days, 3 days, 2 days, 60 hours, 48 hours, 36 hours, 24 hours, or 12 hours. In some embodiments, cells cultivated while enclosed, connected, and/or under control of a bioreactor reach or achieve a threshold expansion, cell count, and/or density at least 50%, at least 60%, at least 70%, at least 80%, at least 90%, at least 95%, at least 100%, at least 150%, at least 1-fold, at least 2-fold, at least 3-fold, at least 4-fold, at least 5-fold than cells cultivated in an exemplary and/or alternative process where cells are not cultivated while enclosed, connected, and/or under control of a bioreactor.

In some embodiments, the mixing is or includes rocking and/or motioning. In some embodiments, cells are incubated using containers, e.g., bags, which are used in connection with a bioreactor. In some cases, the bioreactor can be subject to motioning or rocking, which, in some aspects, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some embodiments, at least a portion of the incubation is carried out with rocking. The rocking speed and rocking angle may be adjusted to achieve a desired agitation. In some embodiments the rock angle is or is about 20°, 19°, 18°, 17°, 16°, 15°, 14°, 13°, 12°, 11°, 10°, 9°, 8°, 7°, 6°, 5°, 4°, 3°, 2° or 1°. In certain embodiments, the rock angle is between 6-16°. In other embodiments, the rock angle is between 7-16°. In other embodiments, the rock angle is between 8-12°. In some embodiments, the rock rate is 1, 2, 3, 4, 5, 6, 7, 8, 9, 10, 11, 1 12, 13, 14 15, 16, 17, 18, 19, 20, 21, 22, 23, 24, 25, 26, 27, 28, 29, 30, 31, 32, 33, 34, 35, 36, 37, 38, 39, 40 rpm. In some embodiments, the rock rate is between 4 and 12 rpm, such as between 4 and 6 rpm, inclusive. At least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

In some embodiments, a composition comprising cells, such as engineered cells, e.g. engineered T cells, engineered CD3+ T cells, engineered CD4+ T cells or engineered CD8+ T cells, is cultivated in the presence of a surfactant. In particular embodiments, cultivating the cells of the composition reduces the amount of shear stress that may occur during the cultivation, e.g., due to mixing, rocking, motion, and/or perfusion. In particular embodiments, the composition of cells, such as engineered cells, e.g. engineered T cells, engineered CD3+ T cells, engineered CD4+ T cells or engineered CD8+ T cells, is cultivated with the surfactant and at least 50%, at least 60%, at least 70%, at least 75%, at least 80%, at least 85%, at least 90%, at least 95%, at least 99%, or at least 99.9% of the T cells survive, e.g., are viable and/or do not undergo necrosis, programed cell death, or apoptosis, during or at least 1 day, 2 days, 3 days, 4 days, 5 days, 6 days, 7 days, or more than 7 days after the cultivation is complete. In particular embodiments, the composition of cells, such as engineered T cells, e.g. engineered CD3+ T cells, engineered CD4+ T cells or engineered CD8+ T cells, is cultivated in the presence of a surfactant and less than 50%, less than 40%, less than 30%, less than 25%, less than 20%, less than 15%, less than 10%, less than 5%, less than 1%, less than 0.1% or less than 0.01% of the cells undergo cell death, e.g., programmed cell death, apoptosis, and/or necrosis, such as due to shearing or shearing-induced stress.

In particular embodiments, a composition of cells, such as engineered T cells, e.g. engineered CD4+ T cells or engineered CD8+ T cells, is cultivated in the presence of between 0.1 µl/ml and 10.0 µl/ml, between 0.2 µl/ml and 2.5 µl/ml, between 0.5 µl/ml and 5 µl/ml, between 1 µl/ml and 3 µl/ml, or between 2 µl/ml and 4 µl/ml of the surfactant. In some embodiments, the composition of cells, such as engineered T cells, e.g. engineered CD4+ T cells or engineered CD8+ T cells, is cultivated in the presence of, of about, or at least 0.1 µl/ml, 0.2 µl/ml, 0.4 µl/ml, 0.6 µl/ml, 0.8 µl/ml, 1 µl/ml, 1.5 µl/ml, 2.0 µl/ml, 2.5 µl/ml, 5.0 µl/ml, 10 µl/ml, 25 µl/ml, or 50 µl/ml of the surfactant. In certain embodiments, the composition of cells is cultivated in the presence of or of about 2 µl/ml of the surfactant.

In some embodiments, a surfactant is or includes an agent that reduces the surface tension of liquids and/or solids. For example, a surfactant includes a fatty alcohol (e.g., steryl alcohol), a polyoxyethylene glycol octylphenol ether (e.g., Triton X-100), or a polyoxyethylene glycol sorbitan alkyl ester (e.g., polysorbate 20, 40, 60). In certain embodiments the surfactant is selected from the group consisting of Polysorbate 80 (PS80), polysorbate 20 (PS20), poloxamer 188 (P188). In an exemplary embodiment, the concentration of the surfactant in chemically defined feed media is about 0.0025% to about 0.25% (v/v) of PS80; about 0.0025% to about 0.25% (v/v) of PS20; or about 0.1% to about 5.0% (w/v) of P188.

In some embodiments, the surfactant is or includes an anionic surfactant, a cationic surfactant, a zwitterionic surfactant, or a nonionic surfactant added thereto. Suitable anionic surfactants include but are not limited to alkyl sulfonates, alkyl phosphates, alkyl phosphonates, potassium laurate, triethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inosine, phosphatidylinositol, diphosphatidylglycerol, phosphatidylserine, phosphatidic acid and their salts, sodium carboxymethylcellulose, cholic acid and other bile acids (e.g., cholic acid, deoxycholic acid, glycocholic acid, taurocholic acid, glycodeoxycholic acid) and salts thereof (e.g., sodium deoxycholate).

In some embodiments, suitable nonionic surfactants include: glyceryl esters, polyoxyethylene fatty alcohol ethers, polyoxyethylene sorbitan fatty acid esters (polysorbates), polyoxyethylene fatty acid esters, sorbitan esters, glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (poloxamers), poloxamines, methylcellulose, hydroxymethyl cellulose, hydroxypropyl cellulose, hydroxypropylmethyl cellulose, noncrystalline cellulose, polysaccharides including starch and starch derivatives such as hydroxyethylstarch (HES), polyvinyl alcohol, and polyvinylpyrrolidone. In certain embodiments, the nonionic surfactant is a polyoxyethylene and polyoxypropylene copolymer and preferably a block copolymer of propylene glycol and ethylene glycol. Such polymers are sold under the tradename POLOXAMER, also sometimes referred to as PLURONIC^{®} F68 or Kolliphor^{®} P188. Among polyoxyethylene fatty acid esters is included those having short alkyl chains. One example of such a surfactant is SOLUTOL^{®} HS 15, polyethylene-660-hydroxystearate.

In some embodiments, suitable cationic surfactants may include, but are not limited to, natural phospholipids, synthetic phospholipids, quaternary ammonium compounds, benzalkonium chloride, cetyltrimethyl ammonium bromide, chitosans, lauryl dimethyl benzyl ammonium chloride, acyl carnitine hydrochlorides, dimethyl dioctadecyl ammomium bromide (DDAB), dioleyoltrimethyl ammonium propane (DOTAP), dimyristoyl trimethyl ammonium propane (DMTAP), dimethyl amino ethane carbamoyl cholesterol (DC-Chol), 1,2-diacylglycero-3-(O-alkyl) phosphocholine, O-alkylphosphatidylcholine, alkyl pyridinium halides, or long-chain alkyl amines such as, for example, n-octylamine and oleylamine.

Zwitterionic surfactants are electrically neutral but possess local positive and negative charges within the same molecule. Suitable zwitterionic surfactants include but are not limited to zwitterionic phospholipids. Suitable phospholipids include phosphatidylcholine, phosphatidylethanolamine, diacyl-glycero-phosphoethanolamine (such as dimyristoyl-glycero-phosphoethanolamine (DMPE), dipalmitoyl-glycero-phosphoethanolamine (DPPE), distearoyl-glycero-phosphoethanolamine (DSPE), and dioleolyl-glycero-phosphoethanolamine (DOPE)). Mixtures of phospholipids that include anionic and zwitterionic phospholipids may be employed in this invention. Such mixtures include but are not limited to lysophospholipids, egg or soybean phospholipid or any combination thereof. The phospholipid, whether anionic, zwitterionic or a mixture of phospholipids, may be salted or desalted, hydrogenated or partially hydrogenated or natural semi-synthetic or synthetic.

In certain embodiments, the surfactant is poloxamer, e.g., poloxamer 188. In some embodiments, a composition of cells is cultivated in the presence of between 0.1 µl/ml and 10.0 µl/ml, between 0.2 µl/ml and 2.5 µl/ml, between 0.5 µl/ml and 5 µl/ml, between 1 µl/ml and 3 µl/ml, or between 2 µl/ml and 4 µl/ml of poloxamer. In some embodiments, the composition of cells is cultivated in the presence of, of about, or at least 0.1 µl/ml, 0.2 µl/ml, 0.4 µl/ml, 0.6 µl/ml, 0.8 µl/ml, 1 µl/ml, 1.5 µl/ml, 2.0 µl/ml, 2.5 µl/ml, 5.0 µl/ml, 10 µl/ml, 25 µl/ml, or 50 µl/ml of the surfactant. In certain embodiments, the composition of cells is cultivated in the presence of or of about 2 µl/ml of poloxamer.

In some aspects, engineered T cells populations (e.g., CD4, CD8) may be expanded separately or expanded together until they each reach a threshold amount or cell density. In particular embodiments, the cultivation ends, such as by harvesting cells, when cells achieve a threshold amount, concentration, and/or expansion. In particular embodiments, the cultivation ends when the cell achieve or achieve about or at least a 1.5-fold expansion, a 2-fold expansion, a 2.5-fold expansion, a 3-fold expansion, a 3.5-fold expansion, a 4-fold expansion, a 4.5-fold expansion, a 5-fold expansion, a 6-fold expansion, a 7-fold expansion, a 8-fold expansion, a 9-fold expansion, a 10-fold expansion, or greater than a 10-fold expansion, e.g., with respect and/or in relation to the amount of density of the cells at the start or initiation of the cultivation. In some embodiments, the threshold expansion is a 4-fold expansion, e.g., with respect and/or in relation to the amount of density of the cells at the start or initiation of the cultivation. In some embodiments, the cultivation ends, such as by harvesting cells, when the cells achieve a threshold total amount of cells, e.g., threshold cell count. In some embodiments, the cultivation ends when the cells achieve a threshold total nucleated cell (TNC) count. In some embodiments, the cultivation ends when the cells achieve a threshold viable amount of cells, e.g., threshold viable cell count. In some embodiments, the threshold cell count is or is about or is at least of 50 x10⁶ cells, 100 x10⁶ cells, 200 x10⁶ cells, 300 x10⁶ cells, 400 x10⁶ cells, 600 x10⁶ cells, 800 x10⁶ cells, 1000 x10⁶ cells, 1200 x10⁶ cells, 1400 x10⁶ cells, 1600 x10⁶ cells, 1800 x10⁶ cells, 2000 x10⁶ cells, 2500 x10⁶ cells, 3000 x10⁶ cells, 4000 x10⁶ cells, 5000 x10⁶ cells, 10,000 x10⁶ cells, 12,000 x10⁶ cells, 15,000 x10⁶ cells or 20,000 x10⁶ cells, or any of the foregoing threshold of viable cells.

In particular embodiments, the cultivation ends when the cells achieve a threshold cell count. In some embodiments, the cultivation ends at, at about, or within 6 hours, 12 hours, 24 hours, 36 hours, 1 day, 2 days, 3 days, 4 days, 5 days, 6 days, or 7 or more days, after the threshold cell count is achieved. In particular embodiments, the cultivation is ended at or about 1 day after the threshold cell count is achieved. In certain embodiments, the threshold density is, is about, or is at least 0.1 x10⁶ cells/ml, 0.5 x10⁶ cells/ml, 1 x10⁶ cells/ml, 1.2 x10⁶ cells/ml, 1.5 x10⁶ cells/ml, 1.6 x10⁶ cells/ml, 1.8 x10⁶ cells/ml, 2.0 x10⁶ cells/ml, 2.5 x10⁶ cells/ml, 3.0 x10⁶ cells/ml, 3.5 x10⁶ cells/ml, 4.0 x10⁶ cells/ml, 4.5 x10⁶ cells/ml, 5.0 x10⁶ cells/ml, 6 x10⁶ cells/ml, 8 x10⁶ cells/ml, or 10 x10⁶ cells/ml, or any of the foregoing threshold of viable cells. In particular embodiments, the cultivation ends when the cells achieve a threshold density. In some embodiments, the cultivation ends at, at about, or within 6 hours, 12 hours, 24 hours, 36 hours, 1 day, 2 days, 3 days, 4 days, 5 days, 6 days, or 7 or more days, after the threshold density is achieved. In particular embodiments, the cultivation is ended at or about 1 day after the threshold density is achieved.

In some embodiments, at least a portion of the cultivation is carried out under static conditions. In some embodiments, at least a portion of the cultivation is carried out with perfusion, such as to perfuse out spent media and perfuse in fresh media during the culture. In some embodiments, the method includes a step of perfusing fresh culture medium into the cell culture, such as through a feed port. In some embodiments, the culture media added during perfusion contains the one or more stimulating agents, e.g. one or more recombinant cytokine, such as IL-2, IL-7 and/or IL-15. In some embodiments, the culture media added during perfusion is the same culture media used during a static incubation.

In some embodiments, subsequent to the incubation, the container, e.g., bag, is re-connected to a system for carrying out the one or more other processing steps of for manufacturing, generating or producing the cell therapy, such as is re-connected to the system containing the centrifugal chamber. In some aspects, cultured cells are transferred from the bag to the internal cavity of the chamber for formulation of the cultured cells.

In some embodiments, the cells are monitored during the incubation step, e.g., under expanded (e.g., cultivation) or minimally expanded/non-expanded (e.g., incubation). Monitoring may be performed, for example, to ascertain (e.g., measure, quantify) cell morphology, cell phenotype, cell viability, cell death, and/or cell concentration (*e.g*., viable cell concentration). In some embodiments, the monitoring is performed manually, such as by a human operator. In some embodiments, the monitoring is performed by an automated system. The automated system may require minimal or no manual input to monitor the cultivated cells. In some embodiments, the monitoring is performed both manually and by an automated system.

### G. Harvesting and Collecting Cells (not part of the claimed invention)

In some embodiments, the cells are harvested or collected, which is not in accordance with the claimed invention,. In particular embodiments, the cells are collected or harvested after the completion of the incubation as described in Section II-F. In certain embodiments, the collected or harvested cells are the cells of an output population. In some embodiments, the output population includes cells that are viable, CD3+, CD4+, CD8+, and/or positive for a recombinant receptor, e.g., CAR+. In particular embodiments, the harvested CD4+ T cells and formulated CD8+ T cells are the output CD4+ and CD8+ T cells. In particular embodiments, a formulated cell population, e.g., a formulated population of enriched CD4+ and CD8+ cells, is an output cell population, e.g., an output population of enriched CD4+ and CD8+ cells.

In some embodiments, the cells or cell population that is harvested, collected, or formulated have not undergone any expansion, e.g., any conditions where the cells were incubated or cultivated under conditions that increase the amount of viable cells during the incubation or cultivation. For example, in some aspects, the cells that are harvested have not undergone any incubation or cultivation where the amount of total viable cells is increased at the end of the incubation or cultivation as compared to the number of total viable cells at the beginning of the incubation or cultivation. In some embodiments, the collected, harvested, or formulated cells have not previously undergone an incubation or cultivation that was performed in a bioreactor, or under conditions where the cells were rocked, rotated, shaken, or perfused for all or a portion of the incubation or cultivation.

In some embodiments, a cell selection, isolation, separation, enrichment, and/or purification step is performed before the cells or cell population is harvested, collected, or formulated. In some embodiments, the cell selection, isolation, separation, enrichment, and/or purification step is carried out using chromatography as disclosed herein. In some embodiments, a T cell selection step by chromatography is performed after T cell transduction, but prior to harvesting, prior to collecting, and/or prior to formulating the cells. In some embodiments, a T cell selection step by chromatography is performed immediately prior to harvesting the cells.

In certain embodiments, the amount of time from the initiation of the stimulation (e.g., on-column stimulation) to collecting, harvesting, or formulating the cells is, is about, or is less than 24 hours, 36 hours, 42 hours, 48 hours, 54 hours, 60 hours, 72 hours, 84 hours, 96 hours, 108 hours, or 120 hours. In some embodiments, the amount of time from the initiation of the stimulation to collecting, harvesting, or formulating the cells for generating engineered cells, from the initiation of the stimulation to collecting, harvesting, or formulating the cells is between or between about 12 hours and 24 hours, 36 hours and 120 hours, 48 hours and 96 hours, or 48 hours and 72 hours, inclusive. In particular embodiments, the amount of time from the initiation of incubation to harvesting, collecting, or formulating the cells is, is about, or is less than 48 hours, 72 hours, or 96 hours. In particular embodiments, the amount of time from the initiation of incubation to harvesting, collecting, or formulating the cells is 48 hours ± 6 hours, 72 hours ± 6 hours, or 96 hours ± 6 hours.

In certain embodiments, one or more populations of enriched T cells are formulated. In particular embodiments, one or more populations of enriched T cells are formulated after the one or more populations have been engineered and/or cultivated. In particular embodiments, the one or more populations are input populations or output compositions. In some embodiments, the one or more input populations or output compositions have been previously cryoprotected and stored, and are thawed prior to the incubation (e.g., incubation as described in Section I-F).

In certain embodiments, the cells are harvested prior to, prior to about, or prior to at least one, two, three, four, five, six, eight, ten, twenty, or more cell doublings of the cell population, e.g., doublings that occur during the incubating.

In particular embodiments, the cells are harvested or collected at a time before the total number of cells, e.g., total number of incubated cells or cells undergoing the incubation (e.g., incubation as described in Section II-F), is greater than or than about one, two, three, four, five, six, eight, ten, twenty, or more than twenty times the number of cells of the input population, e.g., the total number of cells that were contacted with the stimulatory reagent. In some embodiments, the cells are harvested or collected at a time before the total number of incubated cells is greater than or than about one, two, three, four, five, six, eight, ten, twenty, or more than twenty times the total number of cells that were transformed, transduced, or spinoculated, e.g., the total number of cells that were contacted with a viral vector. In certain embodiments, the cells are T cells, viable T cells, CD3+ T cells, CD4+ T cells, CD8+ T cells, CAR expressing T cells, or a combination of any of the foregoing. In particular embodiments, the cells are harvested or collected at a time before the total number of cells is greater than the total number of cells of the input population. In various embodiments, the cells are harvested or collected at a time before the total number of viable CD3+ T cells is greater than the total number of viable CD3+ cells of the input population. In particular embodiments, the cells are harvested or collected at a time before the total number of cells is greater than the total number of cells of the transformed, transduced, or spinoculated cells. In various embodiments, the cells are harvested or collected at a time before the total number of viable CD3+ T cells is greater than the total number of viable CD3+ of the transformed, transduced, or spinoculated cells.

In certain embodiments, the formulated cells are output cells. In some embodiments, a formulated population of enriched T cells is an output population of enriched T cells. In particular embodiments, the formulated CD4+ T cells and formulated CD8+ T cells are the output CD4+ and CD8+ T cells. In particular embodiments, a formulated cell population, e.g., a formulated population of enriched CD4+ and CD8+ cells, is an output cell population, e.g., an output population of enriched CD4+ and CD8+ cells.

In some embodiments, cells can be formulated into a container, such as a bag or vial.

In some embodiments, the cells are formulated in a pharmaceutically acceptable buffer, which may, in some aspects, include a pharmaceutically acceptable carrier or excipient, which is not in accordance with the claimed invention,. In some embodiments, the processing includes exchange of a medium into a medium or formulation buffer that is pharmaceutically acceptable or desired for administration to a subject. In some embodiments, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some embodiments contains the cells in amounts effective to treat or prevent the disease or condition, such as a therapeutically effective or prophylactically effective amount.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, *e.g.,* by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, *e.g*., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

Compositions in some embodiments are provided as sterile liquid preparations, e.g., isotonic aqueous solutions, suspensions, emulsions, dispersions, or viscous compositions, which may in some aspects be buffered to a selected pH. Liquid compositions can comprise carriers, which can be a solvent or dispersing medium containing, for example, water, saline, phosphate buffered saline, polyol (for example, glycerol, propylene glycol, liquid polyethylene glycol) and suitable mixtures thereof. Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (*e.g*., methylcellulose), pH buffering agents, gelling or viscosity enhancing additives, preservatives, flavoring agents, and/or colors, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

In some embodiments, the formulation buffer contains a cryopreservative. In some embodiments, the cell are formulated with a cyropreservative solution that contains 1.0% to 30% DMSO solution, such as a 5% to 20% DMSO solution or a 5% to 10% DMSO solution. In some embodiments, the cryopreservation solution is or contains, for example, PBS containing 20% DMSO and 8% human serum albumin (HSA), or other suitable cell freezing media. In some embodiments, the cryopreservative solution is or contains, for example, at least or about 7.5% DMSO. In some embodiments, the processing steps can involve washing the transduced and/or expanded cells to replace the cells in a cryopreservative solution. In some embodiments, the cells are frozen, e.g., cryoprotected or cryopreserved, in media and/or solution with a final concentration of or of about 12.5%, 12.0%, 11.5%, 11.0%, 10.5%, 10.0%, 9.5%, 9. 0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular embodiments, the cells are frozen, e.g., cryoprotected or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and -5%, between 0.25% and 4%, between 0.5% and 2%, or between 1% and 2% HSA.

In particular embodiments, the composition of enriched T cells, e.g., T cells that have been stimulated, engineered, and/or cultivated, are formulated, cryoprotected, and then stored for an amount of time. In certain embodiments, the formulated, cryoprotected cells are stored until the cells are released for infusion. In particular embodiments, the formulated cryoprotected cells are stored for between 1 day and 6 months, between 1 month and 3 months, between 1 day and 14 days, between 1 day and 7 days, between 3 days and 6 days, between 6 months and 12 months, or longer than 12 months. In some embodiments, the cells are cryoprotected and stored for, for about, or for less than 1 days, 2 days, 3 days, 4 days, 5 days, 6 days, or 7 days. In certain embodiments, the cells are thawed and administered to a subject after the storage. In certain embodiments, the cells are stored for or for about 5 days.

In some embodiments, the formulation is carried out using one or more processing step including washing, diluting or concentrating the cells, such as the cultured or expanded cells. In some embodiments, the processing can include dilution or concentration of the cells to a desired concentration or number, such as unit dose form compositions including the number of cells for administration in a given dose or fraction thereof. In some embodiments, the processing steps can include a volume-reduction to thereby increase the concentration of cells as desired. In some embodiments, the processing steps can include a volume-addition to thereby decrease the concentration of cells as desired. In some embodiments, the processing includes adding a volume of a formulation buffer to transduced and/or expanded cells. In some embodiments, the volume of formulation buffer is from or from about 10 mL to 1000 mL, such as at least or about at least or about or 50 mL, 100 mL, 200 mL, 300 mL, 400 mL, 500 mL, 600 mL, 700 mL, 800 mL, 900 mL or 1000 mL.

In some embodiments, such processing steps for formulating a cell composition are carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax^{®} or Sepax 2^{®} cell processing systems. An exemplary system and process is described in International Publication Number WO2016/073602. In some embodiments, the method includes effecting expression from the internal cavity of the centrifugal chamber a formulated composition, which is the resulting composition of cells formulated in a formulation buffer, such as pharmaceutically acceptable buffer, in any of the above embodiments as described. In some embodiments, the expression of the formulated composition is to a container, such as a bag that is operably linked as part of a closed system with the centrifugal chamber. In some embodiments, the container, such as bag, is connected to a system at an output line or output position.

In some embodiments, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some aspects, a desired number or plurality of output containers, e.g., bags, can be sterilely connected to one or more, generally two or more, such as at least 3, 4, 5, 6, 7, 8 or more of the ports of the multi-port output. For example, in some embodiments, one or more containers, e.g., bags can be attached to the ports, or to fewer than all of the ports. Thus, in some embodiments, the system can effect expression of the output composition into a plurality of output bags.

In some aspects, cells can be expressed to the one or more of the plurality of output bags in an amount for dosage administration, such as for a single unit dosage administration or multiple dosage administration. For example, in some embodiments, the output bags may each contain the number of cells for administration in a given dose or fraction thereof. Thus, each bag, in some aspects, may contain a single unit dose for administration or may contain a fraction of a desired dose such that more than one of the plurality of output bags, such as two of the output bags, or 3 of the output bags, together constitute a dose for administration.

Thus, the containers, e.g., output bags, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subject.

In some embodiments, each of the containers, e.g., bags, individually comprises a unit dose of the cells. Thus in some embodiments, each of the containers comprises the same or approximately or substantially the same number of cells. In some embodiments, each unit dose contains at least or about at least 1 x 10⁶, 2 x 10⁶, 5 x 10⁶, 1 x 10⁷, 5 x 10⁷, or 1 x 10⁸ engineered cells, total cells, T cells, or PBMCs. In some embodiments, the volume of the formulated cell composition in each bag is 10 mL to 100 mL, such as at least or about at least 20 mL, 30 mL, 40 mL, 50 mL, 60 mL, 70 mL, 80 mL, 90 mL or 100 mL.

In some embodiments, such cells produced by the method, or a composition comprising such cells, are administered to a subject for treating a disease or condition.

### H. Removal of Stimulatory Reagents (not part of the claimed invention)

In some embodiments, the stimulatory reagent (e.g., oligomeric stimulatory reagent) is removed or separated from the collected cells or cell populations after collecting, harvesting, or formulating the cells, which is not in accordance with the claimed invention,. In some embodiments, the stimulatory reagents are removed or separated from the cells or cell populations after collection from the chromatography column, e.g., after the step of elution and cell collection as described in Section II-E. In some embodiments, the stimulatory reagents are removed or separated from the cells or cell populations after or during the incubation, e.g., an incubation described herein such as in Section II-F. In certain embodiments, the cells or cell population undergoes a process, procedure, step, or technique to remove the stimulatory reagent (e.g., oligomeric stimulatory reagent) after the incubation but prior to steps for collecting, harvesting, or formulating the cells. In particular embodiments, the cells or cell population undergoes a process, procedure, step, or technique to remove the stimulatory reagent (e.g., oligomeric stimulatory reagent) after the incubation. In some aspects, when stimulatory reagent (e.g., oligomeric stimulatory reagent) is separated or removed from the cells during the incubation, the cells are returned to the same incubation conditions as prior to the separation or removal for the remaining duration of the incubation.

In certain embodiments, the stimulatory reagent (e.g., oligomeric stimulatory reagent) is removed and/or separated from the cells. Without wishing to be bound by theory, particular embodiments contemplate that the binding and/or association between a stimulatory reagent (e.g., oligomeric stimulatory reagent) and cells may, in some circumstances, be reduced over time during the incubation. In certain embodiments, one or more agents may be added to reduce the binding and/or association between the stimulatory reagent and the cells. In particular embodiments, a change in cell culture conditions, e.g., the addition of an agent (e.g., a substance such as a competition agent or free binding agent), may reduce the binding and/or association between the stimulatory reagent and the cells. Thus, in some embodiments, the stimulatory reagent (e.g., oligomeric stimulatory reagent) may be removed from an incubation, cell culture system, and/or a solution separately from the cells, e.g., without removing the cells from the incubation, cell culture system, and/or a solution as well.

In certain embodiments, the stimulatory reagent (e.g., oligomeric stimulatory reagent) is separated and/or removed from the cells after an amount of time. In particular embodiments, the amount of time is an amount of time from the initiation of the stimulation. In particular embodiments the start of the incubation is considered at or at about the time the cells are contacted with the stimulatory reagent and/or a media or solution containing the stimulatory reagent. In particular embodiments, the stimulatory reagent is removed or separated from the cells within or within about 120 hours, 108 hours, 96 hours, 84 hours, 72 hours, 60 hours, 48 hours, 36 hours, 24 hours, 12 hours, 6, hours, 5 hours, 4 hours, 3 hours, or 2 hours, inclusive, of the initiation of the stimulation. In particular embodiments, the stimulatory reagent (e.g., oligomeric stimulatory reagent) is removed or separated from the cells at or at about 48 hours after the stimulation is initiated. In certain embodiments, the stimulatory reagent is removed or separated from the cells at or at about 72 hours after the stimulation is initiated. In some embodiments, the stimulatory reagent is removed or separated from the cells at or at about 96 hours after the stimulation is initiated.

### 1. Removal of Oligomeric reagents (not part of the claimed invention)

In some embodiments, the population of stimulated cells (i.e., cells having undergone selection with column chromatography and on-column stimulation as described herein) which was produced or generated in accord with any of the methods provided herein, are treated to remove the oligomeric stimulatory reagent and/or reduce the ability of the oligomeric stimulatory reagent to deliver a signal in the cells, which is not in accordance with the claimed invention,. For instance, the reversibility of the one or more stimulatory agents bound to the reagent via the streptavidin-binding peptide of the one or more agents to the streptactin mutein of the reagent can be carried out by use of a competition agent or free binding partner to disrupt the interaction. As a result, the one or more stimulatory agents that had been multimerized on the reagent is released from the reagent backbone and their ability to deliver a stimulatory signal is reduced or terminated.

In certain embodiments, the one or more stimulatory agents (e.g., agents that stimulate or activate a TCR and/or a costimulatory molecule) associate with, such as are reversibly bound to, the oligomeric reagent, such as via the plurality of the particular binding sites (e.g., binding sites Z) present on the oligomeric reagent. In some cases, this results in the stimulatory agents being closely arranged to each other such that an avidity effect can take place if a target cell having (at least two copies of) a cell surface molecule that is bound by or recognized by the stimulatory agent is brought into contact with the agent. In some aspects, the stimulatory agent has a low affinity towards the molecule of the cell at binding site B, such that the receptor binding reagent dissociates from the cell in the presence of the competition reagent. Thus, in some embodiments, the stimulatory agents are removed from the cells in the presence of the competition reagent.

In some embodiments, the oligomeric stimulatory reagent is a streptavidin mutein oligomer with reversibly attached anti-CD3 and anti-CD28 Fabs. In some embodiments, the Fabs are attached contain streptavidin binding domains, e.g., that allow for the reversible attachment to the streptavidin mutein oligomer. In some cases, anti-CD3 and anti-CD28 Fabs are closely arranged to each other such that an avidity effect can take place if a T cell expressing CD3 and/or CD28 is brought into contact with the oligomeric stimulatory reagent with the reversibly attached Fabs. In some aspects, the Fabs have a low affinity towards CD3 and CD28, such that the Fabs dissociate from the cell in the presence of the competition reagent, e.g., biotin or a biotin variant or analogue. Thus, in some embodiments, the Fabs are removed or dissociated from the cells in the presence of the competition reagent, e.g., D-biotin.

In some embodiments, the population of stimulated cells (i.e., cells having undergone selection with column chromatography and on-column stimulation as described herein) is provided or added a substance, such as a competition agent or free binding agent, such as to lessen and/or terminate, the signaling of the stimulatory agent or agents. In some embodiments, the addition of the competition agent or free binding agent is carried out following an elution step as described herein (*see* Section II-E). In some embodiments, the addition of the competition agent or free binding agent is carried out following a genetic engineering step as described herein. In some embodiments, the addition of the competition agent or free binding agent is carried out following a harvesting step as described herein.

Thus, in some embodiments, the population of the stimulated cells contains the presence of a substance, such as a competition agent, e.g. biotin or a biotin analog, e.g. D-Biotin. In some embodiments, the substance, such as a competition agent, e.g. biotin or a biotin analog, e.g. D-Biotin, is present in an amount that is at least 1.5-fold greater, at least 2-fold, at least 3-fold, at least 4-fold, at least 5-fold, at least 10-fold, at least 100-fold, at least 1000-fold or more greater than the amount of the substance in a reference population or preparation of cultured cells (e.g., T cells) in which the substance was not added exogenously during one of the aforementioned steps. In some embodiments, the amount of the substance, such as a competition agent, e.g. biotin or a biotin analog, e.g. D-Biotin, in the population of stimulated cells is from or from about 10 µM to 100 µM, 100 µM to 1 mM, 100 µM to 500 µM or 10 µM to 100 µM. In some embodiments, 10 µM or about 10 µM of biotin or a biotin analog, e.g., D-biotin, is added to the cells or the cell population to separate or remove the oligomeric stimulatory reagent from the cells or cell population.

In some embodiments, the oligomeric stimulatory reagent, e.g., the oligomeric stimulatory streptavidin mutein reagent, is removed or separated from the cells or cell populations prior to harvesting or formulating the cells. In some embodiments, oligomeric stimulatory reagent, e.g., the oligomeric stimulatory streptavidin mutein reagent, is removed or separated from the cells or cell populations by contact or exposure to a competition reagent, e.g., biotin or a biotin analog such as D-biotin, after or during the incubation, e.g., an incubation described herein such as in Section II-D. In certain embodiments, the cells or cell population are contacted or exposed to a competition reagent, e.g., biotin or a biotin analog such as D-biotin, to remove the oligomeric stimulatory reagent, e.g., the stimulatory oligomeric streptavidin mutein reagent, after the incubation but prior to steps for genetically engineering, harvesting, or formulating the cells. In particular embodiments, the cells or cell population are contacted or exposed to a competition reagent, e.g., biotin or a biotin analog such as D-biotin, to remove the oligomeric stimulatory reagent, e.g., the oligomeric stimulatory streptavidin mutein reagent, after the incubation. In some aspects, when oligomeric stimulatory reagent, e.g., the oligomeric stimulatory streptavidin mutein reagent, is separated or removed from the cells during the incubation, e.g., by contact or exposure to a competition reagent, e.g., biotin or a biotin analog such as D-biotin, the cells are returned to the same incubation conditions as prior to the separation or removal for the remaining duration of the incubation.

In some embodiments, the cells are contacted with, with about, or with at least 0.01 µM, 0.05 µM, 0. 1 µM, 0.5 µM, 1 µM, 2 µM, 3 µM, 4 µM, 5 µM, 10 µM, 100 µM, 500 µM, 0.01 µM, 1 mM, or 10 mM of the competition reagent to remove or separate the oligomeric stimulatory reagent from the cells. In various embodiments, the cells are contacted with, with about, or with at least 0.01 µM, 0.05 µM, 0. 1 µM, 0.5 µM, 1 µM, 2 µM, 3 µM, 4 µM, 5 µM, 10 µM, 100 µM, 500 µM, 0.01 µM, 1 mM, or 10 mM of biotin or a biotin analog such as D-biotin, to remove or separate the stimulatory streptavidin mutein oligomers with reversibly attached anti-CD3 and anti-CD28 Fabs from the cells.

In particular embodiments, the oligomeric stimulatory reagent, e.g., the oligomeric stimulatory streptavidin mutein reagent, is removed or separated from the cells within or within about 120 hours, 108 hours, 96 hours, 84 hours, 72 hours, 60 hours, 48 hours, 36 hours, 24 hours, or 12 hours, inclusive, of the initiation of the stimulation. In particular embodiments, the oligomeric stimulatory reagent, e.g., the oligomeric stimulatory streptavidin mutein reagent, is removed or separated from the cells at or at about 48 hours after the stimulation is initiated. In certain embodiments, the oligomeric stimulatory reagent, e.g., the oligomeric stimulatory streptavidin mutein reagent, is removed or separated from the cells at or at about 72 hours after the stimulation is initiated. In some embodiments, the oligomeric stimulatory reagent, e.g., the oligomeric stimulatory streptavidin mutein reagent is removed or separated from the cells at or at about 96 hours after the stimulation is initiated.

In some embodiments, the cells can be washed, e.g. with cell media, to remove or dilute the one or more stimulatory agents (e.g. anti-CD3/anti-CD28 Fab), reagent (e.g. streptactin mutein) and/or competition agent from the cell composition.

### I. Sequential Selection and Polishing (not part of the claimed invention)

The methods, which are not in accordance with the claimed invention, provided herein allow for multiple selection steps, for example by column chromatography, to isolate and/or enrich a target cell population (e.g., T cells, CD3+, CD4+, CD8+ T cells). In some embodiments, one or more selection steps are carried out at one or more time points or following certain steps of the process for creating an output therapeutic cell composition, for example a process as described by sections above. In some embodiments, selection steps that occur following initial cell selection, for example as described in Section I-C, are referred to as polishing steps. Polishing steps may be performed for a variety of purposes, including, but not limited to, further purification of the cell composition, selection of specific cell subtypes (e.g., CD28+, CD62L+, CCR7+, CD27+, CD127+, CD4+, CD8+, CD45RA+, and/or CD45RO+ T cells), removal of dead cells (e.g., selection of viable cells), selection of successfully engineered cells (e.g., cells expressing a transgene (e.g., chimeric antigen receptor (CAR), T cell receptor (TCR), etc.), or for adjusting the ratio, total number, or concentration of specific cell types (e.g., CD4+ to CD8+ cells, CAR+ or TCR+ cells to CAR-or TCR- cells, or total number or concentration of CD4+, CD8+, CAR+ , TCR+, and/or viable cells). In some embodiments, a selection step (e.g., polishing step) is useful for increasing product control and/or decreasing between patient variance.

In some embodiments, a selection step (e.g., an initial selection step and/or a polishing step) includes multiple selection steps for, for example, further purifying the cell composition, selection of specific cell subtypes, selection of viable cells, selection of engineered cells, and/or adjusting the ratio, total number, or concentration of cells.

The methods, which are not in accordance with the claimed invention, provided herein allow for multiple selection steps (e.g. initial selection and/or polishing steps), for example by column chromatography, to isolate and/or enrich a target cell population (e.g., T cells, CD3+, CD4+, CD8+ T cells). In some aspects, such methods, which are not in accordance with the claimed invention, are achieved by a single process stream, such as in a closed system, by employing sequential selections in which a plurality of different cell populations from a sample, as provided herein, are enriched and/or isolated. In some aspects, carrying out the separation or isolation in the same vessel or set of vessels, e.g., tubing set, is achieved by carrying out sequential positive and negative selection steps, the subsequent step subjecting the negative and/or positive fraction from the previous step to further selection, where the entire process is carried out in the same tube or tubing set. In one embodiment, a sample containing target cells (e.g. composition of stimulated and/or engineered, such as transduced cells) is subjected to a sequential selection in which a first selection is effected to enrich for one of the CD4+ or CD8+ populations, and the non-selected cells from the first selection are used as the source of cells for a second selection to enrich for the other of the CD4+ or CD8+ populations. In some embodiments, a further selection or selections can be effected to enrich for sub-populations of one or both of the CD4+ or CD8+ population, for example, central memory T (T_{CM}) cells or naive T cells. In one embodiment, a sample containing target cells (e.g. composition of stimulated and/or engineered, such as transduced cells) is subjected to a sequential selection in which a first selection is effected to enrich for a CD3+ population. In some embodiments, a further selection or selections can be effected to enrich for sub-populations of the CD3+ population, for example, CD4+ cells. In some embodiments, a further selection or selections can be effected to enrich for sub-populations of the CD3+ population, for example, CD8+ cells. In some embodiments, specific subpopulations of T cells (e.g., CD3+, CD4+, CD8+ cells), such as cells positive or expressing high levels of one or more surface markers, e.g., CD28+, CD62L+, CCR7+, CD27+, CD127+, CD4+, CD8+, CD45RA+, and/or CD45RO+ T cells, are selected by positive or negative selection techniques during a selection step (e.g., an initial selection step and/or a polishing step). In some embodiments, a cell population (e.g., composition of stimulated and/or engineered cells, such as transduced cells) containing target cells is subjected to a sequential selection in which the polishing step selects for viable cells. In some embodiments, selecting viable cells includes or consists of removing dead cells from the cell population (e.g., output composition of stimulated and/or engineered cells or subpopulations thereof). In some embodiments, the polishing step allows for controlling or adjusting the ratio or total number of cells in the cell composition.

In some embodiments, a first selection step can be carried out using beads labeled with selection agents as described herein, and the positive and negative fractions from the first selection step can be retained, followed by further positive selection of the positive fraction to enrich for a second selection marker, such as by using beads labeled with a second selection agent or by subjecting the positive fraction to column chromatography as described above.

The methods, which are not in accordance with the claimed invention, provided herein further allow for the selection and enrichment of successfully stimulated and engineered or transduced cells. For example, in some embodiments, the sequential selection, parallel selection, or single selection procedures described above may be used to identify or enrich cells engineered (e.g. transduced) with a recombinant receptor (e.g., CARs, TCRs). Selection agents for selecting or enriching engineered cells (e.g. CAR or TCR engineered cells) including any selection agent able to bind to a surrogate marker of the enginnered cells or to the recombinant receptor. In some embodiments, nucleic acids encoding recombinant receptors that are introduced into cells are generated to co-express a surrogate marker that will be co-expressed on the engineered cell with the recombinant receptor. In some embodiments, the surrogate marker is a truncated receptor, such as described herein. In particular embodiments, the truncated receptor is truncated EGFR (EGFRt). In some embodiments, the selection agent for selecting or enriching engineered cells (e.g. CAR) is an anti-idiotype antibody against the antigen-binding domain of the CAR. Various anti-idiotype antibodies are known. Exemplary anti-idiotype antibodies against anti-CD19 binding domains, such as FMC63 or SJ25C1, are described in WO2018/023100. In some embodiments, cells expressing the recombinant receptor (e.g., CAR) can be further enriched (e.g., polished) for sub-population cells, e.g., CD4+ CAR+ T cells, CD8+ CAR+ T cells, CD28+, CD62L+, CCR7+, CD27+, CD127+, CD45RA+, CD45RO+ T cells, and/or viable cells. In some embodiments, cells expressing the recombinant receptor (e.g., CAR) can be further depleted for (e.g., polished) CD57+. In some embodiments, the selection step (e.g., initial selection and/or polishing step) allows control or adjustment of the ratio, concentration, or total number of cells expressing a recombinant receptor (e.g., CAR, TCR) and/or subpopulations thereof. In some embodiments, enriched (e.g., polished) populations can be formulated for use (e.g., administration) for cell therapy.

In some aspects, isolating the plurality of populations in a single or in the same isolation or separation vessel or set of vessels, such as a single column or set of columns, and/or same tube, or tubing set or using the same separation matrix or media or reagents, such as the same magnetic matrix, affinity-labeled solid support, or antibodies or other binding partners, include features that streamline the isolation, for example, resulting in reduced cost, time, complexity, need for handling of samples, use of resources, reagents, or equipment. In some aspects, such features are advantageous in that they minimize cost, efficiency, time, and/or complexity associated with the methods, and/or avoid potential harm to the cell product, such as harm caused by infection, contamination, and/or changes in temperature. The methods provided herein allow for multiple selection steps to enrich target populations both prior to or following cell selection combined with on-column stimulation.

The methods provided herein further allow for the selection and enrichment of successfully stimulated and engineered cells. For example, in some embodiments, the sequential selection procedures described above may be used to identify stimulated cells expressing recombinant receptor (e.g., CARs). In some embodiments, cells expressing the recombinant receptor (e.g., CAR) can be enriched for sub-population cells, e.g., CD4+ CAR+ T cells, CD8+ CAR+ T cells. In some embodiments, enriched populations can be formulated for use (e.g., administration) for cell therapy.

### III. RECOMBINANT PROTEINS (not part of the claimed invention)

In some embodiments, the cells that are treated, processed, engineered, and/or produced by the methods, which are not in accordance with the claimed invention, using the device provided herein contain or express, or are engineered to contain or express, a recombinant protein, such as a recombinant receptor, e.g., a chimeric antigen receptor (CAR), or a T cell receptor (TCR). In certain embodiments, the methods, which are not in accordance with the claimed invention, provided herein produce and/or a capable of producing cells, or populations or compositions containing and/or enriched for cells, that are engineered to express or contain a recombinant protein.

### A. Recombinant Receptors (not part of the claimed invention)

In some embodiments, provided are engineered cells, such as immune cells, such as T cells, that express one or more recombinant receptor(s). Among the receptors are antigen receptors and receptors containing one or more component thereof. The recombinant receptors may include chimeric receptors, such as those containing ligand-binding domains or binding fragments thereof and intracellular signaling domains or regions, functional non-TCR antigen receptors, chimeric antigen receptors (CARs), T cell receptors (TCRs), such as recombinant or transgenic TCRs, chimeric autoantibody receptor (CAAR) and components of any of the foregoing. The recombinant receptor, such as a CAR, generally includes the extracellular antigen (or ligand) binding domain linked to one or more intracellular signaling components, in some aspects via linkers and/or transmembrane domain(s). In some embodiments, the engineered cells express two or more receptors that contain different components, domains or regions. In some aspects, two or more receptors allows spatial or temporal regulation or control of specificity, activity, antigen (or ligand) binding, function and/or expression of the recombinant receptors.

### 1. Chimeric Antigen Receptors (CARs) (not part of the claimed invention)

In some embodiments of the provided methods, which are not in accordance with the claimed invention, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some embodiments, the intracellular signaling domain is an activating intracellular domain portion, such as a T cell activating domain, providing a primary activation signal. In some embodiments, the intracellular signaling domain contains or additionally contains a costimulatory signaling domain to facilitate effector functions. In some embodiments, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, . 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy (VH) chain region and/or variable light (VL) chain region of the antibody, e.g., an scFv antibody fragment.

In some embodiments, the antigen targeted by the receptor is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In some embodiments, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-Al, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30. In some embodiments, the antigen is or includes a pathogen-specific or pathogen-expressed antigen. In some embodiments, the antigen is a viral antigen (such as a viral antigen from HIV, HCV, HBV, etc.), bacterial antigens, and/or parasitic antigens.

Antigens targeted by the receptors in some embodiments include antigens associated with a B cell malignancy, such as any of a number of known B cell marker. In some embodiments, the antigen targeted by the receptor is CD20, CD19, CD22, ROR1, CD45, CD21, CD5, CD33, Igkappa, Iglambda, CD79a, CD79b or CD30.

In some embodiments, the antigen or antigen binding domain is CD19. In some embodiments, the scFv contains a VH and a VL derived from an antibody or an antibody fragment specific to CD19. In some embodiments, the antibody or antibody fragment that binds CD19 is a mouse derived antibody such as FMC63 and SJ25C1. In some embodiments, the antibody or antibody fragment is a human antibody, e.g., as described in U.S. Patent Publication No. US 2016/0152723.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, heavy chain variable (V_{H}) regions capable of specifically binding the antigen, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e.g*., bispecific or trispecific, antibodies, diabodies, triabodies, and tetrabodies, tandem discFv, tandem tri-scFv. Unless otherwise stated, the term "antibody" should be understood to encompass functional antibody fragments thereof also referred to herein as "antigen-binding fragments." The term also encompasses intact or full-length antibodies, including antibodies of any class or sub-class, including IgG and sub-classes thereof, IgM, IgE, IgA, and IgD.

The terms "complementarity determining region," and "CDR," synonymous with "hypervariable region" or "HVR," are known in the art to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known in the art to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 1,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 1. Boundaries of CDRs according to various numbering schemes.**

| **CDR** | **Kabat** | **Chothia** | **AbM** | **Contact** |
|---|---|---|---|---|
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32..34 | H26--H35B | H30--H35B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93--H101 |

| | | | | |
|---|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | | |

Thus, unless otherwise specified, a "CDR" or "complementary determining region," or individual specified CDRs (*e.g.,* CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR (*e.g*., a CDR-H3) contains the amino acid sequence of a corresponding CDR in a given V_{H} or V_{L} region amino acid sequence, it is understood that such a CDR has a sequence of the corresponding CDR (*e.g*., CDR-H3) within the variable region, as defined by any of the aforementioned schemes, or other known schemes. In some embodiments, specific CDR sequences are specified. Exemplary CDR sequences of provided antibodies are described using various numbering schemes, although it is understood that a provided antibody can include CDRs as described according to any of the other aforementioned numbering schemes or other numbering schemes known to a skilled artisan.

Likewise, unless otherwise specified, a FR or individual specified FR(s) (*e.g*., FR-H1, FR-H2, FR-H3, FR-H4), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, AbM or Contact method, or other known schemes. In other cases, the particular amino acid sequence of a CDR or FR is given.

The term "variable region" or "variable domain" refers to the domain of an antibody heavy or light chain that is involved in binding the antibody to antigen. The variable regions of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g*., Portolano et al., J. Immunol. 150:880-887 (1993); Clarkson et al., Nature 352:624-628 (1991).

Among the antibodies included in the provided CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In some embodiments, the antigen-binding domain in the provided CARs is or comprises an antibody fragment comprising a variable heavy chain (V_{H}) and a variable light chain (V_{L}) region. In particular embodiments, the antibodies are single-chain antibody fragments comprising a heavy chain variable (V_{H}) region and/or a light chain variable (V_{L}) region, such as scFvs.

In some embodiments, the scFv is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some embodiments, the FMC63 antibody comprises CDRH1 and H2 set forth in SEQ ID NOS: 51and 52, respectively, and CDRH3 set forth in SEQ ID NO: 53 or 54 and CDRL1 set forth in SEQ ID NO: 55 and CDR L2 set forth in SEQ ID NO: 55 or 57 and CDR L3 set forth in SEQ ID NO: 58 or 59. In some embodiments, the FMC63 antibody comprises the heavy chain variable region (V_{H}) comprising the amino acid sequence of SEQ ID NO: 60 and the light chain variable region (V_{L}) comprising the amino acid sequence of SEQ ID NO: 61.

In some embodiments, the scFv comprises a variable light chain containing the CDRL1 sequence of SEQ ID NO: 55, a CDRL2 sequence of SEQ ID NO: 56, and a CDRL3 sequence of SEQ ID NO: 58 and/or a variable heavy chain containing a CDRH1 sequence of SEQ ID NO: 51, a CDRH2 sequence of SEQ ID NO: 52, and a CDRH3 sequence of SEQ ID NO: 53. In some embodiments, the scFv comprises a variable heavy chain region set forth in SEQ ID NO:60 and a variable light chain region set forth in SEQ ID NO:61. In some embodiments, the variable heavy and variable light chains are connected by a linker. In some embodiments, the linker is set forth in SEQ ID NO: 62. In some embodiments, the scFv comprises, in order, a V_{H}, a linker, and a V_{L}. In some embodiments, the scFv comprises, in order, a V_{L}, a linker, and a V_{H}. In some embodiments, the scFv is encoded by a sequence of nucleotides set forth in SEQ ID NO: 63 or a sequence that exhibits at least 85%, 86%, 87%, 88%, 89%, 90%, 91%, 92%, 93%, 94%, 95%, 96%, 97%, 98%, or 99% sequence identity to SEQ ID NO: 63. In some embodiments, the scFv comprises the sequence of amino acids set forth in SEQ ID NO: 64 or a sequence that exhibits at least 85%, 86%, 87%, 88%, 89%, 90%, 91%, 92%, 93%, 94%, 95%, 96%, 97%, 98%, or 99% sequence identity to SEQ ID NO:64.

In some embodiments the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some embodiments, the SJ25C1 antibody comprises CDRH1, H2 and H3 set forth in SEQ ID NOS: 65-67, respectively, and CDRL1, L2 and L3 sequences set forth in SEQ ID NOS:68-70, respectively. In some embodiments, the SJ25C1 antibody comprises the heavy chain variable region (V_{H}) comprising the amino acid sequence of SEQ ID NO: 71 and the light chain variable region (V_{L}) comprising the amino acid sequence of SEQ ID NO: 72.

In some embodiments, the scFv comprises a variable light chain containing the CDRL1 sequence of SEQ ID NO:73, a CDRL2 sequence of SEQ ID NO: 74, and a CDRL3 sequence of SEQ ID NO:75 and/or a variable heavy chain containing a CDRH1 sequence of SEQ ID NO:76, a CDRH2 sequence of SEQ ID NO:77, and a CDRH3 sequence of SEQ ID NO:78. In some embodiments, the scFv comprises a variable heavy chain region set forth in SEQ ID NO: 71 and a variable light chain region set forth in SEQ ID NO:72. In some embodiments, the variable heavy and variable light chain are connected by a linker. In some embodiments, the linker is set forth in SEQ ID NO:79. In some embodiments, the scFv comprises, in order, a V_{H}, a linker, and a V_{L}. In some embodiments, the scFv comprises, in order, a V_{L}, a linker, and a V_{H}. In some embodiments, the scFv comprises the sequence of amino acids set forth in SEQ ID NO:80 or a sequence that exhibits at least 85%, 86%, 87%, 88%, 89%, 90%, 91%, 92%, 93%, 94%, 95%, 96%, 97%, 98%, or 99% sequence identity to SEQ ID NO:80.

In some embodiments, the antigen or antigen binding domain is BCMA. In some embodiments, the scFv contains a VH and a VL derived from an antibody or an antibody fragment specific to BCMA. In some embodiments, the antibody or antibody fragment that binds BCMA is or contains a VH and a VL from an antibody or antibody fragment set forth in International Patent Applications, Publication Number WO 2016/090327 and WO 2016/090320.

In some embodiments, the antigen or antigen binding domain is GPRC5D. In some embodiments, the scFv contains a VH and a VL derived from an antibody or an antibody fragment specific to GPRC5D. In some embodiments, the antibody or antibody fragment that binds GPRC5D is or contains a VH and a VL from an antibody or antibody fragment set forth in International Patent Applications, Publication Number WO 2016/090329 and WO 2016/090312.

In some embodiments, the antigen is CD20. In some embodiments, the scFv contains a VH and a VL derived from an antibody or an antibody fragment specific to CD20. In some embodiments, the antibody or antibody fragment that binds CD20 is an antibody that is or is derived from Rituximab, such as is Rituximab scFv.

In some embodiments, the antigen is CD22. In some embodiments, the scFv contains a VH and a VL derived from an antibody or an antibody fragment specific to CD22. In some embodiments, the antibody or antibody fragment that binds CD22 is an antibody that is or is derived from m971, such as is m971 scFv.

In some embodiments, the chimeric antigen receptor includes an extracellular portion containing an antibody or antibody fragment. In some aspects, the chimeric antigen receptor includes an extracellular portion containing the antibody or fragment and an intracellular signaling domain. In some embodiments, the antibody or fragment includes an scFv.

In some embodiments, the antibody portion of the recombinant receptor, e.g., CAR, further includes at least a portion of an immunoglobulin constant region, such as a hinge region, e.g., an IgG4 hinge region, and/or a CH1/CL and/or Fc region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some embodiments, the constant region or portion is of a human IgG, such as IgG4 or IgG1. In some embodiments, the spacer has the sequence ESKYGPPCPPCP (set forth in SEQ ID NO: 81), and is encoded by the sequence set forth in SEQ ID NO: 82. In some embodiments, the spacer has the sequence set forth in SEQ ID NO: 83. In some embodiments, the spacer has the sequence set forth in SEQ ID NO: 84. In some embodiments, the constant region or portion is of IgD. In some embodiments, the spacer has the sequence set forth in SEQ ID NO: 85. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 81, 83, 84 or 85. In some embodiments, the spacer has the sequence set forth in SEQ ID NOS: 86-94. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 86-94.

In some embodiments, the antigen receptor comprises an intracellular domain linked directly or indirectly to the extracellular domain. In some embodiments, the chimeric antigen receptor includes a transmembrane domain linking the extracellular domain and the intracellular signaling domain. In some embodiments, the intracellular signaling domain comprises an ITAM. For example, in some aspects, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some embodiments, the chimeric receptor comprises a transmembrane domain linked or fused between the extracellular domain (e.g. scFv) and intracellular signaling domain. Thus, in some embodiments, the antigen-binding component (e.g., antibody) is linked to one or more transmembrane and intracellular signaling domains.

In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s). In some aspects, the transmembrane domain contains a transmembrane portion of CD28.

In some embodiments, the extracellular domain and transmembrane domain can be linked directly or indirectly. In some embodiments, the extracellular domain and transmembrane are linked by a spacer, such as any described herein. In some embodiments, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some aspects described as being mediated by two classes of cytoplasmic signaling sequences: those that initiate antigen-dependent primary activation through the TCR (primary cytoplasmic signaling sequences), and those that act in an antigenindependent manner to provide a secondary or co-stimulatory signal (secondary cytoplasmic signaling sequences). In some aspects, the CAR includes one or both of such signaling components.

The receptor, e.g., the CAR, generally includes at least one intracellular signaling component or components. In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some embodiments, cytoplasmic signaling molecule(s) in the CAR contain(s) a cytoplasmic signaling domain, portion thereof, or sequence derived from CD3 zeta.

In some embodiments, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some embodiments, cell signaling modules include CD3 transmembrane domain, CD3 intracellular signaling domains, and/or other CD3 transmembrane domains. In some embodiments, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some embodiments, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling domain of an antigen receptor component or costimulatory molecule is used in place of an intact immunostimulatory chain, for example, if it transduces the effector function signal. In some embodiments, the intracellular signaling domain or domains include the cytoplasmic sequences of the T cell receptor (TCR), and in some aspects also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

In some embodiments, the chimeric antigen receptor contains an intracellular domain of a T cell costimulatory molecule. In some embodiments, the CAR includes a signaling domain and/or transmembrane portion of a costimulatory receptor, such as CD28, 4-1BB, OX40, DAP10, and ICOS. In some aspects, the same CAR includes both the activating and costimulatory components. In some embodiments, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

In some embodiments, the activating domain is included within one CAR, whereas the costimulatory component is provided by another CAR recognizing another antigen. In some embodiments, the CARs include activating or stimulatory CARs, costimulatory CARs, both expressed on the same cell (see WO2014/055668). In some aspects, the cells include one or more stimulatory or activating CAR and/or a costimulatory CAR. In some embodiments, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In some embodiments, the two receptors induce, respectively, an activating and an inhibitory signal to the cell, such that ligation of one of the receptor to its antigen activates the cell or induces a response, but ligation of the second inhibitory receptor to its antigen induces a signal that suppresses or dampens that response. Examples are combinations of activating CARs and inhibitory CARs (iCARs). Such a strategy may be used, for example, to reduce the likelihood of off-target effects in the context in which the activating CAR binds an antigen expressed in a disease or condition but which is also expressed on normal cells, and the inhibitory receptor binds to a separate antigen which is expressed on the normal cells but not cells of the disease or condition.

In some aspects, the chimeric receptor is or includes an inhibitory CAR (e.g. iCAR) and includes intracellular components that dampen or suppress an immune response, such as an ITAM- and/or co stimulatory-promoted response in the cell. Exemplary of such intracellular signaling components are those found on immune checkpoint molecules, including PD-1, CTLA4, LAG3, BTLA, OX2R, TIM-3, TIGIT, LAIR-1, PGE2 receptors, EP2/4 Adenosine receptors including A2AR. In some aspects, the engineered cell includes an inhibitory CAR including a signaling domain of or derived from such an inhibitory molecule, such that it serves to dampen the response of the cell, for example, that induced by an activating and/or costimulatory CAR.

In certain embodiments, the intracellular signaling domain comprises a CD28 transmembrane and signaling domain linked to a CD3 (e.g., CD3-zeta) intracellular domain. In some embodiments, the intracellular signaling domain comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some embodiments, the antigen receptor further includes a marker and/or cells expressing the CAR or other antigen receptor further includes a surrogate marker, such as a cell surface marker, which may be used to confirm transduction or engineering of the cell to express the receptor. In some aspects, the marker includes all or part (e.g., truncated form) of CD34, a NGFR, or epidermal growth factor receptor, such as truncated version of such a cell surface receptor (e.g., tEGFR). In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 43 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 43 or 44. An exemplary T2A linker sequence comprises the sequence of amino acids set forth in SEQ ID NO: 47 or 48 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 47 or 48.

In some embodiments, the marker is a molecule, e.g., cell surface protein, not naturally found on T cells or not naturally found on the surface of T cells, or a portion thereof. In some embodiments, the molecule is a non-self molecule, e.g., non-self protein, i.e., one that is not recognized as "self" by the immune system of the host into which the cells will be adoptively transferred.

In some embodiments, the marker serves no therapeutic function and/or produces no effect other than to be used as a marker for genetic engineering, e.g., for selecting cells successfully engineered. In other embodiments, the marker may be a therapeutic molecule or molecule otherwise exerting some desired effect, such as a ligand for a cell to be encountered *in vivo,* such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

For example, in some embodiments, the CAR contains an antibody, e.g., an antibody fragment, such as an scFv, specific to an antigen including any as described, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, such as an scFv, specific to an antigen including any as described, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 95 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 95; in some embodiments, the transmembrane-domain containing portion of the recombinant receptor comprises the sequence of amino acids set forth in SEQ ID NO: 96 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some embodiments, the intracellular signaling component(s) of the recombinant receptor, *e.g*. the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 97 or 98 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 97 or 98. In some embodiments, the intracellular domain comprises an intracellular costimulatory signaling domain of 4-1BB (e.g. (Accession No. Q07011.1) or functional variant or portion thereof, such as the sequence of amino acids set forth in SEQ ID NO: 99 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 99.

In some embodiments, the intracellular signaling domain of the recombinant receptor, *e.g.* the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some embodiments, the intracellular signaling domain comprises the sequence of amino acids as set forth in SEQ ID NO: 100, 101 or 102 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 100, 101 or 102.

In some aspects, the spacer contains only a hinge region of an IgG, such as only a hinge of IgG4 or IgG1, such as the hinge only spacer set forth in SEQ ID NO: 81. In other embodiments, the spacer is or contains an Ig hinge, e.g., an IgG4-derived hinge, optionally linked to a CH2 and/or CH3 domains. In some embodiments, the spacer is an Ig hinge, e.g., an IgG4 hinge, linked to CH2 and CH3 domains, such as set forth in SEQ ID NO: 84. In some embodiments, the spacer is an Ig hinge, e.g., an IgG4 hinge, linked to a CH3 domain only, such as set forth in SEQ ID NO: 83. In some embodiments, the spacer is or comprises a glycine-serine rich sequence or other flexible linker such as known flexible linkers.

For example, in some embodiments, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some embodiments, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in 43 or 44) or a prostate-specific membrane antigen (PSMA) or modified form thereof. tEGFR may contain an epitope recognized by the antibody cetuximab (Erbitux^{®}) or other therapeutic anti-EGFR antibody or binding molecule, which can be used to identify or select cells that have been engineered to express the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, *e.g.* surrogate marker, includes all or part (*e.g.,* truncated form) of CD34, a NGFR, a CD19 or a truncated CD19, e.g., a truncated non-human CD19, or epidermal growth factor receptor (*e.g.,* tEGFR). In some embodiments, the marker is or comprises a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In some embodiments, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In some embodiments, the marker is a resistance marker or selection marker. In some embodiments, the resistance marker or selection marker is or comprises a polypeptide that confers resistance to exogenous agents or drugs. In some embodiments, the resistance marker or selection marker is an antibiotic resistance gene. In some embodiments, the resistance marker or selection marker is an antibiotic resistance gene confers antibiotic resistance to a mammalian cell. In some embodiments, the resistance marker or selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

In some embodiments, the nucleic acid encoding the marker is operably linked to a polynucleotide encoding for a linker sequence, such as a cleavable linker sequence, *e.g.,* a T2A. For example, a marker, and optionally a linker sequence, can be any as disclosed in PCT Pub. No. WO2014031687.

In some embodiments, nucleic acid molecules encoding such CAR constructs further includes a sequence encoding a T2A ribosomal skip element and/or a tEGFR sequence, e.g., downstream of the sequence encoding the CAR. In some embodiments, the sequence encodes a T2A ribosomal skip element set forth in SEQ ID NO: 47 or 48, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 47 or 48.

In some embodiments, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some embodiments, the sequence encodes an tEGFR sequence set forth in SEQ ID NO: 43 or 44, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 43 or 44. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 45), equine rhinitis A virus (E2A, *e.g.,* SEQ ID NO: 46), Thosea asigna virus (T2A, *e.g.,* SEQ ID NO: 47 or 48), and porcine teschovirus-1 (P2A, *e.g.,* SEQ ID NO: 49 or 50) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some embodiments, the cells express a CAR that specifically binds to an antigen expressed by a cell or tissue of the disease or condition or associated with the disease or condition.

### 2. Chimeric Auto-Antibody Receptor (CAAR) (not part of the claimed invention)

In some embodiments, the recombinant receptor is a chimeric autoantibody receptor (CAAR). In some embodiments, the CAAR binds, e.g., specifically binds, or recognizes, an autoantibody. In some embodiments, a cell expressing the CAAR, such as a T cell engineered to express a CAAR, can be used to bind to and kill autoantibody-expressing cells, but not normal antibody expressing cells. In some embodiments, CAAR-expressing cells can be used to treat an autoimmune disease associated with expression of self-antigens, such as autoimmune diseases. In some embodiments, CAAR-expressing cells can target B cells that ultimately produce the autoantibodies and display the autoantibodies on their cell surfaces, mark these B cells as disease-specific targets for therapeutic intervention. In some embodiments, CAAR-expressing cells can be used to efficiently targeting and killing the pathogenic B cells in autoimmune diseases by targeting the disease-causing B cells using an antigen-specific chimeric autoantibody receptor. In some embodiments, the recombinant receptor is a CAAR, such as any described in U.S. Patent Application Pub. No. US 2017/0051035.

In some embodiments, the CAAR comprises an autoantibody binding domain, a transmembrane domain, and one or more intracellular signaling region or domain (also interchangeably called a cytoplasmic signaling domain or region). In some embodiments, the intracellular signaling region comprises an intracellular signaling domain. In some embodiments, the intracellular signaling domain is or comprises a primary signaling domain, a signaling domain that is capable of stimulating and/or inducing a primary activation signal in a T cell, a signaling domain of a T cell receptor (TCR) component (e.g. an intracellular signaling domain or region of a CD3-zeta (CD3ζ) chain or a functional variant or signaling portion thereof), and/or a signaling domain comprising an immunoreceptor tyrosine-based activation motif (ITAM).

In some embodiments, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some embodiments, the autoimmune disease includes pemphigus vulgaris (PV). Exemplary autoantigens include desmoglein 1 (Dsgl) and Dsg3.

### 3. T Cell Receptors (TCRs) (not part of the claimed invention)

In some embodiments, engineered cells, such as T cells, are provided that express a T cell receptor (TCR) or antigen-binding portion thereof that recognizes an peptide epitope or T cell epitope of a target polypeptide, such as an antigen of a tumor, viral or autoimmune protein.

In some embodiments, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some embodiments, the variable domains of the TCR contain hypervariable loops, or complementarity determining regions (CDRs), which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction.

In some embodiments, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or Cβ, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains.

In some embodiments, the TCR chains contain a transmembrane domain. In some embodiments, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling device or complex. The intracellular tails of CD3 signaling subunits (e.g. CD3y, CD36, CD3ε and CD3ζ chains) contain one or more immunoreceptor tyrosine-based activation motif or ITAM that are involved in the signaling capacity of the TCR complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds.

In some embodiments, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some embodiments, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some embodiments, the TCR is obtained from a biological source, such as from cells such as from a T cell (e.g. cytotoxic T cell), T-cell hybridomas or other publicly available source. In some embodiments, the T-cells can be obtained from in vivo isolated cells. In some embodiments, the TCR is a thymically selected TCR. In some embodiments, the TCR is a neoepitope-restricted TCR. In some embodiments, the T- cells can be a cultured T-cell hybridoma or clone. In some embodiments, the TCR or antigen-binding portion thereof or antigen-binding fragment thereof can be synthetically generated from knowledge of the sequence of the TCR.

In some embodiments, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4+ or CD8+ T cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific. Alternatively, in some embodiments, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some embodiments, the TCR or antigen-binding portion thereof is one that has been modified or engineered. In some embodiments, directed evolution methods are used to generate TCRs with altered properties, such as with higher affinity for a specific MHC-peptide complex. In some embodiments, directed evolution is achieved by display methods including, but not limited to, yeast display (Holler et al. (2003) Nat Immunol, 4, 55-62; Holler et al. (2000) Proc Natl Acad Sci U S A, 97, 5387-92), phage display (Li et al. (2005) Nat Biotechnol, 23, 349-54), or T cell display (Chervin et al. (2008) J Immunol Methods, 339, 175-84). In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some embodiments, peptides of a target polypeptide for use in producing or generating a TCR of interest are known or can be readily identified. In some embodiments, peptides suitable for use in generating TCRs or antigen-binding portions can be determined based on the presence of an HLA-restricted motif in a target polypeptide of interest, such as a target polypeptide described below. In some embodiments, peptides are identified using available computer prediction models. In some embodiments, for predicting MHC class I binding sites, such models include, but are not limited to, ProPred1 (Singh and Raghava (2001) Bioinformatics 17(12):1236-1237, and SYFPEITHI (see Schuler et al. (2007) Immunoinformatics Methods in Molecular Biology, 409(1): 75-93 2007). In some embodiments, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vo1 409(1): 75-93 2007).

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal. A TCR may be cell-bound or in soluble form. In some embodiments, for purposes of the provided methods, the TCR is in cell-bound form expressed on the surface of a cell.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). In some embodiments, a dTCR or scTCR have the structures as described in WO 03/020763, WO 04/033685, WO2011/044186.

In some embodiments, the TCR contains a sequence corresponding to the transmembrane sequence. In some embodiments, the TCR does contain a sequence corresponding to cytoplasmic sequences. In some embodiments, the TCR is capable of forming a TCR complex with CD3. In some embodiments, any of the TCRs, including a dTCR or scTCR, can be linked to signaling domains that yield an active TCR on the surface of a T cell. In some embodiments, the TCR is expressed on the surface of cells.

In some embodiments a dTCR contains a first polypeptide wherein a sequence corresponding to a TCR α chain variable region sequence is fused to the N terminus of a sequence corresponding to a TCR a chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native inter-chain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant a domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR. Typically, a scTCR can be generated using methods known, See e.g., Soo Hoo, W. F. et al. PNAS (USA) 89, 4759 (1992); Wiilfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some embodiments, a scTCR contains an introduced non-native disulfide interchain bond to facilitate the association of the TCR chains (see e.g. International published PCT No. WO 03/020763). In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, a scTCR contains a first segment constituted by an amino acid sequence corresponding to a TCR a chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an α chain variable region sequence fused to the N terminus of an a chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, the linker of a scTCRs that links the first and second TCR segments can be any linker capable of forming a single polypeptide strand, while retaining TCR binding specificity. In some embodiments, the linker sequence may, for example, have the formula -P-AA-P- wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from 10 to 45 amino acids or from about 10 to about 45 amino acids, such as 10 to 30 amino acids or 26 to 41 amino acids residues, for example 29, 30, 31 or 32 amino acids. In some embodiments, the linker has the formula -PGGG-(SGGGG)5-P- wherein P is proline, G is glycine and S is serine (SEQ ID NO:38). In some embodiments, the linker has the sequence GSADDAKKDAAKKDGKS (SEQ ID NO:39).

In some embodiments, the scTCR contains a covalent disulfide bond linking a residue of the immunoglobulin region of the constant domain of the α chain to a residue of the immunoglobulin region of the constant domain of the β chain. In some embodiments, the interchain disulfide bond in a native TCR is not present. For example, in some embodiments, one or more cysteines can be incorporated into the constant region extracellular sequences of the first and second segments of the scTCR polypeptide. In some cases, both a native and a non-native disulfide bond may be desirable.

In some embodiments of a dTCR or scTCR containing introduced interchain disulfide bonds, the native disulfide bonds are not present. In some embodiments, the one or more of the native cysteines forming a native interchain disulfide bonds are substituted to another residue, such as to a serine or alanine. In some embodiments, an introduced disulfide bond can be formed by mutating non-cysteine residues on the first and second segments to cysteine. Exemplary non-native disulfide bonds of a TCR are described in published International PCT No. WO2006/000830.

In some embodiments, the TCR or antigen-binding fragment thereof exhibits an affinity with an equilibrium binding constant for a target antigen of between or between about 10-5 and 10-12 M and all individual values and ranges therein. In some embodiments, the target antigen is an MHC-peptide complex or ligand.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech). In some embodiments, a viral vector is used, such as a retroviral vector.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other MHC-peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, to generate a vector encoding a TCR, the α and β chains are PCR amplified from total cDNA isolated from a T cell clone expressing the TCR of interest and cloned into an expression vector. In some embodiments, the α and β chains are cloned into the same vector. In some embodiments, the α and β chains are cloned into different vectors. In some embodiments, the generated α and β chains are incorporated into a retroviral, e.g. lentiviral, vector.

### B. Nucleic Acids, Vectors and Methods for Genetic Engineering (not part of the claimed invention)

Using a device disclosed herein, provided herein are methods, which are not in accordance with the claimed invention, where the cells, e.g., T cells, are genetically engineered to express a recombinant receptor. In some embodiments, the engineering is carried out by introducing one or more polynucleotide(s) that encode the recombinant receptor or portions or components thereof. Also provided are polynucleotides encoding a recombinant receptor, and vectors or constructs containing such nucleic acids and/or polynucleotides.

In some embodiments, the polynucleotide encoding the recombinant receptor contains at least one promoter that is operatively linked to control expression of the recombinant receptor. In some examples, the polynucleotide contains two, three, or more promoters operatively linked to control expression of the recombinant receptor. In some embodiments, polynucleotide can contain regulatory sequences, such as transcription and translation initiation and termination codons, which are specific to the type of host (e.g., bacterium, fungus, plant, or animal) into which the polynucleotide is to be introduced, as appropriate and taking into consideration whether the polynucleotide is DNA- or RNA-based. In some embodiments, the polynucleotide can contain regulatory/control elements, such as a promoter, an enhancer, an intron, a polyadenylation signal, a Kozak consensus sequence, internal ribosome entry sites (IRES), a 2A sequence, and splice acceptor or donor. In some embodiments, the polynucleotide can contain a nonnative promoter operably linked to the nucleotide sequence encoding the recombinant receptor and/or one or more additional polypeptide(s). In some embodiments, the promoter is selected from among an RNA pol I, pol II or pol III promoter. In some embodiments, the promoter is recognized by RNA polymerase II (e.g., a CMV, SV40 early region or adenovirus major late promoter). In another embodiment, the promoter is recognized by RNA polymerase III (e.g., a U6 or H1 promoter). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, the promoter is or comprises a constitutive promoter. Exemplary constitutive promoters include, e.g., simian virus 40 early promoter (SV40), cytomegalovirus immediate-early promoter (CMV), human Ubiquitin C promoter (UBC), human elongation factor 1α promoter (EF1α), mouse phosphoglycerate kinase 1 promoter (PGK), and chicken β-Actin promoter coupled with CMV early enhancer (CAGG). In some embodiments, the constitutive promoter is a synthetic or modified promoter. In some embodiments, the promoter is or comprises an MND promoter, a synthetic promoter that contains the U3 region of a modified MoMuLV LTR with myeloproliferative sarcoma virus enhancer (see Challita et al. (1995) J. Virol. 69(2):748-755). In some embodiments, the promoter is a tissue-specific promoter. In another embodiment, the promoter is a viral promoter. In another embodiment, the promoter is a non-viral promoter. In some embodiments, exemplary promoters can include, but are not limited to, human elongation factor 1 alpha (EF1α) promoter or a modified form thereof or the MND promoter.

In another embodiment, the promoter is a regulated promoter (e.g., inducible promoter). In some embodiments, the promoter is an inducible promoter or a repressible promoter. In some embodiments, the promoter comprises a Lac operator sequence, a tetracycline operator sequence, a galactose operator sequence or a doxycycline operator sequence, or is an analog thereof or is capable of being bound by or recognized by a Lac repressor or a tetracycline repressor, or an analog thereof. In some embodiments, the polynucleotide does not include a regulatory element, e.g. promoter.

In some cases, the nucleic acid sequence encoding the recombinant receptor contains a signal sequence that encodes a signal peptide. In some aspects, the signal sequence may encode a signal peptide derived from a native polypeptide. In other aspects, the signal sequence may encode a heterologous or non-native signal peptide, such as the exemplary signal peptide of the GMCSFR alpha chain set forth in SEQ ID NO:40 and encoded by the nucleotide sequence set forth in SEQ ID NO:41. In some cases, the nucleic acid sequence encoding the recombinant receptor, e.g., chimeric antigen receptor (CAR) contains a signal sequence that encodes a signal peptide. Non-limiting exemplary signal peptides include, for example, the GMCSFR alpha chain signal peptide set forth in SEQ ID NO: 40 and encoded by the nucleotide sequence set forth in SEQ ID NO:40, or the CD8 alpha signal peptide set forth in SEQ ID NO:42.

In some embodiments, the polynucleotide contains a nucleic acid sequence encoding one or more additional polypeptides, e.g., one or more marker(s) and/or one or more effector molecules. In some embodiments, the one or more marker(s) includes a transduction marker, a surrogate marker and/or a resistance marker or selection marker. Among additional nucleic acid sequences introduced, e.g., encoding for one or more additional polypeptide(s), include nucleic acid sequences that can improve the efficacy of therapy, such as by promoting viability and/or function of transferred cells; nucleic acid sequences to provide a genetic marker for selection and/or evaluation of the cells, such as to assess *in vivo* survival or localization; nucleic acid sequences to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also WO 1992008796 and WO 1994028143 describing the use of bifunctional selectable fusion genes derived from fusing a dominant positive selectable marker with a negative selectable marker, and US Patent No. 6,040,177.

In some embodiments, the marker is a transduction marker or a surrogate marker. A transduction marker or a surrogate marker can be used to detect cells that have been introduced with the polynucleotide, e.g., a polynucleotide encoding a recombinant receptor. In some embodiments, the transduction marker can indicate or confirm modification of a cell. In some embodiments, the surrogate marker is a protein that is made to be co-expressed on the cell surface with the recombinant receptor, e.g. CAR. In particular embodiments, such a surrogate marker is a surface protein that has been modified to have little or no activity. In certain embodiments, the surrogate marker is encoded on the same polynucleotide that encodes the recombinant receptor. In some embodiments, the nucleic acid sequence encoding the recombinant receptor is operably linked to a nucleic acid sequence encoding a marker, optionally separated by an internal ribosome entry site (IRES), or a nucleic acid encoding a self-cleaving peptide or a peptide that causes ribosome skipping, such as a 2A sequence. Extrinsic marker genes may in some cases be utilized in connection with engineered cell to permit detection or selection of cells and, in some cases, also to promote cell elimination and/or cell suicide.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 43 or 44) or a prostate-specific membrane antigen (PSMA) or modified form thereof, such as a truncated PSMA (tPSMA). In some aspects, tEGFR may contain an epitope recognized by the antibody cetuximab (Erbitux^{®}) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, *e.g.* surrogate marker, includes all or part (*e.g.,* truncated form) of CD34, a NGFR, a CD19 or a truncated CD19, e.g., a truncated non-human CD19. An exemplary polypeptide for a truncated EGFR (*e.g.* tEGFR) comprises the sequence of amino acids set forth in SEQ ID NO: 43 or 44 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 43 or 44.

In some embodiments, the marker is or comprises a detectable protein, such as a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, codon-optimized, stabilized and/or enhanced variants of the fluorescent proteins. In some embodiments, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof. In some aspects, expression of the enzyme can be detected by addition of a substrate that can be detected upon the expression and functional activity of the enzyme.

In some embodiments, the marker is a resistance maker or selection marker. In some embodiments, the resistance maker or selection marker is or comprises a polypeptide that confers resistance to exogenous agents or drugs. In some embodiments, the resistance marker or selection marker is an antibiotic resistance gene. In some embodiments, the resistance marker or selection marker is an antibiotic resistance gene confers antibiotic resistance to a mammalian cell. In some embodiments, the resistance marker or selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

Any of the recombinant receptors and/or the additional polypeptide(s) described herein can be encoded by one or more polynucleotides containing one or more nucleic acid sequences encoding recombinant receptors, in any combinations, orientation or arrangements. For example, one, two, three or more polynucleotides can encode one, two, three or more different polypeptides, e.g., recombinant receptors or portions or components thereof, and/or one or more additional polypeptide(s), e.g., a marker and/or an effector molecule. In some embodiments, one polynucleotide contains a nucleic acid sequence encoding a recombinant receptor, e.g., CAR, or portion or components thereof, and a nucleic acid sequence encoding one or more additional polypeptide(s). In some embodiments, one vector or construct contains a nucleic acid sequence encoding a recombinant receptor, e.g., CAR, or portion or components thereof, and a separate vector or construct contains a nucleic acid sequence encoding one or more additional polypeptide(s). In some embodiments, the nucleic acid sequence encoding the recombinant receptor and the nucleic acid sequence encoding the one or more additional polypeptide(s) are operably linked to two different promoters. In some embodiments, the nucleic acid encoding the recombinant receptor is present upstream of the nucleic acid encoding the one or more additional polypeptide(s). In some embodiments, the nucleic acid encoding the recombinant receptor is present downstream of the nucleic acid encoding one or more additional polypeptide(s).

In certain cases, one polynucleotide contains nucleic acid sequences encode two or more different polypeptide chains, e.g., a recombinant receptor and one or more additional polypeptide(s), e.g., a marker and/or an effector molecule. In some embodiments, the nucleic acid sequences encoding two or more different polypeptide chains, e.g., a recombinant receptor and one or more additional polypeptide(s), are present in two separate polynucleotides. For example, two separate polynucleotides are provided, and each can be individually transferred or introduced into the cell for expression in the cell. In some embodiments, the nucleic acid sequences encoding the marker and the nucleic acid sequences encoding the recombinant receptor are present or inserted at different locations within the genome of the cell. In some embodiments, the nucleic acid sequences encoding the marker and the nucleic acid sequences encoding the recombinant receptor are operably linked to two different promoters.

In some embodiments, such as those where the polynucleotide contains a first and second nucleic acid sequence, the coding sequences encoding each of the different polypeptide chains can be operatively linked to a promoter, which can be the same or different. In some embodiments, the nucleic acid molecule can contain a promoter that drives the expression of two or more different polypeptide chains. In some embodiments, such nucleic acid molecules can be multicistronic (bicistronic or tricistronic, see *e.g.,* U.S. Patent No. 6,060,273). In some embodiments, the nucleic acid sequences encoding the recombinant receptor and the nucleic acid sequences encoding the one or more additional polypeptide(s) are operably linked to the same promoter and are optionally separated by an internal ribosome entry site (IRES), or a nucleic acid encoding a self-cleaving peptide or a peptide that causes ribosome skipping, such as a 2A element. For example, an exemplary marker, and optionally a ribosome skipping sequence sequence, can be any as disclosed in PCT Pub. No. WO2014031687.

In some embodiments, transcription units can be engineered as a bicistronic unit containing an IRES, which allows coexpression of gene products (*e.g.* encoding the recombinant receptor and the additional polypeptide) by a message from a single promoter. Alternatively, in some cases, a single promoter may direct expression of an RNA that contains, in a single open reading frame (ORF), two or three genes (e.g. encoding the marker and encoding the recombinant receptor) separated from one another by sequences encoding a self-cleavage peptide (e.g., 2A sequences) or a protease recognition site (e.g., furin). The ORF thus encodes a single polypeptide, which, either during (in the case of 2A) or after translation, is processed into the individual proteins. In some cases, the peptide, such as a T2A, can cause the ribosome to skip (ribosome skipping) synthesis of a peptide bond at the C-terminus of a 2A element, leading to separation between the end of the 2A sequence and the next peptide downstream (see, e.g., de Felipe, Genetic Vaccines and Ther. 2:13 (2004) and de Felipe et al. Traffic 5:616-626 (2004)). Various 2A elements are known. Examples of 2A sequences that can be used in the methods and system disclosed herein, without limitation, 2A sequences from the foot-and-mouth disease virus (F2A, *e.g.,* SEQ ID NO: 45), equine rhinitis A virus (E2A, *e.g.,* SEQ ID NO: 46), Thosea asigna virus (T2A, *e.g.,* SEQ ID NO: 47 or 48), and porcine teschovirus-1 (P2A, *e.g.,* SEQ ID NO: 49 or 50) as described in U.S. Patent Pub. No. 20070116690.

In some embodiments, the polynucleotide encoding the recombinant receptor and/or additional polypeptide is contained in a vector or can be cloned into one or more vector(s). In some embodiments, the one or more vector(s) can be used to transform or transfect a host cell, e.g., a cell for engineering. Exemplary vectors include vectors designed for introduction, propagation and expansion or for expression or both, such as plasmids and viral vectors. In some aspects, the vector is an expression vector, e.g., a recombinant expression vector. In some embodiments, the recombinant expression vectors can be prepared using standard recombinant DNA techniques.

In some embodiments, the vector can be a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1 149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech).

In some embodiments, the vector is a viral vector, such as a retroviral vector. In some embodiments, the polynucleotide encoding the recombinant receptor and/or additional polypeptide(s) are introduced into the cell via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy 18:1748-1757; and Hackett et al. (2010) Molecular Therapy 18:674-683).

In some embodiments, one or more polynucleotide(s) are introduced into cells using recombinant infectious virus particles, such as, e.g., vectors derived from simian virus 40 (SV40), adenoviruses, adeno-associated virus (AAV). In some embodiments, one or more polynucleotide(s) are introduced into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the vector is a retroviral vector. In some aspects, a retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505. In some embodiments, the polynucleotide encoding the recombinant receptor and/or one or more additional polypeptide(s), is introduced into a composition containing cultured cells, such as by retroviral transduction, transfection, or transformation.

In some embodiments, one or more polynucleotide(s) are introduced into a T cell using electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material, e.g., polynucleotides and/or vectors, into immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987) and other approaches described in, e.g., International Pat. App. Pub. No. WO 2014055668, and U.S. Patent No. 7,446,190.

In some embodiments, the one or more polynucleotide(s) or vector(s) encoding a recombinant receptor and/or additional polypeptide(s) may be introduced into cells, e.g., T cells, either during or after expansion. This introduction of the polynucleotide(s) or vector(s) can be carried out with any suitable retroviral vector, for example. Resulting genetically engineered cells can then be liberated from the initial stimulus (e.g., anti-CD3/anti-CD28 stimulus) and subsequently be stimulated in the presence of a second type of stimulus (e.g., via a de novo introduced recombinant receptor). This second type of stimulus may include an antigenic stimulus in form of a peptide/MHC molecule, the cognate (cross-linking) ligand of the genetically introduced receptor (e.g. natural antigen and/or ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

### IV. COMPOSITIONS, FORMULATIONS AND METHODS OF ADMINISTRATION (not part of the claimed invention)

Also provided are compositions containing the stimulated and selected cells, optionally genetically engineered (e.g., engineered antigen receptor), such as CAR or TCR, and compositions containing the cells, including pharmaceutical compositions and formulations. Also provided are methods, which are not in accordance with the claimed invention, of using and uses of the compositions, such as in the treatment of diseases, conditions, and disorders in which the antigen is expressed, or in detection, diagnostic, and prognostic methods.

### A. Compositions and Formulations (not part of the claimed invention)

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some embodiments contains agents or cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells or agent. In some embodiments, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some embodiments suitably administered to the subject at one time or over a series of treatments.

The cells or agents may be administered using standard administration techniques, formulations, and/or devices. Provided are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some embodiments, the agent or cell populations are administered parenterally. The term "parenteral," as used herein, includes intravenous, intramuscular, subcutaneous, rectal, vaginal, and intraperitoneal administration. In some embodiments, the agent or cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

### B. Methods of Treatment and Uses (not part of the claimed invention)

Provided herein are methods, which are not in accordance with the claimed invention, of treatment, e.g., including administering any of the engineered cells (e.g. CAR-expressing cells) or compositions containing engineered cells (e.g. CAR-expressing cells) described herein. In some aspects, also provided are methods of administering any of the engineered cells (e.g. CAR-expressing cells) or compositions containing engineered cells described herein to a subject, such as a subject that has a disease or disorder. In some aspects, also provided are uses of any of the engineered cells (e.g. CAR-expressing cells) or compositions containing engineered cells described herein for treatment of a disease or disorder. In some aspects, also provided are uses of any of the engineered cells (e.g. CAR-expressing cells) or compositions containing engineered cells described herein for the manufacture of a medicament for the treatment of a disease or disorder. In some aspects, also provided are any of the engineered cells (e.g. CAR-expressing cells) or compositions containing engineered cells described herein, for use in treatment of a disease or disorder, or for administration to a subject having a disease or disorder.

Methods for administration of cells for adoptive cell therapy are known and may be used in connection with the provided methods and compositions. For example, adoptive T cell therapy methods are described, e.g., in US Pat. App. Pub. No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular embodiments, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some embodiments, the disease, disorder or condition is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM). In some embodiments, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some embodiments, the disease or condition is NHL and the NHL is selected from the group consisting of aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B).

In some embodiments, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some embodiments, the antigen associated with the disease or disorder is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen _{1B} (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT) vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30. In some embodiments, the antigen is or includes a pathogen-specific or pathogen-expressed antigen, such as a viral antigen (e.g., a viral antigen from HIV, HCV, HBV), bacterial antigens, and/or parasitic antigens.

In some embodiments, the antibody or an antigen-binding fragment (e.g. scFv or V_{H} domain) specifically recognizes an antigen, such as CD19. In some embodiments, the antibody or antigen-binding fragment is derived from, or is a variant of, antibodies or antigen-binding fragment that specifically binds to CD19. In some embodiments, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells. In some embodiments, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some embodiments suitably administered to the subject at one time or over a series of treatments.

In some embodiments, a dose of cells is administered to subjects in accord with the provided methods, and/or with the provided articles of manufacture or compositions. In some embodiments, the size or timing of the doses is determined as a function of the particular disease or condition in the subject. In some cases, the size or timing of the doses for a particular disease in view of the provided description may be empirically determined.

In some embodiments, the dose of cells comprises between at or about 2 x 10⁵ of the cells/kg and at or about 2 x 10⁶ of the cells/kg, such as between at or about 4 x 10⁵ of the cells/kg and at or about 1 x 10⁶ of the cells/kg or between at or about 6 x 10⁵ of the cells/kg and at or about 8 x 10⁵ of the cells/kg. In some embodiments, the dose of cells comprises no more than 2 x 10⁵ of the cells (e.g. antigen-expressing, such as CAR-expressing cells) per kilogram body weight of the subject (cells/kg), such as no more than at or about 3 x 10⁵ cells/kg, no more than at or about 4 x 10⁵ cells/kg, no more than at or about 5 x 10⁵ cells/kg, no more than at or about 6 x 10⁵ cells/kg, no more than at or about 7 x 10⁵ cells/kg, no more than at or about 8 x 10⁵ cells/kg, no more than at or about 9 x 10⁵ cells/kg, no more than at or about 1 x 10⁶ cells/kg, or no more than at or about 2 x 10⁶ cells/kg. In some embodiments, the dose of cells comprises at least or at least about or at or about 2 x 10⁵ of the cells (e.g. antigen-expressing, such as CAR-expressing cells) per kilogram body weight of the subject (cells/kg), such as at least or at least about or at or about 3 x 10⁵ cells/kg, at least or at least about or at or about 4 x 10⁵ cells/kg, at least or at least about or at or about 5 x 10⁵ cells/kg, at least or at least about or at or about 6 x 10⁵ cells/kg, at least or at least about or at or about 7 x 10⁵ cells/kg, at least or at least about or at or about 8 x 10⁵ cells/kg, at least or at least about or at or about 9 x 10⁵ cells/kg, at least or at least about or at or about 1 x 10⁶ cells/kg, or at least or at least about or at or about 2 x 10⁶ cells/kg.

In some embodiments, the dose of cells is a flat dose of cells or fixed dose of cells such that the dose of cells is not tied to or based on the body surface area or weight of a subject.

In some embodiments, for example, where the subject is a human, the dose includes fewer than about 5 x 10⁸ total recombinant receptor (e.g., CAR)-expressing cells, T cells, or peripheral blood mononuclear cells (PBMCs), e.g., in the range of about 1 x 10⁶ to 5 x 10⁸ such cells, such as 2 x 10⁶, 5 x 10⁶, 1 x 10⁷, 5 x 10⁷, 1 x 10³, or 5 x 10⁸ total such cells, or the range between any two of the foregoing values.

In some embodiments, the dose of genetically engineered cells comprises from or from about 1 x 10⁵ to 5 x 10⁸ total CAR-expressing T cells, 1 x 10⁵ to 2.5 x 10⁸ total CAR-expressing T cells, 1 x 10⁵ to 1 x 10⁸ total CAR-expressing T cells, 1 x 10⁵ to 5 x 10⁷ total CAR-expressing T cells, 1 x 10⁵ to 2.5 x 10⁷ total CAR-expressing T cells, 1 x 10⁵ to 1 x 10⁷ total CAR-expressing T cells, 1 x 10⁵ to 5 x 10⁶ total CAR-expressing T cells, 1 x 10⁵ to 2.5 x 10⁶ total CAR-expressing T cells, 1 x 10⁵ to 1 x 10⁶ total CAR-expressing T cells, 1 x 10⁶ to 5 x 10⁸ total CAR-expressing T cells, 1 x 10⁶ to 2.5 x 10⁸ total CAR-expressing T cells, 1 x 10⁶ to 1 x 10⁸ total CAR-expressing T cells, 1 x 10⁶ to 5 x 10⁷ total CAR-expressing T cells, 1 x 10⁶ to 2.5 x 10⁷ total CAR-expressing T cells, 1 x 10⁶ to 1 x 10⁷ total CAR-expressing T cells, 1 x 10⁶ to 5 x 10⁶ total CAR-expressing T cells, 1 x 10⁶ to 2.5 x 10⁶ total CAR-expressing T cells, 2.5 x 10⁶ to 5 x 10⁸ total CAR-expressing T cells, 2.5 x 10⁶ to 2.5 x 10⁸ total CAR-expressing T cells, 2.5 x 10⁶ to 1 x 10⁸ total CAR-expressing T cells, 2.5 x 10⁶ to 5 x 10⁷ total CAR-expressing T cells, 2.5 x 10⁶ to 2.5 x 10⁷ total CAR-expressing T cells, 2.5 x 10⁶ to 1 x 10⁷ total CAR-expressing T cells, 2.5 x 10⁶ to 5 x 10⁶ total CAR-expressing T cells, 5 x 10⁶ to 5 x 10⁸ total CAR-expressing T cells, 5 x 10⁶ to 2.5 x 10⁸ total CAR-expressing T cells, 5 x 10⁶ to 1 x 10⁸ total CAR-expressing T cells, 5 x 10⁶ to 5 x 10⁷ total CAR-expressing T cells, 5 x 10⁶ to 2.5 x 10⁷ total CAR-expressing T cells, 5 x 10⁶ to 1 x 10⁷ total CAR-expressing T cells, 1 x 10⁷ to 5 x 10⁸ total CAR-expressing T cells, 1 x 10⁷ to 2.5 x 10⁸ total CAR-expressing T cells, 1 x 10⁷ to 1 x 10⁸ total CAR-expressing T cells, 1 x 10⁷ to 5 x 10⁷ total CAR-expressing T cells, 1 x 10⁷ to 2.5 x 10⁷ total CAR-expressing T cells, 2.5 x 10⁷ to 5 x 10⁸ total CAR-expressing T cells, 2.5 x 10⁷ to 2.5 x 10⁸ total CAR-expressing T cells, 2.5 x 10⁷ to 1 x 10⁸ total CAR-expressing T cells, 2.5 x 10⁷ to 5 x 10⁷ total CAR-expressing T cells, 5 x 10⁷ to 5 x 10⁸ total CAR-expressing T cells, 5 x 10⁷ to 2.5 x 10⁸ total CAR-expressing T cells, 5 x 10⁷ to 1 x 10⁸ total CAR-expressing T cells, 1 x 10⁸ to 5 x 10⁸ total CAR-expressing T cells, 1 x 10⁸ to 2.5 x 10⁸ total CAR-expressing T cells, or 2.5 x 10⁸ to 5 x 10⁸ total CAR-expressing T cells.

In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agents include a cytokine, such as IL-2, for example, to enhance persistence. In some embodiments, the methods comprise administration of a chemotherapeutic agent.

In some embodiments, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some aspects can improve the effects of adoptive cell therapy (ACT).

Thus, in some embodiments, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some embodiments, the subject is administered a preconditioning agent no more than 7 days prior, such as no more than 6, 5, 4, 3, or 2 days prior, to the initiation of the cell therapy.

In some embodiments, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some embodiments, the cyclophosphamide can be administered in a single dose or can be administered in a plurality of doses, such as given daily, every other day or every three days. In some embodiments, the cyclophosphamide is administered once daily for one or two days. In some embodiments, where the lymphodepleting agent comprises cyclophosphamide, the subject is administered cyclophosphamide at a dose between or between about 100 mg/m² and 500 mg/m², such as between or between about 200 mg/m² and 400 mg/m², or 250 mg/m² and 350 mg/m², inclusive. In some instances, the subject is administered about 300 mg/m² of cyclophosphamide. In some embodiments, the cyclophosphamide can be administered in a single dose or can be administered in a plurality of doses, such as given daily, every other day or every three days. In some embodiments, cyclophosphamide is administered daily, such as for 1-5 days, for example, for 3 to 5 days. In some instances, the subject is administered about 300 mg/m² of cyclophosphamide, daily for 3 days, prior to initiation of the cell therapy.

In some embodiments, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 40 mg/m², or 24 mg/m² and 35 mg/m², inclusive. In some instances, the subject is administered about 30 mg/m² of fludarabine. In some embodiments, the fludarabine can be administered in a single dose or can be administered in a plurality of doses, such as given daily, every other day or every three days. In some embodiments, fludarabine is administered daily, such as for 1-5 days, for example, for 3 to 5 days. In some instances, the subject is administered about 30 mg/m² of fludarabine, daily for 3 days, prior to initiation of the cell therapy.

In some embodiments, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (~2 g/m²) of cyclophosphamide and 3 to 5 doses of 25 mg/m² fludarabine prior to the first or subsequent dose.

Following administration of the cells, the biological activity of the engineered cell populations in some embodiments is measured. e.g., by any of a number of known methods. Parameters to assess include specific binding of an engineered or natural T cell or other immune cell to antigen, in vivo, e.g., by imaging, or ex vivo, e.g., by ELISA or flow cytometry. In certain embodiments, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells is measured by assaying expression and/or secretion of one or more cytokines, such as CD107a, IFNγ, IL-2, and TNF. In some aspects the biological activity is measured by assessing clinical outcome, such as reduction in tumor burden or load.

In certain embodiments, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616.

In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agent includes a cytokine, such as IL-2, for example, to enhance persistence.

### V. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known. Reference to "about" a value or parameter herein includes (and describes) embodiments that are directed to that value or parameter per se. For example, description referring to "about X" includes description of "X".

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, a composition refers to any mixture of two or more products, substances, or compounds, including cells. It may be a solution, a suspension, liquid, powder, a paste, aqueous, non-aqueous or any combination thereof.

As used herein, a "subject" is a mammal, such as a human or other animal, and typically is human.

### VI. EXAMPLES

The following examples are included for illustrative purposes only and are not intended to limit the scope of the invention.

### Example 1: Methods for preparing an anti-CD3/anti-CD28 Fab conjugated oligomeric reagent comprising a streptavidin mutein.

An oligomeric reagent was prepared by polymerizing an exemplary streptavidin mutein designated STREP-TACTIN^{®} M2 (a streptavidin homo-tetramer containing the mutein sequence of amino acids set forth in SEQ ID NO:6, see e.g. U.S. Patent No. 6,103,493 and Voss and Skerra (1997) Protein Eng., 1:975-982, and Argarana et al. (1986) Nucleic Acids Research, 1871-1882). To prepare streptavidin muteins for oligomerization, streptavidin muteins containing one or more reactive thiol groups were incubated with maleimide activated streptavidin muteins. To prepare the thiolated streptavidin mutein, about 100 mg of streptavidin mutein was thiolated by incubation with 2-iminothiolane hydrochloride at a molar ratio of 1:100 at a pH of about 8.5 at 24°C for 1 hour in 100 mM Borate buffer in a total volume of 2.6 mL. For the activation reaction, about 400 mg of streptavidin mutein was incubated with Succinimidyl-6-[(β-maleimidopropionamido) hexanoate (SMPH) at a molar ratio of 1:2 at a pH of about 7.2 at 24°C for 1 hour in a total volume of about 10.4 mL in a sodium phosphate buffer. The thiolation and activation reactions were coordinated to start at about the same time, and the duration of the reactions was controlled

After the reactions, the 2-Iminothiolane hydrochloride and SMPH were removed from the samples by individually carrying out gel filtration of the samples with PD-10 desalting columns (GE Healthcare). For each 2.5 mL volume of sample, a 1 mL PD-10 column was equilibrated and loaded with either thiolated mutein streptavidin or maleimide mutein streptavidin and elution was carried out by adding 3.5 mL of coupling buffer (100 mM NaH₂P)₄, 150 mM NaCl, 5 mM EDTA, pH 7.2). Gel filtration of the maleimide mutein streptavidin was carried out on 4 columns to account for the > 10 mL volume and eluates were pooled. The timing of the activation and thiolation reactions and the timing between the end of the activation and thiolation reactions and the start of the oligomerization reactions were carefully controlled. Generally, no more than ten minutes was allowed to pass from the start of gel filtrations, i.e. the end of the activation and thiolation reactions, to when oligomerization reaction was initiated.

For oligomerization, the maleimide streptavidin mutein and thiolated streptavidin mutein samples were then combined into an overall volume of about 17.5 mL and incubated for 1 hour at a pH of 7.2 at 24°C under stirring conditions at about 600 rpm. Because four times more streptavidin mutein was incubated with SMPH than with 2-iminothiolane hydrochloride, the molar ratio of thiolated streptavidin mutein and maleimide streptavidin mutein was 1:4 during the oligomerization reaction. After the reaction, remaining SH groups of the oligomerized streptavidin mutein reagent were saturated by incubation with N-Ethylmaleimide (NEM)for 15 min at 24°C with stirring (about 600 rpm) followed by incubation for a further 16-20 hours at 4°C.

After incubation with NEM, the sample containing oligomerized streptavidin mutein was centrifuged and the supernatant was filtered through a 0.45 µm membrane (Millex-HP 0.45 µm from Merck Millopore). The filtered solution was then loaded into a column (Sephacryl S-300 HR HiPrep 26/60, GE Healthcare) for size exclusion chromatography (SEC) with an AKTA Explorer chromatography system (GE Healthcare). Fractions with a milli absorbance unit (mAU) greater than or equal to 1500 mAU were pooled.

The pooled sample containing oligomeric streptavidin mutein was treated with 100 mM hydroxylamine at a pH of 6.35 for 15 minutes at room temperature. To remove the hydroxylamine after treatment, sample was loaded onto a PD10 column (2.5 mL per column) and eluted with 3.5 mL of buffer containing 100 mM NaH₂PO₄, 140 mM NaCl, 1 mM EDTA, pH 7.2. The PDI10 elutes were pooled and sterile filtered with a 0.45 µm filter followed by a 0.22 µm filter and then samples were frozen and stored at -80°C. Prior to freezing, the final concentration of the oligomeric streptavidin mutein reagent was measured and the size of the oligomeric streptavidin mutein reagent was determined by dynamic light scattering (DLS).

To evaluate the consistency of the oligomerization process, 10 oligomeric streptavidin mutein reagents were prepared using the methods described above from five different lots of streptavidin mutein (SAM). The average size, percent yield (determined by measuring absorbance at 280 nm without baseline correction), and activity (biotin binding) of the oligomers were assessed and the results are shown in Table E1. The results indicated that the resulting oligomeric streptavidin mutein reagents were consistent in these parameters with an average radius of 97 nm ± 10 nm and biotin binding of 40 nmol/mg ± 3 nmol/mg.

**Table E1: Comparison of oligomerized STREP-TACTIN from different batches.**

| | SAM lot | Radius (nm) | Yield(%) | Biotin Binding (nmol/mg) |
|---|---|---|---|---|
| Batch 1 | 1 | 92 | 74 | 41 |
| Batch 2 | 2 | 100 | 68 | 40 |
| Batch 3 | 2 | 106 | 82 | 37 |
| Batch 4 | 2 | 94 | 73 | 39 |
| Batch 5 | 3 | 87 | 79 | 41 |
| Batch 6 | 3 | 90 | 81 | 39 |
| Batch 7 | 4 | 97 | 84 | 43 |
| Batch 8 | 4 | 97 | 76 | 43 |
| Batch 9 | 5 | 102 | 85 | 42 |
| Batch 10 | 5 | 87 | 63 | 42 |

The average molecular weight (MW) of three oligomeric streptavidin mutein reagents generated as described above was measured by asymmetrical flow field-flow fractionation (AF4) performed with an HPLC system (AGILENT 1100 and Wyatt ECLIPSE DUALTEC) with UV detection (Agilent UV detector coupled with MALLS DAWN HELEOS (Wyatt)). The measurements by AF4 allowed for the calculation of the average number of streptavidin mutein tetramers in each oligomeric reagent assuming the average molecular weight of a streptavidin mutein tetramer of 52,500 g/mol (52.5 kDa) (Table E2).

**Table E2: Size and Molecular Weight of oligomeric streptavidin mutein reagents**

| Radius (nm) | MW (g/mol) | Number of Tetramers |
|---|---|---|
| 102 | 1.65x10⁸ | 3150 |
| 82 | 1.08 x10⁸ | 2050 |
| 92 | 1.26 x10⁸ | 2280 |

Stimulatory agents (anti-CD3 and anti-CD28 Fab fragments) were multimerized by reversible binding to oligomeric streptavidin mutein reagent generated as described above. Anti-CD3 and anti-CD28 Fab fragments were reversibly bound to the streptavidin mutein oligomer via a streptavidin peptide-binding partner fused to each Fab fragment. The anti-CD3 Fab fragment was derived from the CD3 binding monoclonal antibody produced by the hybridoma cell line OKT3 (ATCC^{®} CRL-8001^{™}; see also U.S. Patent No. 4,361,549), and contained the heavy chain variable domain and light chain variable domain of the anti-CD3 antibody OKT3 described in Arakawa et al J. Biochem. 120, 657-662 (1996). These sequences are set forth in SEQ ID NOS: 31 and 32, respectively. The anti-CD28 Fab fragment was derived from antibody CD28.3 (deposited as a synthetic single chain Fv construct under GenBank Accession No. AF451974.1; see also Vanhove et al., BLOOD, 15 July 2003, Vol. 102, No. 2, pages 564-570) and contained the heavy and light chain variable domains of the anti-CD28 antibody CD28.3 set forth in SEQ ID NOS: 33 and 34, respectively. The Fab fragments were individually fused at the carboxy-terminus of their heavy chain to a streptavidin peptide-binding sequence containing a sequential arrangement of two streptavidin binding modules having the sequence of amino acids SAWSHPQFEKGGGSGGGSGGSAWSHPQFEK (SEQ ID NO: 16). The peptide-tagged Fab fragments were recombinantly produced (see International Patent App. Pub. Nos. WO 2013/011011 and WO 2013/124474).

To effect reversible binding, peptide-tagged anti-CD3 and anti-CD28 Fab fragments were mixed with the oligomeric streptavidin mutein reagent at approximately room temperature, thereby reversibly binding them to the reagent via interaction between twin-strep-tags on the Fab fragments, which were binding partners capable of reversibly binding to binding sites on the reagent. In some cases, the peptide-tagged Fab fragments were premixed prior to immobilization onto the oligomeric streptavidin mutein reagent, which, in some instances, can result in a more uniform distribution of the different Fab molecules. Binding of the peptide-tagged anti-CD3 and anti-CD28 to the oligomeric streptavidin mutein reagent can be disrupted, or reversed, by addition of D-biotin. D-biotin competes with the strep-tag on the agents for binding to the binding partner on the streptavidin mutein, thereby disrupting binding.

### Example 2: Activity Assessment of oligomerized anti-CD3 and anti-CD28 Fab fragments reversibly bound to streptavidin mutein oligomers.

Anti-CD3 and anti-CD28 Fab fragments, reversibly bound to various oligomeric streptavidin reagents from each of the batches described in Table E1 by the process described in Example 1, were assessed for the ability to stimulate T cells. These oligomeric streptavidin reagents had an average radius of about 95 nm. Metabolic activity of cells as an indicator of cell proliferation was assessed by colorimetric monitoring of cleavage of the stable tetrazolium salt WST-1 to a soluble formazan dye complex.

T cells, from three different donors, were incubated with the anti-CD3/anti-CD28 multimerized Fab fragments reversibly bound on an oligomeric streptavidin reagent. Cells were also incubated with control oligomeric reagents that had either an average radius of 101 (internal reference) or 36 nm, which also were reversibly bound to anti-CD3/anti-CD28 Fab fragments.

After the incubation, WST-1 reagent was applied to the cells and the levels of metabolic activity were assessed by measuring the absorbance at 450 nm as a readout. The results were normalized to the number of cells in the culture being assayed and depicted as the ratio of WST-1 per cell number.

As shown in **FIG. 3B****,** mean WST-1 activity (WST ratio) of T cells stimulated with each of the tested reagents were comparable. Moreover, the degree of stimulation was similar for all tested reagents and was comparable to a similarly sized internal reference reagent (varying generally within ±2 standard deviations). **FIG. 3A** shows the WST-1 activity (WST ratio) depicted as a separate data point for each reagent. **FIG. 3A** and **FIG. 3B** indicate that stimulation of T cells, as observed by WST-1 activity, was lower using anti-CD3/anti-CD28 Fabs multimerized on a smaller 36 nm oligomeric streptavidin mutein reagent backbone.

### Example 3: Selection and stimulation of T cells via column chromatography.

On-column T cell selection and stimulation were performed via column chromatography, and the selected and stimulated T cells were collected, as detailed below. An exemplary process of on-column T cell selection and stimulation is shown in **FIG. 4****.**

### A. Column preparation.

Sephadex^{®} G-50 (Sigma) was used as a stationary phase and was covalently coupled with Strep-tactin^{®} M2 (SEQ ID NO: 6) using a cyanogen bromide (CNBr) method for column activation. A 50% suspension of the Sephadex^{®} G-50 resin contained approximately 70 µg of covalently coupled Strep-tactin^{®} per mL of the resin bead suspension.

Following immobilization of Strep-tactin^{®} onto the Sephadex^{®} G-50 resin, 2 mL of the suspension of Sephadex^{®} G-50 with immobilized Strep-tactin^{®} was incubated with 10 µg of a selection agent specific to a T cell surface selection marker for 20 min at 4°C. The selection agent comprised an anti-CD3 Fab fragment. The CD3 binding Fab fragment was derived from the CD3 binding monoclonal antibody produced by the hybridoma cell line OKT3 (ATCC^{®} CRL-8001^{™}; *see also* U.S. Patent No. 4,361,549), and contained the heavy chain variable domain (SEQ ID NO: 31) and light chain variable domain (SEQ ID NO: 32) of the anti-CD3 antibody OKT3 described in Arakawa et al., J. Biochem. 120, 657-662 (1996). In this example, the heavy chain of the CD3 binding Fab fragment was carboxy-terminally fused with a Twin Strep-Tag^{®} (SAWSHPQFEK(GGGS)₂GGSA WSHPQFEK, SEQ ID NO: 16) containing a sequential arrangement of two streptavidin binding modules, which facilitated binding of the CD3 binding Fab fragment to the immobilized Strep-tactin^{®} on the resin.

The resin suspension was then filled into a heating/gas column with a 90 micrometer frit at the bottom. The heating/gas column used in this example differed from a reference column in that the heating/gas column comprised a heating element and a gas supply element. The heating element comprised a heating coil with inlet and outlet for external warm water supply, and the gas supply element comprised a gas supply connector for screw-on air filters (*see e.g.* **FIG. 1A** and **FIG. 1B**). The heating/gas column was equilibrated with PBS (phosphate buffered saline) containing 0.5% bovine serum albumin (PBSA buffer) to give a bed volume of 1 mL. The heating/gas column was used for T cell selection and stimulation, prior to their collection from the column as described below.

### B. On-Column Selection and Stimulation

An apheresis sample from a human donor was loaded onto the affinity column, and CD3+ T cells in the sample were allowed to interact with the CD3 binding Fab fragment immobilized on the resin. The column was washed two times with PBSA buffer to remove cells that did not intereact with the immobilized CD3 binding Fab fragment. After approximately 15 minutes from the time of loading the sample onto the column, a stimulatory reagent was added to the column by loading onto the column at least 400 µg of an anti-CD3/anti-CD28 oligomeric reagent (multimerized anti-CD3 Fab fragments and anti-CD28 Fab fragment stimulatory agents reversibly bound to an oligomeric streptavidin mutein reagent), described in Examples 1 and 2 above, in 3 mL of serum free basal media containing glutamine and recombinant IL-2, IL-15, and IL-7, and the cells were incubated on the column under conditions to stimulate the cells. During the incubation, the heating element of the column maintained the temperature of the water passing through the heating coil to a temperature at about 37°C, and the gas exchange element supplied air under control of open sterile filter which were connected during the incubation.

After the addition of the oligomeric reagent to the column, column content was heated up to 37°C and the temperature was maintained during full activation process. Temperature within the column was constantly monitored by a temperature sensor. Gas exchenge was continued throughout the complete selection procedure using a serile filter as a passive element.

During the incubation with the anti-CD3/anti-CD28 oligomeric reagent, the selected CD3+ T cells spontaneously detached from the column by activation induced detachment (AID) without the need to add a competition reagent to disrupt the binding between the cells and the resin. Within approximately 4.5 hours after adding the stimulatory reagent, spontaneously detached cells were collected from the column in a single step by passing through the column approximately 80 mL of serum free basal media containing glutamine and recombinant IL-2, IL-15, and IL-7, and the cells were collected by gravity flow. No further competition substance was added to the column to disrupt the binding of cells on the column prior to their collection by gravity flow.

The number of collected CD3+ T cells after on-column selection and stimulation using the heat/gas column was determined and compared to a theoretical estimate, a control group, and those collected by a similar process but using the reference column that did not contain a heating element and gas supply element (i.e. the cells were incubated in the reference column at room temperature, without air exchange between the cells and the outside invironment). In the control group, an apheresis sample was loaded onto the standard anti-CD3 affinity column for selection of CD3+ T cells, but the cells were not incubated in the presence of the anti-CD3/anti-CD28 oligomeric stimulatory reagent; after 4.5 hours, D-biotin was added to disrupt binding between the Twin Strep-Tag^{®} of the anti-CD3 Fab and the Strep-tactin^{®} on the resin, thereby releasing the selected CD3+ T cells from the column resin.

The results are shown in **FIG. 5****,** where the estimate (grey bar) was the theoretical number of captured cells that could be eluted assuming 100% efficiency. As shown in **FIG. 5****,** elution efficiency using the heat/gas column having a heating element and a gas supply element was approximately two-fold of that using the reference column. The results in **FIG. 5** indicate that the heat/gas column with the heating element and the gas supply element supports on-column T cell selection and substantially more efficient collection of stimulated T cells from the resin, as compared to the reference column. In addition, the heat/gas column achieves similar T cell recovery as compared to the control group, but without the need of an additional competition substance to disrupt binding. Thus, the heat/gas column can be used to significantly reduce time required for T cell selection and/or stimulation, while maintaining a high recovery rate of the selected and stimulated T cells.

### Example 4: Analysis of T cells during and after selection and stimulation via column chromatography.

On-column T cell selection and stimulation were preformed as described in Example 3, using a column having a heating element and a gas supply element. An apheresis sample from a human donor, as the starting material, was loaded onto the affinity column with CD3 Fab-loaded resin, prior to the addition of the anti-CD3/anti-CD28 oligomeric stimulatory reagent substantially as described in Example 3. The selected and stimulated cells were collected into a culture bag under gravity flow, without the addition of a competition agent as described in Example 3.

Cells in the starting material, the negative fraction (unbound cells that were washed prior to loading the stimulatory reagent), or the positive fraction (cells collected by the process described in Example 3) were analyzed. The cells were stained with antibodies recognizing surface markers including CD3, CD4, CD8, CD45 and CD14 and quantified by flow cytometry. Debris was excluded and the samples were pre-gated on CD45+ live cells.

The flow cytometry results are shown in **FIG. 6****,** where about 64% of all CD3+ cells in the starting sample were enriched and present in the positive fraction, and the remaining cells were in the negative fraction. Of the cells in the positive fraction, greater than 94% of the cells were CD4+ and CD8+ T cells.

The cells collected into the culture bag were further incubated at 37°C in serum free basal media containing glutamine and recombinant IL-2, IL-15, and IL-7 for up to 3 days. During the incubation, the oligomeric stimulatory reagent was not removed; however, no additional stimulatory reagent was added.

The cells were monitored, at Day 1, Day 3, and Day 3 during the subsequent incubation, for cell number and cell surface expression by flow cytometry after staining the cells with antibodies recognizing CD3, CD4, CD8, and the activation markers CD69 and CD25. As shown in **FIG. 7A****,** 98.8% of the cultured cells were CD3+ at Day 3, and surface expression of activation markers CD25 and CD69 was also observed in the majority of the T cells. The purity of CD3+ cells increased at day 3 compared to at the time of harvest from the column, while the ratio of CD4:CD8 T cells was maintained. Assessment for cell number and fold-expansion following the subsequent incubation showed that at Day 3, the selected and stimulated T cells expressed activation markers and had started to increase in number consistent with the ability of the cells to proliferate (**FIG. 7B**). These results demonstrate that T cells collected directly from the column following selection and stimulation by the on-column process described in Example 3 exhibit features supporting their continued proliferation in culture.

### Example 5: On-column selection of T cells from a cryopreserved apheresis starting sample.

On-column T cell selection was performed essentially as described in Example 3, except using a cryopreserved apheresis sample as the starting sample. Cryopreserved apheresis samples generally have high monocyte content (greater than 20%). Results were compared to a process carried out on a fresh apheresis sample. The monocyte contents (% of live CD45+ cells) of cryopreserved apheresis samples and fresh apheresis samples are shown in **FIG. 8A** for a plurality of different donors.

The starting material and enriched cells in the positive fraction that were collected from the column were stained with antibodies recognizing CD3 and CD14 (monocyte marker) and quantified by flow cytometry. **FIG. 8B** depicts the percentage of cells positive for CD3 or CD14 in the starting material and positive fraction. As shown, although monocyte content in the starting material was high, the positive fraction resulted in 94% purity of CD3+ T cells with only 1% CD14+ monocyte detected, representing a drop in monocyte: T cell ratio from 0.33 to 0.01. The numbers of T cells selected using the chromatography column are shown in **FIG. 8C****.**

### Example 6: Selection and stimulation of T cells using sequential or parallel columns.

On-column T cell selection and stimulation were performed essentially as described in Example 3 using a column having a heating element and a gas supply element, in which was immobilized anti-CD3 Fab fragment for selection of cells. In one study, selection and stimulation was carried out in a system involving two identical columns that were arranged sequentially, while in another study the selection and stimulation was carried out in a system with two identical columns that were arranged in parallel (see **FIG. 9**). In each study, approximately 1/5 of an apheresis sample from the same donor were loaded onto each system of columns as the starting material.

The starting material, the negative fraction, and the positive fraction were stained with antibodies recognizing surface markers including CD3, CD4, CD8, and CD14 and quantified by flow cytometry. The flow cytometry results are shown in **FIG. 10A****.** As shown, similar enrichment of CD3+ T cells was achieved by either strategy.

Cells from the positive fraction (cells collected by the process described in Example 3 except using parallel or sequential columns) were harvested into a culture bag, and the collected cells from each study were incubated at 37° C in serum free basal media containing glutamine and recombinant IL-2, IL-15, and IL-7 for up to 6 days. During the incubation, the oligomeric stimulatory reagent was not removed; however, no additional anti-CD3/anti-CD28 stimulatory reagent was added.

The cells were monitored at Day 0, Day 1, Day 2, Day 3, and Day 6 during the incubation, for cell number and cell surface expression by flow cytometry after staining the cells with antibodies recognizing CD3, CD4. CD8, and the activation markers CD69 and CD25. As shown in **FIG. 10B****,** left panel, the cells from each chromatography study exhibited similar expression of activation markers at Day 1, and cell numbers similarly started to increase during the incubation. Representative results for cell number in Run 1 (■) and Run 2 (●) are shown in **FIG. 10B****,** right panel.

These results indicate that sequential or parallel column chromatography may be used for on-column selection and stimulation of T cells.

### Example 7: On-column selection and stimulation of T cells from concentrated blood.

On-column T cell selection and stimultion was preformed essentially as described in Example 3, except using a concentrated blood sample as the starting sample. Further, a parallel CD3 selection and stimulation was carried out using a system substantially as described in Example 6. Lightly concentrated whole blood with reduced content of thrombocytes and serum was pre-diluted 1:1 in PBSA buffer. No erythrocute lysis was performed. 160 mL (80 mL per column) of the diluted sample was loaded onto two parallel columns each containing 4 mL of CD3 Fab-loaded resin, the columns were washed and then the anti-CD3/anti-CD28 oligomeric stimulatory reagent was added to each column.

Cells in the starting material, the negative fraction, and the positive fraction from the CD3+ T cell selection were stained with antibodies recognizing surface markers including CD45, CD3, CD4, CD8, and CD14 and quantified by flow cytometry. The flow cytometry results are shown in **FIG. 11A****.** As shown, cells in the positive fraction (cells collected by the process described in Example 3 using two parallel columns) showed a high enrichment of CD3+ T cells with a purity of CD3+ T cells of greater than 93%.

Cells from the positive fraction (cells collected by the process described in Example 3 except using parallel columns) were harvested into a culture bag, and were incubated at 37°C in serum free basal media containing glutamine and recombinant IL-2, IL-15, and IL-7 for up to 6 days. During the incubation, the oligomeric stimulatory reagent was not removed; however, no additional anti-CD3/anti-CD28 stimulatory reagent was added. The cells were monitored at 72 hours following the intiation of the subsequent incubation for cell surface expression of CD4 and CD8 and the activation markers CD69 and CD25. As shown in **FIG. 11A****,** the resulting cells maintained the ratio of CD4 and CD8 T cells present in the positive fraction prior to the subsequent incubation (compare positive fraction of **FIG. 11A** and **FIG. 11B****,** left panel), and a majority of the cells were positive for activation makers CD25 and/or CD69 (**FIG. 11B****,** right panel).

### Example 8: Selection of T cells directly from whole blood.

An agarose resin (100µl resin) was functionalized with Strep-tactin^{®} for immobililzation of an anti-CD3 Fab fragment carboxy-terminally fused with a Twin Strep-Tag^{®} (SAWSHPQFEK(GGGS)₂GGSAWSHPQFEK, SEQ ID NO: 16), by a process similar to described in Example 3 using Sephadex^{®} G-50 as the resin. 1 mL of fresh undiluted blood draw was loaded onto the column. No erythrocyte lysis was performed. The column was washed and the negative fraction was collected. D-biotin was added to disrupt binding between the Twin Strep-Tag^{®} of the anti-CD3 Fab and the Strep-tactin^{®} on the resin, thereby releasing the selected CD3+ T cells from the column resin in the positive fraction.

The starting material, the negative fractions, and the positive fractions from the CD3+ T cell selection were stained with propidium iodine (PI) and a CD3 antibody and quantified by flow cytometry. The flow cytometry results are shown in **FIG. 12****.** The positive fractions contained >80% CD3+ cells, as compared to 0.182% CD3+ cells in the fresh undiluted whole blood. This result supports the feasibility of on-column T cell selection directly from fresh blood.

The results indicate that the on-column T cell selection performs well with whole blood samples.

### Example 9: Selection and stimulation of T cells via column chromatography.

A study was carried out to enrich T cells by column-based affinity chromatography with on-column stimulation in the presence of an anti-CD3/anti-CD28 oligomeric stimulatory agent.

In this study, a Sephadex G50 (Sigma) was used as stationary phase and was covalently coupled with STREP-TACTIN^{®} M2 (SEQ ID NO: 6) using a cyanogen bromide (CNBr) activated resin. A 50% suspension of Sephadex G50 contained approximately 70 µg of covalently coupled 7 Strep-tactin^{®}/mL of the bead suspension. Following immobilization of STREP-TACTIN^{®} onto the stationary phase, two mL of the suspension of Sephadex G50 with Strep-tactin^{®} was incubated with 10 µg of a selection agent specific to a T cell surface selection marker for 20 min at 4° C in order to allow binding of the Fab fragment to the immobilized Strep-tactin^{®} reagent. The suspension was then filled in a plastic minicolumn with a 90 micrometer frit at the bottom. The column was equilibrated with PBS (phosphate buffered saline) containing 0.5% bovine serum albumin (PBSA buffer) to give a bed volume of 1 mL.

In these studies, experiments were carried out using as the selection agent either an anti-CD3 binding Fab fragment, an anti-CD4 binding Fab fragment, or an anti-CD8 binding Fab fragment as the selection agent. The CD3 binding Fab fragment was derived from the CD3 binding monoclonal antibody produced by the hybridoma cell line OKT3 (ATCC^{®} CRL-8001^{™}; see also U.S. Patent No. 4,361,549), and contained the heavy chain variable domain (SEQ ID NO:31) and light chain variable domain (SEQ ID NO:32) of the anti-CD3 antibody OKT3 described in Arakawa et al J. Biochem. 120, 657-662 (1996). The CD4 binding Fab fragment was obtained from m13B8.2 (e.g. US Patent 7,482,000) and contained the heavy chain variable domain (SEQ ID NO:29) and light chain variable domain (SEQ ID NO:30) of the anti-CD4 antibody m13B8.2. The CD8 binding Fab fragment was obtained from OKT8 (e.g. ATCC CRL-8014) and contained the heavy chain variable domain (SEQ ID NO:9) and light chain variable domain (SEQ ID NO:10) of the anti-CD8 antibody OKT8. The heavy chain of each of the Fab fragment was carboxy-terminally fused with a Twin Strep-Tag^{®} (SEQ ID NO:16) containing a sequential arrangement of two streptavidin binding modules.

An apheresis sample from a human donor was loaded onto the affinity column and two wash steps performed After more than 30 minutes passed from the time of loading the sample, 40 µg of multimerized anti-CD3 anti-CD28 Fab fragments reversibly bound to an oligomeric streptavidin mutein reagent (anti-CD3/anti-CD28 oligomeric reagent), generated as described in Example 1, was loaded onto the column in 3 mL of serum free basal media containing glutamine and recombinant IL-2, IL-15, and IL-7 and incubated at 37 °C. After approximately 24 hours, cells were collected from the column in a single step by passing approximately 80 mL of serum free basal media containing glutamine and recombinant IL-2, IL-15, and IL-7 , without the addition of a further competition substance to disrupt the binding, through the column. As a control, an apheresis sample was loaded onto the anti-CD3 affinity column but without incubation in the presence of the anti-CD3/anti-CD28 oligomeric stimulatory reagent. For the control condition, after 24 hours D-biotin was added to disrupt binding between the Twin Strep-Tag^{®} of the anti-CD3 Fab and the STREP-TACTIN^{®}, and released cells were collected by gravity flow.

Approximately 24 hours after the initiation of stimulation with the anti-CD3/anti-CD28 oligomeric reagent, collected cells were analyzed for surface expression of the selection marker. As shown in **FIG. 13****,** downregulation of CD3, CD4, and CD8 was observed at 24 hours following on-column selection using the respective selection agent specific to the selection marker, and incubation with the stimulatory reagent. In contrast, for the control condition in which cells were not incubated with the oligomeric stimulatory reagent, receptor downregulation of the selection marker was not observed. These results are consistent with an observation that stimulation of the T cells with an anti-CD3/anti-CD8 stimulatory reagent resulted in downregulation of surface expression of the receptor, thereby permitting spontaneous detachment of the cells from the column without the need to add a competition reagent.

A similar study was carried out in which T cells were selected on an anti-CD3 STREP-TACTIN^{®} affinity column and subjected to on-column stimulation with an anti-CD3/anti-CD28 oligomeric stimulatory reagent as described above. Cells were collected by gravity flow at various times after addition of the oligomeric stimulatory reagent. Collected cells were immediately stained with an antibody against alpha-beta TCR chains and monitored by flow cytometry to detect surface expression of CD3. FIG. 14 illustrates exemplary kinetics of CD3/TCR complex downregulation and re-expression following the initiation of the stimulation in the presence of the anti-CD3/anti-CD28 oligomeric stimulatory reagent. As shown, a rapid downregulation of CD3/TCR complex surface expression was observed during the first 24 hours of stimulation, with a maximum reduction in CD3/TCR complex surface expression observed after only 12 hours. Incubation in the presence of the oligomeric stimulatory reagent for longer than 36 hours resulted in re-expression of surface CD3/TCR complex with maximal CD3/TCR complex surface re-expression achieved within about 72 hours after the initiation of the stimulation with the stimulatory reagent. These results support that substantial on-column stimulation-mediated spontaneous T cell detachment can occur within 4 to 6 hours, with maximal release occurring at or approximately at 12 hours, after initiation of the on-column incubation with the stimulatory reagent.

Cells that were spontaneously detached at about 24 hours after adding the anti-CD3/anti-CD28 oligomeric stimulatory reagent were cultured at 37° C in serum free basal media containing glutamine and recombinant IL-2, IL-15, and IL-7 for up to 9 days. During the incubation, the oligomeric stimulatory reagent was not removed; however, it was diluted as the cells continued to expand during the incubation. The cells were monitored, at 24 hours and at 5 days during the subsequent incubation, for size and expression of CD3 and activation markers CD69 and CD25. As shown in **FIG. 15A****,** further incubation for up to 5 days resulted in re-expression of CD3 following CD3 early downregulation observed at 24 hours. In addition, an increase in cell size (left panels of **FIG. 15A****)** and surface expression of activation markers CD25 and CD69 also was observed at 5 days compared to at the 24 hour time point. Assessment for cell number and fold-expansion following the subsequent incubation for up to 9 days showed that spontaneously detached cells demonstrated high proliferative capacity **(****FIG. 15B****).** These results are consistent with an observation that T cells that have undergone on-column selection and short-term stimulation are able to be further incubated to attain a desired stimulation, activation and/or expansion.

These results support the use of on-column selection and stimulation as a rapid means (e.g., less than 24 hours) of isolating and stimulating target cells prior to, or in connection with, downstream processes (e.g. transduction) for producing an engineered T cell composition.

### Example 10: Assessment of T cell Phenotype and Function in Engineered T cells Produced in the Presence of a Small Molecule mTOR Kinase Inhibitor.

CD4+ and CD8+ T cells were isolated by immunoaffinity-based enrichment from leukapheresis samples from human donor subjects. At day 1, isolated CD4+ and CD8+ T cells were mixed 1:1 and stimulated with an anti-CD3/anti-CD28 oligomeric stimulatory reagent generated as described in Example 1 in serum-free media supplemented with recombinant IL-2 (100 IU/mL), recombinant IL-7 (600 IU/mL), and recombinant IL-15 (100 IU/mL) with or without 1 µM of 2-(3-hydroxyphenyl)-9-(2-isopropylphenyl)-8-oxo-8,9-dihydro-7H-purine-6-carboxamide (Compound 63). The T cells cultured with our without Compound 63 were incubated overnight (approximately 24 hr) at 37 °C, and were then transduced in serum-free culture media with our without Compound 63 (1 mM) with a lentiviral vector encoding an anti-CD19 CAR. The CAR contained an scFv antigen-binding domain specific for CD19 (derived from FMC63), a CD28 transmembrane region, a 4-1BB costimulatory signaling region, and a CD3-zeta derived intracellular signaling domain. Following transduction, the cells were incubated in serum free media without recombinant cytokines (basal media) and with or without Compound 63 (1 mM) and allowed to incubate at about 37.0 °C in an incubator for up to 96 hours after initiation of the stimulation with the anti-CD3/anti-CD28 oligomeric stimulatory reagent (until day 5 of the process). At approximately 24 hours after beginning the incubation (day 3 of the process), 1 mM biotin was added. Following incubation CD4+ and CD8+ T cells from each donor were harvested, formulated, and cryofrozen.

The cryofrozen engineered CD4+ and CD8+ T cells were thawed, and T cells were assessed for intracellular S6 phosphorylation, a ribosomal protein and marker of mTOR inhibition, and co-stained for surface expression of CD4 or CD8 and for CCR7 and CD45RA as markers of memory subsets. pS6 expression in live CD8+ T cells by memory subsets was shown by the expression of CCR7 and CD45RA as shown in **FIG. 16A****.** As shown, incubation with Compound 63 decreased the mean fluorescence intensity of stimulated memory T cell subsets, Temra, Tem, and Tcm cells, indicating an inhibition of mTOR, while having no significant effect on the percentage or total number of viable cells over time. Mean fluorescence intensity (MFI) of PS6 in CD8+ T cells is shown in **FIG. 16B****.** As shown in **FIG. 16C** and **FIG. 16D****,** the presence (black lines) or absence (gray lines) of compound 63 did not impact the percent viable cells or the total live cells, respectively, in the generated composition.

Thawed cells generated by the process described were assessed for a marker of apoptosis (e.g., percentage of caspase positive CAR-T cells), phenotypic profile, and ability to produce intracellular cytokines following stimulation with PMA/Ionomycin and a Golgi Inhibitor. As shown in **FIG. 17A****,** T cells from samples incubated with compound 63 exhibited less intracellular caspase expression than those not incubated with compound 63, indicating that the presence of compound 63 during the process for generating the engineered cells improved overall cell heath of the T cell composition. Thawed cells also were stained for surface expression of CD27 and CCR7 by flow cytometry. As shown in **FIG. 17B** (CD8+ T cells) and **FIG. 17D** (CD4+ T cells), incubation with compound 63 did not substantially alter the phenotypic subset profile of the cells as assessed by expression of CD27 and/or CCR7. The functional activity of CD4+ and CD8+ T cells produced in the presence of compound 63, as evidenced by the level of intracellular cytokines IL2, IFNg, or TNF, was substantially improved in both the engineered CD8+ T cells **(****FIG. 17C****)** and CD4+ T cells **(****FIG. 17E****)** that had been produced in the presence of Compound 63.

To further assess the functional activity of the cells, the generated T cell compositions were stimulated long-term over 12 days with beads conjugated with an anti-idiotype (ID) antibody against the anti-CD19 CAR, and expansion and survival of the cells were monitored **(****FIG. 17F****,** left panel) and total expansion metric calculated by area under the growth curve **(****FIG. 17F****,** right panel, AUC). As shown, stimulation of the cells in the absence of compound 63 resulted in a decreased expansion of the cells over time consistent with chronic stimulation of the CAR and loss of sustained function following the long-term stimulation. The results show that improved function of the T cells was observed following long-term CAR-specific stimulation in engineered cells that had been produced in the presence of compound 63.

### Example 11: Assessment of phenotypic and functional properties of T cells engineered using a combined selection and on-column stimulation process.

The process combining selection and stimulation steps in a chromatography column (on-column selection and stimulation) was performed substantially as described in Example 9, but at a larger scale. The on-column selection and stimulation process was compared to an alternative process that was substantially similar but in which the stimulation step was carried out separately from selection and in solution.

### A. On-column selection and stimulation.

On day 0, an apheresis sample from a human donor was loaded onto an affinity column containing a Sephadex G50 (Sigma) stationary phase covalently coupled to StrepTactin^{®} (SEQ ID NO: 6) using a cyanogen bromide (CNBr) activated resin. The 20 mL stationary phase was capable of accommodating up to 2 billion ± 0.5 billion cells. The selection agent, an anti-CD3 binding Fab fragment as described in Example 3, was immobilized on the stationary phase through a heavy chain carboxy-terminally fused streptavidin binding peptide (Twin Strep-Tag^{®};SEQ ID NO:16) capable of binding to StrepTactin.

After approximately 60 minutes from the time of loading the sample, multimerized anti-CD3 anti-CD28 Fab fragments reversibly bound to an oligomeric streptavidin mutein reagent (anti-CD3/anti-CD28 oligomeric reagent), generated as described in Example 1, were loaded onto the column at a fixed dose of 0.2-0.3x (1-2 µg/1 million cells) in serum free media containing recombinant IL-2 (e.g. 100 IU/mL), IL-15 (e.g. 100 IU/mL), and IL-7 (e.g. 600 IU/mL) and incubated at 37 °C in the column for approximately 4.5 hours. During the incubation, the stimulation with the anti-CD3/anti-CD28 oligomeric reagent resulted in detachment or release of immobilized cells from the selection agent on the column. Released cells were eluted from the column by gravity flow with the same serum free media. The media did not include biotin or any competition substance to disrupt the binding between the StrepTactin^{®} on the stationary phase and the streptavidin binding peptide fused to the anti-CD3 antibody used to immobilize the cells on the stationary phase of the column.

The released and collected cells were then transduced to express a chimeric antigen receptor (CAR) by incubation for 1 hour in the same serum free media with a lentiviral vector encoding an exemplary anti-CD19 CAR. The exemplary CAR contained an anti-CD19 scFv derived from a murine antibody FMC63, an immunoglobulin spacer, a transmembrane domain derived from CD28, a costimulatory region derived from 4-1BB, and a CD3-zeta intracellular signaling domain. For transduction, the culture volume was adjusted to 1 x 10⁶ cells/mL.

The transduced cells were washed and then further incubated at 37 °C. About 48 hours after initiation of the on-column stimulation with the anti-CD3/anti-CD28 oligomeric reagent, 1.0 mM D-biotin was added and mixed with the cells to dissociate the anti-CD3 and anti-CD28 Fabs from the soluble oligomeric streptavidin mutein reagent.

After addition of the biotin, the cells were further incubated at 37 °C for an additional about 24 hours. The cells were then divided into two subsets. In a first subset, the cells were directly formulated with a cryoprotectant. In a second subset, the volume of the cells was adjusted to 0.5 x 10⁶ cells/mL in serum free media containing twice the concentration of IL-2, IL-7 and IL-15 as used during the incubation and transduction steps. The cells of this second subset were further incubated for expansion by cultivation for another 5 days at 37 °C in static culture with media exchange, and then were formulated with a cryprotectant.

### B. Alternative process: separate selection and stimulation (in solution).

The alternative process proceeded generally as described above, but the steps for selection and stimulation were not combined in the column. An apheresis sample from the same human donor was loaded onto an affinity column containing an anti-CD3 selection reagent as described above for selection of CD3+ T cells. To elute the selected cells, 1.0 mM D-biotin was added to the column and the eluted cells were collected. D-biotin acted as a competition substance to disrupt the binding between the streptavidin binding peptide fused to the anti-CD3 Fab and the StrepTactin^{®} on the stationary phase to release the cells from the column and the anti-CD3 Fab.

At day 0 of the alternative process, the selected cells were washed, diluted to 1 x 10⁶/mL, and stimulated by incubation with anti-CD3/anti-CD28 Fab conjugated oligomeric streptavidin mutein reagents, generated as described in Example 1, at a fixed dose (0.3x, approximately 2 µg/1 million cells). The stimulation was carried out for between about 18-30 hours (24 ± 6 hours) in serum-free media containing recombinant IL-2 (e.g. 100 IU/mL), recombinant IL-7 (e.g. 600 IU/mL), and recombinant IL-15 (e.g. 100 IU/mL).

After stimulation, the cells were transduced by spinoculation for 30 minutes in the same serum free media with a lentiviral vector encoding the same exemplary anti-CD19 CAR as described above.

After spinoculation, the cells were washed and then further incubated at 37 °C. About 48 ± 6 hours after initiation of the stimulation with the anti-CD3/anti-CD28 oligomeric reagent, 1.0 mM D-biotin was added and mixed with the cells to dissociate the anti-CD3 and anti-CD28 Fabs from the oligomeric streptavidin reagent.

After addition of the biotin, the cells were further incubated at 37 °C for an additional about 24 hours. The cells were then divided into two subsets similar to above. In a first subset, the cells were directly formulated with a cryoprotectant. In a second subset, the cells were further incubated for expansion by cultivation for a further 5 days at 37 °C in static culture with media exchange, and then were formulated with a cryprotectant.

### C. Assessment of Cell Recovery and Phenotype.

Yield of CD3+, CD4+ and CD8+ T cells that had been released from the column following on-column selection and stimulation were compared against the cells that had been released from the column in the alternative process in which only the selection was carried out on the column with the addition of biotin to release the cells. As shown in **FIG. 18A****,** the CD3+ T cell yield, as well as the CD4+ and CD8+ T cell yield, was similar in both processes. Total cell count and percentage of live cells after stimulation among cells that had been released from the column following on-column selection and stimulation were compared against cells after separate stimulation of selected cells in the alternative process **FIG. 18B** and **FIG. 18C** show the total cell counts and percentage of live cells, respectively, after stimulation for both processes. In this experiment, cell recovery was greater for the process using on-column stimulation.

For processes that included an additional 5 day cultivation, the quality and phenotype of cell populations engineered by each process were assessed on day 5 in culture (day 8 from the start of the process). **FIG. 19A** and **FIG. 19B** show the percentage of live T cells recovered and the percentage of live cells expressing the exemplary CAR (CAR+), respectively, for each process. Samples from the compositions generated from both processes after the further cultivation for 5 days (day 8 from start of process) were assessed by flow cytometry for surface expression of markers that included CD4, CD8, CD27 and CCR7. **FIG. 19C** provides a comparison of the percentage of CD4+ T cells obtained from the selection step (alternative process) or combined selection and stimulation step (on-column stimulation) against the percentage of live CD4+ T cells present in cell compositions on the last day of culture after the further cultivation (day 5).

**FIG. 19D** shows the percentage of CD27-CCR7-, CD27+CCR7-, CD27+CCR7+ and CD27-CCR7+ T cells in the compositions generated by the on-column stimulation process was similar to the percentages present in a cell composition produced by the alternative process.

### D. Assessing Function of Engineered T Cells Manufactured Using On-Column Stimulation.

The functional capabilities of engineered T cells manufactured using a process including on-column stimulation were assessed both *in vitro* and *in vivo.* The results were compared against engineered T cells manufactured using the alternative process.

### 1. In vitro Functional Analysis

Cytolytic activity was assessed by co-culturing the engineered anti-CD19 CAR T cells with HEK cells expressing CD19 (HEK CD19) at an effector to target ratio of 5:1. Cell lysis was determined by impedance measurements taken at multiple time points during culture. Control conditions included incubation of T cells expressing an alternative CAR directed against a different target (BCMA) (HEK CD19+ BCMA CAR), target cells only (HEK CD19+ only), or non-target cells incubated with anti-CD19 CAR T cells (HEK CD19-& CD19 CAR). As shown in **FIG. 20****,** cytolytic activity (as indicated by HEK cell lysis) was specific to target cells, and CAR T cells manufactured by the on-column stimulation process exhibited potent cytolytic activity that was similar to cells engineered by the alternative process. These data demonstrate that the engineered T cells manufactured using on-column stimulation have comparable potency to engineered T cells manufactured using the alternative process.

The cytokine activity of the engineered T cells was assessed by monitoring cytokine accumulation following antigen-specific stimulation with a target cell line (CD19+ HEK cells) in the presence of Golgi inhibitor. Cytokine production was assessed by flow cytometry following intracellular cytokine staining for IFNg, IL-2, and TNF-alpha in cells that were also co-stained for surface CD4, CD8 or the anti-CD19 CAR. **FIGS. 21A-21C** show the percentages of CD4+ and CD8+ T cell subsets derived from each manufacturing process that expressed IFNg, IL-2, and TNF-alpha, respectively. T cells manufactured according to the two methods, but lacking CAR expression were used as controls. These data demonstrate that CAR T cell antigen-specific cytokine production is comparable in cells produced between the manufacturing processes.

### 2. In vivo Functional Analysis

The anti-tumor activity of T cell compositions containing anti-CD19 CAR T cells generated from the on-column stimulation and alternative engineering processes were compared *in vivo.*

Immunocompromised NSG mice were injected (i.v.) with 5 x 10⁵ B cell lymphoma cell line (Raji) at day 0. On day 7, mice were injected with 0.75 x 10⁶ CAR+ T cells from CAR+ engineered compositions produced by either the on-column stimulation or alternative process. Three manufacturing runs were completed for each process from three different donors and each produced engineered CAR+ T therapeutic cell composition was tested. **FIGS. 22A-22C** show CD4:CD8 ratio, transduction efficiency, and percentage of viable cells, respectively, of each engineered therapeutic composition prior to injection. As shown, cells from both processes produced comparable engineered cells for each donor, although some donor variability was observed.

Tumor burden was measured *in vivo* by in-life luminescence imaging at different time points up to 41 days following administration of CAR-T cells. Six days following tumor injection, animals in all treatment groups showed similar tumor burden **(****FIG. 23****).** As shown in **FIG. 24****,** tumor burden was substantially reduced over time across all treatment groups. These results demonstrate comparable anti-tumor efficacy between the CAR+ engineered therapeutic T cells produced by the manufacturing processes.

### E. Conclusion

Together, these data indicate that on-column selection and stimulation can be used in a process for producing engineered T cells (e.g. CAR+ T cells), including in a process in which selected T cells are collected from the column within 4.5 hours after initiation of stimulation with anti-CD3/anti-CD28 oligomeric reagent for use in subsequent steps of the process including transduction. The results demonstrate that the on-column selection and stimulation process results in an engineered (e.g. CAR+ cells) cell composition that exhibits phenotypic and functional features that are comparable to the alternative process, yet can be carried out more efficiently and in a shorter time due to the ability to combine the selection and stimulation in a single step.

### Example 12: Selection and stimulation of T cells via column chromatography.

A study was carried out to enrich T cells by column-based affinity chromatography with on-column stimulation in the presence of an anti-CD3/anti-CD28 oligomeric stimulatory agent. This study examined whether selection using the further exemplary cell surface marker CD27 was permissive of spontaneous cell detachment as induced by on-column stimulation. This study also examined the ability to select and stimulate T cells by on-column T cell selection and stimulation using a chromatography column heated by heating elements configured outside the chromatography column elements.

In phosphate buffered saline (PBS) buffer, 10 mg of multimerized strepativin mutein (e.g. STREP-TACTIN^{®} M2, SEQ ID NO: 6) were coupled to 8 g (with respect to initial dry weight) of epoxy-activated polystytrene resin (CY17030; Cytosorbents) via solvent-exposed primary amine groups. Subsequently, 1.8 mg of an anti-CD27 Fab selection agent comprising a streptavidin binding peptide (e.g., Twin-Strep-tag^{®},SEQ ID NO: 16) carboxyl-terminally fused to the heavy chain of the Fab fragment were added to a 50% suspension (resin bed volume vs. total volume) of the STREP-TACTIN^{®}-coated resin. The suspension was incubated under gentle shaking for 1 hour at room temperature in order to allow - via the Twin-Strep-tag^{®} - reversible binding of the Fab fragment to the immobilized STREP-TACTIN^{®} M2 multimer reagent. The anti-CD27 Fab fragment functionalized resin was then added to a plastic full-scale column to give a bed volume of 18-20 mL. Prior to use, the column was equilibrated with PBS containing 0.5% bovine serum albumin (PBSA buffer). The column also included a gas supply element, specifically a gas supply connector for screw-on air filters, in order to permit the presence of air in the column.

The heating elements for the column were part of a jacket member designed to enclose the column. The jacket member included two jacket components that together were connected to surround the column, in which each jacket component contained a heating coil arranged along its interior surface. Each heating coil had an inlet and an outlet for an external warm water supply. Each jacket component with heating coil was designed to surround half of the circumference of the column from top to bottom, and the heating coil within the jacket component also similarly surrounded the half of the column. Together, the jacket components, and thus the heating coils, enclosed the column except for openings to allow the inlet and outlet of cells and reagents into and out of the column. See, e.g., **FIGS. 25-28** for exemplary schematics.

An apheresis sample from a human donor was loaded onto the affinity column and two wash steps performed. After more than 30 minutes passed from the time of loading the sample, 2000 µg of multimerized anti-CD3 Fab fragments and anti-CD28 Fab fragments reversibly bound to an oligomeric streptavidin mutein reagent (anti-CD3/anti-CD28 oligomeric reagent), generated as described in Example 1, was loaded onto the column in 20 mL of serum free basal media containing glutamine and recombinant IL-2, IL-15, and IL-7 and incubated at 37 °C via the heating coils contained in the jacket member. After approximately 4 hours, cells were collected from the column in a single step by passing approximately 80 mL of serum free basal media containing glutamine and recombinant IL-2, IL-15, and IL-7 through the column. The cells were collected without the addition of a further competition substance (e.g. without adding D-biotin) to disrupt the binding of the streptavidin-binding peptide (e.g. Twin Strep-Tag^{®}) of the anti-CD27 Fab selection agent to the streptavidin mutein (e.g. STREP-TACTIN^{®})-coated stationary phase resin. As a control, an apheresis sample was loaded onto the anti-CD27 affinity column but without incubation in the presence of the anti-CD3/anti-CD28 oligomeric stimulatory reagent. For the control condition, after the selection step, D-biotin was added as a competition substance to disrupt binding between the Twin Strep-Tag^{®} of the anti-CD3 Fab and the STREP-TACTIN^{®}, and released cells were collected by gravity flow.

Approximately 4 hours after the initiation of on-column stimulation with the anti-CD3/anti-CD28 oligomeric reagent, collected cells were analyzed for surface expression of CD27. As shown in **FIG. 32****,** downregulation of CD27 was observed in live CD45+ cells 4 hours following on-column stimulation of T cells that had been immobilized to the affinity column using the anti-CD27 selection agent. In contrast, for the control condition in which cells immobilized on the anti-CD27 affinity column were not incubated with the oligomeric stimulatory reagent, cells collected by elution with D-biotin did not exhibit CD27 downregulation, as evidenced by a high percentage of the collected T cells in the control sample retaining positive expression of CD27. These results are consistent with an observation that stimulation of the T cells with an anti-CD3/anti-CD28 stimulatory reagent resulted in downregulation of surface expression of the selected-for receptor, thereby permitting spontaneous detachment of the cells from the column without the need to add a competition reagent to detach or disrupt binding of the cells to the affinity column.

These results further support that cell surface markers used for selecting cells can be downregulated by on-column stimulation of the cells while immobilized to an affinity column via the cell surface marker, thereby permitting release of the cells from the column without the use of a competition substance to release the cells for cell collection. These results also indicate the utility of a jacket member containing heating elements and configured to surround the column for heating and maintaining column temperature during on-column stimulation of cells.

### Example 13: Selection and stimulation of T cells via sequential column chromatography with multiple selection markers.

On-column T cell selection and stimulation were performed using two separate columns. Each column was prepared using a different exemplary selection agent, and on-column stimulation configured with a heating device to maintain the column temperature at about 37 °C was performed only in the second column. The second column included a gas supply element and was heated with a jacket member containing two heating coils as described in Example 12.

The first column was prepared using an anti-CD27 Fab as described in Example 12. An apheresis sample from a human donor was loaded on the first column, and the column was washed. After more than 30 minutes passed from the time of loading the sample, D-biotin was added to disrupt binding between the Twin Strep-Tag^{®} of the anti-CD27 Fab and the STREP-TACTIN^{®} to release cells from the first column. Released cells were collected by gravity flow, washed to remove residual D-biotin contamination, and passed to a second column prepared as in Example 12, but using an anti-CD3 Fab as the selection agent. The CD3 binding Fab fragment was derived from the CD3 binding monoclonal antibody produced by the hybridoma cell line OKT3 (ATCC^{®} CRL-8001^{™}; see also U.S. Patent No. 4,361,549), and contained the heavy chain variable domain (SEQ ID NO:31) and light chain variable domain (SEQ ID NO:32) of the anti-CD3 antibody OKT3 described in Arakawa et al J. Biochem. 120, 657-662 (1996). After more than 30 minutes passed from the time of loading the released cells into the second column, 2000 µg of multimerized anti-CD3 Fab fragments and anti-CD28 Fab fragments reversibly bound to an oligomeric streptavidin mutein reagent (anti-CD3/anti-CD28 oligomeric reagent), generated as described in Example 1, was loaded onto the second column in 20 mL of serum free basal media containing glutamine and recombinant IL-2, IL-15, and IL-7 and incubated at 37 °C via the heating coils contained in the jacket member. After approximately 4 hours, selected cells were collected from the second column in a single step by passing approximately 80 mL of serum free basal media containing glutamine and recombinant IL-2, IL-15, and IL-7 through the second column. The cells were collected without the addition of a further competition substance (e.g. without adding D-biotin) to disrupt the binding of the streptavidin-binding peptide (e.g. Twin Strep-Tag^{®}) of the anti-CD3 Fab selection agent to the streptavidin mutein (e.g. STREP-TACTIN^{®})-coated stationary phase resin.

Cells from the apheresis sample and the positive fractions of each column were stained with antibodies recognizing surface CD3 and CD27 and quantified by flow cytometry. The flow cytometry results are shown in **FIG. 33****.** In the apheresis sample, 32.3% of cells were CD3+CD27+. CD27 and CD3 selection led to high enrichment of CD3+CD27+ cells, with a purity of 87.5% following CD27 selection and a purity of 96.2% following CD3 selection. On-column stimulation in the second column led to downregulation of CD3 surface expression, while CD27 surface expression was relatively preserved. These results are consistent with an observation that stimulation-induced downregulation of surface receptor expression is specific to the receptor by which selected cells are bound during on-column stimulation.

### Example 14: Selection and stimulation of T cells using different heating configurations.

The ability to select and stimulate T cells by on-column T cell selection and stimulation using chromatography columns heated by different configurations of heating elements disposed outside the chromatography column elements was assessed. Each column was prepared with an anti-CD3 Fab for selection of T cells as described in Example 13, and each column included a gas supply element, specifically a gas supply connector for screw-on air filters.

The heating elements for each column were part of a jacket member designed to enclose the column. In one configuration, the jacket member was as described in Example 12. See, e.g., **FIGS. 25-28** for exemplary schematics.

For the second configuration, the jacket member included three jacket components each designed to surround a third of the circumference of the column from top to bottom, and together the jacket components were connected to surround the column. Each jacket component included an electrical temperature sensor and a metal plate as an electrical heating element. Each metal plate had an aluminum profile, and an electrical insulating layer was placed between each metal plate and its aluminum profile. Each metal plate was mounted at the interior surface of its jacket component, and each jacket component had electrical connection points for its temperature sensor and metal plate. Together, the jacket components enclosed the column except for openings to allow the inlet and outlet of cells and reagents into and out of the column, such that the three metal plates were equally spaced around the circumference of the column and the inside of the jacket was directly in contact with the column. See, e.g., **FIGS. 29-31** for exemplary schematics.

On-column T cell selection and stimulation were performed using a column-based affinity chromatography in which each of the above jacket components were configured around the column under conditions to heat the internal cavity of the column. For each configured column, an apheresis sample from a human donor was loaded onto the affinity column, and CD3+ T cells in the sample were allowed to interact with the CD3 binding Fab fragment immobilized on the resin. The column was washed two times with PBSA buffer to remove cells that did not interact with the immobilized CD3 binding Fab fragment. After approximately 15 minutes from the time of loading the sample onto the column, a stimulatory reagent was added to the column by loading onto the column at least 2000 µg of an anti-CD3/anti-CD28 oligomeric reagent (multimerized anti-CD3 Fab fragments and anti-CD28 Fab fragment stimulatory agents reversibly bound to an oligomeric streptavidin mutein reagent), described in Examples 1 and 2 above, in 20 mL of serum free basal media containing glutamine and recombinant IL-2, IL-15, and IL-7, and the cells were incubated on the column under conditions to stimulate the cells. During incubation, the column heated using metal-based heating elements was maintained at temperatures of 30°C, 35°C, or 37°C. For reference, the column heated using heating coils (e.g., water-based heating elements) was kept at a constant 37°C. The gas supply elements supplied air under control of open sterile filters that were connected during the incubations.

During the on-column stimulation with the anti-CD3/anti-CD28 oligomeric reagent, the selected CD3+ T cells spontaneously detached from the column without the need to add a competition reagent to disrupt the binding between the cells and the resin. Within approximately 4.5 hours after adding the stimulatory reagent, spontaneously detached cells were collected from the column in a single step by passing through the column approximately 80 mL of serum free basal media containing glutamine and recombinant IL-2, IL-15, and IL-7, and the cells were collected by gravity flow. No further competition substance (e.g. no adding D-biotin) was added to the column to disrupt the binding of cells on the column, via the interactions of the streptavidin-binding peptide (e.g. Twin Strep-Tag^{®}) of the anti-CD3 Fab selection agent to the streptavidin mutein (e.g. STREP-TACTIN^{®})-coated stationary phase resin, prior to their collection by gravity flow. Collected cells were then stained and quantified for CD3, CD4, CD8, and CD69 surface expression as well as for the number and percentage of viable cells.

As shown in **FIGS. 34A-34E****,** CD3+ depletion efficiency **(****FIG. 34A****),** CD4 and CD8 expression **(****FIG. 34B****),** and CD69 expression **(****FIG. 34C****)** of cells collected from columns after the on-column selection and stimulation of cells were similar using either of the columns with jacket members (water- and metal-based heating elements). The results also were consistent across incubation temperatures of between 30°C and 37°C. As shown in **FIG. 34D** and **FIG. 34E****,** there was more variability in the percentage **(****FIG. 34D****)** and number of viable cells **(****FIG. 34E****)** collected using the column with metal-based heating elements across incubation temperatures.

Together with the results in the above example, these results indicate the utility of different types of heating elements and configurations for heating and maintaining column temperature during on-column stimulation of cells.

### SEQUENCES

| No. | Sequence | Description |
|---|---|---|
| 1 | DPSKDSKAQVSAAEAGITGTWYNQLGSTFIVTAGADGALTGTYESAVG NAESRYVLTGRYDSAPATDGSGTALGWTVAWKNNYRNAHSATTWSG QYVGGAEARINTQWLLTSGTTEANAWKSTLVGHDTFTKVKPSAASIDA AKKAGVNNGNPLDAVQQ | Streptavidin |
| | | Species: Streptomyces avidinii |
| | | UniProt No. P22629 |
| 2 | MetGluAlaGlyIleThrGlyThrTrpTyrAsnGlnLeuGlySerThrPheIleValThrAlaGly AlaAspGlyAlaLeuThrGlyThrTyrGluSerAlaValGlyAsnAlaGluSerArgTyrValLe uThrGlyArgTyrAspSerAlaProAlaThrAspGlySerGlyThrAlaLeuGlyTrpThrVal AlaTrpLysAsnAsnTyrArgAsnAlaHisSerAlaThrThrTrpSerGlyGlnTyrValGlyGl yAlaGluAlaArgIleAsnThrGlnTrpLeuLeuThrSerGlyThrThrGluAlaAsnAlaTrpL ysSerThrLeuValGlyHisAspThrPheThrLysValLysProSerAlaAlaSer | Minimal streptavidin |
| | | Species: Streptomyces avidinii |
| 3 | DPSKDSKAQVSAAEAGITGTWYNQLGSTFIVTAGADGALTGTYVTARG NAESRYVLTGRYDSAPATDGSGTALGWTVAWKNNYRNAHSATTWSG QYVGGAEARINTQWLLTSGTTEANAWKSTLVGHDTFTKVKPSAASIDA AKKAGVNNGNPLDAVQQ | Mutein Streptavidin Val⁴⁴-Thr⁴⁵-Ala⁴⁶-Arg⁴⁷ |
| | | Species: Streptomyces avidinii |
| 4 | MetGluAlaGlyIleThrGlyThrTrpTyrAsnGlnLeuGlySerThrPheIleValThrAlaGly AlaAspGlyAlaLeuThrGlyThrTyrValThrAlaArgGlyAsnAlaGluSerArgTyrValL euThrGlyArgTyrAspSerAlaProAlaThrAspGlySerGlyThrAlaLeuGlyTrpThrVal AlaTrpLysAsnAsnTyrArgAsnAlaHisSerAlaThrThrTrpSerGlyGlnTyrValGlyGl yAlaGluAlaArgIleAsnThrGlnTrpLeuLeuThrSerGlyThrThrGluAlaAsnAlaTrpL ysSerThrLeuValGlyHisAspThrPheThrLysValLysProSerAlaAlaSer | Mutein Streptavidin Val⁴⁴-Thr⁴⁵-Ala⁴⁶-Arg⁴⁷ |
| | | Species: Streptomyces avidinii |
| 5 | DPSKDSKAQVSAAEAGITGTWYNQLGSTFIVTAGADGALTGTYIGARG NAESRYVLTGRYDSAPATDGSGTALGWTVAWKNNYRNAHSATTWSG QYVGGAEARINTQWLLTSGTTEANAWKSTLVGHDTFTKVKPSAASIDA AKKAGVNNGNPLDAVQQ | Mutein Streptavidin Ile⁴⁴-Gly⁴⁵-Ala⁴⁶-Ar⁴⁷ |
| | | Species: Streptomyces avidinii |
| 6 | MetGluAlaGlyIleThrGlyThrTrpTyrAsnGlnLeuGlySerThrPheIleValThrAlaGly AlaAspGlyAlaLeuThrGlyThrTyrIleGlyAlaArgGlyAsnAlaGluSerArgTyrValLe uThrGlyArgTyrAspSerAlaProAlaThrAspGlySerGlyThrAlaLeuGlyTrpThrVal AlaTrpLysAsnAsnTyrArgAsnAlaHisSerAlaThrThrTrpSerGlyGlnTyrValGlyGl yAlaGluAlaArgIleAsnThrGlnTrpLeuLeuThrSerGlyThrThrGluAlaAsnAlaTrpL ysSerThrLeuValGlyHisAspThrPheThrLysValLysProSerAlaAlaSer | Mutein Streptavidin Ile⁴⁴-Gly⁴⁵-Ala⁴⁶-Ar⁴⁷ |
| | | Species: Streptomyces avidinii |
| 7 | Trp-Arg-His-Pro-Gln-Phe-Gly-Gly | Streptavidin binding peptide, Strep-tag^{®} |
| 8 | WSHPQFEK | Strep-tag^{®} II |
| 9 | His-Pro-Xaa | Streptavidin Binding peptide |
| | | Xaa is selected from Gln, Asp, and Met |
| 10 | His-Pro-Gln-Phe | Streptavidin-binding peptide |
| 11 | Xaa₁-Xaa₂-His-Pro-Gln-Phe-Xaa₃-Xaa₄ | Streptavidin-binding peptide |
| | | Xaa₁ is Trp, Lys or Arg; |
| | | Xaa₂ is any amino acid; |
| | | Xaa₃ is Gly or Glu |
| | | Xaa₄ is Gly, Lys or Arg |
| 12 | -Trp-Xaa₁-His-Pro-Gln-Phe-Xaa₂-Xaa₃- | Streptavidin-binding peptide |
| | | Xaa₁ is any amino acid; |
| | | Xaa₂ is Gly or Glu |
| | | Xaa₃ is Gly, Lys or Arg |
| 13 | Trp-Ser-His-Pro-Gln-Phe-Glu-Lys-(Xaa)n-Trp-Ser-His-Pro-Gln-Phe-Glu-Lys- | Sequential modules of streptavidin-binding peptide |
| | | Xaa is any amino acid; |
| | | n is either 8 or 12 |
| 14 | Trp-Ser-His-Pro-Gln-Phe-Glu-Lys-(GlyGlyGlySer)n-Trp-Ser-His-Pro-Gln- Phe-Glu-Lys | Sequential modules of streptavidin-binding peptide |
| | | n is 2 or 3 |
| 15 | SAWSHPQFEKGGGSGGGSGGGSWSHPQFEK | Twin-Strep-tag |
| 16 | SAWSHPQFEKGGGSGGGSGGSAWSHPQFEK | Twin-Strep-tag |
| 17 | WSHPQFEKGGGSGGGSGGGSWSHPQFEK | Twin-Strep-tag |
| 18 | WSHPQFEKGGGSGGGSWSHPQFEK | Twin-Strep-tag |
| 19 | WSHPQFEKGGGSGGGSGGSAWSHPQFEK | Twin-Strep-tag |
| 20 | Tyr-Pro-Tyr-Asp-Val-Pro-Asp-Tyr-Ala | HA-tag |
| 21 | Tyr-Thr-Asp-Ile-Glu-Met-Asn-Arg-Leu-Gly-Lys | VSV-G-tag |
| 22 | Gln-Pro-Glu-Leu-Ala-Pro-Glu-Asp-Pro-Glu-Asp | HSV-tag |
| 23 | Ala-Ser-Met-Thr-Gly-Gly-Gln-Gln-Met-Gly | T7 epitope |
| 24 | Gln-Pro-Glu-Leu-Ala-Pro-Glu-Asp-Pro-Glu-Asp | HSV epitope |
| 25 | Glu-Gln-Lys-Leu-Ile-Ser-Glu-Glu-Asp-Leu | Myc epitope |
| 26 | Gly-Lys-Pro-Ile-Pro-Asn-Pro-Leu-Leu-Gly-Leu-Asp-Ser-Thr | V5-tag |
| 27 | EAGITGTWYNQLGSTFIVTAGADGALTGTYVTARGNAESRYVLTGRYD SAPATDGSGTALGWTVAWKNNYRNAHSATTWSGQYVGGAEARINTQ WLLTSGTTEENAGYSTLVGHDTFTKVKPSAAS | Mutein Streptavidin Val⁴⁴-Thr⁴⁵-Ala⁴⁶-Arg⁴⁷ and Glu¹¹⁷, Gly¹²⁰, Tyr¹²¹ (mutein m1-9) |
| | | Species: Streptomyces avidinii |
| 28 | DPSKDSKAQVSAAEAGITGTWYNQLGSTFIVTAGADGALTGTYVTARG NAESRYVLTGRYDSAPATDGSGTALGWTVAWKNNYRNAHSATTWSG QYVGGAEARINTQWLLTSGTTEENAGYSTLVGHDTFTKVKPSAAS | Mutein Streptavidin Val⁴⁴-Thr⁴⁵-Ala⁴⁶-Arg⁴⁷ and Glu¹¹⁷, Gly¹²⁰, Tyr¹²¹ (mutein m1-9) |
| | | Species: Streptomyces avidinii |
| 29 | AMQVQLKQSG PGLVQPSQSL SITCTVSGFS LTTFGVHWVR QSPGKGLEWL GVIWASGITD YNVPFMSRLS ITKDNSKSQV FFKLNSLQPD DTAIYYCAKN DPGTGFAYWG QGTLVTVSAG STKGPSVFPL APSSKSTSGG TAALGCLVKD YFPEPVTVSW NSGALTSGVH TFPAVLQSSG LYSLSSVVTV PSSSLGTQTY ICNVNHKPSN TKVDKKVEPK SCGSAWSHPQ FEKGGGSGGG SGGSAWSHPQ FEK | Variable Heavy chain of Fab fragment m13B8.2 |
| 30 | AMDIQMTQSP ASLSASVGET VTFTCRASEM IYSYLAWYQQ KQGKSPQLLV HDAKTLAEGV PSRFSGGGSG TQFSLKINTL QPEDFGTYYC QAHYGNPPTF GGGTKLEIKR GIAAPSVFIF PPSDEQLKSG TASVVCLLNN FYPREAKVQW KVDNALQSGN SQESVTEQDS KDSTYSLSST LTLSKADYEK HKVYACEVTH QGLSSPVTKS FNRGECGS | Variable Light chain of Fab Fragment m13B8.2 |
| 31 | | Variable Heavy chain of anti-CD3 antibody OKT3 |
| 32 | | Variable Light chain of anti-CD3 antibody OKT3 |
| 33 | Leu Gln Gln Ser Gly Ala Glu Leu Val Lys Pro Gly Ala Ser Val Arg Leu Ser Cys Lys Ala Ser Gly Tyr Thr Phe Thr Glu Tyr Ile Ile His Trp Ile Lys Leu Arg Ser Gly Gln Gly Leu Glu Trp Ile Gly Trp Phe Tyr Pro Gly Ser Asn Asp Ile Gln Tyr Asn Ala Lys Phe Lys Gly Lys Ala Thr Leu Thr Ala Asp Lys Ser Ser Ser Thr Val Tyr Met Glu Leu Thr Gly Leu Thr Ser Glu Asp Ser Ala Val Tyr Phe Cys Ala Arg Arg Asp Asp Phe Ser Gly Tyr Asp Ala Leu Pro Tyr Trp Gly Gln Gly Thr Met Val Thr Val | Variable Heavy chain of anti-CD28 antibody CD28.3 |
| 34 | Asp Ile Gln Met Thr Gln Ser Pro Ala Ser Leu Ser Val Ser Val Gly Glu Thr Val Thr Ile Thr Cys Arg Thr Asn Glu Asn Ile Tyr Ser Asn Leu Ala Trp Tyr Gln Gln Lys Gln Gly Lys Ser Pro Gln Leu Leu Ile Tyr Ala Ala Thr His Leu Val Glu Gly Val Pro Ser Arg Phe Ser Gly Ser Gly Ser Gly Thr Gln Tyr Ser Leu Lys Ile Thr Ser Leu Gln Ser Glu Asp Phe Gly Asn Tyr Tyr Cys Gln His Phe Trp Gly Thr Pro Cys Thr Phe Gly Gly Gly Thr Lys Leu Glu Ile Lys Arg | Variable Light chain of anti-CD28 antibody CD28.3 |
| 35 | His-Asn-Hi-Arg-His-Lys-His-Gly-Gly-Gly-Cys | MAT tag |
| 36 | Gln Val Gln Leu Val Gln Ser Gly Ala Glu Val Lys Lys Pro Gly Ala1 Thr Val Lys Ile Ser Cys Lys Val Ser Gly Phe Asn Ile Lys Asp Thr Tyr Ile His Trp Val Gln Gln Ala Pro Gly Lys Gly Leu Glu Trp Met Gly Arg Ile Asp Pro Ala Asn Asp Asn Thr Leu Tyr Ala Ser Lys Phe Gln Gly Arg Val Thr Ile Thr Ala Asp Thr Ser Thr Asp Thr Ala Tyr65 Met Glu Leu Ser Ser Leu Arg Ser Glu Asp Thr Ala Val Tyr Tyr Cys Ala Arg Gly Tyr Gly Tyr Tyr Val Phe Asp His Trp Gly Gln Gly Thr Leu Val Thr Val Ser Ser | Variable Heavy chain of huOKT8 |
| 37 | Asp Val Gln Ile Thr Gln Ser Pro Ser Ser Leu Ser Ala Ser Val Gly Asp Arg Val Thr Ile Thr Cys Arg Thr Ser Arg Ser Ile Ser Gln Tyr Leu Ala Trp Tyr Gln Gln Lys Pro Gly Lys Val Pro Lys Leu Leu Ile Tyr Ser Gly Ser Thr Leu Gln Ser Gly Val Pro Ser Arg Phe Ser Gly Ser Gly Ser Gly Thr Asp Phe Thr Leu Thr Ile Ser Ser Leu Gln Pro Glu Asp Val Ala Thr Tyr Tyr Cys Gln Gln His Asn Glu Asn Pro Leu Thr Phe Gly Gly Gly Thr Lys Val Glu Ile Lys | Variable Light chain of huOKT8 |
| 38 | -PGGG-(SGGGG)₅-P- | Linker |
| | | P is proline, G is glycine and S is serine |
| 39 | GSADDAKKDAAKKDGKS | Linker |
| 40 | MLLLVTSLLLCELPHPAFLLIP | GMCSFR alpha chain (amino acid) |
| 41 | atgcttctcctggtgacaagccttctgctctgtgagttaccacacccagcattcctcctgatccca | GMCSFR alpha chain (nucleic acid) |
| 42 | MALPVTALLLPLALLLHA | CD8 alpha signal peptide |
| 43 | MLLLVTSLLLCELPHPAFLLIPRKVCNGIGIGEFKDSLSINATNIKHFKNC TSISGDLHILPVAFRGDSFTHTPPLDPQELDILKTVKEITGFLLIQAWPEN RTDLHAFENLEIIRGRTKQHGQFSLAVVSLNITSLGLRSLKEISDGDVIIS GNKNLCYANTINWKKLFGTSGQKTKIISNRGENSCKATGQVCHALCSP EGCWGPEPRDCVSCRNVSRGRECVDKCNLLEGEPREFVENSECIQCHP ECLPQAMNITCTGRGPDNCIQCAHYIDGPHCVKTCPAGVMGENNTLV WKYADAGHVCHLCHPNCTYGCTGPGLEGCPTNGPKIPSIATGMVGAL LLLLVVALGIGLFM | truncated epidermal growth factor receptor (tEGFR) |
| 44 | RKVCNGIGIGEFKDSLSINATNIKHFKNCTSISGDLHILPVAFRGDSFTHT PPLDPQELDILKTVKEITGFLLIQAWPENRTDLHAFENLEIIRGRTKQHG QFSLAVVSLNITSLGLRSLKEISDGDVIISGNKNLCYANTINWKKLFGTS GQKTKIISNRGENSCKATGQVCHALCSPEGCWGPEPRDCVSCRNVSRG | truncated epidermal growth factor receptor (tEGFR) |
| | RECVDKCNLLEGEPREFVENSECIQCHPECLPQAMNITCTGRGPDNCIQ CAHYIDGPHCVKTCPAGVMGENNTLVWKYADAGHVCHLCHPNCTYG CTGPGLEGCPTNGPKIPSIATGMVGALLLLLVVALGIGLFM | |
| 45 | VKQTLNFDLLKLAGDVESNPGP | F2A |
| 46 | QCTNYALLKLAGDVESNPGP | E2A |
| 47 | LEGGGEGRGSLLTCGDVEENPGPR | T2A |
| 48 | EGRGSLLTCGDVEENPGP | T2A |
| 49 | GSGATNFSLLKQAGDVEENPGP | P2A |
| 50 | ATNFSLLKQAGDVEENPGP | P2A |
| 51 | DYGVS | CDR H1 |
| 52 | VIWGSETTYYNSALKS | CDR H2 |
| 53 | YAMDYWG | CDR H3 |
| 54 | HYYYGGSYAMDY | CDR H3 |
| 55 | RASQDISKYLN | CDR L1 |
| 56 | SRLHSGV | CDR L2 |
| 57 | HTSRLHS | CDR L2 |
| 58 | GNTLPYTFG | CDR L3 |
| 59 | QQGNTLPYT | CDR L3 |
| 60 | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCAK HYYYGGSYAMDYWGQGTSVTVSS | VH |
| 61 | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTFG GGTKLEIT | VL |
| 62 | GSTSGSGKPGSGEGSTKG | Linker |
| 63 | gacatccagatgacccagaccacctccagcctgagcgccagcctgggcgaccgggtgaccatcagctgccgg gccagccaggacatcagcaagtacctgaactggtatcagcagaagcccgacggcaccgtcaagctgctgatct accacaccagccggctgcacagcggcgtgcccagccggtttagcggcagcggctccggcaccgactacagc ctgaccatctccaacctggaacaggaagatatcgccacctacttttgccagcagggcaacacactgccctacacc tttggcggcggaacaaagctggaaatcaccggcagcacctccggcagcggcaagcctggcagcggcgaggg cagcaccaagggcgaggtgaagctgcaggaaagcggccctggcctggtggcccccagccagagcctgagc gtgacctgcaccgtgagcggcgtgagcctgcccgactacggcgtgagctggatccggcagccccccaggaag ggcctggaatggctgggcgtgatctggggcagcgagaccacctactacaacagcgccctgaagagccggctg accatcatcaaggacaacagcaagagccaggtgttcctgaagatgaacagcctgcagaccgacgacaccgcc atctactactgcgccaagcactactactacggcggcagctacgccatggactactggggccagggcaccagcgt gaccgtgagcagc | Sequence encoding scFv |
| 64 | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTFG GGTKLEITGSTSGSGKPGSGEGSTKGEVKLQESGPGLVAPSQSLSVTCT VSGVSLPDYGVSWIRQPPRKGLEWLGVIWGSETTYYNSALKSRLTIIKD NSKSQVFLKMNSLQTDDTAIYYCAKHYYYGGSYAMDYWGQGTSVTV SS | scFv |
| 65 | SYWMN | CDR H1 |
| 66 | QIYPGDGDTNYNGKFKG | CDR H2 |
| 67 | KTISSVVDFYFDY | CDR H3 |
| 68 | QQYNRYPYT | CDR L3 |
| 69 | SYWMN | CDR H1 |
| 70 | QIYPGDGDTNYNGKFKG | CDR H2 |
| 71 | EVKLQQSGAELVRPGSSVKISCKASGYAFSSYWMNWVKQRPGQGLEW IGQIYPGDGDTNYNGKFKGQATLTADKSSSTAYMQLSGLTSEDSAVYF CARKTISSVVDFYFDYWGQGTTVTVSS | VH |
| 72 | DIELTQSPKFMSTSVGDRVSVTCKASQNVGTNVAWYQQKPGQSPKPLI YSATYRNSGVPDRFTGSGSGTDFTLTITNVQSKDLADYFCQQYNRYPY TSGGGTKLEIKR | VL |
| 73 | KASQNVGTNVA | CDR L1 |
| 74 | SATYRNS | CDR L2 |
| 75 | QQYNRYPYT | CDR L3 |
| 76 | SYWMN | CDR H1 |
| 77 | QIYPGDGDTNYNGKFKG | CDR H2 |
| 78 | KTISSVVDFYFDY | CDR H3 |
| 79 | GGGGSGGGGSGGGGS | Linker |
| 80 | EVKLQQSGAELVRPGSSVKISCKASGYAFSSYWMNWVKQRPGQGLEW IGQIYPGDGDTNYNGKFKGQATLTADKSSSTAYMQLSGLTSEDSAVYF CARKTISSVVDFYFDYWGQGTTVTVSSGGGGSGGGGSGGGGSDIELTQ SPKFMSTSVGDRVSVTCKASQNVGTNVAWYQQKPGQSPKPLIYSATYR NSGVPDRFTGSGSGTDFTLTITNVQSKDLADYFCQQYNRYPYTSGGGT KLEIKR | scFv |
| 81 | ESKYGPPCPPCP | spacer (IgG4hinge) (aa) |
| | | Homo sapiens |
| 82 | GAATCTAAGTACGGACCGCCCTGCCCCCCTTGCCCT | spacer (IgG4hinge) (nt) homo sapiens |
| 83 | ESKYGPPCPPCPGQPREPQVYTLPPSQEEMTKNQVSLTCLVKGFYPSDI AVEWESNGQPENNYKTTPPVLDSDGSFFLYSRLTVDKSRWQEGNVFSC SVMHEALHNHYTQKSLSLSLGK | Hinge-CH3 spacer |
| | | Homo sapiens |
| 84 | ESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTV DKSRWQEGNVFSCSVMHEALHNHYTQKSLSLSLGK | Hinge-CH2-CH3 spacer |
| | | Homo sapiens |
| 85 | RWPESPKAQASSVPTAQPQAEGSLAKATTAPATTRNTGRGGEEKKKEK EKEEQEERETKTPECPSHTQPLGVYLLTPAVQDLWLRDKATFTCFVVG SDLKDAHLTWEVAGKVPTGGVEEGLLERHSNGSQSQHSRLTLPRSLW NAGTSVTCTLNHPSLPPQRLMALREPAAQAPVKLSLNLLASSDPPEAAS WLLCEVSGFSPPNILLMWLEDQREVNTSGFAPARPPPQPGSTTFWAWS VLRVPAPPSPQPATYTCVVSHEDSRTLLNASRSLEVSYVTDH | IgD-hinge-Fc |
| | | Homo sapiens |
| 86 | Glu Val Val Val Lys Tyr Gly Pro Pro Cys Pro Pro Cys Pro | Exemplary IgG Hinge |
| 87 | X₁PPX₂P X₁ is glycine, cysteine or arginine X₂ is cysteine or threonine | Exemplary IgG Hinge |
| 88 | Glu Pro Lys Ser Cys Asp Lys Thr His Thr Cys Pro Pro Cys Pro | Exemplary IgG Hinge |
| 89 | Glu Arg Lys Cys Cys Val Glu Cys Pro Pro Cys Pro | Exemplary IgG Hinge |
| 90 | ELKTPLGDTHTCPRCPEPKSCDTPPPCPRCPEPKSCDTPPPCPRCPEPKSC DTPPPCPRCP | Exemplary IgG Hinge |
| 91 | Glu Ser Lys Tyr Gly Pro Pro Cys Pro Ser Cys Pro | Exemplary IgG Hinge |
| 92 | Glu Ser Lys Tyr Gly Pro Pro Cys Pro Pro Cys Pro | Exemplary IgG Hinge |
| 93 | Tyr Gly Pro Pro Cys Pro Pro Cys Pro | Exemplary IgG Hinge |
| 94 | Lys Tyr Gly Pro Pro Cys Pro Pro Cys Pro | Exemplary IgG Hinge |
| 95 | FWVLVVVGGVLACYSLLVTVAFIIFWV | CD28 (amino acids 153-179 of Accession No. P10747) |
| | | Homo sapien |
| 96 | IEVMYPPPYLDNEKSNGTIIHVKGKHLCPSPLFPGPSKPFWVLVVVGGV LACYSLLVTVAFIIFWV | CD28 (amino acids 114-179 of Accession No. P10747) |
| | | Homo sapiens |
| 97 | RSKRSRLLHSDYMNMTPRRPGPTRKHYQPYAPPRDFAAYRS | CD28 (amino acids 180-220 of P10747) |
| | | Homo sapiens |
| 98 | RSKRSRGGHSDYMNMTPRRPGPTRKHYQPYAPPRDFAAYRS | CD28 (LL to GG) |
| | | Homo sapiens |
| 99 | KRGRKKLLYIFKQPFMRPVQTTQEEDGCSCRFPEEEEGGCEL | 4-1BB (amino acids 214-255 of Q07011.1) |
| | | Homo sapiens |
| 100 | RVKFSRSADAPAYQQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | CD3 zeta |
| | | Homo sapiens |
| 101 | RVKFSRSAEPPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR | CD3 zeta |
| | | Homo sapiens |
| 102 | RVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | CD3 zeta |
| | | Homo sapiens |
| 103 | GluAlaGlyIleThrGlyThrTrpTyrAsnGlnLeuGlySerThrPheIleValThrAlaGlyAla AspGlyAlaLeuThrGlyThrTyrGluSerAlaValGlyAsnAlaGluSerArgTyrValLeuT hrGlyArgTyrAspSerAlaProAlaThrAspGlySerGlyThrAlaLeuGlyTrpThrValAla TrpLysAsnAsnTyrArgAsnAlaHisSerAlaThrThrTrpSerGlyGlnTyrValGlyGlyAl aGluAlaArgIleAsnThrGlnTrpLeuLeuThrSerGlyThrThrGluAlaAsnAlaTrpLysS erThrLeuValGlyHisAspThrPheThrLysValLysProSerAlaAlaSer | Minimal streptavidin without initial methionine |
| | | Species: Streptomyces avidinii |
| 104 | GluAlaGlyIleThrGlyThrTrpTyrAsnGlnLeuGlySerThrPheIleValThrAlaGlyAla AspGlyAlaLeuThrGlyThrTyrValThrAlaArgGlyAsnAlaGluSerArgTyrValLeuT hrGlyArgTyrAspSerAlaProAlaThrAspGlySerGlyThrAlaLeuGlyTrpThrValAla TrpLysAsnAsnTyrArgAsnAlaHisSerAlaThrThrTrpSerGlyGlnTyrValGlyGlyAl aGluAlaArgIleAsnThrGlnTrpLeuLeuThrSerGlyThrThrGluAlaAsnAlaTrpLysS erThrLeuValGlyHisAspThrPheThrLysValLysProSerAlaAlaSer | Mutein Streptavidin Val⁴⁴-Thr⁴⁵-Ala⁴⁶-Arg⁴⁷ without initial methionine |
| | | Species: Streptomyces avidinii |
| 105 | GluAlaGlyIleThrGlyThrTrpTyrAsnGlnLeuGlySerThrPheIleValThrAlaGlyAla AspGlyAlaLeuThrGlyThrTyrIleGlyAlaArgGlyAsnAlaGluSerArgTyrValLeuTh rGlyArgTyrAspSerAlaProAlaThrAspGlySerGlyThrAlaLeuGlyTrpThrValAlaT rpLysAsnAsnTyrArgAsnAlaHisSerAlaThrThrTrpSerGlyGlnTyrValGlyGlyAla GluAlaArgIleAsnThrGlnTrpLeuLeuThrSerGlyThrThrGluAlaAsnAlaTrpLysSe rThrLeuValGlyHisAspThrPheThrLysValLysProSerAlaAlaSer | Mutein Streptavidin Ile44-Gly45-Ala-46-Arg47 without initial methionine |
| | | Species: Streptomyces avidinii |
| 106 | ASTKGPSVFPLAPCSRSTSESTAALGCLVKDYFPEPVTVSWNSGALTSG VHTFPAVLQSSGLYSLSSVVTVPSSNFGTQTYTCNVDHKPSNTKVDKT VERKCCVECPPCPAPPVAGPSVFLFPPKPKDTLMISRTPEVTCVVVDVS HEDPEVQFNWYVDGVEVHNAKTKPREEQFNSTFRVVSVLTVVHQDWL NGKEYKCKVSNKGLPAPIEKTISKTKGQPREPQVYTLPPSREEMTKNQV SLTCLVKGFYPSDISVEWESNGQPENNYKTTPPMLDSDGSFFLYSKLTV DKSRWQQGNVFSCSVMHEALHNHYTQKSLSLSPGK | Human IgG2 Fc (Uniprot P01859) |
| 107 | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKR VESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDW LNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLT VDKSRWQEGNVFSCSVMHEALHNHYTQKSLSLSLGK | Human IgG4 Fc (Uniprot P01861) |
| 108 | GVQVETISPGDGRTFPKRGQTCVVHYTGMLEDGKKMDSSRDRNKPFK FMLGKQEVIRGWEEGVAQMSVGQRAKLTISPDYAYGATGHPGIIPPHA TLVFDVELLKLE | FKBP |
| 109 | GVQVETISPGDGRTFPKRGQTCVVHYTGMLEDGKKVDSSRDRNKPFKF MLGKQEVIRGWEEGVAQMSVGQRAKLTISPDYAYGATGHPGIIPPHAT LVFDVELLKLE | FKBP12v36 |
| 110 | MGSNKSKPKDASQRRR | Modified acylation motif |
| 111 | Met-Gly-Cys-Xaa-Cys | dual acylation motif |
| 112 | Cys-Ala-Ala-Xaa | acylation region |

## Claims

1. A chromatography column, comprising a housing assembly (1) for column chromatography, the housing assembly comprising:
an inlet housing member (2) and an outlet housing member (3), wherein at least the inlet housing member and the outlet housing member form an internal cavity configured to house a stationary phase for column chromatography, wherein:
the internal cavity comprises the stationary phase for column chromatography; and
the stationary phase comprises an affinity chromatography matrix;
a temperature control member configured to provide heat to the stationary phase in the internal cavity; and
a connector (6) configured to operably connect the internal cavity to a gas source, thereby permitting or effecting intake of gas into the internal cavity.

2. The chromatography column of claim 1, the housing assembly further comprising a side wall member (7), wherein the inlet housing member, the outlet housing member, and the side wall member form the internal cavity.

3. The chromatography column of claim 1 or claim 2, wherein the connector comprises one or more filter(s), and optionally, wherein the one or more filter(s) is a gas filter.

4. The chromatography column of any one of claims 1-3,
wherein the inlet housing member comprises one or more inlet(s) operably connected to the internal cavity to permit intake of an input composition into the internal cavity; and/or
wherein the outlet housing member comprises one or more outlet(s) operably connected to the internal cavity to permit or effect discharge of an output composition from the internal cavity.

5. The chromatography column of any of claims 1-4, further comprising:
a first porous member (9) configured to separate the stationary phase and an inlet of the internal cavity,
wherein the first porous member is optionally between the inlet housing member and the side wall member, and further optionally,
wherein the first porous member is independently a cell strainer or a cell sieve; and/or
a second porous member (9) configured to separate the stationary phase and an outlet of the internal cavity,
wherein the second porous member is optionally between the outlet housing member and the side wall member, and further optionally,
wherein the second porous member is independently a cell strainer or a cell sieve.

6. The chromatography column of any one of claims 1-5, wherein the temperature control member is configured:
to heat the stationary phase to a target temperature between about 30°C and about 39°C, and optionally, wherein the target temperature is between about 35°C and about 39°C, and further optionally at or about 37°C, and/or
to maintain the stationary phase at the target temperature.

7. The chromatography column of any one of claims 1-6, wherein the temperature control member comprises one or more heating element(s), and optionally, wherein the one or more heating element(s) are configured to uniformly heat the stationary phase.

8. The chromatography column of claim 7, wherein at least one of the one or more heating element(s) is:
a) an electromagnetic induction heating element, and the electromagnetic induction heating element comprises an induction heating coil surrounding a magnetizable core configured to provide heat to the stationary phase in the internal cavity; or
b) an electric heating element (17) and optionally,
wherein the electric heating element comprises a metal plate, a metal rod, a metal wire, or a combination thereof; and/or
wherein the electric heating element is configured to connect to a power source external to the housing assembly; or
c) a non-electric heating element, and the non-electric heating element comprises a heating channel comprising an inlet and an outlet for a heated fluid, and optionally, wherein the heating channel is a heating coil and/or wherein the heated fluid is heated water, and further optionally, wherein the inlet for heated water is configured to connect to an external reservoir of heated water.

9. The chromatography column of any one of claims 7-8,
wherein at least one of the one or more heating element(s) is disposed along and/or around a central axis of the internal cavity, and/or
wherein at least one of the one or more heating element(s) is disposed inside the internal cavity, outside the internal cavity, or partially inside and partially outside the internal cavity.

10. The chromatography column of any one of claims 7-9, wherein at least one of the one or more heating element(s) surrounds at least a portion of the inlet housing member, at least a portion of the outlet housing member, and/or at least a portion of the side wall member.

11. The chromatography column of any one of claims 7-10 when each is dependent on claim 2, wherein at least a portion of at least one of the one or more heating element(s) is in contact with at least a portion of the inlet housing member, at least a portion of the outlet housing member, and/or at least a portion of the side wall member, optionally at least a portion of the side wall member.

12. The chromatography column of any one of claims 7-10 when each is dependent on claim 1, wherein at least a portion of at least one of the one or more heating element(s) is in contact with at least a portion of the inlet housing member, at least a portion of the outlet housing member, and/or at least a portion of a side wall member, optionally at least a portion of the side wall member.

13. The chromatography column of any one of claim 10 or of claims 11 or 12 when dependent on claim 10, wherein the housing assembly further comprises an insulation layer between at least one of the one or more heating element(s) and at least a portion of the inlet housing member, at least a portion of the outlet housing member, and/or at least a portion of the side wall member, and optionally,
wherein the insulation layer comprises
a gas, optionally air, or
a liquid.

14. The chromatography column of any one of claims 7-13, wherein the housing assembly further comprises a jacket member (12) comprising at least one of the one or more heating element(s), wherein the jacket member is configured to surround at least a portion of the inlet housing member, at least a portion of the outlet housing member, and/or at least a portion of the side wall member, and optionally:
wherein the jacket member is releasably connected together to surround the at least a portion of the inlet housing member, the at least a portion of the outlet housing member, and/or the at least a portion of the side wall member; and/or
wherein the jacket member is configured to surround at least a portion of the side wall member, optionally is configured to entirely surround the side wall member.

15. The chromatography column of any preceding claim, wherein the stationary phase comprises or is a non-magnetic material, a non-ferromagnetic material, or non-paramagnetic material.

16. The chromatography column of any preceding claim, wherein the stationary phase is configured to immobilize target cells thereon, optionally wherein the target cells are T cells, optionally CD3+, CD4+, or CD8+ T cells.

17. The chromatography column of any preceding claim, wherein the stationary phase comprises a selection agent (32) immobilized thereon.

18. The chromatography column of claim 17, wherein the selection agent is capable of specific binding to a selection marker (34) on the surface of one or more cell(s), and optionally wherein the one or more cell(s) are immune cells, further optionally T cells.

19. The chromatography column of claim 18, wherein the selection agent is or comprises an agent selected from the group consisting of antibody fragments, monovalent antibody fragments, proteinaceous binding molecules with immunoglobulin-like functions, molecules containing Ig domains, cytokines, chemokines, aptamers, MHC molecules, MHC-peptide complexes; receptor ligands; and binding fragments thereof; and/or
the selection agent comprises an antibody fragment;
the selection agent is or comprises a Fab fragment;
the selection agent is or comprises a single domain antibody, optionally a VHH antibody;
the selection agent is selected from the group of divalent antibody fragments consisting of F(ab')₂ fragments and divalent single-chain Fv (scFv) fragments;
the selection agent is a monovalent antibody fragment selected from the group consisting of Fab fragments, Fv fragments, and scFvs; and/or
the selection agent is a proteinaceous binding molecule with antibody-like binding properties, selected from the group consisting of aptamers, muteins based on a polypeptide of the lipocalin family, glubodies, proteins based on the ankyrin scaffold, proteins based on the crystalline scaffold, adnectins, and avimers.

20. The chromatography column of one of claims 18 or 19, wherein:
the selection marker is a T cell coreceptor;
the selection marker is or comprises a member of a T cell antigen receptor complex;
the selection marker is or comprises a CD3 complex;
the selection marker is or comprises a CD3 chain;
the selection marker is or comprises a CD3γ, CD3δ, CD3ε, or CD3ζ chain;
the selection marker is or comprises CD8;
the selection marker is or comprises CD4;
the selection marker is or comprises CD45RA;
the selection marker is or comprises CD27;
the selection marker is or comprises CD28; and/or
the selection marker is or comprises CCR7.

21. The chromatography column of any one of claims 18-20, wherein the selection agent comprises or is an anti-CD3 Fab, an anti-CD8 Fab, an anti-CD4 Fab, or an anti-CD27 Fab.

22. The chromatography column of any one of claims 18-21, wherein the selection agent is bound indirectly to the stationary phase through a selection reagent (31) to which the selection agent reversibly binds.

23. The chromatography column of claim 22, wherein the selection reagent comprises or is a mutein of streptavidin that reversibly binds a streptavidin-binding peptide and optionally,
wherein the streptavidin-binding peptide is selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18), and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19); and/or,
wherein the streptavidin mutein comprises the amino acid sequence Val⁴⁴-Thr⁴⁵-Ala⁴⁶-Arg⁴⁷ at sequence positions corresponding to positions 44 to 47 of SEQ ID NO: 1, or the streptavidin mutein comprises the amino acid sequence Ile⁴⁴-Gly⁴⁵-Ala⁴⁶-Arg⁴⁷ at sequence positions corresponding to positions 44 to 47 of SEQ ID NO: 1; and/or
wherein the N-terminal amino acid residue of the streptavidin mutein is in the region of amino acids 10 to 16 of SEQ ID NO: 1, and the C-terminal amino acid residue of the streptavidin mutein is in the region of amino acids 133 to 142 of SEQ ID NO: 1, and/or
wherein the streptavidin mutein comprises the amino acid sequence set forth in any of SEQ ID NOs: 3-6, 27, 28, 104, and 105.

24. The chromatography column of any of claims 17-23, wherein the selection agent comprises a streptavidin-binding peptide, optionally wherein the streptavidin-binding peptide is selected from the group consisting of Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18), and Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19).

## Patentansprüche

1. Eine Chromatographiesäule, umfassend eine Gehäuseanordnung (1) für die Säulenchromatographie, wobei die Gehäuseanordnung umfasst:
ein Einlassgehäuseelement (2) und ein Auslassgehäuseelement (3), wobei mindestens das Einlassgehäuseelement und das Auslassgehäuseelement einen inneren Hohlraum bilden, der so konfiguriert ist, dass eine stationäre Phase für die Säulenchromatographie aufgenommen wird, wobei:
der innere Hohlraum die stationäre Phase für die Säulenchromatographie umfasst; und
die stationäre Phase eine Affinitätschromatographie-Matrix umfasst;
ein Temperaturkontrollelement, das so konfiguriert ist, dass es Wärme an die stationäre Phase im inneren Hohlraum liefert; und
ein Verbindungselement (6), das so konfiguriert ist, dass es den inneren Hohlraum funktionsfähig mit einer Gasquelle verbindet, wodurch die Zufuhr von Gas in den inneren Hohlraum ermöglicht oder bewirkt wird.

2. Die Chromatographiesäule nach Anspruch 1, die Gehäuseanordnung, umfasst außerdem ein Seitenwandstück (7), wobei das Einlassgehäuseelement, das Auslassgehäuseelement und das Seitenwandstück den inneren Hohlraum bilden.

3. Die Chromatographiesäule nach Anspruch 1 oder Anspruch 2, wobei das Verbindungselement einen oder mehrere Filter umfasst und optional, wobei ein oder mehrere Filter ein Gasfilter ist/sind.

4. Die Chromatographiesäule nach einem der Ansprüche 1-3,
wobei das Einlassgehäuseelement einen oder mehrere Einlässe umfasst, die funktionsfähig mit dem inneren Hohlraum verbunden sind, um die Aufnahme einer Eingangszusammensetzung in den inneren Hohlraum zu ermöglichen; und/oder
wobei das Auslassgehäuse-Element einen oder mehrere Auslässe umfasst, die funktionsfähig mit dem inneren Hohlraum verbunden sind, um die Abgabe einer Ausgangszusammensetzung aus dem inneren Hohlraum zu ermöglichen oder zu bewirken.

5. Die Chromatographiesäule nach einem der Ansprüche 1 bis 4, die ferner umfasst:
ein erstes poröses Element (9), das so konfiguriert ist, dass es die stationäre Phase und einen Einlass des inneren Hohlraums trennt,
wobei das erste poröse Element optional zwischen dem Einlassgehäuseelement und dem Seitenwandteil angeordnet ist, und ferner optional,
wobei das erste poröse Element unabhängig voneinander ein Zellfilter oder ein Zellsieb ist; und/oder
ein zweites poröses Element (9), das so konfiguriert ist, dass es die stationäre Phase und einen Auslass des inneren Hohlraums trennt,
wobei das zweite poröse Element optional zwischen dem Auslassgehäuseelement und dem Seitenwandteil angeordnet ist, und weiter optional
wobei das zweite poröse Element unabhängig davon ein Zellfilter oder ein Zellsieb ist.

6. Die Chromatographiesäule nach einem der Ansprüche 1-5, wobei das Temperaturkontrollelement so konfiguriert ist, dass es
die stationäre Phase auf eine Zieltemperatur zwischen etwa 30 °C und etwa 39 °C erwärmt, wobei die Zieltemperatur optional zwischen etwa 35 °C und etwa 39 °C und weiter optional bei oder etwa 37 °C liegt, und/oder
die stationäre Phase auf der Zieltemperatur hält.

7. Die Chromatographiesäule nach einem der Ansprüche 1 bis 6, wobei das Temperaturkontrollelement ein oder mehrere Heizelemente umfasst und wobei das eine oder die mehreren Heizelemente optional so konfiguriert sind, dass sie die stationäre Phase gleichmäßig erwärmen.

8. Die Chromatographiesäule nach Anspruch 7, wobei mindestens eines der einen oder mehreren Heizelemente:
a) ein elektromagnetisches Induktionsheizelement ist und das elektromagnetische Induktionsheizelement eine Induktionsheizspule umfasst, die einen magnetisierbaren Kern umgibt, der so konfiguriert ist, dass er der stationären Phase in dem inneren Hohlraum Wärme zuführt; oder
b) ein elektrisches Heizelement (17) ist und optional,
wobei das elektrische Heizelement eine Metallplatte, einen Metallstab, einen Metalldraht oder eine Kombination davon umfasst; und/oder
wobei das elektrische Heizelement so konfiguriert ist, dass es mit einer außerhalb der Gehäuseanordnung befindlichen Stromquelle verbunden werden kann; oder
c) ein nicht-elektrisches Heizelement ist, wobei das nicht-elektrische Heizelement einen Heizkanal umfasst, der einen Einlass und einen Auslass für ein erwärmtes Fluid umfasst, und optional, wobei der Heizkanal eine Heizspule ist und/oder wobei das erwärmte Fluid erwärmtes Wasser ist, und weiter optional, wobei der Einlass für erwärmtes Wasser so konfiguriert ist, dass er mit einem externen Vorratsbehälter für erwärmtes Wasser verbunden werden kann.

9. Die Chromatographiesäule nach einem der Ansprüche 7-8,
wobei mindestens eines der einen oder mehreren Heizelemente entlang und/oder um eine Mittelachse des inneren Hohlraums angeordnet ist und/oder
wobei mindestens eines der einen oder mehreren Heizelemente innerhalb des inneren Hohlraums, außerhalb des inneren Hohlraums oder teilweise innerhalb und teilweise außerhalb des inneren Hohlraums angeordnet ist.

10. Die Chromatographiesäule nach einem der Ansprüche 7 bis 9, wobei mindestens eines der einen oder mehreren Heizelemente mindestens einen Teil des Einlassgehäuseelements, mindestens einen Teil des Auslassgehäuseelements und/oder mindestens einen Teil des Seitenwandteils umgibt.

11. Die Chromatographiesäule nach einem der Ansprüche 7 bis 10, wobei jeder Anspruch von Anspruch 2 abhängt, wobei mindestens ein Teil mindestens eines der einen oder mehreren Heizelemente mit mindestens einem Teil des Einlassgehäuseelements, mindestens einem Teil des Auslassgehäuseelements und/oder mindestens einem Teil des Seitenwandteils, optional mindestens einem Teil des Seitenwandteils, in Kontakt steht.

12. Die Chromatographiesäule nach einem der Ansprüche 7 bis 10, wobei jeder Anspruch von Anspruch 1 abhängt, wobei mindestens ein Teil mindestens eines der einen oder mehreren Heizelemente mit mindestens einem Teil des Einlassgehäuseelements, mindestens einem Teil des Auslassgehäuseelements und/oder mindestens einem Teil eines Seitenwandteils, optional mindestens einem Teil des Seitenwandteils, in Kontakt steht

13. Die Chromatographiesäule nach einem der Ansprüche 10 oder nach den Ansprüchen 11 oder 12, wenn sie von Anspruch 10 abhängig ist, wobei die Gehäuseanordnung ferner eine Isolierschicht zwischen mindestens einem der einen oder mehreren Heizelemente und mindestens einem Teil des Einlassgehäuseelements, mindestens einem Teil des Auslassgehäuseelements und/oder mindestens einem Teil des Seitenwandteils umfasst, und optional
wobei die Isolierschicht
ein Gas, optional Luft, oder
eine Flüssigkeit umfasst.

14. Die Chromatographiesäule nach einem der Ansprüche 7 bis 13, wobei die Gehäuseanordnung ferner ein Mantelelement (12) umfasst, das mindestens eines der einen oder mehreren Heizelemente umfasst, wobei das Mantelelement so konfiguriert ist, dass es mindestens einen Teil des Einlassgehäuseelements, mindestens einen Teil des Auslassgehäuseelements und/oder mindestens einen Teil des Seitenwandteils umgibt, und optional:
wobei das Mantelelement lösbar miteinander verbunden ist, um mindestens einen Teil des Einlassgehäuseelements, mindestens einen Teil des Auslassgehäuseelements und/oder mindestens einen Teil des Seitenwandelements zu umgeben; und/oder
wobei das Mantelelement so konfiguriert ist, dass es mindestens einen Teil des Seitenwandelements umgibt, optional so konfiguriert ist, dass es das Seitenwandelement vollständig umgibt.

15. Die Chromatographiesäule nach einem der vorstehenden Ansprüche, wobei die stationäre Phase ein nichtmagnetisches Material, ein nichtferromagnetisches Material oder ein nichtparamagnetisches Material umfasst oder ein solches ist.

16. Die Chromatographiesäule nach einem der vorstehenden Ansprüche, wobei die stationäre Phase so konfiguriert ist, dass sie Zielzellen darauf immobilisiert, wobei die Zielzellen optional T-Zellen, optional CD3+-, CD4+- oder CD8+-T-Zellen, sind.

17. Die Chromatographiesäule nach einem der vorstehenden Ansprüche, wobei die stationäre Phase ein darauf immobilisiertes Selektionsmittel (32) umfasst.

18. Die Chromatographiesäule nach Anspruch 17, wobei das Selektionsmittel in der Lage ist, spezifisch an einen Selektionsmarker (34) auf der Oberfläche einer oder mehrerer Zellen zu binden, und wobei die eine oder mehreren Zellen optional Immunzellen, weiter optional T-Zellen sind.

19. Die Chromatographiesäule nach Anspruch 18, wobei das Selektionsmittel ein Mittel ist oder umfasst, das aus der Gruppe ausgewählt ist, bestehend aus Antikörperfragmenten, monovalenten Antikörperfragmenten, proteinhaltigen Bindungsmolekülen mit immunoglobulinähnlichen Funktionen, Molekülen, die Ig-Domänen enthalten, Zytokinen, Chemokinen, Aptameren, MHC-Molekülen, MHC-Peptidkomplexen, Rezeptorliganden und Bindungsfragmenten davon; und/oder
das Selektionsmittel ein Antikörperfragment umfasst;
das Selektionsmittel ein Fab-Fragment ist oder ein solches umfasst;
das Selektionsmittel ein Einzeldomänen-Antikörper, optional ein VHH-Antikörper, ist oder ein solches umfasst;
das Selektionsmittel aus der Gruppe der zweiwertigen Antikörperfragmente ausgewählt ist, bestehend aus F(ab')₂-Fragmenten und zweiwertigen einkettigen Fv (scFv)-Fragmenten;
das Selektionsmittel ein monovalentes Antikörperfragment ist ausgewählt aus der Gruppe bestehend die aus Fab-Fragmenten, Fv-Fragmenten und scFvs; und/oder
das Selektionsmittel ein proteinhaltiges Bindungsmolekül mit antikörperähnlichen Bindungseigenschaften ist, ausgewählt aus der Gruppe bestehend aus Aptameren, Muteinen auf Basis eines Polypeptids der Lipocalin-Familie, Glubodies, Proteinen auf Basis des Ankyrin-Gerüsts, Proteinen auf Basis des Kristallgerüsts, Adnektinen und Avimeren.

20. Die Chromatographiesäule nach einem der Ansprüche 18 oder 19, wobei:
der Selektionsmarker ein T-Zell-Korezeptor ist;
der Selektionsmarker ein Mitglied eines T-Zell-Antigenrezeptorkomplexes ist oder umfasst;
der Selektionsmarker ein CD3-Komplex ist oder umfasst;
der Selektionsmarker eine CD3-Kette ist oder umfasst;
der Selektionsmarker eine CD3y-, CD3δ-, CD3ε- oder CD3ζ-Kette ist oder umfasst;
der Selektionsmarker CD8 ist oder umfasst;
der Selektionsmarker CD4 ist oder umfasst;
der Selektionsmarker CD45RA ist oder umfasst;
der Selektionsmarker CD27 ist oder umfasst;
der Selektionsmarker CD28 ist oder umfasst; und/oder
der Selektionsmarker CCR7 ist oder umfasst.

21. Die Chromatographiesäule nach einem der Ansprüche 18 bis 20, wobei das Selektionsmittel ein Anti-CD3-Fab, ein Anti-CD8-Fab, ein Anti-CD4-Fab oder ein Anti-CD27-Fab umfasst oder ein solches ist.

22. Die Chromatographiesäule nach einem der Ansprüche 18 bis 21, wobei das Selektionsmittel indirekt über ein Selektionsreagenz (31), an das das Selektionsmittel reversibel bindet, an die stationäre Phase gebunden ist.

23. Die Chromatographiesäule nach Anspruch 22, wobei das Selektionsreagenz ein Mutein von Streptavidin umfasst oder ein solches ist, das reversibel an ein Streptavidin-bindendes Peptid bindet, und optional
wobei das Streptavidin-bindende Peptid aus der Gruppe ausgewählt ist, bestehend aus Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) und Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19); und/oder,
wobei das Streptavidin-Mutein die Aminosäuresequenz Val⁴⁴-Thr⁴⁵-Ala⁴⁶-Arg⁴⁷ an Sequenzpositionen umfasst, die den Positionen 44 bis 47 von SEQ ID NO: 1, oder das Streptavidin-Mutein die Aminosäuresequenz Ile⁴⁴-Gly⁴⁵-Ala⁴⁶-Arg⁴⁷ an Sequenzpositionen umfasst, die den Positionen 44 bis 47 von SEQ ID NO: 1 entsprechen; und/oder
wobei der N-terminale Aminosäurerest des Streptavidin-Muteins im Bereich der Aminosäuren 10 bis 16 von SEQ ID NO: 1 liegt und der C-terminale Aminosäurerest des Streptavidin-Muteins im Bereich der Aminosäuren 133 bis 142 von SEQ ID NO: 1 liegt, und/oder
wobei das Streptavidin-Mutein die Aminosäuresequenz umfasst, die in einer der SEQ ID NOs: 3-6, 27, 28, 104 und 105 angegeben ist.

24. Die Chromatographiesäule nach einem der Ansprüche 17 bis 23, wobei das Selektionsmittel ein Streptavidin-bindendes Peptid umfasst, wobei das Streptavidin-bindende Peptid optional ausgewählt ist aus der Gruppe bestehend aus Trp-Ser-His-Pro-Gln-Phe-Glu-Lys (SEQ ID NO: 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO: 15), Trp-Ser-His-Pro-Gln-Phe-Glu-Lys-(GlyGlyGlySer)₃-Trp-Ser-His-Pro-Gln-Phe-Glu-Lys (SEQ ID NO: 17), SAWSHPQFEKGGGSGGGSGGSAWSHPQFEK (SEQ ID NO: 16), Trp-Ser-His-Pro-Gln-Phe-Glu-Lys-(GlyGlyGlySer)₂-Trp-Ser-His-Pro-Gln-Phe-Glu-Lys (SEQ ID NO: 18) und Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO: 19).

## Revendications

1. Une colonne de chromatographie, comprenant un ensemble de boîtier (1) pour la chromatographie sur colonne, l'ensemble de boîtier comprenant :
un élément de boîtier d'entrée (2) et un élément de boîtier de sortie (3), dans lequel au moins l'élément de boîtier d'entrée et l'élément de boîtier de sortie forment une cavité interne configurée pour loger une phase stationnaire pour la chromatographie sur colonne, dans laquelle :
la cavité interne comprend la phase stationnaire pour la chromatographie sur colonne ; et
la phase stationnaire comprend une matrice de chromatographie d'affinité ;
un élément de contrôle de la température configuré pour fournir de la chaleur à la phase stationnaire dans la cavité interne ; et
un connecteur (6) configuré pour relier de manière opérationnelle la cavité interne à une source de gaz, permettant ou effectuant ainsi l'admission de gaz dans la cavité interne.

2. La colonne chromatographique selon la revendication 1, l'ensemble de boîtier comprenant en outre un élément de paroi latérale (7), dans lequel l'élément de boîtier d'entrée, l'élément de boîtier de sortie et l'élément de paroi latérale forment la cavité interne.

3. La colonne chromatographique selon la revendication 1 ou la revendication 2, dans laquelle le connecteur comprend un ou plusieurs filtres, et éventuellement, dans laquelle le ou les filtres sont des filtres à gaz.

4. Colonne chromatographique selon l'une quelconque des revendications 1 à 3,
dans laquelle l'élément de boîtier d'entrée comprend une ou plusieurs entrées reliées de manière opérationnelle à la cavité interne pour permettre l'admission d'une composition d'entrée dans la cavité interne ; et/ou
dans laquelle l'élément de boîtier de sortie comprend une ou plusieurs sorties reliées de manière opérationnelle à la cavité interne pour permettre ou effectuer la décharge d'une composition de sortie de la cavité interne.

5. La colonne chromatographique selon l'une quelconque des revendications 1 à 4, comprenant en outre :
un premier élément poreux (9) configuré pour séparer la phase stationnaire et une entrée de la cavité interne,
dans lequel le premier élément poreux est éventuellement situé entre l'élément de boîtier d'entrée et l'élément de paroi latérale, et éventuellement en outre,
dans lequel le premier élément poreux est indépendamment un tamis cellulaire ou un tamis à cellules ; et/ou
un deuxième élément poreux (9) configuré pour séparer la phase stationnaire et une sortie de la cavité interne,
dans lequel le deuxième élément poreux est éventuellement situé entre l'élément de boîtier de sortie et l'élément de paroi latérale, et éventuellement en outre,
dans lequel le deuxième élément poreux est indépendamment un filtre cellulaire ou un tamis cellulaire.

6. La colonne de chromatographie selon l'une quelconque des revendications 1 à 5, dans laquelle l'élément de contrôle de la température est configuré :
pour chauffer la phase stationnaire à une température cible comprise entre environ 30 °C et environ 39 °C, et éventuellement, dans laquelle la température cible est comprise entre environ 35 °C et environ 39 °C, et éventuellement à environ 37 °C, et/ou
pour maintenir la phase stationnaire à la température cible.

7. La colonne chromatographique selon l'une quelconque des revendications 1 à 6, dans laquelle l'élément de contrôle de la température comprend un ou plusieurs éléments chauffants, et éventuellement, dans laquelle le ou les éléments chauffants sont configurés pour chauffer uniformément la phase stationnaire.

8. La colonne chromatographique selon la revendication 7, dans laquelle au moins un des éléments chauffants comprend :
a) un élément chauffant à induction électromagnétique, et l'élément chauffant à induction électromagnétique comprend une bobine de chauffage par induction entourant un noyau magnétisable configuré pour fournir de la chaleur à la phase stationnaire dans la cavité interne ; ou
b) un élément chauffant électrique (17) et, éventuellement,
dans lequel l'élément chauffant électrique comprend une plaque métallique, une tige métallique, un fil métallique ou une combinaison de ceux-ci ; et/ou
dans lequel l'élément chauffant électrique est configuré pour se connecter à une source d'alimentation externe à l'ensemble de boîtier ; ou
c) un élément chauffant non électrique, et l'élément chauffant non électrique comprend un canal de chauffage comprenant une entrée et une sortie pour un fluide chauffé, et éventuellement, dans lequel le canal de chauffage est une bobine chauffante et/ou dans lequel le fluide chauffé est de l'eau chauffée, et éventuellement en outre, dans lequel l'entrée pour l'eau chauffée est configurée pour se connecter à un réservoir externe d'eau chauffée.

9. La colonne chromatographique selon l'une quelconque des revendications 7 à 8,
dans laquelle au moins un des un ou plusieurs éléments chauffants est disposé le long et/ou autour d'un axe central de la cavité interne, et/ou
dans laquelle au moins un des un ou plusieurs éléments chauffants est disposé à l'intérieur de la cavité interne, à l'extérieur de la cavité interne, ou partiellement à l'intérieur et partiellement à l'extérieur de la cavité interne.

10. La colonne chromatographique selon l'une quelconque des revendications 7 à 9, dans laquelle au moins l'un des éléments chauffants entoure au moins une partie de l'élément de boîtier d'entrée, au moins une partie de l'élément de boîtier de sortie et/ou au moins une partie de l'élément de paroi latérale.

11. La colonne de chromatographie selon l'une quelconque des revendications 7 à 10, lorsque chacune dépend de la revendication 2, dans laquelle au moins une partie d'au moins un des éléments chauffants est en contact avec au moins une partie de l'élément de boîtier d'entrée, au moins une partie de l'élément de boîtier de sortie et/ou au moins une partie de l'élément de paroi latérale, éventuellement au moins une partie de l'élément de paroi latérale.

12. La colonne chromatographique de l'une quelconque des revendications 7 à 10, lorsque chacune dépend de la revendication 1, dans laquelle au moins une partie d'au moins un ou plusieurs éléments chauffants est en contact avec au moins une partie de l'élément de boîtier d'entrée, au moins une partie de l'élément de boîtier de sortie et/ou au moins une partie d'un élément de paroi latérale, éventuellement au moins une partie de l'élément de paroi latérale.

13. La colonne chromatographique selon l'une quelconque des revendications 10, 11 ou 12 lorsqu'elle dépend de la revendication 10, dans laquelle l'ensemble de boîtier comprend en outre une couche isolante entre au moins l'un des éléments chauffants et au moins une partie de l'élément de boîtier d'entrée, au moins une partie de l'élément de boîtier de sortie et/ou au moins une partie de l'élément de paroi latérale, et éventuellement,
dans laquelle la couche isolante comprend
un gaz, éventuellement de l'air, ou
un liquide.

14. La colonne de chromatographie selon l'une quelconque des revendications 7 à 13, dans laquelle l'ensemble de boîtier comprend en outre un élément d'enveloppe (12) comprenant au moins un ou plusieurs éléments chauffants, dans laquelle l'élément d'enveloppe est configuré pour entourer au moins une partie de l'élément de boîtier d'entrée, au moins une partie de l'élément de boîtier de sortie et/ou au moins une partie de l'élément de paroi latérale, et éventuellement :
dans laquelle l'élément d'enveloppe est relié de manière amovible pour entourer au moins une partie de l'élément de boîtier d'entrée, au moins une partie de l'élément de boîtier de sortie et/ou au moins une partie de l'élément de paroi latérale ; et/ou
dans laquelle l'élément d'enveloppe est configuré pour entourer au moins une partie de l'élément de paroi latérale, éventuellement configuré pour entourer entièrement l'élément de paroi latérale.

15. La colonne chromatographique selon l'une quelconque des revendications précédentes, dans laquelle la phase stationnaire comprend ou est constituée d'un matériau non magnétique, d'un matériau non ferromagnétique ou d'un matériau non paramagnétique.

16. La colonne chromatographique selon l'une quelconque des revendications précédentes, dans laquelle la phase stationnaire est configurée pour immobiliser des cellules cibles sur celle-ci, les cellules cibles étant éventuellement des cellules T, éventuellement des cellules T CD3+, CD4+ ou CD8+.

17. La colonne chromatographique selon l'une quelconque des revendications précédentes, dans laquelle la phase stationnaire comprend un agent de sélection (32) immobilisé sur celle-ci.

18. La colonne chromatographique selon la revendication 17, dans laquelle l'agent de sélection est capable de se lier spécifiquement à un marqueur de sélection (34) à la surface d'une ou plusieurs cellules, et éventuellement dans laquelle la ou les cellules sont des cellules immunitaires, éventuellement des cellules T.

19. La colonne chromatographique selon la revendication 18, dans laquelle l'agent de sélection est ou comprend un agent choisi dans le groupe constitué par des fragments d'anticorps, des fragments d'anticorps monovalents, des molécules de liaison protéiques ayant des fonctions de type immunoglobuline, des molécules contenant des domaines Ig, des cytokines, des chimiokines, des aptamères, des molécules MHC, des complexes MHC-peptide ; des ligands de récepteurs ; et des fragments de liaison de ceux-ci ; et/ou l'agent de sélection comprend un fragment d'anticorps ;
l'agent de sélection est ou comprend un fragment Fab ;
l'agent de sélection est ou comprend un anticorps à domaine unique, éventuellement un anticorps VHH ;
l'agent de sélection est choisi dans le groupe des fragments d'anticorps divalents constitués de fragments F(ab')2 et de fragments Fv à chaîne unique divalents (scFv) ; l'agent de sélection est un fragment d'anticorps monovalent choisi dans le groupe constitué des fragments Fab, des fragments Fv et des scFv ; et/ou
l'agent de sélection est une molécule de liaison protéique ayant des propriétés de liaison similaires à celles d'un anticorps, choisie dans le groupe constitué par les aptamères, les mutéines basées sur un polypeptide de la famille des lipocalines, les glubodies, les protéines basées sur le squelette de l'ankyrine, les protéines basées sur le squelette cristallin, les adnectines et les avimères.

20. La colonne chromatographique selon l'une des revendications 18 ou 19, dans laquelle :
le marqueur de sélection est un corécepteur de cellules T ;
le marqueur de sélection est ou comprend un membre d'un complexe récepteur d'antigène de cellules T ;
le marqueur de sélection est ou comprend un complexe CD3 ;
le marqueur de sélection est ou comprend une chaîne CD3 ;
le marqueur de sélection est ou comprend une chaîne CD3γ, CD3δ, CD3ε ou CD3ζ ;
le marqueur de sélection est ou comprend CD8 ;
le marqueur de sélection est ou comprend CD4 ;
le marqueur de sélection est ou comprend CD45RA ;
le marqueur de sélection est ou comprend CD27 ;
le marqueur de sélection est ou comprend CD28 ; et/ou
le marqueur de sélection est ou comprend CCR7.

21. La colonne chromatographique selon l'une quelconque des revendications 18 à 20, dans laquelle l'agent de sélection comprend ou est un Fab anti-CD3, un Fab anti-CD8, un Fab anti-CD4 ou un Fab anti-CD27.

22. La colonne de chromatographie selon l'une quelconque des revendications 18 à 21, dans laquelle l'agent de sélection est lié indirectement à la phase stationnaire par l'intermédiaire d'un réactif de sélection (31) auquel l'agent de sélection se lie de manière réversible.

23. La colonne chromatographique selon la revendication 22, dans laquelle le réactif de sélection comprend ou est une mutéine de streptavidine qui se lie de manière réversible à un peptide se liant à la streptavidine et, éventuellement,
dans laquelle le peptide se liant à la streptavidine est choisi dans le groupe constitué par Trp-Ser-His-Pro-Gln-Phe-Glu-Lys (SEQ ID NO : 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO : 15), Trp-Ser-His-Pro-Gln-Phe-Glu-Lys-(GlyGlyGlySer)₃-Trp-Ser-His-Pro-Gln-Phe-Glu-Lys (SEQ ID NO : 17), SAWSHPQFEKGGGSGGGSGGSAWSHPQFEK (SEQ ID NO : 16), Trp-Ser-His-Pro-Gln-Phe-Glu-Lys-(GlyGlyGlySer)₂-Trp-Ser-His-Pro-Gln-Phe-Glu-Lys (SEQ ID NO : 18), et Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO : 19) ; et/ou,
dans lequel la mutéine streptavidine comprend la séquence d'acides aminés Val⁴⁴-Thr⁴⁵-Ala⁴⁶-Arg⁴⁷ aux positions de séquence correspondant aux positions 44 à 47 de SEQ ID NO : 1, ou la mutéine de streptavidine comprend la séquence d'acides aminés Ile⁴⁴-Gly⁴⁵-Ala⁴⁶-Arg⁴⁷ aux positions de séquence correspondant aux positions 44 à 47 de SEQ ID NO : 1 ; et/ou
dans laquelle le résidu d'acide aminé N-terminal de la mutéine de streptavidine se trouve dans la région des acides aminés 10 à 16 de SEQ ID NO : 1, et le résidu d'acide aminé C-terminal de la mutéine de streptavidine se trouve dans la région des acides aminés 133 à 142 de la SEQ ID NO : 1, et/ou
dans laquelle la mutéine de streptavidine comprend la séquence d'acides aminés indiquée dans l'une quelconque des SEQ ID NO : 3-6, 27, 28, 104 et 105.

24. La colonne chromatographique selon l'une quelconque des revendications 17 à 23, dans laquelle l'agent de sélection comprend un peptide se liant à la streptavidine, éventuellement dans laquelle le peptide se liant à la streptavidine est choisi dans le groupe constitué par Trp-Ser-His-Pro-Gln-Phe-Glu-Lys (SEQ ID NO : 8), Ser-Ala-Trp-Ser-His-Pro-Gln-Phe-Glu-Lys-(GlyGlyGlySer)₃-Trp-Ser-His-Pro-Gln-Phe-Glu-Lys (SEQ ID NO : 15), Trp-Ser-His-Pro-Gln-Phe-Glu-Lys-(GlyGlyGlySer)₃-Trp-Ser-His-Pro-Gln-Phe-Glu-Lys (SEQ ID NO : 17), SAWSHPQFEKGGGSGGGSGGSAWSHPQFEK (SEQ ID NO: 16), Trp-Ser-His-Pro-Gln-Phe-Glu-Lys-(GlyGlyGlySer)2-Trp-Ser-His-Pro-Gln-Phe-Glu-Lys (SEQ ID NO : 18) et Trp-Ser-His-Pro-Gln-Phe-Glu-Lys-(GlyGlyGlySer)₂Gly-Gly-Ser-Ala-Trp-Ser-His-Pro-Gln-Phe-Glu-Lys (SEQ ID NO : 19).
